# EUROPEAN PATENT SPECIFICATION

(11) **EP 2 044 051 B1**
(45) Date of publication and mention of the grant of the patent: **27.01.2010**
(21) Application number: 07786791.9
(22) Date of filing: 21.06.2007
(51) Int. Cl.: C07D 401/04, C07D 401/12, C07D 403/12, C07D 403/14, C07D 405/12, C07D 471/04

(54) **PYRIDINE AND PYRAZINE DERIVATIVES AS MNK KINASE INHIBITORS**
PYRIDIN- UND PYRAZINDERIVATE ALS MNK-KINASEINHIBITOREN
DÉRIVÉS DE PYRIDINE ET DE PYRAZINE UTILISÉS EN TANT QU'INHIBITEURS DE LA KINASE MNK

(30) Priority: 22.06.2006 SE 0601379; 06.10.2006 US 850067 P
(43) Date of publication of application: 08.04.2009
(73) Proprietor: BIOVITRUM AB (publ), SE-112 76 Stockholm (SE)
(72) Inventor: JENMALM JENSEN, Annika, S-756 45 Uppsala (SE); RINGOM, Rune, S-754 34 Uppsala (SE); MEDINA, Carmen, S-752 43 Uppsala (SE); SHILVOCK, John, S-172 69 SUNDBYBERG (SE); WIIK, Marie, S-753 25 Uppsala (SE); KOOLMEISTER, Tobias, S-115 27 STOCKHOLM (SE); ANGBRANT, Johan, S-120 57 ÅRSTA (SE); SUTIN, Lori, S-182 66 Danderyd (SE); HENRIKSSON, Martin, S-169 30 Bromma (SE); SANDVALL, Teresa, 113 64 Stockholm (SE); JOHANSSON, Lars, S-163 44 Spånga (SE); NILSSON, Björn, S-112 49 Stockholm (SE)
(86) International application number: PCT/EP2007/056213
(87) International publication number: WO 2007/147874

(56) References cited:
- WO-A-2006/067466
- WO-A-2006/136402
- ION NICOLESCU-DUVAZ, ESTEBAN ROMAN, STEVEN R. WHITTAKER, FRANK FRIEDLOS, RUTH KIRK, IAN J. SCANLON, LAWRENCE C. DAVIES, ET AL.: "Novel inhibitors of B-RAF based on a disubstituted pyrazine scaffold. Generation of a nanomolar lead" J. MED. CHEM., vol. 49, no. 1, 2006, pages 407-416, XP002455215

### SEQUENCE LISTING

<110> Biovitrum AB (publ)
<120> Pyridine and pyrazine derivatives as MNK kinase inhibitors
<130> BV-1131
<140> 07786791.9
   <141> 2007-06-21
<160> 6
<170> PatentIn version 3.3
<210> 1
   <211> 1444
   <212> DNA
   <213> human
<220>
   <221> CDS
   <222> (37)..(1434)
<300>
   <308> GenBank/AF237775
   <309> 2000-10-26
   <313> (1)..(1444)
<400> 1
   cggtcccctc ccccgctggc ggggcccgga cagaag atg gtg cag aag aaa cca         54
                                           Met Val Gln Lys Lys Pro
                                           1               5
   gcc gaa ctt cag ggt ttc cac cgt tcg ttc aag ggg cag aac ccc ttc        102
   Ala Glu Leu Gln Gly Phe His Arg Ser Phe Lys Gly Gln Asn Pro Phe
               10                  15                  20
   gag ctg gcc ttc tcc cta gac cag ccc gac cac gga gac tct gac ttt        150
   Glu Leu Ala Phe Ser Leu Asp Gln Pro Asp His Gly Asp Ser Asp Phe
           25                  30                  35
   ggc ctg cag tgc tca gcc cgc cct gac atg ccc gcc agc cag ccc att        198
   Gly Leu Gln Cys Ser Ala Arg Pro Asp Met Pro Ala Ser Gln Pro Ile
       40                  45                  50
   gac atc ccg gac gcc aag aag agg ggc aag aag aag aag cgc ggc cgg        246
   Asp Ile Pro Asp Ala Lys Lys Arg Gly Lys Lys Lys Lys Arg Gly Arg
   55                  60                  65                  70
   gcc acc gac agc ttc tcg ggc agg ttt gaa gac gtc tac cag ctg cag        294
   Ala Thr Asp Ser Phe Ser Gly Arg Phe Glu Asp Val Tyr Gln Leu Gln
                   75                  80                  85
   gaa gat gtg ctg ggg gag ggc gct cat gcc cga gtg cag acc tgc atc        342
   Glu Asp Val Leu Gly Glu Gly Ala His Ala Arg Val Gln Thr Cys Ile
               90                  95                  100
   aac ctg atc acc agc cag gag tac gcc gtc aag atc att gag aag cag        390
   Asn Leu Ile Thr Ser Gln Glu Tyr Ala Val Lys Ile Ile Glu Lys Gln
           105                 110                 115
   cca ggc cac att cgg agc agg gtt ttc agg gag gtg gag atg ctg tac      438
   Pro Gly His Ile Arg Ser Arg Val Phe Arg Glu Val Glu Met Leu Tyr
       120                 125                 130
   cag tgc cag gga cac agg aac gtc cta gag ctg att gag ttc ttc gag      486
   Gln Cys Gln Gly His Arg Asn Val Leu Glu Leu Ile Glu Phe Phe Glu
   135                 140                 145                 150
   gag gag gac cgc ttc tac ctg gtg ttt gag aag atg cgg gga ggc tcc      534
   Glu Glu Asp Arg Phe Tyr Leu Val Phe Glu Lys Met Arg Gly Gly Ser
                   155                 160                 165
   atc ctg agc cac atc cac aag cgc cgg cac ttc aac gag ctg gag gcc      582
   Ile Leu Ser His Ile His Lys Arg Arg His Phe Asn Glu Leu Glu Ala
               170                 175                 180
   agc gtg gtg gtg cag gac gtg gcc agc gcc ttg gac ttt ctg cat aac      630
   Ser Val Val Val Gln Asp Val Ala Ser Ala Leu Asp Phe Leu His Asn
           185                 190                 195
   aaa ggc atc gcc cac agg gac cta aag ccg gaa aac atc ctc tgt gag      678
   Lys Gly Ile Ala His Arg Asp Leu Lys Pro Glu Asn Ile Leu Cys Glu
       200                 205                 210
   cac ccc aac cag gtc tcc ccc gtg aag atc tgt gac ttc gac ctg ggc      726
   His Pro Asn Gln Val Ser Pro Val Lys Ile Cys Asp Phe Asp Leu Gly
   215                 220                 225                 230
   agc ggc atc aaa ctc aac ggg gac tgc tcc cct atc tcc acc ccg gag      774
   Ser Gly Ile Lys Leu Asn Gly Asp Cys Ser Pro Ile Ser Thr Pro Glu
                   235                 240                 245
   ctg ctc act ccg tgc ggc tcg gcg gag tac atg gcc ccg gag gta gtg      822
   Leu Leu Thr Pro Cys Gly Ser Ala Glu Tyr Met Ala Pro Glu Val Val
               250                 255                 260
   gag gcc ttc agc gag gag gct agc atc tac gac aag cgc tgc gac ctg      870
   Glu Ala Phe Ser Glu Glu Ala Ser Ile Tyr Asp Lys Arg Cys Asp Leu
           265                 270                 275
   tgg agc ctg ggc gtc atc ttg tat atc cta ctc agc ggc tac ccg ccc      918
   Trp Ser Leu Gly Val Ile Leu Tyr Ile Leu Leu Ser Gly Tyr Pro Pro
       280                 285                 290
   ttc gtg ggc cgc tgt ggc agc gac tgc ggc tgg gac cgc ggc gag gcc      966
   Phe Val Gly Arg Cys Gly Ser Asp Cys Gly Trp Asp Arg Gly Glu Ala
   295                 300                 305                 310
   tgc cct gcc tgc cag aac atg ctg ttt gag agc atc cag gag ggc aag     1014
   Cys Pro Ala Cys Gln Asn Met Leu Phe Glu Ser Ile Gln Glu Gly Lys
                   315                 320                 325
   tac gag ttc ccc gac aag gac tgg gcc cac atc tcc tgc gct gcc aaa     1062
   Tyr Glu Phe Pro Asp Lys Asp Trp Ala His Ile Ser Cys Ala Ala Lys
               330                 335                 340
   gac ctc atc tcc aag ctg ctg gtc cgt gac gcc aag cag agg ctg agt     1110
   Asp Leu Ile Ser Lys Leu Leu Val Arg Asp Ala Lys Gln Arg Leu Ser
           345                 350                 355
   gcc gcc caa gtc ctg cag cac ccc tgg gtt cag ggg tgc gcc ccg gag      1158
   Ala Ala Gln Val Leu Gln His Pro Trp Val Gln Gly Cys Ala Pro Glu
       360                 365                 370
   aac acc ttg ccc act ccc atg gtc ctg cag agg aac agc tgt gcc aaa      1206
   Asn Thr Leu Pro Thr Pro Met Val Leu Gln Arg Asn Ser Cys Ala Lys
   375                 380                 385                 390
   gac ctc acg tcc ttc gcg gct gag gcc att gcc atg aac cgg cag ctg      1254
   Asp Leu Thr Ser Phe Ala Ala Glu Ala Ile Ala Met Asn Arg Gln Leu
                   395                 400                 405
   gcc cag cac gac gag gac ctg gct gag gag gag gcc gcg ggg cag ggc      1302
   Ala Gln His Asp Glu Asp Leu Ala Glu Glu Glu Ala Ala Gly Gln Gly
               410                 415                 420
   cag ccc gtc ctg gtc cga gct acc tca cgc tgc ctg cag ctg tct cca      1350
   Gln Pro Val Leu Val Arg Ala Thr Ser Arg Cys Leu Gln Leu Ser Pro
           425                 430                 435
   ccc tcc cag tcc aag ctg gcg cag cgg cgg caa agg gcc agt ctg tcc      1398
   Pro Ser Gln Ser Lys Leu Ala Gln Arg Arg Gln Arg Ala Ser Leu Ser
       440                 445                 450
   tcg gcc cca gtg gtc ctg gtg gga gac cac gcc tga ccctcccatc           1444
   Ser Ala Pro Val Val Leu Val Gly Asp His Ala
   455                 460                 465
<210> 2
   <211> 465
   <212> PRT
   <213> human
<400> 2
   Met Val Gln Lys Lys Pro Ala Glu Leu Gln Gly Phe His Arg Ser Phe
   1               5                   10                  15
   Lys Gly Gln Asn Pro Phe Glu Leu Ala Phe Ser Leu Asp Gln Pro Asp
               20                  25                  30
   His Gly Asp Ser Asp Phe Gly Leu Gln Cys Ser Ala Arg Pro Asp Met
           35                  40                  45
   Pro Ala Ser Gln Pro Ile Asp Ile Pro Asp Ala Lys Lys Arg Gly Lys
       50                  55                  60
   Lys Lys Lys Arg Gly Arg Ala Thr Asp Ser Phe Ser Gly Arg Phe Glu
   65                  70                  75                  80
   Asp Val Tyr Gln Leu Gln Glu Asp Val Leu Gly Glu Gly Ala His Ala
                   85                  90                  95
   Arg Val Gln Thr Cys Ile Asn Leu Ile Thr Ser Gln Glu Tyr Ala Val
               100                 105                 110
   Lys Ile Ile Glu Lys Gln Pro Gly His Ile Arg Ser Arg Val Phe Arg
           115                 120                 125
   Glu Val Glu Met Leu Tyr Gln Cys Gln Gly His Arg Asn Val Leu Glu
       130                 135                 140
   Leu Ile Glu Phe Phe Glu Glu Glu Asp Arg Phe Tyr Leu Val Phe Glu
   145                 150                 155                 160
   Lys Met Arg Gly Gly Ser Ile Leu Ser His Ile His Lys Arg Arg His
                   165                 170                 175
   Phe Asn Glu Leu Glu Ala Ser Val Val Val Gln Asp Val Ala Ser Ala
               180                 185                 190
   Leu Asp Phe Leu His Asn Lys Gly Ile Ala His Arg Asp Leu Lys Pro
           195                 200                 205
   Glu Asn Ile Leu Cys Glu His Pro Asn Gln Val Ser Pro Val Lys Ile
       210                 215                 220
   Cys Asp Phe Asp Leu Gly Ser Gly Ile Lys Leu Asn Gly Asp Cys Ser
   225                 230                 235                 240
   Pro Ile Ser Thr Pro Glu Leu Leu Thr Pro Cys Gly Ser Ala Glu Tyr
                   245                 250                 255
   Met Ala Pro Glu Val Val Glu Ala Phe Ser Glu Glu Ala Ser Ile Tyr
               260                 265                 270
   Asp Lys Arg Cys Asp Leu Trp Ser Leu Gly Val Ile Leu Tyr Ile Leu
           275                 280                 285
   Leu Ser Gly Tyr Pro Pro Phe Val Gly Arg Cys Gly Ser Asp Cys Gly
       290                 295                 300
   Trp Asp Arg Gly Glu Ala Cys Pro Ala Cys Gln Asn Met Leu Phe Glu
   305                 310                 315                 320
   Ser Ile Gln Glu Gly Lys Tyr Glu Phe Pro Asp Lys Asp Trp Ala His
                   325                 330                 335
   Ile Ser Cys Ala Ala Lys Asp Leu Ile Ser Lys Leu Leu Val Arg Asp
               340                 345                 350
   Ala Lys Gln Arg Leu Ser Ala Ala Gln Val Leu Gln His Pro Trp Val
           355                 360                 365
   Gln Gly Cys Ala Pro Glu Asn Thr Leu Pro Thr Pro Met Val Leu Gln
       370                 375                 380
   Arg Asn Ser Cys Ala Lys Asp Leu Thr Ser Phe Ala Ala Glu Ala Ile
   385                 390                 395                 400
   Ala Met Asn Arg Gln Leu Ala Gln His Asp Glu Asp Leu Ala Glu Glu
                   405                 410                 415
   Glu Ala Ala Gly Gln Gly Gln Pro Val Leu Val Arg Ala Thr Ser Arg
               420                 425                 430
   Cys Leu Gln Leu Ser Pro Pro Ser Gln Ser Lys Leu Ala Gln Arg Arg
           435                 440                 445
   Gln Arg Ala Ser Leu Ser Ser Ala Pro Val Val Leu Val Gly Asp His
       450                 455                 460
   Ala
   465
<210> 3
   <211> 1564
   <212> DNA
   <213> human
<220>
   <221> CDS
   <222> (37)..(1281)
<300>
   <308> GenBank/AF237776
   <309> 2000-10-26
   <313> (1)..(1564)
<400> 3
   cggtcccctc ccccgctggc ggggcccgga cagaag atg gtg cag aag aaa cca        54
                                           Met Val Gln Lys Lys Pro
                                           1               5
   gcc gaa ctt cag ggt ttc cac cgt tcg ttc aag ggg cag aac ccc ttc       102
   Ala Glu Leu Gln Gly Phe His Arg Ser Phe Lys Gly Gln Asn Pro Phe
               10                  15                  20
   gag ctg gcc ttc tcc cta gac cag ccc gac cac gga gac tct gac ttt       150
   Glu Leu Ala Phe Ser Leu Asp Gln Pro Asp His Gly Asp Ser Asp Phe
           25                  30                  35
   ggc ctg cag tgc tca gcc cgc cct gac atg ccc gcc agc cag ccc att        198
   Gly Leu Gln Cys Ser Ala Arg Pro Asp Met Pro Ala Ser Gln Pro Ile
       40                  45                  50
   gac atc ccg gac gcc aag aag agg ggc aag aag aag aag cgc ggc cgg        246
   Asp Ile Pro Asp Ala Lys Lys Arg Gly Lys Lys Lys Lys Arg Gly Arg
   55                  60                  65                  70
   gcc acc gac agc ttc tcg ggc agg ttt gaa gac gtc tac cag ctg cag        294
   Ala Thr Asp Ser Phe Ser Gly Arg Phe Glu Asp Val Tyr Gln Leu Gln
                   75                  80                  85
   gaa gat gtg ctg ggg gag ggc gct cat gcc cga gtg cag acc tgc atc        342
   Glu Asp Val Leu Gly Glu Gly Ala His Ala Arg Val Gln Thr Cys Ile
               90                  95                  100
   aac ctg atc acc agc cag gag tac gcc gtc aag atc att gag aag cag        390
   Asn Leu Ile Thr Ser Gln Glu Tyr Ala Val Lys Ile Ile Glu Lys Gln
           105                 110                 115
   cca ggc cac att cgg agc agg gtt ttc agg gag gtg gag atg ctg tac        438
   Pro Gly His Ile Arg Ser Arg Val Phe Arg Glu Val Glu Met Leu Tyr
       120                 125                 130
   cag tgc cag gga cac agg aac gtc cta gag ctg att gag ttc ttc gag        486
   Gln Cys Gln Gly His Arg Asn Val Leu Glu Leu Ile Glu Phe Phe Glu
   135                 140                 145                 150
   gag gag gac cgc ttc tac ctg gtg ttt gag aag atg cgg gga ggc tcc        534
   Glu Glu Asp Arg Phe Tyr Leu Val Phe Glu Lys Met Arg Gly Gly Ser
                   155                 160                 165
   atc ctg agc cac atc cac aag cgc cgg cac ttc aac gag ctg gag gcc        582
   Ile Leu Ser His Ile His Lys Arg Arg His Phe Asn Glu Leu Glu Ala
               170                 175                 180
   agc gtg gtg gtg cag gac gtg gcc agc gcc ttg gac ttt ctg cat aac        630
   Ser Val Val Val Gln Asp Val Ala Ser Ala Leu Asp Phe Leu His Asn
           185                 190                 195
   aaa ggc atc gcc cac agg gac cta aag ccg gaa aac atc ctc tgt gag        678
   Lys Gly Ile Ala His Arg Asp Leu Lys Pro Glu Asn Ile Leu Cys Glu
       200                 205                 210
   cac ccc aac cag gtc tcc ccc gtg aag atc tgt gac ttc gac ctg ggc        726
   His Pro Asn Gln Val Ser Pro Val Lys Ile Cys Asp Phe Asp Leu Gly
   215                 220                 225                 230
   agc ggc atc aaa ctc aac ggg gac tgc tcc cct atc tcc acc ccg gag        774
   Ser Gly Ile Lys Leu Asn Gly Asp Cys Ser Pro Ile Ser Thr Pro Glu
                   235                 240                 245
   ctg ctc act ccg tgc ggc tcg gcg gag tac atg gcc ccg gag gta gtg        822
   Leu Leu Thr Pro Cys Gly Ser Ala Glu Tyr Met Ala Pro Glu Val Val
               250                 255                 260
   gag gcc ttc agc gag gag gct agc atc tac gac aag cgc tgc gac ctg        870
   Glu Ala Phe Ser Glu Glu Ala Ser Ile Tyr Asp Lys Arg Cys Asp Leu
           265                 270                 275
   tgg agc ctg ggc gtc atc ttg tat atc cta ctc agc ggc tac ccg ccc      918
   Trp Ser Leu Gly Val Ile Leu Tyr Ile Leu Leu Ser Gly Tyr Pro Pro
       280                 285                 290
   ttc gtg ggc cgc tgt ggc agc gac tgc ggc tgg gac cgc ggc gag gcc      966
   Phe Val Gly Arg Cys Gly Ser Asp Cys Gly Trp Asp Arg Gly Glu Ala
   295                 300                 305                 310
   tgc cct gcc tgc cag aac atg ctg ttt gag agc atc cag gag ggc aag     1014
   Cys Pro Ala Cys Gln Asn Met Leu Phe Glu Ser Ile Gln Glu Gly Lys
                   315                 320                 325
   tac gag ttc ccc gac aag gac tgg gcc cac atc tcc tgc gct gcc aaa     1062
   Tyr Glu Phe Pro Asp Lys Asp Trp Ala His Ile Ser Cys Ala Ala Lys
               330                 335                 340
   gac ctc atc tcc aag ctg ctg gtc cgt gac gcc aag cag agg ctg agt     1110
   Asp Leu Ile Ser Lys Leu Leu Val Arg Asp Ala Lys Gln Arg Leu Ser
           345                 350                 355
   gcc gcc caa gtc ctg cag cac ccc tgg gtt cag ggg tgc gcc ccg gag     1158
   Ala Ala Gln Val Leu Gln His Pro Trp Val Gln Gly Cys Ala Pro Glu
       360                 365                 370
   aac acc ttg ccc act ccc atg gtc ctg cag agg tgg gac agt cac ttc     1206
   Asn Thr Leu Pro Thr Pro Met Val Leu Gln Arg Trp Asp Ser His Phe
   375                 380                 385                 390
   ctc ctc cct ccc cac ccc tgt cgc atc cac gtg cga cct gga gga ctg     1254
   Leu Leu Pro Pro His Pro Cys Arg Ile His Val Arg Pro Gly Gly Leu
                   395                 400                 405
   gtc aga acc gtt act gtg aat gag tga agatcctgga ggaccctggg           1301
   Val Arg Thr Val Thr Val Asn Glu
               410
   ccccaggcca gctcccatcg ctgggggacg gtgaacggcc atgtgttaat gttacgatgt   1361
   ttttaaaaga caaaaaaaaa aaaaaaacct caaaagtttt tttaaagtgg gggaaaaaca   1421
   tccaagcact ttaattccaa tgtaccaggt gaactgacgg agctcagaag ttttccttta   1481
   caccaactgt caatgccgga attttgtatt ctgttttgta aagatttaat aaaagtcaaa   1541
   aaacttgcaa aaaaaaaaaa aaa                                           1564
<210> 4
   <211> 414
   <212> PRT
   <213> human
   <400> 4
   Met Val Gln Lys Lys Pro Ala Glu Leu Gln Gly Phe His Arg Ser Phe
   1               5                   10                  15
   Lys Gly Gln Asn Pro Phe Glu Leu Ala Phe Ser Leu Asp Gln Pro Asp
               20                  25                  30
   His Gly Asp Ser Asp Phe Gly Leu Gln Cys Ser Ala Arg Pro Asp Met
   35 40 45
   Pro Ala Ser Gln Pro Ile Asp Ile Pro Asp Ala Lys Lys Arg Gly Lys
   50 55 60
   Lys Lys Lys Arg Gly Arg Ala Thr Asp Ser Phe Ser Gly Arg Phe Glu
   65 70 75 80
   Asp Val Tyr Gln Leu Gln Glu Asp Val Leu Gly Glu Gly Ala His Ala
   85 90 95
   Arg Val Gln Thr Cys Ile Asn Leu Ile Thr Ser Gln Glu Tyr Ala Val
   100 105 110
   Lys Ile Ile Glu Lys Gln Pro Gly His Ile Arg Ser Arg Val Phe Arg
   115 120 125
   Glu Val Glu Met Leu Tyr Gln Cys Gln Gly His Arg Asn Val Leu Glu
   130 135 140
   Leu Ile Glu Phe Phe Glu Glu Glu Asp Arg Phe Tyr Leu Val Phe Glu
   145 150 155 160
   Lys Met Arg Gly Gly Ser Ile Leu Ser His Ile His Lys Arg Arg His
   165 170 175
   Phe Asn Glu Leu Glu Ala Ser Val Val Val Gln Asp Val Ala Ser Ala
   180 185 190
   Leu Asp Phe Leu His Asn Lys Gly Ile Ala His Arg Asp Leu Lys Pro
   195 200 205
   Glu Asn Ile Leu Cys Glu His Pro Asn Gln Val Ser Pro Val Lys Ile
   210 215 220
   Cys Asp Phe Asp Leu Gly Ser Gly Ile Lys Leu Asn Gly Asp Cys Ser
   225 230 235 240
   Pro Ile Ser Thr Pro Glu Leu Leu Thr Pro Cys Gly Ser Ala Glu Tyr
   245 250 255
   Met Ala Pro Glu Val Val Glu Ala Phe Ser Glu Glu Ala Ser Ile Tyr
   260 265 270
   Asp Lys Arg Cys Asp Leu Trp Ser Leu Gly Val Ile Leu Tyr Ile Leu
           275                 280                 285
   Leu Ser Gly Tyr Pro Pro Phe Val Gly Arg Cys Gly Ser Asp Cys Gly
       290                 295                 300
   Trp Asp Arg Gly Glu Ala Cys Pro Ala Cys Gln Asn Met Leu Phe Glu
   305                 310                 315                 320
   Ser Ile Gln Glu Gly Lys Tyr Glu Phe Pro Asp Lys Asp Trp Ala His
                   325                 330                 335
   Ile Ser Cys Ala Ala Lys Asp Leu Ile Ser Lys Leu Leu Val Arg Asp
               340                 345                 350
   Ala Lys Gln Arg Leu Ser Ala Ala Gln Val Leu Gln His Pro Trp Val
           355                 360                 365
   Gln Gly Cys Ala Pro Glu Asn Thr Leu Pro Thr Pro Met Val Leu Gln
       370                 375                 380
   Arg Trp Asp Ser His Phe Leu Leu Pro Pro His Pro Cys Arg Ile His
   385                 390                 395                 400
   Val Arg Pro Gly Gly Leu Val Arg Thr Val Thr Val Asn Glu
                   405                 410
<210> 5
   <211> 18
   <212> PRT
   <213> Unknown
<220>
   <221> MOD_RES
   <222> (1)..(18)
   <223> Biotinylated 18 amino acids peptide sequence from the transcription factor CREB
<400> 5
   Ala Gly Ala Gly Lys Arg Arg Glu Ile Leu Ser Arg Arg Pro Ser Tyr
   1               5                   10                  15
   Arg Lys
<210> 6
   <211> 13
   <212> PRT
   <213> Unknown
<220>
   <221> MOD_RES
   <222> (1)..(13)
   <223> Biotinylated ACC2 peptide from Neosystem, part number SPO21928
<400> 6
   Arg Val Pro Thr Met Arg Pro Ser Met Ser Gly Leu His
   1               5                   10

## Description

### TECHNICAL FIELD

The invention relates to certain pyrazine and pyridine compounds that act as inhibitors of the MAP kinase interacting kinases MNK2a, MNK2b and MNK1. The invention further relates to pharmaceutical compositions comprising these compounds, and to the use of the compounds for the preparation of a medicament for the prophylaxis and treatment of type 2 diabetes, obesity and inflammatory conditions, as well as methods of treatment of these disorders.

### BACKGROUND ART

One of the major hormones that influences metabolism is insulin, which is synthesized in the beta cells of the islets of Langerhans of the pancreas. Insulin primarily regulates the direction of metabolism, shifting many processes toward the storage of substrates and away from their degradation (for reviews, see e.g. Shepherd, P.R. et al. (1998) Biochem. J. 333: 471-490; Alessi, D. R. & Downes, C. P. (1998) Biochim. Biophys. Acta 1436: 151-164). Insulin is believed to to be involved in the transport of glucose and amino acids as well as key minerals such as potassium, magnesium, and phosphate from the blood into cells. Insulin is also believed to regulate a variety of enzymatic reactions within the cells, which involve the synthesis of large molecules from smaller building block units. A deficiency in the action of insulin (diabetes mellitus) can cause severe impairment in (i) the storage of glucose in the form of glycogen and the oxidation of glucose for energy; (ii) the synthesis and storage of fat from fatty acids and their precursors and the completion of fatty-acid oxidation; and (iii) the synthesis of proteins from amino acids.

There are two varieties of diabetes. Type I is insulin-dependent diabetes mellitus (IDDM; formerly referred to as juvenile onset diabetes), for which administration of insulin is required. In this type, insulin is not secreted by the pancreas and hence must be administered. Type II diabetes, i.e. non-insulin-dependent diabetes mellitus (NIDDM), is characterized clinically by hyperglycemia and insulin resistance and is commonly associated with obesity. Type II diabetes is a heterogeneous group of disorders in which hyperglycemia typically results from both an impaired insulin secretory response to glucose and decreased insulin effectiveness in stimulating glucose uptake by skeletal muscle and in restraining hepatic glucose production (insulin resistance). Before diabetes develops, patients generally lose the early insulin secretory response to glucose and may secrete relatively large amounts of proinsulin. In established diabetes, although fasting plasma insulin levels may be normal or even increased in type II diabetes patients, glucose-stimulated insulin secretion is clearly decreased. The decreased insulin levels typically reduce insulin-mediated glucose uptake and fail to restrain hepatic glucose production.

Glucose homeostasis depends upon a balance between glucose production by the liver and glucose utilization by insulin-dependent tissues, such as fat and muscle, and insulin-independent tissues, such as brain and kidney. In type II diabetes, the entry of glucose into fat and muscle is reduced and glucose production in the liver is increased, due to insulin resistance in the tissues.

The receptor tyrosine kinases (RTKs) are a class of cell-surface receptors. The ligands for RTKs include peptide/protein hormones including nerve growth factor (NGF), platelet-derived growth factor (PDGF), epidermal growth factor (EGF), and insulin. Binding of a ligand to an RTK is believed to stimulate the receptor's intrinsic protein-tyrosine kinase activity, which subsequently can stimulate a signal-transduction cascade leading to changes in cellular physiology and patterns of gene expression. RTK signaling pathways have a wide spectrum of functions including regulation of cell proliferation and differentiation, promotion of cell survival, and modulation of cellular metabolism.

Ras is a GTP-binding switch protein that acts in a manner similar to a key signaling molecule in pathways triggered by activation of RTKs. In general. Ras-linked RTKs in mammalian cells appear to utilize a highly conserved signal-transduction pathway in which activated Ras induces a kinase cascade that culminates in the activation of MAP kinase (mitogen-activated protein kinase). This serine/threonine kinase, which can translocate into the nucleus, phosphorylates many different proteins including transcription factors that regulate expression of what are considered to be important cell-cycle and differentiation-specific proteins.

The murine MNK1 and MNK2 gene products ("MAP kinase interacting kinase" or "MAP kinase signal-integrating kinase" 1 and 2) are single-domain serine/threonine kinases that share 72% sequence identity (Waskiewicz A.J. et al. (1997) EMBO J. 16: 1909-1920; GenBank Accession Nos. Y11091 and Y11092). Human MNK1 has also been described (Fukunaga, R. et al. (1999) EMBO J. 16: 1921-1933; GenBank Accession No. AB000409). All these three proteins were identified, in part, by their ability to bind tightly to MAP kinases. Both MNK1 and 2 bind the extracellular signal-regulated kinases ERK1 and ERK2, and MNK1 also binds the stress-activated kinase, p38. The eukaryotic initiation factor 4E (eIF4E) has been identified as one of the physiological substrates of MNK1 and MNK2 (Scheper, G.C. et al. (2001) Mol. Cell. Biol. 21: 743-754).

According to the findings of Harris et al. (Blood (2004), vol. 104:5, pp 1314-1323), some eIFs, such as eIF4E, selectively enhance expression of growth-promoting (e.g. cyclin D) and metastasis-related mRNAs (e.g. vascular endothelial growth factor), thus suggesting that translation control through regulation of eiFs may play a role in tumor growth control.

The human mnk2 gene has been identified and characterized through a yeast two-hybrid screen in which the MNK2 protein interacted with the ligand-binding domain of the estrogen receptor (ERβ) (Slentz-Kesler, K. et al. (2000) Genomics 69: 63-71). It was shown that the human mnk2 gene has two C-terminal splice variants, designated mnk2a (the nucleotide and amino acid sequences of mnk2a and MNK2a, respectively, are designated SEQ ID NOS:1 and 2, respectively; GenBank Accession No. AF237775) and mnk2b (the nucleotide and amino acid sequences of mnk2b and MNK2b, respectively, are designated SEQ ID NOS: 3 and 4, respectively; GenBank Accession No. AF237776). The two isoforms have been shown to be identical over the first 385 amino acids of the coding sequence and differ only in the final exon which encodes an additional 80 residues for mnk2a and 29 residues for mnk2b. It was further shown that the MNK2 interaction was selective for estrogen receptor (ER) as opposed to ERl and that the interaction was specific to MNK2b as opposed to MNK2a or MNK 1.

WO02/10336 discloses that MNK2 is involved in the insulin-signaling pathway and features a method for identifying a modulator of glucose uptake.

WO03/037362 suggests that MNK kinases, particularly MNK2 (MNK2a and MNK2b), are involved in the regulation of body-weight and thermogenesis, and thus may be associated with metabolic diseases such as obesity, as well as related disorders such as eating disorder, cachexia, diabetes mellitus, hypertension, coronary heart disease, hypercholesterolemia, dyslipidemia, osteoarthritis, gallstones, and sleep apnea, and disorders related to ROS defence, such as diabetes mellitus, neurodegenerative disorders, and cancer, e.g. cancers of the reproductive organs.

According to WO2005/003785, the MNK kinases are believed to be promising targets for anti-inflammatory therapy.

See also, e.g., Buxadé et al. (Immunity (2005); vol. 23:177-189) according to which heterogeneous nuclear ribonucleoprotein A1, hnRNP A1, is shown to be another substrate for MNK1. hnRNP A1 is involved in the synthesis of TNFα.

The MNK1 protein has also been shown by Worch et al. (Oncogene (2004); 23:9162-9172) to be induced by acute myeloid leukaemia (AML) translocation products, PML-RARα, PLZF-RARα and AML1-ETO, in cell lines, by stabilization of the MNK1 protein. Inhibition of MNK1 enhanced hematopoietic cell differentiation. In AML patients 25 of 99 samples of bone marrow showed MNK1 expression with cytoplasmic localization and in these patients MNK1 expression was associated with the oncogene, c-Myc, protein expression.

To achieve fine-tuned regulation of RTK signalling, cells employ multiple negative feedback mechanisms that intercept RTK pathways at different levels. One such mechanism involves the conserved antagonist of several RTKs, Sprouty (Spry). MNK1 has recently been identified by DaSilva et al. (Mol. Cell. Biology (2006); vol 26:1898-1907) to phosphorylate human Spry2. Deregulation of RTK signalling has been implicated in the establishment of developmental disorders and in the progression of many types of cancer.

### SUMMARY OF INVENTION

This invention relates generally to certain pyrazine and pyridine compounds that can act as inhibitors of the MAP kinase interacting kinases MNK2a, MNK2b and MNK1 (e.g., MNK2a and MNK2b) and related pharmaceutical compositions and methods.

In one aspect this invention relates to a compound of the general formula (I): 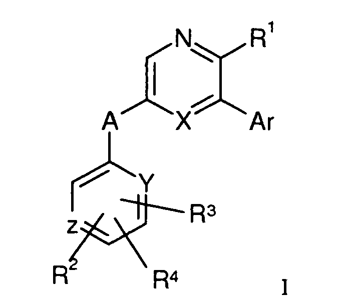 or a pharmaceutically acceptable salt, hydrates, geometrical isomers, racemates, tautomers, optical isomers, N-oxides and prodrug forms thereof, wherein:
X is N or CH;
Y and Z are each selected from N or CH; when Y and/or Z is CH, the hydrogen of the CH moiety can be replaced by any one of R², R³, or R⁴;
A is a bond, -NH- or -N-C₁₋₆ alkyl;
R¹ is H or NH₂;
Ar is benzofuranyl, indolyl, hydroxyphenyl, thienyl, benzothiophenyl, pyrrolyl, aminocarbonylphenyl, or azaindolyl, each of which is unsubstituted or substituted with from 1-2 substituents independently selected from hydroxy, halogen, -CN, -NO₂, C₁₋₆ alkyl, C₁₋₆ alkoxy, acyl, C₁₋₆ alkylsulphonyl, -C(O)NH₂, -NH-C(O)-R⁷, and arylsulphonyl;
R² and R³, are each, independently, located at a position ortho or meta with respect to A; and are each, independently, H, halogen, hydroxy, C₁₋₆ alkyl, hydroxy-C₁₋₆ alkyl, carboxy-C₁₋₆ alkyl, C₁₋₆ alkoxy, C₁₋₆ alkoxy-C₁₋₆ alkoxy, di-C₁₋₆ alkylamino-C₁₋₆ alkoxy, or di-C₁₋₆ alkylaminocarbonyl-C₁₋₆ alkoxy;
R⁴ is located at a position para or meta with respect to A and is -C(O)NR⁵R⁶ or -S(O)₂NR⁵R⁶;
R⁵ is H, C₁₋₆ alkyl, or aryl-C₁₋₆ alkyl;
R⁶ is H, C₁₋₆ alkyl, halo-C₁₋₆ alkyl, di-C₁₋₆ alkylamino-C₁₋₆ alkyl, mono-C₁₋₆ alkylamino-C₁₋₆ alkyl, amino-C₁₋₆ alkyl, hydroxy-C₁₋₆ alkylamino-C₁₋₆ alkyl, di(hydroxy-C₁₋₆ alkyl)amino-C₁₋₆ alkyl, hydroxy-C₁₋₆ alkyl, dihydroxy-C₃₋₆ alkyl, cycloalkyl, heterocyclyl, heterocyclyl-C₁₋₆ alkyl, or heteroaryl-C₁₋₆ alkyl, wherein the heterocyclyl or heteroaryl is unsubstituted or substituted with from 1-2 substituents independently selected from C₁₋₆ alkyl, C₁₋₆ alkyl-OC(O)-or aryl-C₁₋₆ alkyl, said C₁₋₆ alkyl-OC(O)- being attached to a ring N atom; or
R⁵ and R⁶ together with the nitrogen to which they are attached form a 4 to 7 membered heterocyclyl, wherein the 4 to 7 membered heterocyclyl optionally includes a second heteroatom ring member selected from N, S and O (in these embodiments, two of the ring members of the heterocyclyl are heteroatoms: one of the two heteroatoms is the nitrogen atom connecting R⁵ and R⁶, and the other is either N, S or O), and wherein the 4 to 7 membered heterocyclyl is optionally substituted with from 1-2 substituents independently selected from C₁₋₆ alkyl, hydroxy-C₁₋₆ alkyl, C₁₋₂-alkoxy-C₂₋₄-alkyl, di-C₁₋₆ alkylamino, mono-C₁₋₆ alkylamino, amino, di-C₁₋₆ alkylamino-C₁₋₆ alkyl, mono-C₁₋₆ alkylamino-C₁₋₆ alkyl, amino-C₁₋₆ alkyl, C₃₋₇ cycloalkyl, oxo, heterocyclyl-C₁₋₄ alkyl, C₁₋₆ alkyl-OC(O)NH-, and heteroaryl-carbonyl,; or wherein two geminal substituents on said 4- to 7-membered heterocyclyl together form a second 5 to 6 membered heterocyclyl giving a spiro radical, which is optionally substituted; and
R⁷ is H or C₁₋₆ alkyl;
with the provisos that:
when R¹ is NH₂, then A is a bond; and
when A is -NH- or -N-C₁₋₆-alkyl then R¹ is H.

In one embodiment the invention relates to a compound of the general formula (II): 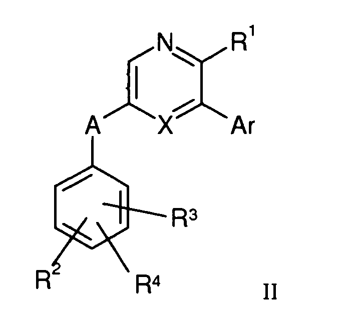 or a pharmaceutically acceptable salt, hydrates, geometrical isomers, racemates, tautomers, optical isomers, N-oxides and prodrug forms thereof, wherein:
X is N or CH (or X can be a carbon atom substituted with any substituent described herein);
A is a bond, -NH-;
R¹ is H or NH₂;
Ar is benzofuranyl, indolyl, hydroxyphenyl, thienyl, benzothiophenyl, pyrrolyl, each of which is unsubstituted or substituted with from 1-2 substituents independently selected from hydroxy, halogen, C₁₋₆ alkyl, C₁₋₆ alkoxy, acyl, C₁₋₆ alkylsulphonyl, and arylsulphonyl;
R² and R³, are each, independently, located at a position ortho or meta with respect to A; and are each, independently, H, halogen, C₁₋₆ alkyl, hydroxy-C₁₋₆ alkyl, carboxy-C₁₋₆ alkyl, or C₁₋₆ alkoxy;
R⁴ is located at a position para or meta with respect to A and is -C(O)NR⁵R⁶ or -S(O)₂NR⁵R⁶;
R⁵ is H, C₁₋₆ alkyl, or aryl-C₁₋₆ alkyl;
R⁶ is H, C₁₋₆ alkyl, halo-C₁₋₆ alkyl, di-C₁₋₆ alkylamino-C₁₋₆ alkyl, mono-C₁₋₆ alkylamino-C₁₋₆ alkyl, amino-C₁₋₆ alkyl, hydroxy-C₁₋₆ alkylamino-C₁₋₆ alkyl, di(hydroxy-C₁₋₆ alkyl)amino-C₁₋₆ alkyl, hydroxy-C₁₋₆ alkyl, dihydroxy-C₃₋₆ alkyl, cycloalkyl, heterocyclyl, heterocyclyl-C₁₋₆ alkyl, or heteroaryl-C₁₋₆ alkyl, wherein the heterocyclyl or heteroaryl is unsubstituted or substituted with from 1-2 substituents independently selected from C₁₋₆ alkyl, C₁₋₆ alkyl-OC(O)-or aryl-C₁₋₆ alkyl, said C₁₋₆ alkyl-OC(O)- being attached to a ring N atom; or
R⁵ and R⁶ together with the nitrogen to which they are attached form a 4 to 7 membered heterocyclyl, wherein the 4 to 7 membered heterocyclyl optionally includes a second heteroatom ring member selected from N, S and O (in these embodiments, two of the ring members of the heterocyclyl are heteroatoms: one of the two heteroatoms is the nitrogen atom connecting R⁵ and R⁶, and the other is either N or O), and wherein the 4 to 7 membered heterocyclyl is optionally substituted with from 1-2 substituents independently selected from C₁₋₆ alkyl, hydroxy-C₁₋₆ alkyl, di-C₁₋₆ alkylamino, mono-C₁₋₆ alkylamino, amino, di-C₁₋₆ alkylamino-C₁₋₆ alkyl, mono-C₁₋₆ alkylamino-C₁₋₆ alkyl, amino-C₁₋₆ alkyl, C₃₋₇ cycloalkyl, C₁₋₆ alkyl-OC(O)NH-, and heteroaryl-carbonyl and C₁₋₆ alkyl-OC(O)NH-;
with the provisos that:
when R¹ is NH₂, then A is a bond; and
when A is -NH- then R¹ is H.

In another aspect, this invention relates to inhibiting the MAP kinase interacting kinases MNK2a, MNK2b and MNK1 (e.g., MNK2a and/or MNK2b) with the compounds described herein. In some embodiments, the methods can include, e.g., contacting one or more of the MAP kinase interacting kinases MNK2a, MNK2b or MNK1 in a sample with a compound having any of the formulae described herein. In other embodiments, the methods can include administering a compound having any of the formulae described herein to a subject (e.g., a subject in need thereof, e.g., a mammal; e.g., a human; e.g., a human having, identified as having, at risk of having, or identified as being at risk of having one or more of the diseases or disorders described herein).

In some embodiments, the compounds of the above formula can exhibit an MNK2 inhibiting activity corresponding to an IC₅₀ of from about 1 nanomolar (nM) to about 3 micromolar (µM), or a lower concentration as tested in an conventional MNK2a in vitro HTRF assay as will be described below.

In some embodiments, the compounds of the above formula can exhibit an MNK2 inhibiting activity corresponding to an IC₅₀ of from about 1 nM to about 3 µM (e.g., from about 1 nM to about 2 µM, from about 1 nM to about 1 µM, from about 1 nM to about 500 nM, from about 1 nM to about 100 nM, from about 1 nM to about 25 nM, from about 1 nM to about 10 nM).

While not wishing to be bound by theory, it is believed that the compounds described herein can be used, e.g., for the treatment or prevention of type 2 diabetes; and/or as anti-inflammatory agents, and/or in treatment of disorders related to energy homeostasis, the regulation of body-weight and thermogenesis, and metabolic diseases and related disorders, and disorders related to ROS defence, neurodegenerative disorders, and cancer.

In a further aspect, this invention relates to a method for the treatment or prophylaxis (e.g., treatment) of a disease, disorder, or condition related to undesired activity of MNK1 and/or MNK2 kinases (e.g., obesity, eating disorder, cachexia, diabetes mellitus, hypertension, coronary heart disease, hypercholesterolemia, dyslipidemia, hyperlipidemia, hyperglycemia, osteoarthritis, gallstones, sleep apnea, neurodegenerative disorders, cancer, inflammatory conditions and type 2 diabetes). The method includes administering to a subject (e.g., a subject in need thereof, e.g., a mammal; e.g., a human; e.g., a human having, identified as having, at risk of having, or identified as being at risk of having one or more of the diseases or disorders described herein) an effective amount of a compound of formula I or a pharmaceutically acceptable salt or prodrug thereof.

In one aspect, this invention relates to a method for the treatment or prophylaxis (e.g., treatment) of type 2 diabetes, which includes administering to a subject (e.g., a subject in need of such treatment as described herein) an effective amount of a compound of formula I or a pharmaceutically acceptable salt or prodrug thereof.

In another aspect, this invention relates to a method for the treatment or prophylaxis (e.g., treatment) of an inflammatory condition, which includes administering to a subject (e.g., a subject in need of such treatment as described herein) an effective amount of a compound of formula I or a pharmaceutically acceptable salt or prodrug thereof.

In a further aspect, this invention relates to a method for the treatment or prophylaxis (e.g., treatment) of cancer, which includes administering to a subject (e.g., a subject in need of such treatment as described herein) an effective amount of a compound of formula I or a pharmaceutically acceptable salt or prodrug thereof.

In one aspect, this invention relates to a method for the treatment or prophylaxis (e.g., treatment) of obesity, which includes administering to a subject (e.g., a subject in need of such treatment as described herein) an effective amount of a compound of formula I or a pharmaceutically acceptable salt or prodrug thereof.

In another aspect, this invention relates to a method for reducing body weight in a subject, which includes administering to a subject (e.g., a subject in need of such treatment as described herein) an effective amount of a compound of formula I or a pharmaceutically acceptable salt or prodrug thereof.

In some embodiments, the subject can be a subject in need thereof (e.g., a subject identified as being in need of such treatment as described herein). Identifying a subject in need of such treatment can be in the judgment of a subject or a health care professional and can be subjective (e.g. opinion) or objective (e.g. measurable by a test or diagnostic method). In some embodiments, the subject can be a mammal. In certain embodiments, the subject is a human.

In a further aspect, this invention relates to the use of a compound of formula I (e.g., as a medicament) or in the manufacture of a medicament containing a compound of formula I for the treatment or prophylaxis (e.g., treatment) of a disease, disorder, or condition related to undesired activity of MNK1 and/or MNK2 kinases as described herein.

In one aspect, the invention relates to a compound (including a pharmaceutically acceptable salt thereof) of any of the formulae delineated herein (e.g., a compound having formula (I), (II), (III), (IV), (V) or (VI) (or subgenera thereof), including the specific compounds described herein); or a composition or formulation (e.g., a pharmaceutical composition or formulation) comprising a compound (including a pharmaceutically acceptable salt thereof) of any of the formulae delineated herein (e.g., a compound having formula (I), (II), (III), (IV), (V) or (VI) (or subgenera thereof), including the specific compounds described herein). In some embodiments, the composition or formulation can further include a pharmaceutically acceptable adjuvant, carrier or diluent. Any such compound can be used in the methods described herein.

In some embodiments, the compound of formula I can be a pyrazine derivative of formula III. 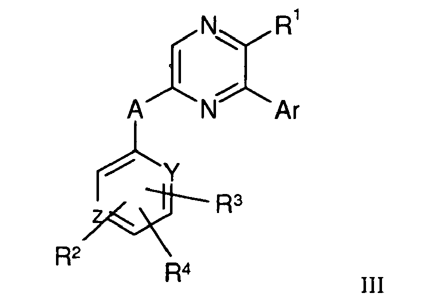 or pharmaceutically acceptable salts, hydrates, geometrical isomers, racemates, tautomers, optical isomers, N-oxides and prodrug forms thereof, wherein: Y, Z, A, Ar, R¹, R², R³, R⁴, R⁵ and R⁶ can be defined anywhere herein. It is provided that when R¹ is NH₂, then A is a bond; and when A is -NH- or - N-C₁₋₆-alkyl then R¹ is H.

In some embodiments, the compound of formula I can be pyridine derivative of formula IV. 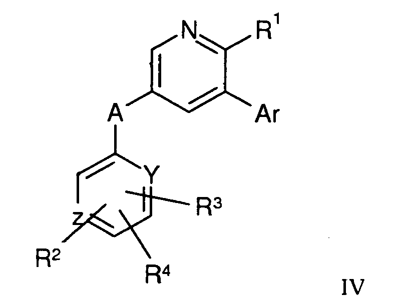 or pharmaceutically acceptable salts, hydrates, geometrical isomers, racemates, tautomers, optical isomers, N-oxides and prodrug forms thereof, wherein: Y, Z, A, Ar, R¹, R², R³, R⁴, R⁵ and R⁶ can be defined anywhere herein. It is provided that when R¹ is NH₂, then A is a bond; and when A is -NH- or - N-C₁₋₆-alkyl then R¹ is H.

The details of one or more embodiments of the invention are set forth in the description below. Other features and advantages of the invention will be apparent from the description and from the claims.

### DETAILED DESCRIPTION OF THE INVENTION

This invention relates generally to certain pyrazine and pyridine compounds that can act as inhibitors of the MAP kinase interacting kinases MNK2a, MNK2b and MNK1 (e.g., MNK2a and MNK2b) and related pharmaceutical compositions and methods.

In some embodiments, certain pyrazine derivatives inhibit the MAP kinase interacting kinases MNK2a and MNK2b. It has surprisingly also been found that, when substituting the pyrazine ring for a pyridine ring, activity was retained for the compounds tested. The present inventors therefore believe the corresponding pyridine derivatives also to exhibit similar activity. The compounds of the present invention are generally represented by the general formula I above.

Additionally, the compounds of the invention have surprisingly been found by the present inventors also to have MNK1 activity. Based on the compounds tested, the MNK1 and MNK2 (tested as MNK2a) activities of the compounds of the invention are believed to be of a similar magnitude, such as generally within an activity ratio of MNK1:MNK2 of 1:20 to 20:1.

According to WO2005/003785, the MNKs (encompassing MNK1 and MNK2) are believed to be promising targets for anti-inflammatory therapy. While not wishing to be bound by theory, since the present compounds have been found to be highly active in inhibiting MNK2, and also MNK1, as described above, the present compounds are believed to be useful in anti-inflammatory therapy.

The present compounds are also believed to be useful in anti-cancer therapy, such as in the treatment of AML.

In one embodiment the invention relates to pyrazine derivatives represented by the general formula (III) below

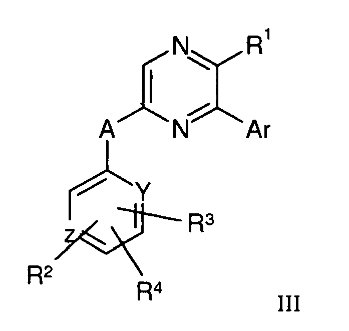

In another embodiment the invention relates to pyridine derivatives represented by the general formula (IV) below

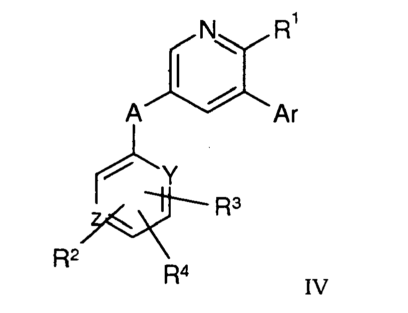

In one embodiment the invention relates to pyrazine derivatives represented by the general formula (V] below

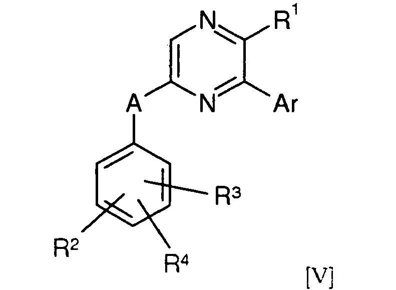

In one embodiment the invention relates to pyridine derivatives represented by the general formula (VI) below

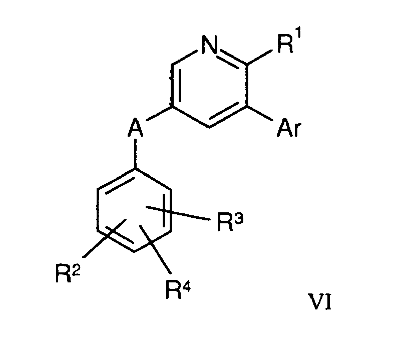

### Definitions

The following definitions shall apply throughout the specification and the appended claims.

Unless otherwise indicated the term "MNK2" as used herein collectively refers to the MAP kinase interacting kinases MNK2a and MNK2b.

The terms "MNK2 related disorder", "disorder or condition associated with the activity of MNK2" and "disorder related to undesired activity of MNK2", have been used interchangeably herein to denote any disorder or symptom wherein the MNK2 is involved in the process or presentation of the disorder or the symptom. The MNK2 related disorders thus e.g. include, but are not limited to, type 2 diabetes and inflammatory conditions.

Similarily, the terms "MNK1 related disorder", "disorder or condition associated with the activity of MNK1" and "disorder related to undesired activity of MNK1", have been used interchangeably herein to denote any disorder or symptom wherein the MNK1 is involved in the process or presentation of the disorder or the symptom. The MNK1 related disorders thus e.g. include, but are not limited to, inflammatory conditions and type 2 diabetes.

Unless otherwise stated or indicated, the term "C₁₋₆-alkyl" denotes a straight or branched alkyl group having from 1 to 6 carbon atoms. For parts of the range "C₁₋₆-alkyl" all subgroups thereof are contemplated such as C₁₋₅-alkyl, C₁₋₄-alkyl, C₁₋₃-alkyl, C₁₋₂-alkyl, C₂₋₆-alkyl, C₂₋₅-alkyl, C₂₋₄-alkyl, C₂₋₃-alkyl, C₃₋₆-alkyl, C₄₋₅-alkyl, etc. Examples of said lower alkyl include methyl, ethyl, n-propyl, iso-propyl, n-butyl, iso-butyl, sec-butyl, t-butyl and straight- and branched-chain pentyl and hexyl. "Halo-C₁₋₆-alkyl" means a C₁₋₆-alkyl group substituted by one or more halogen atoms. Derived expressions such as "C₁₋₆ alkoxy" and "C₁₋₆ alkylamino" are to be construed accordingly where an oxy group, thio group or an amino group, respectively, is bridging the C₁₋₆ alkyl group to the node at which that substituent is substituted. For parts of the range "C₁₋₆-alkoxy" all subgroups thereof are contemplated such as C₁₋₅-alkoxy, C₁₋₄-alkoxy, C₁₋₃-alkoxy, C₁₋₂-alkoxy, C₂₋₆-alkoxy, C₂₋₅-alkoxy, C₂₋₄-alkoxy, C₂₋₃-alkoxy, C₃₋₆-alkoxy, C₄₋₅-alkoxy, etc. Examples of said "C₁₋₆ alkoxy" include methoxy, ethoxy, n-propoxy, iso-propoxy, n-butoxy, iso-butoxy, sec-butoxy, t-butoxy and straight- and branched-chain pentoxy and hexoxy. Subgroups of "C₁₋₆ alkylamino" are to be construed accordingly.

Unless otherwise stated or indicated, the term "cycloalkyl" denotes a cyclic alkyl group having a ring size from 3 to 7 carbon atoms, optionally additionally substituted by C₁₋₃ alkyl. For parts of the range "C₃₋₇-cycloalkyl" all subgroups thereof are contemplated such as C₃₋₆-cycloalkyl, C₃₋₅-cycloalkyl, C₃₋₄-cycloalkyl, C₄₋₇-cycloalkyl, etc. Examples of said cycloalkyl include cyclopropyl, cyclobutyl, cyclopentyl, cyclohexyl, methylcyclohexyl and cycloheptyl.

The term "acyl" is used to specify an optional substituent on Ar which may be straight, branched, cyclic or aromatic, denotes a hydrocarbon having from 1 to 6 carbon atoms and a carbonyl group. For parts of the range "C₁₋₆-acyl" all subgroups thereof are contemplated such as C₁₋₅-acyl, C₁₋₄-acyl, C₁₋₃-acyl, C₁₋₂-acyl, C₂₋₆-acyl, C₂₋₅-acyl, C₂₋₄-acyl, C₂₋₃-acyl, C₃₋₆-a.cyl, C₄₋₅-acyl, etc. Exemplary acyl groups include formyl, acetyl, propanoyl, butanoyl, pentanoyl, hexanoyl, benzoyl, and preferably benzoyl.

Unless otherwise stated or indicated, the term "C₁₋₆ alkylsulphonyl", which may be straight, branched, or cyclic, denotes a hydrocarbon having from 1 to 6 carbon atoms with a sulphonyl group. For parts of the range "C₁₋₆ alkylsulphonyl" all subgroups thereof are contemplated such as C₁₋₅ alkylsulphonyl, C₁₋₄ alkylsulphonyl, C₁₋₃ alkylsulphonyl, C₁₋₂ alkylsulphonyl, C₂₋₆ alkylsulphonyl, C₂₋₅ alkylsulphonyl, C₂₋₄ alkylsulphonyl, C₂₋₃ alkylsulphonyl, C₃₋₆ alkylsulphonyl, C₄₋₅ alkylsulphonyl, etc. Exemplary alkylsulphonyl groups include methylsulphonyl, ethylsulphonyl, propylsulphonyl, n-butylsulphonyl, sec-butylsulphonyl, tert-butylsulphonyl, pentylsulphonyl and hexylsulphonyl.

Unless otherwise stated or indicated, the term "halogen" shall mean fluorine, chlorine, bromine or iodine.

Unless otherwise stated or indicated, the term "aryl" refers to a hydrocarbon ring system selected from phenyl, pentalenyl, indenyl, dihydroindenyl, naphthyl, and fluorenyl, or dehydrogenated derivatives thereof. The aryl rings may optionally be substituted by C₁₋₆-alkyl. Examples of substituted aryl groups are benzyl and 2-methylphenyl. Derived expressions such as "aryloxy" and "aryl carbonyl" should be construed accordingly where an oxy group or a carbonyl group, respectively, is bridging the aryl group to the node at which that substituent is substituted. Examples of and aryloxy group are phenoxy, and naphthoxy, and an example of an aryl carbonyl is benzoyl.

The term "heteroaryl" refers to a hydrocarbon ring system selected from the group consisting of furyl, pyrrolyl, thienyl, oxazolyl, isoxazolyl, imidazolyl, thiazolyl, isothiazolyl, pyridyl, pyrimidinyl, azaindolyl, quinazolinyl, indolyl, isoindolyl, 1,3-dihydro-isoindolyl, pyrazolyl, pyridazinyl, pyrazinyl, quinolinyl, quinoxalinyl, thiadiazolyl, indazolyl, chromanyl, purinyl, benzofuranyl, 2,3-dihydrobenzofuranyl, benzodioxolyl, benzodioxinyl, 2,3-dihydro-1,4-benzodioxinyl, benzothiophenyl, benzothiazolyl, benzothiadiazolyl, and benzotriazolyl groups.

Derived expressions such as "heteroaryloxy" and "heteroaryl carbonyl" should be construed accordingly where an oxy group or a carbonyl group, respectively, is bridging the heteroaryl group to the node at which that substituent is substituted. Exemplary heteroaryl carbonyl groups include furoyl and isonicotinoyl.

Unless otherwise specified, the term "heterocyclyl" refers to a hydrocarbon ring system, containing 4 to 8 ring members that have at least one heteroatom (e.g., S, N, or O) as part of the ring. It includes saturated, partially unsaturated and unsaturated nonaromatic heterocycles. Suitable heterocyclic groups include azetidinyl, tetrahydrofuranyl, pyrrolidinyl, pyrazolidinyl, piperidyl, azepinyl, piperazinyl, diazepanyl, perhydrodiazepinyl, morpholinyl, thiomorpholinyl, pyranyl, and dioxanyl groups. When two geminal substituents on a heterocyclyl together form a second 5- to 6-membered heterocyclyl ring giving a spiro radical, such an exemplary spiro radical is 2,7-diazaspiro[4.5]dec-2-yl.

The term "coupling agent" refers to a substance capable of catalyzing a coupling reaction, such as amidation, or esterification. Examples of coupling agents include, but are not limited to, carbonyldiimidazole, dicyclohexylcarbodimide, pyridine, 4-dimethylaminopyridine, and triphenylphosphine. Another example of a coupling agent is 1-[3-(dimethylaminopropyl)]-3-ethylcarbodiimide hydrochloride, which is used in the presence of hydroxybenzotriazole and a base such as triethylamine.

The term "reducing agent" refers to a substance capable of reducing another substance and it itself is oxidized. Examples of reducing agents include, but are not limited to, hydrogen, sodium, potassium, sodium borohydride, sodium cyanoborohydride, sodium triacetoxyborohydride, lithium aluminiumhydride, and diisobutylaluminium hydride.

"Pharmaceutically acceptable" means being useful in preparing a pharmaceutical composition that is generally safe, non-toxic and neither biologically nor otherwise undesirable and includes being useful for veterinary use as well as human pharmaceutical use.

"Treatment" as used herein includes prophylaxis of the named disorder or condition, or amelioration or elimination of the disorder once it has been established.

The term "mammal" includes organisms, which include mice, rats, cows, sheep, pigs, rabbits, goats, and horses, monkeys, dogs, cats, and preferably humans.

"An effective amount" refers to an amount of a compound that confers a therapeutic effect (e.g., treats, controls, ameliorates, prevents, delays the onset of, or reduces the risk of developing a disease, disorder, or condition or symptoms thereof) on the treated subject. The therapeutic effect may be objective (i.e., measurable by some test or marker) or subjective (i.e., subject gives an indication of or feels an effect).

The term "prodrug forms" means a pharmacologically acceptable derivative, such as an ester or an amide, which derivative is biotransformed in the body to form the active drug. Reference is made to Goodman and Gilman's, The Pharmacological basis of Therapeutics, 8th ed., Mc-Graw-Hill, Int. Ed. 1992, "Biotransformation of Drugs", p. 13-15.

When two of the above-mentioned terms are used together, it is intended that the latter group is substituted by the former. For example, halo-C₁₋₆-alkyl means a C₁₋₆-alkyl group that is substituted by one or more halogen atoms.

In the case of a halo-C₁₋₆₋alkyl group, such as a halo-C₁₋₃ alkyl group, the preferred meaning of halo is fluoro.

In a preferred group of compounds of the above formulae (I), (III), and (IV)
X is as defined above for the respective formula;
Y, Z, Ar, R¹, R², R³, R⁴, R⁵ and R⁶ are as defined for formula (I);
A is a bond or -NH-,
provided that when R¹ is NH₂, then A is a bond; and when A is -NH-then R¹ is H.

In a preferred group of compounds of the above formulae (I), (III) and (IV)
X is as defined above for the respective formula;
Y and Z are as defined for formula (I);
A is a bond or -NH-,
Ar is benzofuranyl, indolyl, hydroxyphenyl, thienyl, benzothiophenyl, pyrrolyl, aminocarbonylphenyl, or azaindolyl, each of which is unsubstituted or substituted with from 1-2 substituents independently selected from hydroxy, halogen, -CN, -NO₂, C₁₋₆ alkyl, C₁₋₆ alkoxy, acyl, C₁₋₆ alkylsulphonyl, -C(O)NH₂, and arylsulphonyl;
R¹, and R⁴ are as defined for formula (I);
R² and R³, are each, independently, located at a position ortho or meta with respect to A and are each, independently, H, halogen, hydroxy, C₁₋₃ alkyl, C₁₋₃ alkoxy, C₁₋₃ alkoxy-C₁₋₃ alkoxy, di-C₁₋₆ alkylamino-C₁₋₆ alkoxy, or di-C₁₋₆ alkylaminocarbonyl-C₁₋₆ alkoxy;
R⁵ is H, C₁₋₃ alkyl, or aryl-C₁₋₃ alkyl;
R⁶ is H, C₁₋₃ alkyl, halo-C₁₋₃ alkyl, di-C₁₋₄ alkylamino-C₁₋₆ alkyl, mono-C₁₋₄ alkylamino-C₁₋₆ alkyl, amino-C₁₋₆ alkyl, hydroxy-C₁₋₃ alkylamino-C₁₋₅ alkyl, di(hydroxy-C₁₋₃ alkyl)amino-C₁₋₅ alkyl, hydroxy-C₁₋₅ alkyl, dihydroxypropyl, cycloalkyl, heterocyclyl, heterocyclyl-C₁₋₄ alkyl, or heteroaryl-C₁₋₃ alkyl, wherein the heterocyclyl or heteroaryl is unsubstituted or substituted with from 1-2 substituents, preferably one, independently selected from C₁₋₃ alkyl, C₁₋₅ alkyl-OC(O)-, or aryl-C₁₋₃ alkyl, said C₁₋₅ alkyl-OC(O)- being attached to a ring N atom, more preferably R⁶ is di-C₁₋₃ alkylamino-C₁₋₄ alkyl, mono-C₁₋₃ alkylamino-C₁₋₄ alkyl, amino-C₁₋₄ alkyl, heterocyclyl, or heterocyclyl-C₁₋₃ alkyl, wherein the heterocyclyl is preferably 4, 5 or 6 membered containing 1 or 2 heteroatoms selected from O and N, wherein the heterocyclyl is unsubstituted or substituted with from 1-2 substituents,, preferably one, independently selected from C₁₋₃ alkyl, C₁₋₅ alkyl-OC(O)-, or aryl-C₁₋₃ alkyl, preferably C₁₋₃ alkyl, said C₁₋₅ alkyl-OC(O)- being attached to a ring N atom; or
R⁵ and R⁶ together with the nitrogen to which they are attached form a 4 to 7 membered heterocyclyl, wherein the 4 to 7 membered heterocyclyl,optionally includes a second heteroatom selected from N and O (in these embodiments, two of the ring members of the heterocyclyl are heteroatoms: one of the two heteroatoms is the nitrogen atom connecting R5 and R6, and the other is either N or O), and wherein the 4 to 7 membered heterocyclyl is unsubstituted or substituted with from 1-2 substituents, preferably one, independently selected from C₁₋₃ alkyl, hydroxy-C₁₋₃ alkyl, C₁₋₂-alkoxy-C₂₋₄-alkyl, di-C₁₋₃ alkylamino, mono-C₁₋₃ alkylamino, amino, di-C₁₋₃ alkylamino-C₁₋₄ alkyl, mono-C₁₋₃ alkylamino-C₁₋₄ alkyl, amino-C₁₋₄ alkyl, C₅₋₆ cycloalkyl, C₁₋₆ alkyl-OC(O)NH-, heterocyclylmethyl, or heteroaryl-carbonyl; or wherein two geminal substituents on said 4 to 7-membered heterocyclyl, preferably 5-membered heterocyclyl, together form a second 5- to 6-membered heterocyclyl giving a spiro radical with the provisos that:
when R¹ is NH₂, then A is a bond; and
when A is -NH- then R¹ is H.

In a preferred group of compounds, one of Y and Z is N.

In another preferred group of compounds, both Y and Z are CH.

In the compounds of the present invention, it is preferred that when both R² and R³ are other than H, then both R² and R³ are not in a position ortho of A.

In some embodiments, when both R² and R³ are other than H, then both R² and R³ are not located in a position that is ortho with respect to A. For example, R² and R³ can each be, independently, halogen, hydroxy, C₁₋₆ alkyl, or C₁₋₆ alkoxy, C₁₋₃ alkoxy-C₁₋₃ alkoxy, di-C₁₋₆ alkylamino-C₁₋₆ alkoxy, or di-C₁₋₆ alkylaminocarbonyl-C₁₋₆ alkoxy; and both of R² and R³ can each be located in a position that is meta with respect to A. When both of R² and R³ are located in a position that is meta with respect to A, then R⁴ is located at a position that is para with respect to A.

In a preferred group of compounds, R² is located at a position that is ortho with respect to A, R³ is located at a position that is meta with respect to A, and R⁴ is located at a position that is para or meta with respect to A; when Ar is a heteraryl, R⁴ is most preferably located para with respect to A; i.e. the substituents R², R³ and R⁴ of the above formulae I, III and IV are located in a position ortho, meta and para, respectively, of A, as shown below 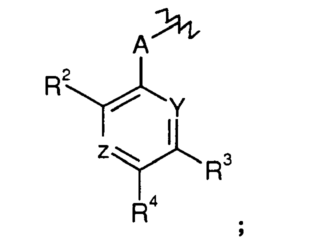 wherein Y and Z can be defined as CH or N.

In another preferred group of compounds the substituents R², R³ and R⁴ of the above formulae I, II, V, and VI are located in a position ortho, meta and para, respectively, of A, as shown below

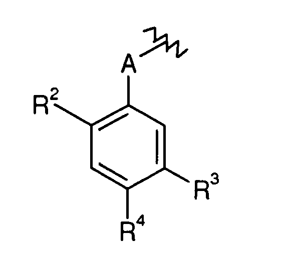

Particularly preferred examples of the substituents R² and R³ in the compounds of the present invention include H, halogen, hydroxy, C₁₋₃ alkyl, C₁₋₃ alkoxy, C₁₋₆ alkoxy-C₁₋₆ alkoxy, di-C₁₋₆ alkylamino-C₁₋₆ alkoxy, or di-C₁₋₆ alkylaminocarbonyl-C₁₋₆ alkoxy, and more preferably H, fluoro, chloro, hydroxy, methyl, methoxy, 2-ethoxyethoxy, 2-(dimethylamino)ethoxy, and dimethylaminocarbonylmethoxy.

In a preferred group of compounds R⁴ is -C(O)NR⁵R⁶.

Preferred examples of R⁵ are H, methyl, ethyl and benzyl.

In a preferred group of compounds R⁶ is selected from di-C₁₋₃ alkylamino-C₁₋₃ alkyl, mono-C₁₋₃ alkylamino-C₁₋₃ alkyl, amino-C₁₋₃ alkyl, heterocyclyl and heterocyclyl-C₁₋₃ alkyl, wherein the heterocyclyl is 4-, 5- or 6-membered containing 1 or 2 heteroatoms selected from O and N, preferably 5 membered and preferably containing one heteroatom atom being an N atom; wherein the heterocyclyl is unsubstituted or substituted with from 1-2 substituents, preferably one, independently selected from C₁₋₃ alkyl, C₁₋₅ alkyl-OC(O)-, or aryl-C₁₋₃ alkyl, and preferably C₁₋₃ alkyl, said C₁₋₅ alkyl-OC(O)- being attached to a ring N atom of the heterocyclyl; or R⁵ and R⁶ together with the nitrogen to which they are attached form a 4-, 5-, 6-, or 7-membered heterocyclyl, which heterocyclyl in the case of a 6-membered heterocyclyl preferably includes a second heteroatom selected from N and O, preferably N, which 4-, 5-, 6-, or 7-membered heterocyclyl is unsubstituted or substituted with from 1-2 substituents, preferably one, independently selected from C₁₋₃ alkyl, hydroxy-C₁₋₃ alkyl, C₁₋₂-alkoxy-C₂₋₄-alkyl, di-C₁₋₃ alkylamino, mono-C₁₋₃ alkylamino, amino, di-C₁₋₃ alkylamino-C₁₋₃ alkyl, mono-C₁₋₃ alkylamino-C₁₋₃ alkyl, amino-C₁₋₃ alkyl, C₅₋₆ cycloalkyl, C₁₋₆ alkyl-OC(O)NH-, heterocyclylmethyl, or heteroaryl-carbonyl; or wherein two geminal substituents on said 4- to 7-membered heterocyclyl together form a second 5 to 6 membered heterocyclyl giving a spiro radical.

Examples of R⁶ include hydrogen, methyl, isopropyl, cyclopentyl, trifluororethyl, hydroxyethyl, hydroxypentyl, dihydroxypropyl, dimethylaminoethyl, diethylaminoethyl, dimethylaminopropyl, 3-dimethylamino-2,2-dimethylpropyl, hydroxypropylaminoethyl, [bis(2-hydroxyethyl)amino]propyl, morpholinylethyl, morpholinylpropyl, azetidinyl, pyrrolidinyl, pyrrolidinylethyl, furylmethyl, pyridinylmethyl, piperidinyl, piperidinylmethyl, tetrahydrofuranylmethyl, imidazolylethyl, pyrazolidinyl, wherein any heterocyclyl or heteroaryl is substituted with from 1-2 substituents, preferably one, independently selected from C₁₋₆ alkyl, C₁₋₆ alkyl-OC(O)- or aryl-C₁₋₆ alkyl, said C₁₋₆ alkyl-OC(O)- being attached to a ring N atom, more preferably methyl, C₄ alkyl-OC(O)- or benzyl, especially methyl, said C₄ alkyl-OC(O)- being attached to a ring N atom.

Specific examples of R⁶ include hydrogen, methyl, isopropyl, cyclopentyl, 2,2,2-trifluororethyl, 2-hydroxyethyl, 1-hydroxymetyl-2-metylpropyl, 2,3-dihydroxypropyl, 2-dimethylaminoethyl, 2-diethylaminoethyl, 3-dimethylaminopropyl, 3-dimethylamino-2,2-dimethylpropyl, 2-(2-hydroxypropylamino)ethyl, 3-[bis(2-hydroxyethyl)amino]propyl, 2-(4-morpholinyl)ethyl, 3-(4-morpholinyl)propyl, *N*-metyl-pyrrolidin-3-yl, 2-(pyrrolidin-1-yl)ethyl, piperidin-3-yl,N-(*t*-butyloxycarbonyl-piperidin)-3-ylmethyl, 2-furylmethyl, pyridine-2-ylmethyl, tetrahydrofuran-2-yl-methyl, 1-benzylpiperidin-4-yl, 2-(1H-imidazol-4-yl)ethyl, 1,2-diethylpyrazolidin-4-yl azetidin-3-yl and N-(t-butyloxycarbonyl)-azetidin-3-yl. Preferably, R⁶ is *N-*metyl-pyrrolidin-3-yl, 2-(pyrrolidin-1-yl)ethyl, N-(*t*-butyloxycarbonyl-piperidin)-3-ylmethyl, pyridin-2-ylmethyl, or 1-benzylpiperidin-4-yl.

Examples of the 4 to 7-membered heterocyclyl formed by R⁵ and R⁶ together with the nitrogen to which they are attached, include azetidinyl, pyrrolidinyl, piperidinyl, morpholinyl, tiomorpholinyl, piperazinyl, and perhydrodiazepinyl which may be unsubstituted or substituted in one or two positions, preferably one, independently with C₁₋₆ alkyl, hydroxy-C₁₋₆ alkyl, di-C₁₋₆ alkylamino, mono-C₁₋₆ alkylamino, amino, di-C₁₋₆ alkylamino-C₁₋₆ alkyl, mono-C₁₋₆ alkylamino-C₁₋₆ alkyl, amino-C₁₋₆ alkyl, C₃₋₇ cycloalkyl, heteroaryl-carbonyl and C₁₋₆ alkyl-OC(O)NH-, preferably with C₁₋₃ alkyl, hydroxy-C₁₋₃ alkyl, C₁₋₂-alkoxy-C₂₋₄-alkyl, di-C₁₋₃ alkylamino, mono-C₁₋₃ alkylamino, amino, di-C₁₋₃ alkylamino-C₁₋₄ alkyl, mono-C₁₋₃ alkylamino-C₁₋₄ alkyl, amino-C₁₋₄ alkyl, C₅₋₇ cycloalkyl, C₁₋₆ alkyl-OC(O)NH-, such as C₄-alkyl-OC(O)NH-, heterocyclylmethyl, and heteroaryl-carbonyl, such as furanyl-carbonyl; or wherein two geminal substituents on said 4 to 7-membered heterocyclyl, preferably 5-membered heterocyclyl, together form a second 5- to 6-membered heterocyclyl giving a spiro radical.

Specific examples of the 4 to 7-membered heterocyclyl formed by R⁵ and R⁶ together with the nitrogen to which they are attached, include 3-aminoazetidin-1-yl, 3-aminopyrrolidin-1-yl, 3,5-dimethylpiperazin-1-yl, 3-methylpiperazin-1-yl, 4-isopropylpiperazin-1-yl, 4-ethylpiperazin-1-yl, 4-(2-methoxyethoxy)piperazin-1-yl, 4-(2-hydroxyethyl)piperazin-1-yl, 2-(pyrrolidin-1-ylmethyl)pyrrolidin-1-yl, 3-(t-butyloxycarbonylamino)-azetidin-1-yl, morpholin-4-yl, piperidin-1-yl, 2-[(dimethylamino)methyl]piperidin-1-yl, 4-(2-furoyl)piperazin-1-yl, 4-[3-(dimethylamino)propyl]piperazin-1-yl, 3-(dimethylamino)pyrrolidin-1-yl, 4-(hydroxymethyl)piperidin-1-yl, thiomor-pholin-4-yl, 4-cyclohexylpiperazin-1-yl, 4-methyl-1,4-diazepan-1-yl, 4-methylpiperazin-1-yl, 4-[2-(dimethylamino)ethyl]piperazin-1-yl, 3-(*t-*butyloxycarbonylamino)-pyrrolidin-1-yl, 2-methoxyethylpiperazin-1-yl, pyrrolidin-1-ylmethyl-pyrrolidin-1-yl and 2,7-diazaspiro[4.5]dec-2-yl.

In a preferred group of compounds of the above formulae (I), (III), and (IV)
X is as defined above for the respective formula;
Y, Z, A, R¹, R², R³, R⁴, R⁵ and R⁶ are as defined for formula (I);
Ar is benzofuranyl, indolyl, hydroxyphenyl, thienyl, benzothiophenyl, pyrrolyl, aminocarbonylphenyl, or azaindolyl, wherein benzofuranyl, indolyl, thienyl, benzothiophenyl, pyrrolyl, and azaindolyl is unsubstituted or substituted with from 1-2 substituents independently selected from hydroxy, halogen, -CN, -NO₂, C₁₋₆ alkyl, C₁₋₆ alkoxy; acyl, C₁₋₆ alkylsulphonyl. -C(O)NH₂, and arylsulphonyl.

Especially preferred compounds of formulae I, III, and IV above are those in which Ar is hydroxyphenyl, methoxyhydroxyphenyl, fluorohydroxyphenyl, (benzoyl)hydroxyphenyl, benzofuranyl, aminocarbonylphenyl or indolyl, more preferably benzofuranyl or indolyl, and most preferably indolyl. The benzofuranyl or indolyl is preferably 2-benzofuranyl and 2- or 5-indolyl, more preferably 2-benzofuranyl and 2-indolyl.

Especially preferred compounds of formulae II, V, and VI above are those in which Ar is hydroxyphenyl, benzofuranyl, aminocarbonylphenyl or indolyl, more preferably benzofuranyl or indolyl, and most preferably indolyl. The benzofuranyl or indolyl is preferably 2-benzofuranyl and 2- or 5-indolyl, more preferably 2-benzofuranyl and 2-indolyl.

Also especially preferred compounds of formulae I, III, IV, V, and VI above are those in which Ar is azaindolyl, more preferably 5-, or 6-azaindol-2-yl,

In a preferred group of compounds when Ar is benzofuranyl, indolyl, thienyl, benzothiophenyl, pyrrolyl, aminocarbonylphenyl or azaindolyl, Ar is unsubstituted or substituted with hydroxy, halogen, -CN, -NO₂ C₁₋₃ alkyl, C₁₋₂ alkoxy, benzoyl, C₁₋₃ alkylsulphonyl, -C(O)NH₂, and phenylsulphonyl; preferably with hydroxy, halogen, cyano, C₁₋₃ alkyl, C₁₋₂ alkoxy, methylsulphonyl, isopropylsulphonyl and -C(O)NH₂; and more preferably with hydroxy, fluoro, chloro, cyano, methyl, methoxy and -C(O)NH₂.

In a preferred group of compounds of the above formulae (I), (III), and (IV)
X is as defined above for the respective formula;
one of Y and Z is N, i.e. giving a pyridine ring;
A is -NH-; and
Ar, R¹, R², R³, R⁴, R⁵ and R⁶ are as defined for formula (I);

Throughout the specification and the appended claims, a given chemical formula or name shall encompass all stereo and optical isomers and racemates thereof where such isomers exist. All diastereomeric forms possible (pure enantiomers, tautomers, racemic mixtures and unequal mixtures of two enantiomers) are within the scope of the invention.

The compounds of the formula (I) may be used as such or, where appropriate, as pharmacologically acceptable salts (acid or base addition salts) thereof. The pharmacologically acceptable addition salts mentioned above are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

The compounds described herein can be used in the treatment or prophylaxis of any disorder or condition associated with the activity of MNK1, MNK2a and/or MNK2b, such as metabolic diseases, e.g. obesity, as well as related disorders such as eating disorder, cachexia, diabetes mellitus, hypertension, coronary heart disease, hypercholesterolemia, dyslipidemia, hyperlipidemia, hyperglycemia, osteoarthritis, gallstones, and sleep apnea, and disorders related to ROS defence, such as diabetes mellitus, neurodegenerative disorders, and cancer, e.g. cancers of the reproductive organs, leukaemia, e.g. acute myeloid leukaemia (AML), and inflammatory conditions. It may also be used in the treatment or prophylaxis of disorders relating to the insulin-signaling pathway. Examples of such disorders are type 2 diabetes. Preferably, the compound is used in the treatment or prophylaxis of type 2 diabetes, cancer, inflammatory conditions, and obesity, and more preferably type 2 diabetes, inflammatory conditions, and obesity.

Another aspect of the present invention is a method for the treatment or prophylaxis of any of the above conditions or disorders, and especially type 2 diabetes, said method comprising administering to a subject (e.g., mammal, human, or animal) in need of such treatment an effective amount of a compound as described above.

A further aspect of the invention relates to a method for the treatment or prophylaxis disorders related to the insulin-signaling pathway, said method comprising administering to a subject (e.g., mammal, human, or animal) in need of such treatment an effective amount of a compound as described above.

Another aspect of the invention relates to a method for the treatment or prophylaxis of anti-inflammatory conditions, said method comprising administering to a subject (e.g., mammal, human, or animal) in need of such treatment an effective amount of a compound as described above Inflammatory conditions can include arthritis, rheumatoid arthritis,spondyloarthopathies, gouty arthritis, osteoarthritis, systemic lupus erythematosus, juvenile arthritis, asthma, bronchitis, menstrual cramps, tendinitis, bursitis, connective tissue injuries or disorders, skin related conditions, psoriasis, eczema, burns, dermatitis,gastrointestinal conditions, inflammatory bowel disease, gastric ulcer, gastric varices, Crohn's disease, gastritis, irritable bowel syndrome, ulcerative colitis, cancer,colorectal cancer, herpes simplex infections, HIV, pulmonary edema, kidney stones, minor injuries, wound healing, vaginitis, candidiasis, lumbar spondylanhrosis, lumbar spondylarthrosis, vascular diseases, migraine headaches, sinus headaches, tension headaches, dental pain, periarteritis nodosa, thyroiditis, plastic anemia, Hodgkin's disease, sclerodoma, rheumatic fever, type I diabetes, myasthenia gravis, multiple sclerosis, sarcoidosis, nephrotic syndrome, Behcet's syndrome, polymyositis, gingivitis, hypersensitivity, swelling occurring after injury, myocardial ischemia, ophthalmic diseases, retinitis, retinopathies, conjunctivitis, uveitis, ocular photophobia, acute injury to the eye tissue, pulmonary inflammation, viral infections, cystic fibrosis, central nervous system disorders, cortical dementias, and Alzheimer's disease.

A further aspect of the present invention relates to a method for the treatment or prophylaxis of cancer, said method comprising administering to a subject (e.g., mammal, human, or animal) in need of such treatment an effective amount of a compound as described above. "Cancer" refers to cellular-proliferative disease states, including but not limited to: Cardiac: sarcoma (angiosarcoma, fibrosarcoma, rhabdomyosarcoma, liposarcoma), myxoma, rhabdomyoma, fibroma, lipoma and teratoma; Lung: bronchogenic carcinoma (squamous cell, undifferentiated small cell, undifferentiated large cell, adenocarcinoma), alveolar (bronchiolar) carcinoma, bronchial adenoma, sarcoma, lymphoma, chondromatous hanlartoma, mesothelioma; Gastrointestinal: esophagus (squamous cell carcinoma, adenocarcinoma, leiomyosarcoma, lymphoma), stomach (carcinoma, lymphoma, leiomyosarcoma), pancreas (ductal adenocarcinoma, insulinoma, glucagonoma, gastrinoma, carcinoid tumors, vipoma), small bowel (adenocarcinoma, lymphoma, carcinoid tumors, Karposi's sarcoma, leiomyoma, hemangioma, lipoma, neurofibroma, fibroma), large bowel (adenocarcinoma, tubular adenoma, villous adenoma, hamartoma, leiomyoma); Genitourinary tract: kidney (adenocarcinoma, Wilm's tumor [nephroblastoma], lymphoma, leukemia), bladder and urethra (squamous cell carcinoma, transitional cell carcinoma, adenocarcinoma), prostate (adenocarcinoma, sarcoma), testis (seminoma, teratoma, embryonal carcinoma, teratocarcinoma, choriocarcinoma, sarcoma, interstitial cell carcinoma, fibroma, fibroadenoma, adenomatoid tumors, lipoma); Liver: hepatoma (hepatocellular carcinoma), cholangiocarcinoma, hepatoblastoma, angiosarcoma, hepatocellular adenoma, hemangioma; Bone: osteogenic sarcoma (osteosarcoma), fibrosarcoma, malignant fibrous histiocytoma, chondrosarcoma, Ewing's sarcoma, malignant lymphoma (reticulum cell sarcoma), multiple myeloma, malignant giant cell tumor chordoma, osteochronfroma (osteocartilaginous exostoses), benign chondroma, chondroblastoma, chondromyxofibroma, osteoid osteoma and giant cell tumors; Nervous system: skull (osteoma, hemangioma, granuloma, xanthoma, osteitis deformans), meninges (meningioma, meningiosarcoma, gliomatosis), brain (astrocytoma, medulloblastoma, glioma, ependymoma, germinoma [pinealoma], glioblastoma multiform, oligodendroglioma, schwannoma, retinoblastoma, congenital tumors), spinal cord neurofibroma, meningioma, glioma, sarcoma); Gynecological: uterus (endometrial carcinoma), cervix (cervical carcinoma, pre-tumor cervical dysplasia), ovaries (ovarian carcinoma [serous cystadenocarcinoma, mucinous cystadenocarcinoma, unclassified carcinoma], granulosa-thecal cell tumors, SertoliLeydig cell tumors, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, botryoid sarcoma (embryonal rhabdomyosarcoma], fallopian tubes (carcinoma); Hematologic: blood (myeloid leukemia [acute and chronic], acute lymphoblastic leukemia, chronic lymphocytic leukemia, myeloproliferative diseases, multiple myeloma, myelodysplastic syndrome), Hodgkin's disease, non-Hodgkin's lymphoma [malignant lymphoma]; Skin: malignant melanoma, basal cell carcinoma, squamous cell carcinoma, Karposi's sarcoma, moles dysplastic nevi, lipoma, angioma, dermatofibroma, keloids, psoriasis; and Adrenal lands: neuroblastoma. Thus, the term "cancerous cell" as provided herein, includes a cell afflicted by any one of the above-identified conditions.

The invention also features a method for reducing body-weight (e.g., treating body-weight disorders). The method includes administering to a subject in need thereof an effective amount of a compound of the formula (I). As used herein, the term "body weight disorders" refers to the disorders caused by an imbalance between energy intake and energy expenditure, resulting in abnormal body (e.g., excessive) weights. Such body weight disorders include obesity.

The methods delineated herein can also include the step of identifying that the subject is in need of treatment of a MNK1 or MNK2-related disorder, such as type 2 diabetes.

A further aspect of the present invention is a method for modulating MNK1 or MNK2 activity (e.g., antagonizing the MNK2), comprising administering to a subject (e.g., mammal, human, or animal) in need thereof an effective amount of a compound as described above or a composition comprising a compound as described above.

Another aspect of the present invention is the use of a compound as described above in the manufacture of a medicament for use in the treatment or prophylaxis of any disorder or condition associated with the activity of MNK1, MNK2a and/or MNK2b, such as the conditions specified above, including metabolic diseases, e.g. obesity, as well as related disorders such as eating disorder, cachexia, diabetes mellitus, hypertension, coronary heart disease, hypercholesterolemia, dyslipidemia, hyperlipidemia, hyperglycemia, osteoarthritis, gallstones, and sleep apnea, and disorders related to ROS defence, such as diabetes mellitus, neurodegenerative disorders, and cancer, e.g. cancers of the reproductive organs, leukaemia, e.g. acute myeloid leukaemia (AML), and inflammatory conditions, and especially type 2 diabetes, cancer, inflammatory conditions, and obesity.

Accordingly, another aspect of the present invention is a pharmaceutical formulation containing a compound as described above as an active ingredient, in combination with a pharmaceutically acceptable diluent or carrier. The pharmaceutical formulation may be used in the treatment or prophylaxis of any of the above conditions, wherein the active ingredient is a compound as described above. Usually the amount of active compounds is between 0.1-95% by weight of the preparation, preferably between 0.2-20% by weight in preparations for parenteral use and preferably between 1 and 50% by weight in preparations for oral administration.

For clinical use, the compounds of the invention are formulated into pharmaceutical formulations for oral, rectal, parenteral or other mode of administration. Pharmaceutical formulations are usually prepared by mixing the active substance, or a pharmaceutically acceptable salt thereof, with conventional pharmaceutical excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like.

The formulations can be further prepared by known methods such as granulation, compression, microencapsulation, spray coating, etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner.

In a further aspect the invention relates to methods of making compounds of any of the formulae herein comprising reacting any one or more of the compounds of the formulae delineated herein, including any processes delineated herein. The compounds of the formula (I) above may be prepared by, or in analogy with, conventional methods.

The processes described above may be carried out to give a compound of the invention in the form of a free base or as an acid addition salt. A pharmaceutically acceptable acid addition salt may be obtained by dissolving the free base in a suitable organic solvent and treating the solution with an acid, in accordance with conventional procedures for preparing acid addition salts from base compounds. Examples of addition salt forming acids are mentioned above.

The compounds of formula (I) may possess one or more chiral carbon atoms, and they may therefore be obtained in the form of optical isomers, e.g. as a pure enantiomer, or as a mixture of enantiomers (racemate) or as a mixture containing diastereomers, as mentioned above. The separation of mixtures of optical isomers to obtain pure enantiomers is well known in the art and may, for example, be achieved by fractional crystallization of salts with optically active (chiral) acids or by chromatographic separation on chiral columns.

The chemicals used in the synthetic routes delineated herein may include, for example, solvents, reagents, catalysts, and protecting group and deprotecting group reagents. The methods described above may also additionally include steps, either before or after the steps described specifically herein, to add or remove suitable protecting groups in order to ultimately allow synthesis of the compounds. In addition, various synthetic steps may be performed in an alternate sequence or order to give the desired compounds. Synthetic chemistry transformations and protecting group methodologies (protection and deprotection) useful in synthesizing applicable compounds are known in the art and include, for example, those described in R. Larock, Comprehensive Organic Transformations, VCH Publishers (1989); T.W. Greene and P.G.M. Wuts, Protective Groups in Organic Synthesis, 3rd Ed., John Wiley and Sons (1999); L. Fieser and M. Fieser, Fieser and Fieser's Reagents for Organic Synthesis, John Wiley and Sons (1994); and L. Paquette, ed., Encyclopedia of Reagents for Organic Synthesis, John Wiley and Sons (1995) and subsequent editions thereof.

The necessary starting materials for preparing the compounds of formula (I) are either known or may be prepared in analogy with the preparation of known compounds. The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

The invention will now be further illustrated by the following non-limiting Examples of the inventive compounds and the methods for their preparation. The compounds of the Examples exhibit an MNK2 inhibiting activity corresponding to an IC₅₀ of from 0.6 µM to about 1 nM, as tested for MNK2a activity according to the *in vitro* MNK2a HTRF assay, which will be described in detail further below.

### METHODS FOR PREPARATION

The following abbreviations have been used:
DCM means dichloromethane;
DMF means dimethylformamide;
HPLC means high performance liquid chromatography;
R.T. (rt.) means room temperature;
TFA means trifluoroacetic acid;
THF means tetrahydrofuran;
NBS means N-bromosuccinimide;
HOBT means 1-hydroxybenzotriazole hydrate;
EDC means 1-(3-dimethylaminopyopyl)-3-ethylcarbodiimide hydrochloride;
TEA means triethylamine;
TBTU means O-benzotriazol-1-yl-N,N,N',N'-tetra-methyluronium tetrafluoroborate;
ACN means acetonitrile.

The specific examples below are to be construed as merely illustrative, and not limitative of the remainder of the disclosure in any way whatsoever. Without further elaboration, it is believed that one skilled in the art can, based on the description herein, utilize the present invention to its fullest extent. All publications cited herein are hereby incorporated by reference in their entirety.

### General Comments

¹H nuclear magnetic resonance (NMR) and ¹³C NMR were recorded on a Bruker PMR 500 spectrometer at 500.1 MHz and 125.1 MHz, respectively, on a Bruker Advance DPX 400 spectrometer at 400.1 and 100.6 MHz, respectively, or on a JEOL eclipse 270 spectrometer at 270.0 MHz and 67.5 MHz, respectively. All spectra were recorded using residual solvent or tetramethylsilane (TMS) as internal standard. Electrospray mass spectrometry (MS) was obtained using an Agilent MSD mass spectrometer. Accurate mass measurements were performed on a Micromass LCT dual probe. Elemental analyses were performed on a Vario El instrument or sent to Mikro Kemi in Uppsala.

Microwave reactions were performed with a Personal Chemistry Smith Creator or Synthesizer using 0.5-2 mL or 2-5 mL Smith Process Vials fitted with aluminum caps and septa.

Analytical HPLC was performed on an Agilent 1100 system equipped with System A: ACE 3 (C8, 50 x 3.0 mm) or System B: YMC ODS-AQ, (33 x 3.0 mm) using the eluent system: water/0.1%TFA and CH₃CN, 1 mL/min, with a gradient time of 3 min.

Preparativè HPLC was performed on a Gilson system equipped with System A: ACE 5 C8 column (50 x 20 mm) gradient time 5 min, system B: YMC ODS-AQ (150 x 30 mm) gradient time 8.5 min, system C: YMC ODS-AQ (50x20 mm) gradient time 5 min, or system D: ACE 5 C8 column (150 x 30 mm) gradient time 8.5 min using the eluent system: water/0.1%TFA and CH₃CN unless otherwise indicated. System E: Xterra MS C18, 5 µm column (19 x 50 mm) gradient time 5 min using the eluent: water/ 10 mM NH₄HCO₃/NH₃ buffer pH 10/MeCN. The compounds were automatically named using ACD6.0 or 10.0

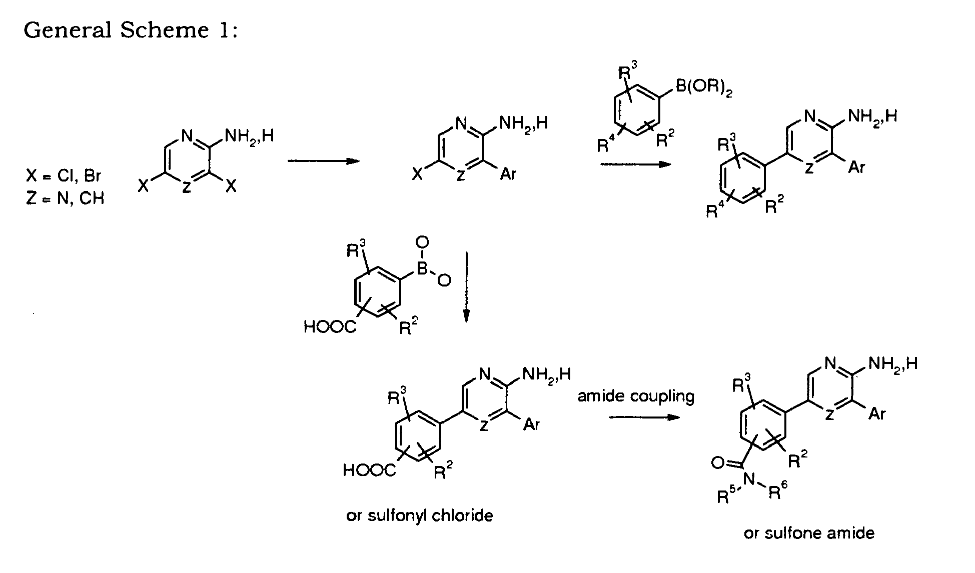

### Intermediate 1

### 3,5-dibromopyrazin-2-amine

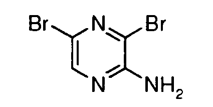

NBS (100 g, 561.8 mmol) was added in small portions to a stirred solution of 2-aminopyrazine (25 g, 263 mmol) in dichloromethane (600 ml) over a period of 1 hour. The reaction was stirred at r.t. for 1h and washed with water. The organic phase was dried (MgSO₄) and evaporated. The crude product was filtered through a plug of silica using 2.5% MeOH in dichloromethane as the eluent.
Yield 25 g (38%). HPLC 99% (System A). MS (electronspray) M+H+ m/z 254.4. 1H NMR (400 MHz, CHLOROFORM-D) δ ppm 5.04 (s, 2 H) 8.03 (s, 1 H).

### Intermediate 2

### 4-(6-Chloropyrazin-2-yl)phenol

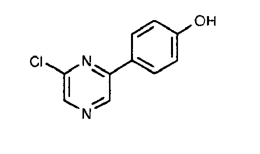

To a tube were added 2,6-dichloropyrazine (149 mg, 1.0 mmol), 4-hydroxyphenylboronic acid (152 mg, 1.1 mmol), palladium tetrakis (58 mg, 0.05 mmol), potassium carbonate (345 mg, 2.5 mmol), 1,4-dioxan (4 ml) and water (1 ml). After heating in a Stemblock at 100°C for 1.5 h, the mixture was evaporated and the residue was partitioned between water (10 ml) and ethyl acetate (2x15 ml). The organic layers were combined and evaporated. Purification by flash chromatography (gradient DCM - DCM/EtOAc (9:1)) yielded a white solid.
54%, HPLC purity = 100%, m/z = 207 (M+H)⁺, 1H NMR (270 MHz, DMSO-D6) d ppm 6.86 - 6.97 (m, 2 H) 7.94 - 8.03 (m, 2 H) 8.60 (s, 1 H) 9.15 (s, 1 H) 10.14 (s, 1 H).

### Intermediate 3

### 4-(2-Amino-5-bromopyrazin-3-yl)phenol

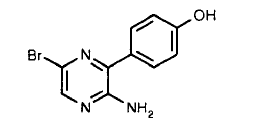

3,5 dibromopyrazine-2-amine (486 mg, 0.054 mmol), 4-hydroxyphenylboronic acid (13 mg, 0.060 mmol), tetrakis(triphenylphosphine)palladium (Pd(tetrakis)) (111 mg, 0.10 mmol) and Na₂CO₃ (509 mg, 4.80 mmol) in 20 mL of dioxane/water 4:1 was heated to 100 deg for 1 h. The rection was diluted with dioxane and filtered through celite. Purification by flash chromatography (DCM/MeOH/Heptane 4:1:5) gave 370 mg (72 %). HPLC purity = 92%, m/z = 267 (M+H)⁺.

***Method A:*** The amide (1 eq), PdCl₂ (dppf) (0.05 eq), KOAc (4 eq) and Bispinacolatoborane (1.5 eq) were dissolved in 2 mL dry DMF and the mixture was heated at 80°C for 1 hrs. Then the reaction was cooled to room temperature and water (0.5 mL), NaHCO₃ (6 eq), PdCl₂ (dppf) (0.05 eq) and 4-(2-amino-5-bromopyrazin-3-yl)phenol (Intermediate 3) (1.2 eq) were added and the resulting mixture was heated at 100°C overnight. The crude reaction was filtered, concentrated and purified using preparative HPLC system D.

***Method B:*** The amide (1 eq), PdCl₂ (dppf) (0.05 eq), KOAc (4 eq) and Bispinacolatoborane (1.5 eq) were dissolved in 2 mL dry DMF and the mixture was heated at 80°C for 1 hrs. Then the reaction was cooled to room temperature and water (0.5 mL), NaHCO₃ (6 eq), PdCl₂ (dppf) (0.05 eq) and 4-(6-chloropyrazin-2-yl)phenol (Intermediate 2) (1.2 eq) were added and the resulting mixture was heated at 100°C overnight. The crude reaction was filtered, concentrated and purified using preparative HPLC system D.

***Method C:*** The sulphonamide (1 eq), PdCl₂ (dppf) (0.05 eq), KOAc (4 eq) and Bispinacolatoborane (1.5 eq) were dissolved in 2 mL dry DMF and the mixture was heated at 80°C for 1 hrs. Then the reaction was cooled to room temperature and water (0.5 mL), NaHCO₃ (6 eq), PdCl₂ (dppf) (0.05 eq), and 4-(2-amino-5-bromopyrazin-3-yl)phenol (Intermediate 3) (1.2 eq) were added and the resulting mixture was heated at 100°C overnight. The crude reaction was then filtered, concentrated and purified using preparative HPLC system D.

***Method D:*** The sulphonamide (1 eq), PdCl₂ (dppf) (0.05 eq), KOAc (4 eq) and Bispinacolatoborane (1.5 eq) were dissolved in 2 mL dry DMF and the mixture was heated at 80°C for 1 hrs. Then the reaction was cooled to room temperature and water (0.5 mL), NaHCO₃ (6 eq), PdCl₂ (dppf) (0.05 eq) and 4-(6-chloropyrazin-2-yl)phenol (Intermediate 2) (1.2 eq) were added and the resulting mixture was heated at 100°C overnight. The crude reaction was filtered, concentrated and purified using preparative HPLC system D.

The amides/sulphonamides in ***Methods A-D*** were made by conventional amide coupling between phenylcarboxylic acids/phenylsulphonyl chlorides and aliphatic amines.

***Method E:*** The boronic acid (1.3 eq), Pd(tetrakis) (0.05 eq), NaHCO₃ (3 eq) were mixed with 3-bromo-5-[4-(morpholin-4-ylcarbonyl)phenyl]pyrazin-2-amine (Intermediate 8) (1 eq) in 1 mL DME and 0.5 mL H₂O and then heated at 120°C for 900 s in microwave. The crude reaction was filtrated and concentrated and then purified using preparative HPLC system A,C or D.

***Method F:*** The boronic acid (1.3 eq), Pd(tetrakis) (0.05 eq), NaHCO₃ (3 eq) were mixed with 3-chloro-5-[4-(morpholin-4-ylcarbonyl)phenyl]pyrazine (1 eq) in 1 mL DME and 0.5 mL H₂O and then heated at 120°C for 900 s in microwave. The crude reaction was filtrated and concentrated and then purified using preparative HPLC system A,C or D.

The central pyrazine scaffold in ***Methods E-F*** were made by a Suzuki coupling between 3-amino-2, 6-dibromopyrazine/2, 6-dicholorpyrazine and [4-(Morpholine-4-carbonyl)phenyl] boronic acid.

***Method G*:** The boronic acid (1.3 eq), Pd(tetrakis) (0.05 eq), NaHCO₃ (3 eq) were mixed with 5-bromo-3-[1H-indol-2-yl]pyrazin-2-amine (1 eq) in 1 mL DME and 0.5 mL H₂O and then heated at 120°C for 900 s in microwave. The crude reaction was filtrated and concentrated and then purified using preparative HPLC system A, C or D.

***Method H:*** The boronic acid (1.3 eq), Pd(tetrakis) (0.05 eq), NaHCO₃ (3 eq) were mixed with 2-(6-chloropyrazin-2-yl)-1H-indole (1 eq) in 1 mL DME and 0.5 mL H₂O and then heated at 120°C for 900 s in microwave. The crude reaction was filtrated and concentrated and then purified using preparative HPLC system A, C or D.

***Method J:*** The boronic acid (1.3 eq), Pd(tetrakis) (0.05 eq), NaHCO₃ (3 eq) were mixed with 3-(1-benzofuran-2-yl)-5-bromopyrazin-2-amine (prepared in Example 54) (1 eq) in 1 mL DME and 0.5 mL H₂O and then heated at 120°C for 900 s in microwave. The crude reaction was filtrated and concentrated and then purified using preparative HPLC system A, C or D.

***Method K:*** The amide (1 eq), PdCl₂ (dppf) (0.05 eq), KOAc (4.5 eq) and bis(neopentyl glycolato)diboron (1.5 eq) were dissolved in 2 mL dry DME and the mixture was heated at 125°C for 1200 s in microwave. Then the reaction was cooled to room temperature and water (0.5 mL), NaHCO₃ (2 eq), Pd(tetrakis) (0.05 eq) and 2-(6-chloropyrazin-2-yl)-1 H-indole (0.95 eq) were added and the resulting mixture was heated at 120°C for 700 s in microwave. The crude reaction was filtered and concentrated and then purified using preparative HPLC system A, C or D.

***Method L:*** The amide (1 eq), PdCl₂ (dppf) (0.05 eq), KOAc (4.5 eq) and bis(neopentyl glycolato)diboron (1.5 eq) were dissolved in 2 mL dry DME and the mixture was heated at 125°C for 1200 s in microwave. Then the reaction was cooled to room temperature and water (0.5 mL), NaHCO₃ (2 eq), Pd(tetrakis) (0.05 eq) and 5-bromo-3-[1H-indol-2-yl]pyrazin-2-amine (0.95 eq) were added and the resulting mixture is heated at 120°C for 700 s in microwave. The crude reaction was filtered and concentrated and then purified using preparative HPLC system A, C or D.

***Method M:*** The amide (1 eq), PdCl₂ (dppf) (0.05 eq), KOAc (4.5 eq) and bis(neopentyl glycolato)diboron (1.5 eq) were dissolved in 2 mL dry DME and the mixture was heated at 125°C for 1200 s in microwave. Then the reaction was cooled to room temperature and water (0.5 mL), NaHCO₃ (2 eq), Pd(tetrakis) (0.05 eq) and 3-(1-benzofuran-2-yl)-5-bromopyrazin-2-amine (0.95 eq) were added and the resulting mixture was heated at 120°C for 700 s in microwave. The crude reaction was filtered and concentrated and then purified using preparative HPLC system A, C or D.

The central pyrazine scaffolds in ***Methods G-M*** were made via a general Suzuki coupling between benzofuran-2-boronic acid/ 1-Boc-indole-2-boronic acid and 3-amino-2, 6-dibromopyrazine/2, 6-dichloropyrazine. The amides in ***Methods K-M*** were made by conventional amide coupling between phenylcarboxylic acids and aliphatic amines.

***Method N:*** 4-[5-amino-6-(1-benzofuran-2-yl)pyrazin-2-yl]benzoic acid (1 eq), the amine (1.5 eq), HOBT (1.3 eq), EDC (1.3 eq) and TEA (1.3 eq) were dissolved in 3 mL THF and stirred at room temperature overnight. The solution was concentrated and then purified using preparative HPLC system A, C or D.

***Method O:*** 4-[6-(1H-indol-2-yl)pyrazin-2-yl]benzoic acid (1 eq), the amine (1.5 eq), HOBT (1.3 eq), EDC (1.3 eq) and TEA (1.3 eq) were dissolved in 3 mL THF and stirred at room temperature overnight. The solution was concentrated and then purified using preparative HPLC system A, C or D.

The central pyrazine scaffolds in ***Methods N and O*** were made via two general Suzuki couplings, the first between benzofuran-2-boronic acid/ 1-Boc-indole-2-boronic acid and 3-amino-2, 6-dibromopyrazine/2, 6-dichloropyrazine, and the second between the corresponding intermediate from the first and 4-carboxyphenylboronic acid.

Any starting materials used in the Examples, the preparation of which has not been described herein, are commercially available and/or can be prepared by the skilled person merely using standard synthesis procedures and pathways known in the field of preparatory organic chemistry.

### Example 1

### 4-{3-amino-6-[3-methyl-4-(morpholin-4-ylcarbonyl)phenyl]pyrazin-2-yl}phenol

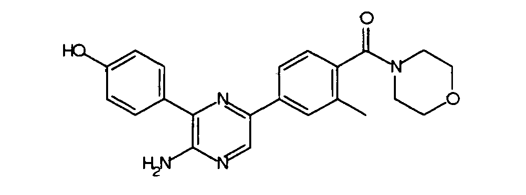

4-(4-bromo-2-methylbenzoyl)morpholine (22 mg, 0.08 mmol)) was treated according to *Method A*, giving the product as a light brown solid in an amount of 4.2 mg (yield 13%).
HPLC (system A) 99 %, RT 1.527 min (10-97 % MeCN over 3 min)
HPLC (system B) 99 %, RT 1.391 min (10-97 % MeCN over 3 min)
MS (ESI⁺) for C₂₂H₂₂N₄O₃ *m*/*z* 391 (M+H⁺)
¹HNMR (400MHz, MeOD) ppm 2.19 (s, 3H); 3.61-3.81 (m, 4H); 3.90-4.06 (m, 4H); 6.72 (d, *J*=9.12, 2H); 7.35 (s, 1H); 7.55 (d, *J*= 7.98, 1H); 8.12 (d, *J*=8.84, 2H); 8.23 (s, 1H); 8.59(d, *J*=8.18, 1H)

### Example 2

### 4-(3-amino-6-{2-methyl-4-[(4-methylpiperazin-1-yl)carbonyl]phenyl}pyrazin-2-yl)phenol

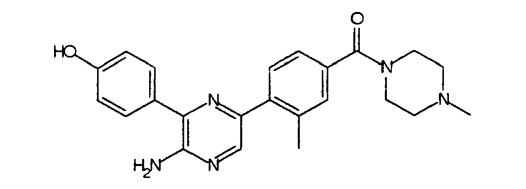

1-(4-bromo-3-methylbenzoyl)-4-methylpiperazine (19 mg, 0.06 mmol) was treated according to *Method A*, to provide the product as a brown solid in an amount of 4.1 mg (yield 17%).
HPLC (system A) 97 %, RT 1.099 min (10-97 % MeCN over 3 min)
HPLC (system B) 99 %, RT 0.956 min (10-97 % MeCN over 3 min)
MS (ESI⁺) for C₂₃H₂₅N₅O₂ *m*/*z* 404 (M+H⁺)

### Example 3

### 4-(6-{2-fluoro-4-[(4-methylpiperazin-1-yl)carbonyl]phenyl}pyrazin-2-yl)phenol

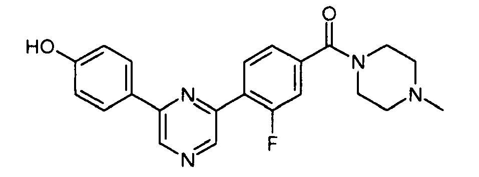

4-(4-bromo-3-fluorobenzoyl)methylpiperazine (32 mg, 0.11 mmol) was treated according to *Method B*, to give the product as a yellow solid in an amount of 2.8 mg (yield 7%).
HPLC (system A) 95 %, RT 1.441 min (10-97 % MeCN over 3 min)
HPLC (system B) 98 %, RT 1.285 min (10-97 % MeCN over 3 min)
MS (ESI⁺) for C₂₂H₂₁FN₄O₂ *m*/*z* 393 (M+H⁺)

### Example 4

### 4-{6-[3-fluoro-4-(morpholin-4-ylsulphonyl)phenyl]pyrazin-2-yl}phenol

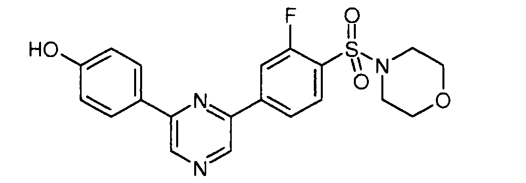

4-[(4-bromo-2-fluorophenyl)sulphonyl]morpholine (30 mg, 0.09 mmol) was treated according to *Method D*, to provide the product as a beige solid in an amount of 7.1 mg (yield 19%).
HPLC (system A) 100 %, RT 2.083 min (10-97 % MeCN over 3 min)
HPLC (system B) 100 %, RT 2.067 min (10-97 % MeCN over 3 min) MS (ESI⁺) for C₂₀H₁₈FN₃O₄S *m*/*z* 416 (M+H⁺)
¹HNMR (400MHz, MeOD) ppm 2.77-2.92 (m, 4H); 3.59-3.70 (m, 4H); 6.83 (d, *J*=8.97, 2H); 7.73 (s, 2H); 7.81-7.90 (m, 1H); 8.11 (d, *J*=9.56, 1H); 8.23 (d, *J*=9.04, 1H); 8.71 (d, *J*=8.77, 2H)

### Example 5

### 4-{3-amino-6-[2 methyl-4-(morpholin-4-ylcarbonyl)phenyl]pyrazin-2-yl}phenol

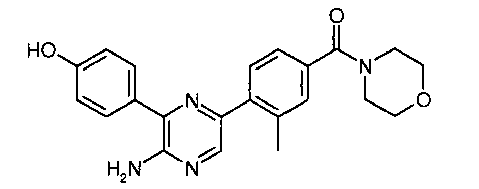

4-(4-bromo-3-methylbenzoyl)morpholine (18 mg, 0.06 mmol) was treated according to *Method A* to provide the product as a light yellow gum in an amount of 1.9 mg (yield 8%).
HPLC (system A) 96 %, RT 1.466 min (10-97 % MeCN over 3 min)
HPLC (system B) 96 %, RT 1.304 min (10-97 % MeCN over 3 min)
MS (ESI⁺) for C₂₂H₂₂N₄O₃ *m*/*z* 391 (M+H⁺)

### Example 6

### 4-{3-amino-6-[2-fluoro-4-(morpholin-4-ylcarbonyl)phenyl]pyrazin-2-yl}phenol

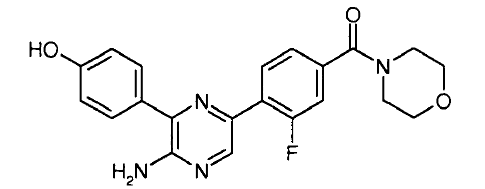

4-(4-bromo-3-fluorobenzoyl)morpholine (17 mg, 0.05 mmol) was treated according to *Method A* to give the product as a light brown solid in an amount of 2.7 mg (yield 14%).
HPLC (system A) 100 %, RT 1.507 min (10-97 % MeCN over 3 min)
HPLC (system B) 100 %, RT 1.389 min (10-97 % MeCN over 3 min)
MS (ESI⁺) for C₂₁H₁₉FN₄O₃ *m*/*z* 395 (M+H⁺)

### Example 7

### 4-(6-{2-methyl-4-[(4-methylpiperazin-1-yl)carbonyl]phenyl}pyrazin-2-yl)phenol

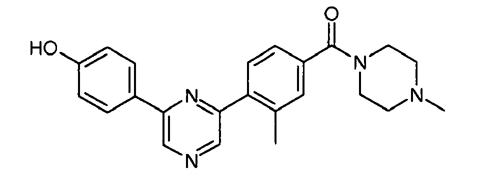

1-(4-bromo-3-methylbenzoyl)-4-methylpiperazine (19 mg, 0.06 mmol) was treated according to *Method B* to give the product as a light yellow solid in amount of 0.8 mg (yield 4%).
HPLC (system A) 97 %, RT 1.397 min (10-97 % MeCN over 3 min)
HPLC (system B) 97 %, RT 1.255 min (10-97 % MeCN over 3 min)
MS (ESI⁺) for C₂₃H₂₄N₄O₂ *m*/*z* 389 (M+H⁺)

### Example 8

### 4-(3-amino-6-{3-fluoro-4-[(4-methylpiperazin-1-yl)sulphonyl]phenyl}pyrazin-2-yl)phenol

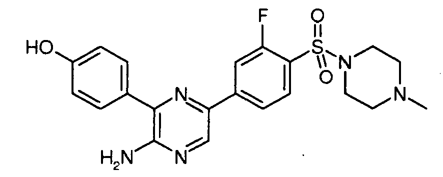

1-[(4-bromo-2-fluorophenyl)sulphonyl]-4-methylpiperazine (30 mg, 0.09 mmol) was treated according to *Method C* to provide the product as a light brown solid in an amount of 3.2 mg (yield 8%).
HPLC (system A) 100 %, RT 1.487 min (10-97 % MeCN over 3 min)
HPLC (system B) 100 %, RT 1.355 min (10-97 % MeCN over 3 min)
MS (ESI⁺) for C₂₁H₂₂FN₅O₃S *m*/*z* 444 (M+H⁺)

### Example 9

### 4-{3-amino-6-[3-methyl-4-(morpholin-4-ylsulphonyl)phenyl]pyrazin-2-yl}phenol

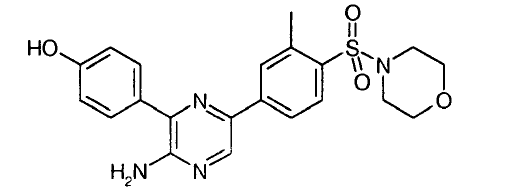

4-[(4-bromo-2-methylphenyl)sulphonyl]morpholine (30 mg, 0.09 mmol) was treated according to *Method C* to provide the product as a brown solid in an amount of 4.7 mg (yield 12%).
HPLC (system A) 95 %, RT 1.851 min (10-97 % MeCN over 3 min)
HPLC (system B) 95 %, RT 1.760 min (10-97 % MeCN over 3 min)
MS (ESI⁺) for C₂₁H₂₂N₄O₄S *m*/*z* 427 (M+H⁺)

### Example 10

### 4-[5-amino-6-(4-hydroxyphenyl)pyrazin-2-yl]-2-methyl-N-(2-pyrrolidin-1-ylethyl)benzenesulphonamide

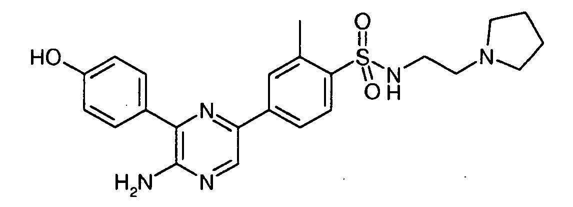

4-bromo-2-methyl-N-(2-pyrrolidin-1-ylethyl)benzenesulphonamide (30 mg, 0.09 mmol) was treated according to *Method C* to give the product as a brown gum in an amount of 1.9 mg (yield 5%).
HPLC (system A) 96 %, RT 1.434 min (10-97 % MeCN over 3 min)
HPLC (system B) 97 %, RT 1.292 min (10-97 % MeCN over 3 min)
MS (ESI⁺) for C₂₃H₂₇N₅O₃S *m*/*z* 454 (M+H⁺)

### Example 11

### 2-fluoro-4-[6-(4-hydroxyphenyl)pyrazin-2-yl]-N-(2-pyrrolidin-1-ylethyl)benzenesulphonamide

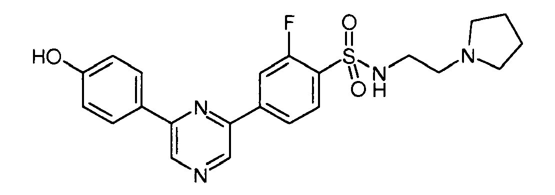

4-bromo-2-fluoro-N-(2-pyrrolidin-1-ylethyl)benzenesulphonamide (30 mg, 0.09 mmol) was treated according to *Method D* to give the product as a yellow gum in an amount of 0.5 mg (yield 1%).
HPLC (system A) 100 %, RT 1.614 min (10-97 % MeCN over 3 min)
HPLC (system B) 100 %, RT 1.473 min (10-97 % MeCN over 3 min)
MS (ESI⁺) for C₂₂H₂₃FN₄O₃S *m*/*z* 443 (M+H⁺)

### Example 12

### 4-{6-[3-methyl-4-(morpholin-4-ylsulphonyl)phenyl]pyrazin-2-yl}phenol

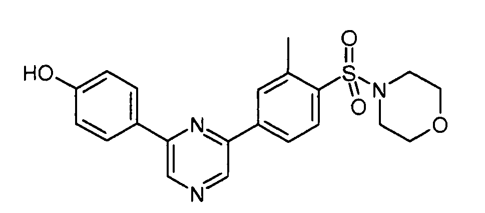

4-[(4-bromo-2-methylphenyl)sulphonyl]morpholine (30 mg, 0.09 mmol) was treated according to *Method D* giving the product as a brown gum in an amount of 6.2 mg (yield 17%).
HPLC (system A) 98 %, RT 2.100 min (10-97 % MeCN over 3 min)
HPLC (system B) 100 %, RT 2.082 min (10-97 % MeCN over 3 min)
MS (ESI⁺) for C₂₁H₂₁N₃O₄S *m*/*z* 412 (M+H⁺)

### Example 13

### 4-[6-(4-hydroxyphenyl)pyrazin-2-yl]-2-methyl-N-(2-pyrrolidin-1-ylethyl)benzenesulphonamide

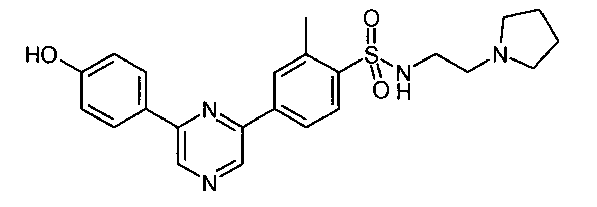

4-bromo-2-methyl-N-(2-pyrrolidin-1-ylethyl)benzenesulphonamide (30 mg, 0.09 mmol) was treated according to *Method D* giving the product as a light yellow solid in an amount of 0.2 mg (yield 0.5%).
HPLC (system A) 93 %, RT 1.655 min (10-97 % MeCN over 3 min)
HPLC (system B) 92 %, RT 1.509 min (10-97 % MeCN over 3 min)
MS (ESI⁺) for C₂₃H₂₆N₄O₃S *m*/*z* 439 (M+H⁺)

### Example 14

### 4-{3-amino-6-[3-fluoro-4-(morpholin-4-ylsulphonyl)phenyl]pyrazin-2-yl}phenol

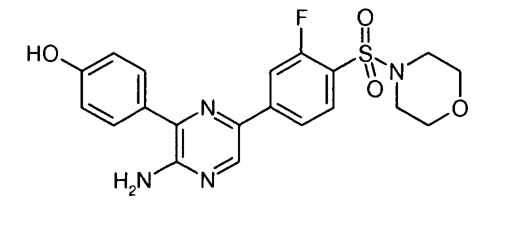

4-[(4-bromo-2-fluorophenyl)sulphonyl]morpholine (30 mg, 0.09 mmol) was treated according to *Method C* giving the product as a beige solid in an amount of 1.3 mg (yield 4%).
HPLC (system A) 96 %, RT 1.863 min (10-97 % MeCN over 3 min)
HPLC (system B) 96 %, RT 1.795 min (10-97 % MeCN over 3 min) MS (ESI⁺) for C₂₀H₁₉FN₄O₄S *m*/*z* 431 (M+H⁺)
¹HNMR (400MHz, MeOD) ppm 2.72-2.87 (m, 4H); 3.55-3.69 (m, 4H); 6.71 (d, *J*=8.77, 2H); 7.77 (d, *J*=10.14, 1H); 7.96 (d, *J*=9.06, 1H); 8.12 (d, *J*=8.70, 2H); 8.19-8.27 (m. 1H); 8.30 (s, 1H)

### Example 15

### 4-[5-amino-6-(4-hydroxyphenyl)pyrazin-2-yl]-2-fluoro-N-(2-pyrrolidin-1-ylethyl)benzenesuliphonamide

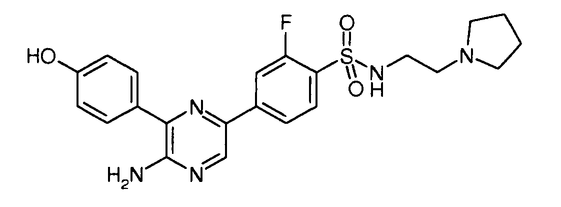

4-bromo-2-fluoro-N-(2-pyrrolidin-1-ylethyl)benzenesulphonamide (30 mg, 0.09 mmol) was treated according to *Method C* giving the product as a yellow solid in an amount of 4.3 mg (yield 10%).
HPLC (system A) 100 %, RT 1.418 min (10-97 % MeCN over 3 min)
HPLC (system B) 100 %, RT 1.283 min (10-97 % MeCN over 3 min) MS (ESI⁺) for C₂₂H₂₄FN₅O₃S *m*/*z* 458 (M+H⁺)
¹HNMR (400MHz, MeOD) ppm 1.67-1.75 (m, 4H); 2.37-2.50 (m, 6H); 2.95-3.04 (m, 2H); 6.73 (d, *J*=7.80, 2H); 7.74 (d, *J*=8.12, 1H); 8.00 (d, *J*=7.97, 1H); 8.09 (d, *J*=8.36, 2H); 8.15-8.24 (m, 1H); 8.30 (s, 1H)

### Example 16

### 4-(3-amino-6-{3-methyl-4-[(4-methylpiperazin-1-yl)sulphonyl]phenyl}pyrazin-2-yl)phenol

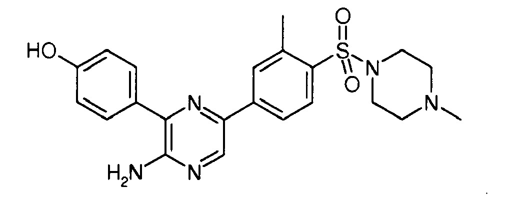

1-[(4-bromo-2-methylphenyl)sulphonyl]-4-methylpiperazine (30 mg, 0.09 mmol) was treated according to *Method C* giving the product as a beige solid in an amount of 1.1 mg (yield 3%).
HPLC (system A) 100 %, RT 1.467 min (10-97 % MeCN over 3 min)
HPLC (system B) 100 %, RT 1.335 min (10-97 % MeCN over 3 min) MS (ESI⁺) for C₂₂H₂₅N₅O₃S *m*/*z* 440 (M+H⁺)
¹HNMR (400MHz, MeOD) ppm 2.39-2.52 (m, 7H); 2.71 (s, 3H); 3.08-3.14 (m, 4H); 6.72 (d, *J*=8.73, 2H); 7.57 (d, *J*=8.58, 2H); 7.94 (s, 1H); 8.10 (d, *J*=8.64, 2H); 8.27 (s, 1H)

### Example 17

### 4-(6-{3-fluoro-4-[(4-methylpiperazin-1-yl)sulphonyl]phenyl}pyrazin-2-yl)phenol

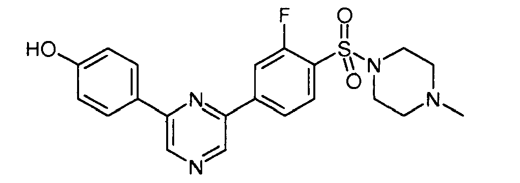

1-[(4-bromo-2-fluorophenyl)sulphonyl]-4-methylpiperazine (29 mg, 0.09 mmol) was treated according to *Method D* giving the product as a brown solid in an amount of 3.8 mg (yield 10%).
HPLC (system A) 100 %, RT 1.641 min (10-97 % MeCN over 3 min)
HPLC (system B) 100 %, RT 1.510 min (10-97 % MeCN over 3 min) MS (ESI⁺) for C₂₁H₂₁FN₄O₃S *m*/*z* 429 (M+H⁺)
¹HNMR (500MHz, MeOD) ppm 2.40-2.52 (m, 7H); 3.00-3.14 (m, 4H); 6.84 (d, *J*=8.77, 2H); 7.68 (s, 1H); 7.76 (d, *J*=8.69, 2H); 7.96 (s, 1H); 8.13 (d, *J*=8.70, 1H); 8.38 (d, *J*=8.82, 2H)

### Example 18

### 4-(3-amino-6-{3-methyl-4-[(4-methylpiperazin-1-yl)carbonyl]phenyl}pyrazin-2-yl)phenol trifluoroacetate (salt)

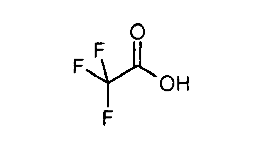

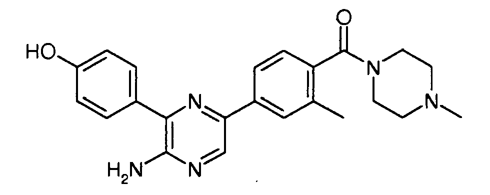

1-(4-bromo-2-methylbenzoyl)-4-methylpiperazine (19 mg, 0.06 mmol) was treated according to *Method A,* but purified using system C, to give the product as a yellow gum in an amount of 2.8 mg (yield 12%).
HPLC (system A) 100 %, RT 1.121 min (10-97 % MeCN over 3 min)
HPLC (system B) 100 %, RT 1.005 min (10-97 % MeCN over 3 min) MS (ESI⁺) for C₂₃H₂₅N₅O₂ *m*/*z* 404 (M+H⁺)
¹HNMR (400MHz, MeOD) ppm 2.11 (s, 3H); 2.25 (s, 3H); 2.73-2.81 (m, 4H); 3.78-3.86 (m, 4H); 6.75 (d, *J*=8.50, 2H); 7.35 (d, *J*=8.61, 1H); 7.47 (s, 1H); 8.13 (d, *J*=8.59, 2H); 8.23 (s, 1H); 8.49 (d, *J*=8.40, 1H)

### Example 19

### 4-(3-amino-6-{3-fluoro-4-[(4-methylpiperazin-1-yl)carbonyl]phenyl}pyrazin-2-yl)phenol

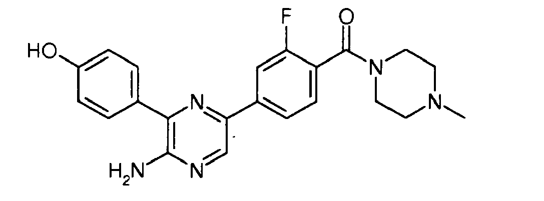

1-(4-bromo-2-fluorobenzoyl)-4-methylpiperazine (19 mg, 0.06 mmol) was treated according to *Method A* giving the product as a beige solid inan amount of 2.5 mg (yield 10%).
HPLC (system A) 100 %, RT 1.223 min (10-97 % MeCN over 3 min)
HPLC (system B) 100 %, RT 1.116 min (10-97 % MeCN over 3 min)
MS (ESI⁺) for C₂₂H₂₂FN₅O₂ *m*/*z* 408 (M+H⁺)
¹HNMR (400MHz, MeOD) ppm 2.25 (s, 3H); 2.67-2.81 (m, 4H); 3.77-3.88 (m, 4H); 6.73 (d, *J*=8.68, 2H); 7.70-7.78 (m, 2H); 8.12 (d, *J*=8.70, 2H); 8.26 (s, 1H); 8.57 (s, 1H)

### Example 20

### 4-(3-amino-6-{3-chloro-4-[(4-methylipiperazin-1-yl)carbonyl]phenyl}pyrazin-2-yl)phenol

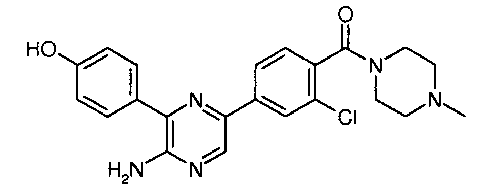

1-(4-bromo-2-chlorobenzoyl)-4-methylpiperazine (9 mg, 0.03 mmol) was treated according to *Method A* giving the product as a beige solid in an amount of 0.6 mg (yield 5%).
HPLC (system A) 100 %, RT 1.278 min (10-97 % MeCN over 3 min)
HPLC (system B) 100 %, RT 1.164 min (10-97 % MeCN over 3 min)
MS (ESI⁺) for C₂₂H₂₂ClN₅O₂ *m*/*z* 424 (M+H⁺)
¹HNMR (400MHz, MeOD) ppm 2.25 (s, 3H); 2.67-2.81 (m. 4H); 3.78-3.89 (m, 4H); 6.71 (d, *J*=8.78, 2H); 7.58 (d, *J*=8.18, 1H); 7.70 (d, *J*=8.71, 1H); 8.13 (d, *J*=8.71, 2H); 8.36 (s, 1H); 8.47 (s, 1H)

### Example 21

### 4-[5-amino-6-(4-hydroxyphenyl)pyrazin-2-yl]-2-chloro-N-(2-pyrrolidin-1-ylethyl)benzamide

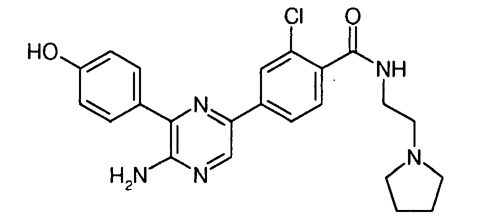

4-bromo-2-chloro-N-(2-pyrrolidin-1-ylethyl)benzamide (17 mg, 0.05 mmol) was treated according to *Method A* giving the product as a yellow solid in an amount of 0.5 mg (yield 2%).
HPLC (system A) 100 %, RT 1.355 min (10-97 % MeCN over 3 min)
HPLC (system B) 100 %, RT 1.233 min (10-97 % MeCN over 3 min)
MS (ESI⁺) for C₂₃H₂₄ClN₅O₂ *m*/*z* 438 (M+H⁺)
¹HNMR (400MHz, MeOD) ppm 1.70-1.81 (m, 4H); 2.46-2.61 (m, 6H); 3.37 (d, *J*=5.71, 2H); 6.73 (d, *J*=8.70, 2H); 7.62 (d, *J*=8.21, 1H); 7.73 (d, *J*=8.67, 1H); 8.14 (d, *J*=8.71, 2H); 8.36 (s, 1H); 8.62 (s, 1H)

### Example 22

### 4-{6-[3,5-difluoro-4-(morpholin-4-ylcarbonyl)phenyl]pyrazin-2-yl}phenol

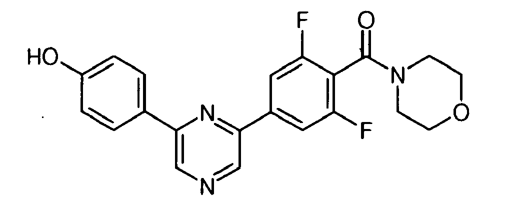

4-(4-bromo-2,6-difluorobenzoyl)morpholine (15 mg, 0.05 mmol) was treated according to *Method B* giving the product as a beige solid in an amount of 4.0 mg (yield 20%).
HPLC (system A) 100 %, RT 1.882 min (10-97 % MeCN over 3 min)
HPLC (system B) 98 %, RT 1.812 min (10-97 % MeCN over 3 min)
MS (ESI⁺) for C₂₁H₁₇F₂N₃O₃ *m*/*z* 398 (M⁺H⁺)

### Example 23

### 5-[4-(morpholin-4-ylcarbonyl)phenyl]-3-(3-thienyl)pyrazin-2-amine

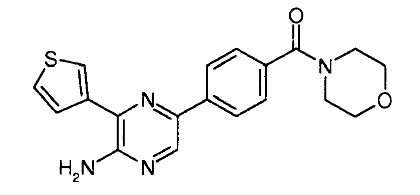

3-Thiopenic boronic acid (9 mg, 0.0715 mmol) was treated according to *Method E* giving the product as a light brown solid in an amount of 3.1 mg (yield 15%).
HPLC (system A) 96 %, RT 1.772 min (10-97 % MeCN over 3 min)
HPLC (system B) 98 %, RT 1.652 min (10-97 % MeCN over 3 min)
MS (ESI⁺) for C₁₉H₁₈N₄O₂S *m*/*z* 367 (M+H⁺)

### Example 24

### 5-[4-(morpholin-4-ylcarbonyl)phenyl]-3-[1-(phenylsulphonyl)-1H-indol-2-yl]pyrazin-2-amine

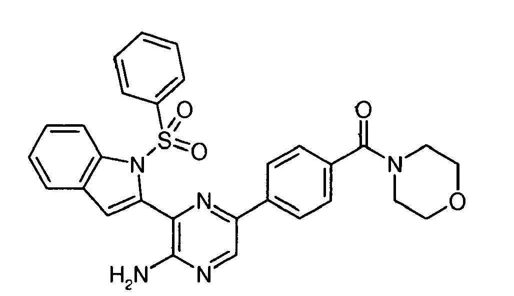

1-(Phenylsulphonyl)-1H-indol-2-yl boronic acid (22 mg,0.0715 mmol) was treated according to *Method E* giving the product as a light green solid in an amount of 0.1 mg (yield 0.4%).
HPLC (system A) 96 %, RT 2.259 min (10-97 % MeCN over 3 min)
HPLC (system B) 95 %, RT 2.028 min (10-97 % MeCN over 3 min)
MS (ESI⁺) for C₂₉H₂₅N₅O₄S *m*/*z* 540 (M+H⁺)
¹HNMR (400MHz, MeOD) ppm 3.39-3.77 (m, 8H); 6.92 (s, 1H); 7.19-7.35 (m, 4H); 7.42-7.54 (m, 3H); 7.71 (d, *J*=9.13, 2H); 7.85-8.00 (m, 3H); 8.06-8.13 (m, 2H)

### Example 25

### 4-{4-[6-(1-benzothien-2-yl)pyrazin-2-yl]benzoyl}morpholine

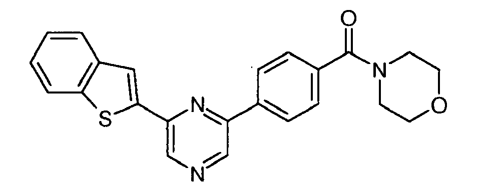

Benzothiophene-2-boronic acid (15 mg, 0.0858 mmol) was treated according to *Method F* giving the product as a light yellow solid in an amount of 2.2 mg (yield 9%).
HPLC (system A) 99 %, RT 2.401 min (10-97 % MeCN over 3 min)
HPLC (system B) 98 %, RT 2.247 min (10-97 % MeCN over 3 min)
MS (ESI⁺) for C₂₃H₁₉N₃O₂S *m*/*z* 402 (M+H⁺)
¹HNMR (400MHz, MeOD) ppm 3.37-3.81 (m, 8H); 7.31-7.35 (m, 2H); 7.56 (d, *J*=8.71, 2H); 7.80-7.88 (m, 2H); 8.14 (s, 1H); 8.24 (d, *J*=9.21, 2H); 8.98 (s, 1H); 9.10 (s, 1H)

### Example 26

### 4-{4-[6-(5-chloro-2-thienyl)pyrazin-2-yl]benzoyl}morpholine

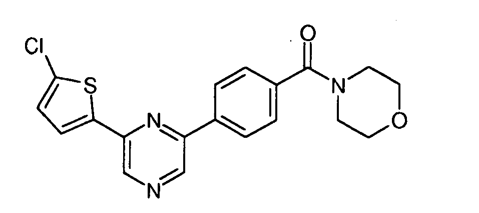

5-Chloro-2-thiophene boronic acid (15 mg, 0.0858 mmol) was treated according to *Method F* giving the product as a yellow solid in an amount of 6.5 mg (yield 25%).
HPLC (system A) 99 %, RT 2.351 min (10-97 % MeCN over 3 min)
HPLC (system B) 98 %, RT 2.160 min (10-97 % MeCN over 3 min)
MS (ESI⁺) for C₁₉H₁₆ClN₃O₂S *m*/*z* 386 (M+H⁺)

### Example 27

### 4-{4-[6-(1H-pyrrol-2-yl)pyrazin-2-yl]benzoyl}morpholine

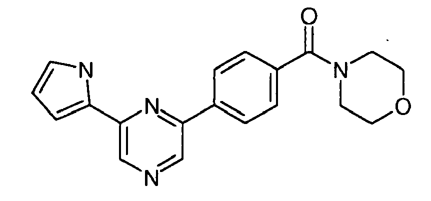

1-Boc-pyrrole-2-boronic acid (18 mg, 0.0858 mmol) was treated according to *Method F* giving the product (which was deprotected during synthesis) as a yellow gum in an amount of 2.1 mg (yield 9%).
HPLC (system A) 98 %, RT 1.892 min (10-97 % MeCN over 3 min)
HPLC (system B) 98 %, RT 1.661 min (10-97 % MeCN over 3 min)
MS (ESI⁺) for C₁₉H₁₈N₄O₂ *m*/*z* 335 (M+H⁺)
¹HNMR (400MHz, MeOD) ppm 3.38-3.74 (m, 8H); 6.19-6.22 (m, 1H); 6.86-6.91 (m, 1H); 6.94-6.98 (m, 1H); 7.52 (d, *J*=9.16, 2H); 8.27 (d, *J*=9.21, 2H); 8.73 (d, *J*= 8.47, 2H);

### Example 28

### 5-methoxy-2-{6-[4-(morpholin-4-ylcarbonyl)phenyl]pyrazin-2-yl}-1H-indole hydrochloride

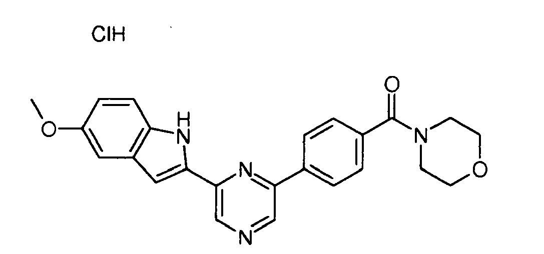

1-Boc-5-methoxyindole-2-boronic acid (24 mg, 0.0858 mmol) is treated according to *Method F* giving the product (which is deprotected using conc HCl and gentle heating) as a orange solid in an amount of 1.2 mg (yield 4%).
HPLC (system A) 100 %, RT 2.160 min (10-97 % MeCN over 3 min)
HPLC (system B) 100 %, RT 2.155 min (10-97 % MeCN over 3 min)
MS (ESI⁺) for C₂₄H₂₂N₄O₃ *m*/*z* 415 (M+H⁺),

### Example 29

### 2-{6-[4-(morpholin-4-ylcarbonyl)phenyl]pyrazin-2-yl}-1H-indole hydrochloride

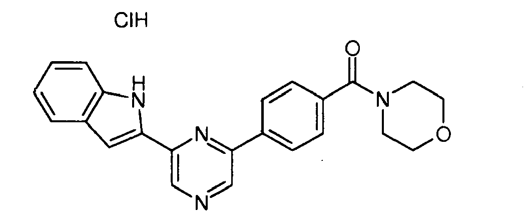

1-Boc-indole-2-boronic acid (22 mg, 0.0858 mmol) was treated according to *Method F* giving the product (which was deprotected using conc HCl and gentle heating) as a light yellow gum in an amount of 0.1 mg (yield 0.4%).
HPLC (system A) 100 %, RT 2.272 min (10-97 % MeCN over 3 min)
HPLC (system B) 100 %, RT 2.245 min (10-97 % MeCN over 3 min)
MS (ESI⁺) for C₂₃H₂₀N₄O₂ *m*/*z* 385 (M+H⁺)

### Example 30

### 3-(1H-indol-2-yl)-5-[4-(morpholin-4-ylcarbonyl)phenyl]pyrazin-2-amine trifluoroacetatete

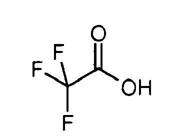

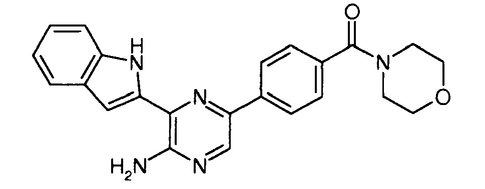

4-(morpholin-4-ylcarbonyl)phenyl boronic acid (193 mg 0.82 mmol) was treated according to *Method G* (but with the indole scaffold containing Boc group which partly falls off during reaction) giving the product as a brown gum in an amount of 18.1 mg (yield 6%).
HPLC (system A) 98 %, RT 2.094 min (10-97 % MeCN over 3 min)
HPLC (system B) 100 %, RT 2.037 min (10-97 % MeCN over 3 min)
MS (ESI⁺) for C₂₃H₂₁N₅O₂ *m*/*z* 400(M+H⁺)
¹HNMR (400MHz, MeOD) ppm 3.50-3.78 (m, 8H); 6.43-6.49 (m, 1H); 7.04-7.08 (m, 2H); 7.42 (s, 1H); 7.66 (d, *J*=8.28, 2H); 8.46 (d, *J*=8.29, 2H); 8.99 (s, 1H); 9.12 (s, 1H)

### Example 31

### 5-{6-[4-(morpholin-4-ylcarbonyl)phenyl]pyrazin-2-yl}-1H-indole

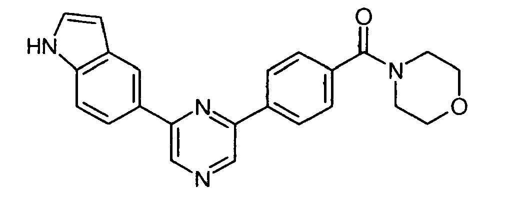

1H-indol-5 boronic acid (15 mg, 0.09 mmol) was treated according to *Method F* giving the product as a light yellow gum in an amount of 21.2 mg (yield 7%). HPLC (system A) 100 %, RT 1.851 min (10-97 % MeCN over 3 min)
HPLC (system B) 97 %, RT 1.692 min (10-97 % MeCN over 3 min)
MS (ESI⁺) for C₂₃H₂₀N4O₂ *m*/*z* 385 (M+H⁺)
¹HNMR (400MHz, MeOD) ppm 3.50-3.78 (m, 8H); 6.43-6.49 (m, 1H); 7.04-7.08 (m, 2H); 7.42 (s, 1H); 7.66 (d, *J*=8.28, 2H); 8.46 (d, *J*=8.29, 2H); 8.99 (s, 1H); 9.12 (s, 1H)

### Example 32

### 2-(6-{2-fluoro-4-[(4-methylpiperazin-1-yl)carbonyl]phenyl}pyrazin-2-yl)-1H-indole

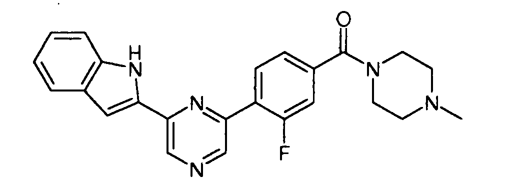

4-(4-bromo-3-fluorobenzoyl)methylpiperazine (100 mg 0.33 mmol) was treated according to *Method K* giving the product as a light green gum in an amount of 8.2 mg (yield 7%).
HPLC (system A) 100 %, RT 1.937 min (10-97 % MeCN over 3 min)
HPLC (system B) 100 %, RT 1.768 min (10-97 % MeCN over 3 min)
MS (ESI⁺) for C₂₄H₂₂FN₅O *m*/*z* 416(M+H⁺)
¹HNMR (400MHz, MeOD) ppm 3.50-3.78 (m, 8H); 6.43-6.49 (m, 1H); 7.04-7.08 (m, 2H); 7.42 (s, 1H); 7.66 (d, *J*=8.28, 2H); 8.46 (d, *J*=8.29, 2H); 8.99 (s, 1H); 9.12 (s, 1H)

### Example 33

### 5-{2-fluoro-4-[(4-methylpiperazin-1-yl)carbonyl]phenyl}-3-(1H-indol-2-yl)pyrazin-2-amine

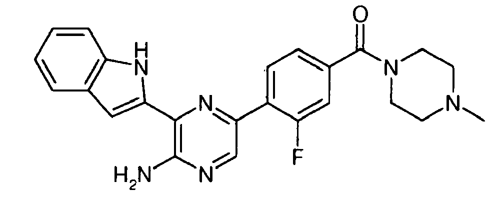

4-(4-bromo-3-fluorobenzoyl)methylpiperazine (100 mg 0.33 mmol) was treated according to *Method L* giving the product as a light yellow gum in an amount of 10.7 mg (yield 8%).
HPLC (system A) 100 %, RT 1.790 min (10-97 % MeCN over 3 min)
HPLC (system B) 100 %, RT 1.632 min (10-97 % MeCN over 3 min)
MS (ESI⁺) for C₂₄H₂₂FN₆O *m*/*z* 431(M+H⁺)
¹HNMR (400MHz, MeOD) ppm 3.50-3.78 (m, 8H); 6.43-6.49 (m, 1H); 7.04-7.08 (m, 2H); 7.42 (s, 1H); 7.66 (d, *J*=8.28, 2H); 8.46 (d, *J*=8.29, 2H); 8.99 (s, 1H); 9.12 (s, 1H)

### Example 34

### 2-{6-[2-fluoro-4-(morpholin-4-ylcarbonyl)phenyl]pyrazin-2-yl}-1H-indole

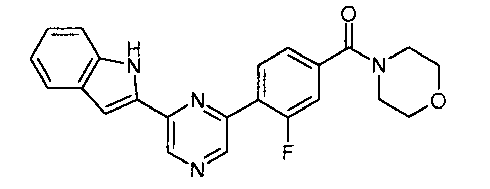

4-(4-bromo-3-fluorobenzoyl)morpholine (100 mg 0.33 mmol) was treated according to *Method K* giving the product as a light yellow gum in an amount of 1 mg (yield 0.8%).
HPLC (system A) 100 %, RT 2.342 min (10-97 % MeCN over 3 min)
HPLC (system B) 100 %, RT 2.350min (10-97 % MeCN over 3 min)
MS (ESI⁺) for C₂₃H₁₉FN₄O₂ *m*/*z* 403(M+H⁺)

### Example 35

### 5-[2-fluoro-4-(morpholin-4-ylcarbonyl)phenyl]-3-(1H-indol-2-yl)pyrazin-2-amine

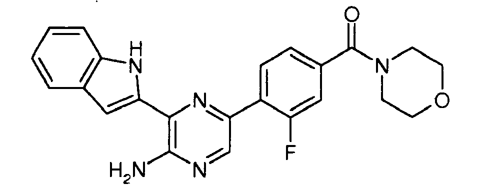

4-(4-bromo-3-fluorobenzoyl)morpholine (100 mg 0.33 mmol) was treated according to *Method L* giving the product as a light yellow gum in an amount of 1.2 mg (yield 0.9%).
HPLC (system A) 95 %, RT 2.098 min (10-97 % MeCN over 3 min)
HPLC (system B) 95 %, RT 2.057min (10-97 % MeCN over 3 min)
MS (ESI⁺) for C₂₃H₂₀FN₅O₂ *m*/*z* 418(M+H⁺)

### Example 36

### 6-methyl-2-{6-[4-(morpholin-4-ylcarbonyl)phenyl]pvrazin-2-yl}-1H-indole

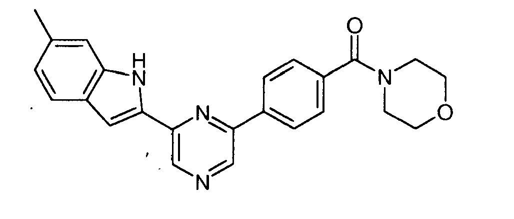

[4-(morpholine-4-carbonyl)phenyl]boronic acid (3.1 mg 0.013 mmol) is treated according to *Method H* (but with 2-(6-chloropyrazin-2-yl)-6-methyl-1H-indole as central scaffold) giving the product as a yellow gum in an amount of 2.2 mg (yield 45%).
HPLC (system A) 99 %, RT 2.346 min (10-97 % MeCN over 3 min)
HPLC (system B) 99 %, RT 2.329min (10-97 % MeCN over 3 min)
MS (ESI⁺) for C₂₄H₂₂N₄O₂ *m*/*z* 399 (M+H⁺)

### Example 37

### 5-fluoro-2-{6-[4-(morpholin-4-ylcarbonyl)phenyl]pyrazin-2-yl}-1H-indole

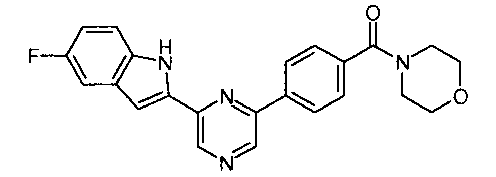

[4-(morpholine-4-carbonyl)phenyl]boronic acid (3.6 mg 0.0154 mmol) was treated according to *Method H* (but with 2-(6-chloropyrazin-2-yl)-5-fluoro-1H-indole as central scaffold) giving the product as a yellow gum in an amount of 1.5 mg (yield 24%).
HPLC (system A) 99 %, RT 2.257 min (10-97 % MeCN over 3 min)
HPLC (system B) 99 %, RT 2.223min (10-97 % MeCN over 3 min)
MS (ESI⁺) for C₂₃H₁₉FN₄O₂ *m*/*z* 403 (M+H⁺)

### Example 38

### 1-(methylsulphonyl)-2-{6-[4-(morpholin-4-ylcarbonyl)phenyl]pyrazin-2-yl}-1H-indole

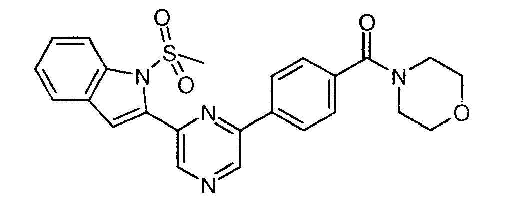

2-{6-[4-(morpholin-4-ylcarbonyl)phenyl]pyrazin-2-yl}-1H-indole (the hydrochloride of which has been discribed in Example 29) (25 mg, 0.065 mmol) was dissolved in 2 mL DCM, NaOH (3 mg, 0.09 mmol) was added, and the mixture was stirred for 10 min. Then methyl sulphonyl chloride (11 mg, 0.095 mmol) was added and the mixture was stirred overnight and then purified using preparative HPLC system C or D, to give the product as a light brown gum, 2.2mg (yield 7%).
HPLC (system A) 100 %, RT 2.198 min (10-97 % MeCN over 3 min)
HPLC (system B) 100 %, RT 2.102 min (10-97 % MeCN over 3 min)
MS (ESI⁺) for C₂₄H₂₂N₄O₄S *m*/*z* 463(M+H⁺)

### Example 39

### 1-(isopropylsulphonyl)-2-{6-[4-(morpholin-4-ylcarbonyl)phenyl]pyrazin-2-yl}-1H-indole

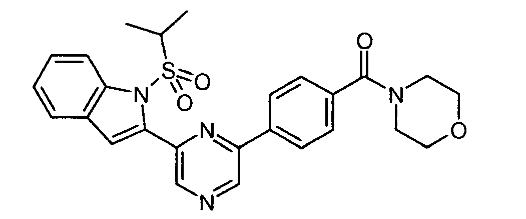

2-{6-[4-(morpholin-4-ylcarbonyl)phenyl]pyrazin-2-yl}-1H-indole (the hydrochloride of which has been discribed in Example 29) (25 mg, 0.065 mmol) was dissolved in 2mL DCM, NaOH (3 mg, 0.09 mmol) was added, and the mixture was stirred for 10 min. Thereafter isopropyl sulphonyl chloride (13 mg, 0.095 mmol) was added and the mixture was stirred overnight and then purified using preparative HPLC system C or D, to give the product as a light yellow gum, 1.0mg (yield 3%).
HPLC (system A) 100 %, RT 2.372 min (10-97 % MeCN over 3 min)
HPLC (system B) 100 %, RT 2.290 min (10-97 % MeCN over 3 min)
MS (ESI⁺) for C₂₆H₂₆N₄O₄S *m*/*z* 491(M+H⁺)

### Example 40

### 4-[5-amino-6-(1-benzofuran-2-yl)pyrazin-2-yl]-N-(2,3-dihydroxypropyl)benzamide

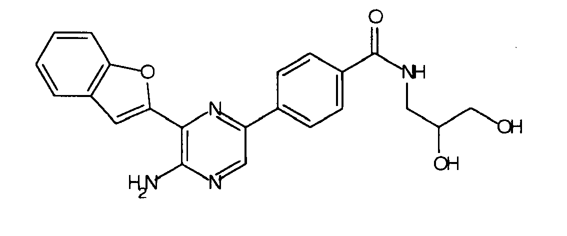

3-amino-1,2-dipropanediol (8.2 mg, 0.09 mmol) was treated according to *Method N* to give the product as a yellow solid in an amount of 0.6 mg (yield 2.5%).
HPLC (system A) 100 %, RT 1.805 min (10-97 % MeCN over 3 min)
HPLC (system B) 100 %, RT 1.701 min (10-97 % MeCN over 3 min)
MS (ESI⁺) for C₂₂H₂₀N₄O₄ *m*/*z* 405(M+H⁺)

### Example 41

### 4-[5-amino-6-(1-benzofuran-2-yl)pyrazin-2-yl]-N-[1-(hydroxymethyl)-2-methylpropyl]benzamide

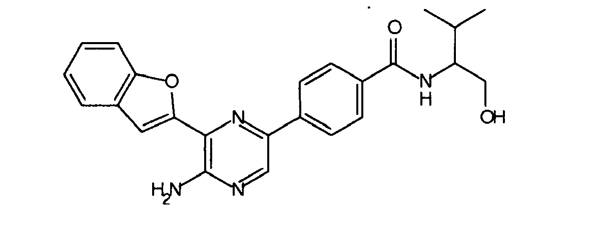

2-Amino-3-methyl-1-butanol (9.3 mg, 0.09 mmol) was treated according to *Method N* giving the product as a yellow solid in an amount of 1.3 mg (yield 5%).
HPLC (system A) 100 %, RT 2.181 min (10-97 % MeCN over 3 min)
HPLC (system B) 100 %, RT 2.136 min (10-97 % MeCN over 3 min)
MS (ESI⁺) for C₂₄H₂₄N₄O₃ *m*/*z* 417(M+H⁺)
¹HNMR (400MHz, MeOD) ppm 0.92-0.97 (m, 6H); 1.94-1.99 (m, 1H); 3.64-3.68 (m, 2H); 3.85-3.89 (m, 1H); 7.22-7.33 (m, 2H); 7.52-7.65 (m, 4H); 7.88 (d, *J*=8.53, 1H); 8.08 (d, *J*=8.78, 1H); 8.27 (s, 1H); 8.52 (s, 1H)

### Example 42

### 4-[5-amino-6-(1-benzofuran-2-yl)pyrazin-2-yl]-N-[(2S)-2,3-dihydroxypropyl]benzamide

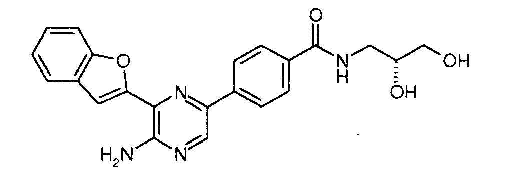

(S)-(-)-3-Amino-1,2-propandiol (8.2 mg, 0.09 mmol) was treated according to *Method N* giving the product as a yellow solid in an amount of 1.6 mg (yield 7%).

HPLC (system A) 100 %, RT 1.805 min (10-97 % MeCN over 3 min)
HPLC (system B) 100 %, RT 1.701 min (10-97 % MeCN over 3 min)
MS (ESI⁺) for C₂₂H₂₀N₄O₄ *m*/*z* 405(M+H⁺)
¹HNMR (400MHz, MeOD) ppm 3.44-3.51 (m, 2H); 3.56-3.64 (m, 2H); 3.86-3-91 (m, 1H); 7.32-7.43 (m, 2H); 7.67-7.76 (m, 4H); 7.98 (d, *J*=8.53, 1H); 8.18 (d, *J*=8.79, 1H); 8.62 (s, 2H)

### Example 43

### tert-butyl (1-{4-[5-amino-6-(1-benzofuran-2-yl)pyrazin-2-yl]benzoyl}pyrrolidin-3-yl)carbamate

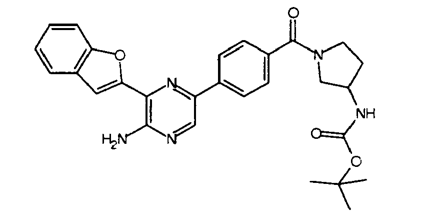

3-(tert-Butoxycarbonylamino)pyrrolidine (16.7 mg, 0.09 mmol) was treated according to *Method N* giving the product as a yellow gum in an amount of 3.2 mg (yield 11%).
HPLC (system A) 97 %, RT 2.407 min (10-97 % MeCN over 3 min)
HPLC (system B) 93 %, RT 2.383 min (10-97 % MeCN over 3 min)
MS (ESI⁺) for C₂₈H₂₉N₅O₄ *m*/*z* 500(M+H⁺)
¹HNMR (400MHz, MeOD) ppm 1.42 (s, 9H); 2.05-2.20 (m, 2H); 3.49-3.89 (m, 4H); 4.05-4.11 (m, 1H); 7.32-7.46 (m, 2H); 7.61-7.70 (m, 4H); 7.74 (d, *J*=7.78, 1H); 8.16 (d, *J*=8.54, 1H); 8.59 (s, 2H)

### Example 44

### 4-[5-amino-6-(1-benzofuran-2-yl)pyrazin-2-yl]-N-{3-[bis(2-hydroxyethyl)amino]propyl}benzamide

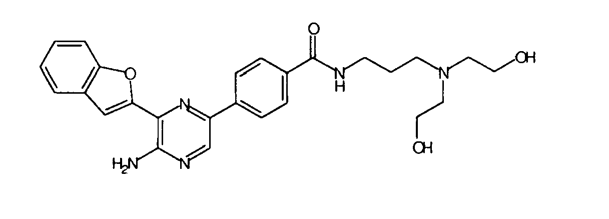

N-(3-Aminopropyl)diethanolamine (14.6 mg, 0.09 mmol) was treated according to *Method N* giving the product as a yellow solid in an amount of 5.3 mg (yield 19%).
HPLC (system A) 94 %, RT 1.739 min (10-97 % MeCN over 3 min)
HPLC (system B) 94 %, RT 1.582 min (10-97 % MeCN over 3 min)
MS (ESI⁺) for C₂₆H₂₉N₅O₄ *m*/*z* 476(M+H⁺)
¹HNMR (400MHz, MeOD) ppm 1.98-2.05 (m, 2H); 3.04-3.10 (m, 4H); 3.50-3.58 (m, 4H); 3.76-3.81 (m, 4H); 7.32-7.43 (m, 2H); 7.66-7.75 (m, 4H); 7.97 (d, *J*=8.53, 1H); 8.18 (d, *J*=8.79, 1H); 8.61 (s, 2H)

### Example 45

### N-(2,3-dihydroxypropyl)-4-[6-(1H-indol-2-yl)pyrazin-2-yl]benzamide

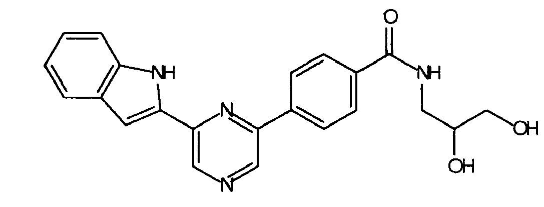

3-amino-1,2-dipropanediol (8.6 mg, 0.095 mmol) was treated according to *Method O* giving the product as a yellow gum in an amount of 2.5 mg (yield 10%).
HPLC (system A) 100 %, RT 1.972 min (10-97 % MeCN over 3 min)
HPLC (system B) 100 %, RT 1.897 min (10-97 % MeCN over 3 min)
MS (ESI⁺) for C₂₂H₂₀N₄O₃ *m*/*z* 389(M+H⁺)

### Example 46

### N-[(2S)-2,3-dihydroxypropyl]-4-[6-(1H-indol-2-yl)pyrazin-2-yl]benzamide

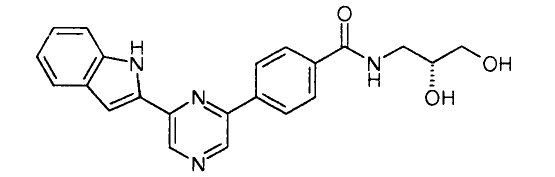

(S)-(-)-3-Amino-1,2-propandiol (8.6 mg, 0.095 mmol) was treated according to *Method O* to give the product as a yellow gum in an amount of 1.0 mg (yield 4%).
HPLC (system A) 100 %, RT 1.975 min (10-97 % MeCN over 3 min)
HPLC (system B) 100 %, RT 1.900 min (10-97 % MeCN over 3 min)
MS (ESI⁺) for C₂₂H₂₀N₄O₃ *m*/*z* 389(M+H⁺)

### Example 47

### tert-butyl (1-{4-[6-(1H-indol-2-yl)pyrazin-2-yl]benzoyl}pyrrolidin-3-yl)carbamate

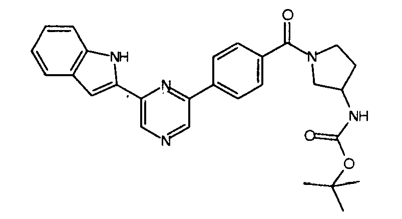

3-(tert-Butoxycarbonylamino)pyrrolidine (17.7 mg, 0.095 mmol) was treated according to *Method O* giving the product as a yellow gum in an amount of 4.2 mg (yield 14%).
HPLC (system A) 100 %, RT 2.507 min (10-97 % MeCN over 3 min)
HPLC (system B) 100 %, RT 2.461 min (10-97 % MeCN over 3 min)
MS (ESI⁺) for C₂₈H₂₉N₅O₃ *m*/*z* 484 (M+H⁺)
¹HNMR (400MHz, MeOD) ppm 1.82 (s, 9H); 2.06-2.18 (m, 2H); 3.34-3.80 (m, 4H); 3.99-4.04 (m, 1H); 6.98 (t, *J*=14.30, 1H); 7.12 (t, *J*=15.31, 1H); 7.22 (s, 1H); 7.44 (d, *J*=7.53, 2H); 7.55 (d, *J*=8.03, 2H); 7.64 (t, *J*=17.82, 1H); 8.32-8.35 (m, 1H); 8.89 (s, 1H); 9.01 (s, 1H)

### Example 48

### tert-butyl 3-[({4-[6-(1H-indol-2-yl)pyrazin-2-yl]benzoyl}amino)methyl]piperidine-1-carboxylate

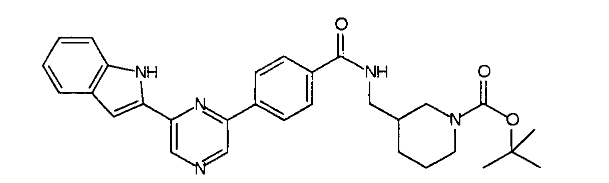

3-(Aminomethyl)-1-N-Boc-piperidine (20.3 mg, 0.095 mmol) was treated according to *Method O* giving the product as a yellow gum in an amount of 3.9 mg (yield 14%).
HPLC (system A) 91 %, RT 2.683 min (10-97 % MeCN over 3 min)
HPLC (system B) 90 %, RT 2.749 min (10-97 % MeCN over 3 min)
MS (ESI⁺) for C₃₀H₃₃N₅O₃ *m*/*z* 412 (M-Boc+H⁺)
¹HNMR (400MHz, MeOD) ppm 1.32-1.38 (m, 3H); 1.53-1.59 (m, 2H); 1.76-1.85 (m, 4H); 1.90 (s, 9H); 1.95 (s, 2H); 7.00 (t, *J*=15.06, 1H); 7.15 (t, *J*=15.31, 1H); 7.21 (s, 1H); 7.43 (d, *J*=8.28, 2H); 7.56 (d, *J*=8.03, 2H); 7.94 (d, *J*=6.78, 1H); 8.36 (d, *J*=8.54, 1H); 8.85 (s, 2H)

### Example 49

### 4-[5-amino-6-(1-benzofuran-2-yl)pyrazin-2-yl]-N-ethyl-N-(2-hydroxyethyl)benzamide

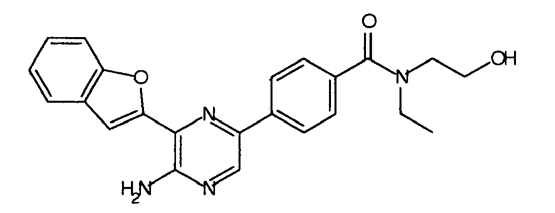

2-(Ethylamino)ethanol (8.0 mg, 0.09 mmol) was treated according to *Method N* giving the product as a light yellow solid in an amount of 0.7 mg (yield 3%). HPLC (system A) 100 %, RT 2.061 min (10-97 % MeCN over 3 min)
HPLC (system B) 100 %, RT 1.982 min (10-97 % MeCN over 3 min)
MS (ESI⁺) for C₂₃H₂₂N₄O₃ *m*/*z* 403 (M+H⁺)

### Example 50

### N-ethyl-N-(2-hydroxyethyl)-4-[6-(1H-indol-2-yl)pyrazin-2-yl]benzamide

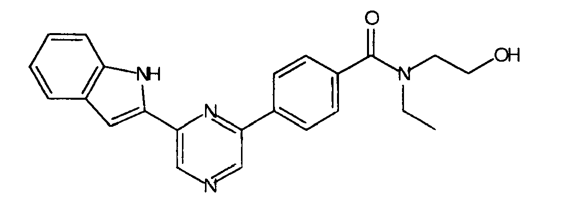

2-(Ethylamino)ethanol (8.5 mg, 0.095 mmol) was treated according to *Method O* giving the product as a light yellow gum in an amount of 0.6 mg (yield 2%). HPLC (system A) 97 %, RT 2.143 min (10-97 % MeCN over 3 min)
HPLC (system B) 98 %, RT 2.073 min (10-97 % MeCN over 3 min)
MS (ESI⁺) for C₂₃H₂₂N₄O₂ *m*/*z* 387 (M+H⁺)

### Example 51

### N-[3-(dimethylamino)propyl]-4-[6-(1H-indol-2-yl)pyrazin-2-yl]-N-methylbenzamide

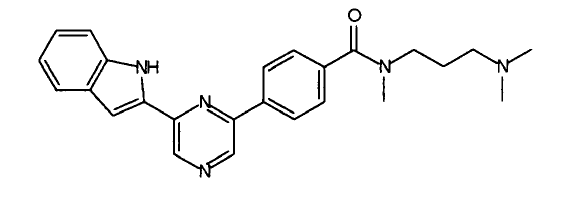

N,N,N'-Trimethyl-1,3-propanediamine (11.2 mg, 0.095 mmol) was treated according to *Method O* to give the product as a yellow gum in an amount of 1.3 mg (yield 5%).
HPLC (system A) 100 %, RT 1.915 min (10-97 % MeCN over 3 min)
HPLC (system B) 100 %, RT 1.733 min (10-97 % MeCN over 3 min)
MS (ESI⁺) for C₂₅H₂₇N₅O *m*/*z* 414 (M+H⁺)

### Example 52

### 3-(4-{4-[6-(1H-indol-2-yl)pyrazin-2-yl]benzoyl}piperazin-1-yl)-N,N-dimethylpropan-1-amine

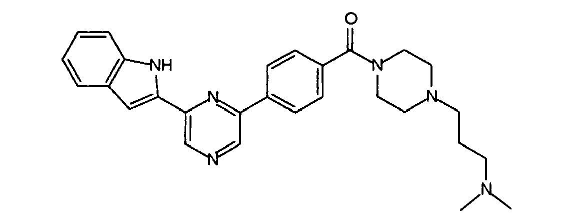

1-[3-(Dimethylamino)propyl]piperazine (16.2 mg, 0.095 mmol) was treated according to *Method O* giving the product as a yellow gum in an amount of 6.0 mg (yield 20%).
HPLC (system A) 100 %, RT 1.723 min (10-97 % MeCN over 3 min)
HPLC (system B) 100 %, RT 1. 528 min (10-97 % MeCN over 3 min)
MS (ESI⁺) for C₂₈H₃₂N₆O *m*/*z* 469 (M+H⁺)
¹HNMR (400MHz, MeOD) ppm 2.24-2.32 (m, 2H); 2.72-2.80 (m, 2H); 2.95(s, 8H); 3.24-3.30 (m, 4H); 3.40-3.54 (m, 4H); 7.09 (t, *J*=15.06, 1H); 7.23 (t, *J*=115.06, 1H); 7.34 (s, 1H); 7.54 (d, *J*=8.28, 1H); 7.66 (d, *J*=7.78, 1H); 7.73(d, *J*=8.28, 2H); 8.49 (d, *J*=8.28, 2H); 9.01 (s, 1H); 9.13 (s, 1H)

### Example 53

### 5-fluoro-2-(6-{4-[(4-methylpiperazin-1-yl)carbonyl]phenyl}pyrazin-2-yl)-1H-indole

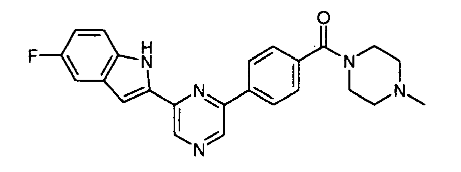

200 mg (1.35 mmol) of 2, 6-dichloropyrazine was mixed with 232 mg (1.4 mmol) 4-carboxyphenyl boronic acid, 146 mg (4.7 mmol) of NaHCO₃ and 7 mg Pd(tetrakis) in 3.5 mL DME and 1 mL H₂O. The mixture was heated in microwave for 900 s at 120°C. To the same microwave tube was 360 mg (1.2 mmol) of 1-Boc-5-fluoroindole-2-boronic acid and 7 mg of Pd (tetrakis) added. The mixture was heated at 120°C for 900 s in microwave. The solution was concentrated, diluted in MeOH, and filtrated through celite. Thereafter concentrated to a yellow solid, consisting of both unprotected and protected 4-[6-(5-flouro-1(H/Boc)-indol-2-yl)pyrazin-2-yl]benzoic acid in an amount of 250 mg.

250 mg (0.75 mmol) of 4-[6-(5-flouro-1(H/Boc)-indol-2-yl)pyrazin-2-yl]benzoic acid was dissolved in 25 mL DMF, and TBTU 481 mg (1.5 mmol) and TEA 114 mg (1.1 mmol) were added. The reaction mixture was stirred at room temperature for 3 hrs, then concentrated to a redbrown gum. Purification with preparative HPLC which gives the product as a yellow gum in an amount of 27.3 mg (yield 9%).
HPLC (system A) 100 %, RT 1.950 min (10-97 % MeCN over 3 min)
HPLC (system B) 100 %, RT 1.765 min (10-97 % MeCN over 3 min)
MS (ESI⁺) for C₂₄H₂₂FN₅O *m*/*z* 416 (M+H⁺)
¹HNMR (400MHz, MeOD) ppm 2.27 (s, 3H); 2.35-3.44 (m, 4H); 2.45-2.51 (m, 4H); 6.88-6.93 (m, 1H); 7.20-7.23 (m, 1H); 7.39-7.44 (m, 1H); 7.54 (d, *J*=8.53, 2H); 7.60(d, *J*=8.28, 2H); 8.36 (t, *J*=16.56, 1H); 8.91-8.94 (m, 1H); 9.00-9.04 (m, 1H)

### Example 54

### 3-(1-benzofuran-2-yl)-5-{2-fluoro-4-[(4-methylpiperazin-1-yl)carbonyl]phenyl}pyrazin-2-amine

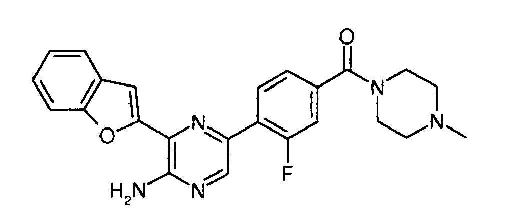

1 g (3.9 mmol) of 3-amino- 2, 6-dibromopyrazine was mixed with 704 mg (4.3 mmol) of benzofuran-2-boronic acid, 363 mg (11.7 mmol) of NaHCO₃ and 22 mg of Pd(tetrakis) in 25 mL DME and 5 mL H₂O. The mixture was heated at 100°C overnight. The reaction was cooled to room temperature and then portioned between water and DCM. The organic phase was filtrated through celite and concentrated to give 3-(1-benzofuran-2-yl)-5-bromopyrazin-2-amine as a brown orange solid in an amount of 960 mg.

14 mg (0.045 mmol) of 1-(4-bromo-3-fluorobenzoyl)-4-methylpiperazine was mixed with 15 mg (0.068 mmol) of bis(neopentyl glycolato)diboron, 20 mg (0.2 mmol) of KOAc, 3 mg of PdCl₂(dppf) which were then dissolved in 1.5 mL dry DME and heated in the microwave for 750 s at 125°C. To the same microwave tube was then 12 mg (0.04 mmol) of 3-(1-benzofuran-2-yl)-5-bromopyrazin-2-amine, 37 mg (0.09 mmol) of NaHCO₃, 7 mg of Pd(tetrakis) and 0.5 mL H₂O added. The mixture was heated in the microwave for 600 s at 120°C. The reaction mixture was filtrated, concentrated and purified by preparative HPLC giving the product as a brown gum in an amount of 4.1 mg (yield 21%).

HPLC (system A) 94 %, RT 1.770 min (10-97 % MeCN over 3 min)
HPLC (system B) 95 %, RT 1.618 min (10-97 % MeCN over 3 min)
MS (ESI⁺) for C₂₄H₂₂FN₅O₂ *m*/*z* 432 (M+H⁺)
¹HNMR (400MHz, CDCl₃) ppm 2.29 (s, 3H); 3.40-3.53 (m, 4H); 3.71-3.82 (m, 4H); 5.95 (s, 1H); 7.22-7.33 (m, 2H); 7.47-7.55 (m, 2H); 7.62 (d, *J*=7.03, 2H); 8.07-8.11 (m, 1H); 8.52 (s, 1H)

### Intermediate 4

### 2-(6-chloropyrazin-2-yl)-5-fluoro-1H-indole

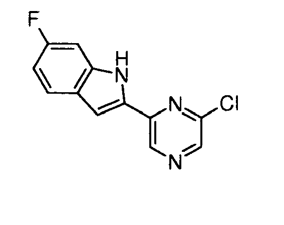

530 mg (3.6 mmol) of 2, 6-dichloropyrazine was mixed with 1 g (3.6 mmol) of 1-Boc-5-fluoroindole-2-boronic acid, 390 mg (12.6 mmol) of NaHCO₃ and 20 mg of Pd(tetrakis) in 10 mL DME and 2 mL H₂O. The mixture was heated at 100°C overnight, then filtrated through celite and concentrated to give 2-(6-chloropyrazin-2-yl)-5-fluoro-1 H-indole as a yellow solid in an amount of 850 mg.

### Example 55

### 3-fluoro-4-[6-(6-fluoro-1H-indol-2-yl)pyrazin-2-yl]-N-methyl-N-(1-methylpyrrolidin-3-yl)benzamide

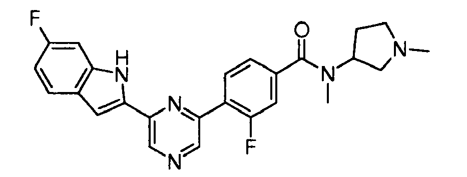

40 mg (0.18 mmol) of 4-bromo-3-fluoro benzoic acid was dissolved in 2 mL DMF and 21 mg (0.22 mmol) of N,N'-Dimethyl-3-aminopyrrolidine, 88 mg (0.27 mmol) TBTU and 22 mg (0.22 mmol) of TEA were added. The mixture was stirred at room temperature overnight, then concentrated and purified with preparative HPLC giving 4-bromo-3-fluoro-N-methyl-N-(1-methylpyrrolidin-3-yl)benzamide as a yellow gum in an amount of 81 mg.

20 mg (0.063 mmol) of 4-bromo-3-fluoro-N-methyl-N-(1-methylpyrrolidin-3-yl)benzamide, 22 mg (0.1 mmol) of bis(neopentyl glycolato) diboron, 30 mg (0.30 mmol) of KOAc and 4 mg of PdCl₂ were heated at 125°C for 900 s in microwave. To the same microwave tube were then 15 mg (0.06 mmol) of 2-(6-chloropyrazin-2-yl)-5-fluoro-1H-indole, 4 mg (0.12 mmol) of NaHCO₃ and 4 mg of Pd(tetrakis) added. The mixture was heated at 120°C for 700 s then filtrated through celite. Finally purification using preparative HPLC giving the product as a brown gum in an amount of 1 mg (yield 3.5%).

HPLC (system A) 90 %, RT 1.983 min (10-97 % MeCN over 3 min)
HPLC (system B) 94 %, RT 1.862 min (10-97 % MeCN over 3 min)
MS (ESI⁺) for C₂₅H₂₃F₂N₅O *m*/*z* 448(M+H⁺)

### Example 56

### 2-(4-{3-fluoro-5-[6-(6-fluoro-1H-indol-2-yl)pyrazin-2-yl]benzoyl}piperazin-1-yl)-N,N-dimethylethanamine

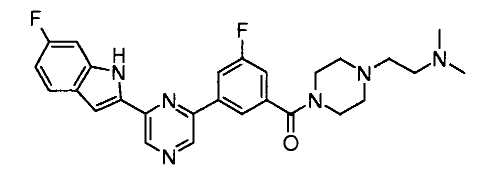

40 mg (0.18 mmol) of 3-bromo-5-fluoro benzoic acid was dissolved in 2 mL DMF and 29 mg (0.22 mmol) of 1-[2-(Dimethylamino)ethyl]piperazine, 88 mg (0.27 mmol) TBTU and 22 mg (0.22 mmol) of TEA were added. The mixture was stirred at room temperature overnight, then concentrated and purified with preparative HPLC giving 2-[4-(3-bromo-5-fluorobenzoyl)piperazin-1-yl]-N,N-dimethylethanamine as a yellow gum in an amount of 85 mg.

21 mg (0.062 mmol) of 2-[4-(3-bromo-5-fluorobenzoyl)piperazin-1-yl]-N,N-dimethylethanamine, 22 mg (0.1 mmol) of bis(neopentyl glycolato) diboron, 30 mg (0.30 mmol) of KOAc and 4 mg of PdCl₂ were heated at 125°C for 900 s in microwave. To the same microwave tube were then 15 mg (0.06 mmol) of 2-(6-chloropyrazin-2-yl)-5-fluoro-1H-indole, 4 mg (0.12 mmol) of NaHCO₃ and 4 mg of Pd(tetrakis) added. The mixture was heated at 120°C for 700 s then filtrated through celite. Finally purification using preparative HPLC giving the product as a light brown gum in an amount of 0.9 mg (yield 3%).
HPLC (system A) 94 %, RT 1.874 min (10-97 % MeCN over 3 min)
HPLC (system B) 98 %, RT 1.748 min (10-97 % MeCN over 3 min)
MS (ESI⁺) for C₂₇H₂₈F₂N₆O *m*/*z* 491(M+H⁺)

### Example 57

### 3-fluoro-5-[6-(6-fluoro-1H-indol-2-yl)pyrazin-2-yl]-N-methyl-N-(1-methylpyrrolidin-3-yl)benzamide

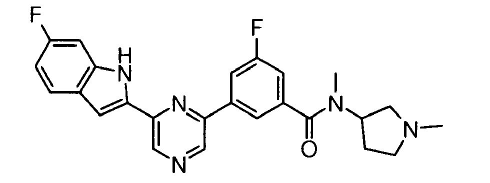

40 mg (0.18 mmol) of 3-bromo-5-fluoro benzoic acid was dissolved in 2 mL DMF and 21 mg (0.22 mmol) of N,N'-Dimethyl-3-aminopyrrolidine, 88 mg (0.27 mmol) TBTU and 22 mg (0.22 mmol) of TEA were added. The mixture was stirred at room temperature overnight, then concentrated and purified with preparative HPLC giving 3-bromo-5-fluoro-N-methyl-N-(1-methylpyrrolidin-3-yl)benzamide as a yellow gum in an amount of 81 mg.

20 mg (0.063 mmol) of 4-bromo-3-fluoro-N-methyl-N-(1-methylpyrrolidin-3-yl)benzamide, 22 mg (0.1 mmol) of bis(neopentyl glycolato) diboron, 30 mg (0.30 mmol) of KOAc and 4 mg of PdCl₂ were heated at 125°C for 900 s in microwave. To the same microwave tube were then 15 mg (0.06 mmol) of 2-(6-chloropyrazin-2-yl)-5-fluoro-1H-indole, 4 mg (0.12 mmol) of NaHCO₃ and 4 mg of Pd(tetrakis) added. The mixture was heated at 120°C for 700 s then filtrated through celite. Finally purification using preparative HPLC giving the product as a brown gum in an amount of 2.2 mg (yield 8%).
HPLC (system A) 95 %, RT 1.997 min (10-97 % MeCN over 3 min)
HPLC (system B) 97 %, RT 1.885 min (10-97 % MeCN over 3 min)
MS (ESI⁺) for C₂₅H₂₃F₂N₅O *m*/*z* 448(M+H⁺)

### Example 58

### 3-[5-amino-6-(4-hydroxyphenyl)pyrazin-2-yl]benzamide

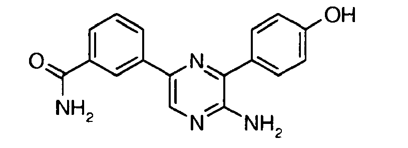

4-(2-amino-5-bromopyrazin-3-yl)phenol (15 mg, 0.06 mmol), (3-aminocarbonylphenyl)boronic acid (15 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5µm 19 x 50 mm, flow 25 ml/min, 50 mM pH10 NH₄HCO₃ / ACN, fractions collected based on UV-signal (254 nm). Yield: 10.6 mg (62%). Light yellow solid. MS (electronspray) M+H+ m/z 307.1.

### Example 59

### 4-[5-amino-6-(4-hydroxyphenyl)pyrazin-2-yl]-N-(2-furylmethyl)benzamide

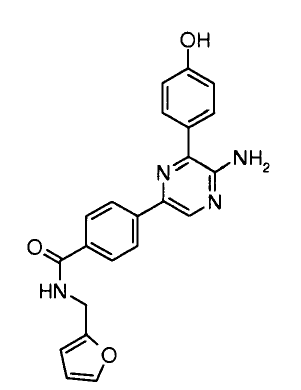

4-(2-amino-5-bromopyrazin-3-yl)phenol (15 mg, 0.06 mmol), ([4-(furfurylaminocarbonyl)phenyl]boronic acid (21 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5µm 19 x 50mm, flow 25 ml/min, 50 mM pH10 NH₄HCO₃ / ACN, fractions collected based on UV-signal (254 nm). Yield: 6.9 mg (33%). Light yellow solid. MS (electronspray) M+H+ m/z 387.0.

### Example 60

### 4-{3-amino-6-[3-(morpholin-4-ylcarbonyl)phenyl]pyrazin-2-yl}phenol

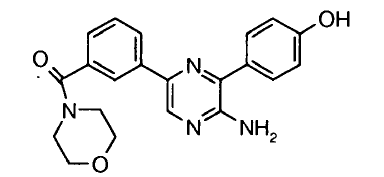

4-(2-amino-5-bromopyrazin-3-yl)phenol (15 mg, 0.06 mmol), [3-(morpholine-4-carbonyl)phenyl]boronic acid (20 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5 µm 19 x 50 mm, flow 25 ml/min, 50 mM pH10 NH₄HCO₃ / ACN, fractions collected based on UV-signal (254 nm) Yield: 12.0 mg (57%). Light yellow solid. MS (electronspray) M+H+ m/z 377.1

### Example 61

### 3-[5-amino-6-(4-hydroxyphenyl)pyrazin-2-yl]-N-(2-furylmethyl)benzamide

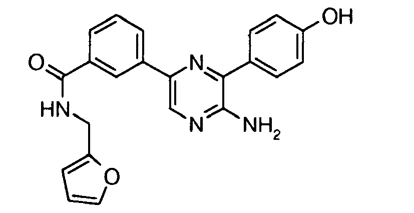

4-(2-amino-5-bromopyrazin-3-yl)phenol (15 mg, 0.06 mmol), [3-(Furfurylaminocarbonyl)phenyl]boronic acid (21 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5µm 19 x 50mm, flow 25 ml/min, 50 mM pH10 NH₄HCO₃ / ACN, fractions collected based on UV-signal (254 nm). Yield: 13.2 mg (61%). Light yellow solid. MS (electronspray) M+H+ m/z 387.0.

### Example 62

### 4-{3-amino-6-[4-(morpholin-4-ylcarbonyl)phenyl]pyrazin-2-yl}phenol

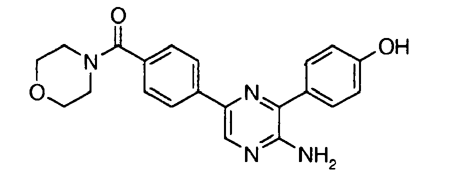

4-(2-amino-5-bromopyrazin-3-yl)phenol (15 mg, 0.06 mmol), [4-(Morpholine-4-carbonyl)phenyl]boronic acid (20.2 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5µm 19 x 50 mm, flow 25 ml/min, 50 mM pH10 NH₄HCO₃ / ACN, fractions collected based on UV-signal (254 nm). Yield: 3.2 mg (15%). Light yellow solid. MS (electronspray) M+H+ m/z 377.1.

### Intermediate 5

### 4-[5-amino-6-(4-hydroxyphenyl)pyrazin-2-yl]benzoic acid

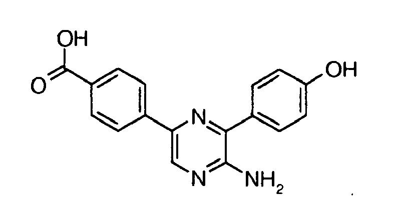

4-(2-amino-5-bromopyrazin-3-yl)phenol (15 mg, 0.06 mmol), (4-carboxybenzene boronic acid (21 mg, 0.086 mmol), tetrakis(triphenylphosphine)palladium (4 mg, 0.0034 mmol) and Na₂CO₃ (aq) (0.2 ml, 0.2 mmol) were suspended in DME (1 ml) and heated in the microwave to 120°C for 15 minutes. The mixture was evaporated and dissolved in MeOH (1 ml) and purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 8.8 mg (51%). Light yellow solid. MS (electronspray) M+H+ m/z 308.1.

### General method for the preparation of Examples 63-76:

4-[5-amino-6-(4-hydroxyphenyl)pyrazin-2-yl]benzoic acid (Intermediate 5) was dissolved in THF (15.5 ml) and (1 ml, 0.033 mmol) was pipetted into 15 individual vials. To each vial was added the requisite amine (0.066 mmol), triethylamine (10 mg, 0.1 mmol), hydroxybenzotriazole (6.76 mg, 0.050 mmol) and EDC (9.59 mg, 0.050 mmol). The reactions were stirred at room temperature overnight. The mixtures were evaporated and dissolved in MeOH water (9:1) (1.5 ml), filtered and purified as described below.

### Example 63

### 4-{3-amino-6-[4-(piperidin-1-ylcarbonyl)phenyl]pyrazin-2-yl}phenol

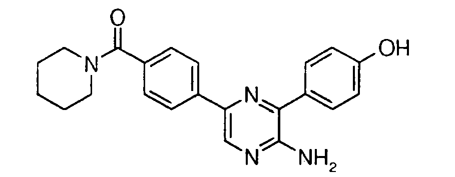

Amine: piperidine (5.6 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 5.6 mg (45%). Yellow solid.
HPLC 100% (system A. 10-97% MeCN over 3 minutes. MS (electronspray) M+H+ m/z 375.4.
1 H NMR (500 MHz, DMSO-D6) δ ppm 1.39 - 1.71 (m, *J*=3.65 Hz, 8 H) 2.73 - 3.10 (m, 2 H) 6.22 (s, 2 H) 6.90 (d, *J*=8.53 Hz, 2 H) 7.42 (d, *J*=7.92 Hz, 2 H) 7.65 (d, *J*=8.53 Hz, 2 H) 8.03 (d, *J*=8.53 Hz, 2 H) 8.52 (s, 1 H) 9.69 (s, 1 H)

### Example 64

### 4-[5-amino-6-(4-hydroxyphenyl)pyrazin-2-yl]-N-(2-morpholin-4-ylethyl)benzamide

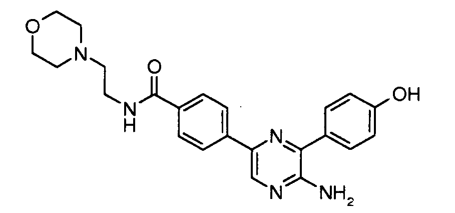

Amine: N-(2-aminoethyl)morpholine (8.6 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 9.2 mg (66%). Yellow solid. HPLC 100% (system A. 10-97% MeCN over 3 minutes. MS (electronspray) M+H+ m/z 420.4

### Example 65

### 4-[5-amino-6-(4-hydroxyphenyl)pyrazin-2-yl]-N-(3-morpholin-4-ylpropyl)benzamide

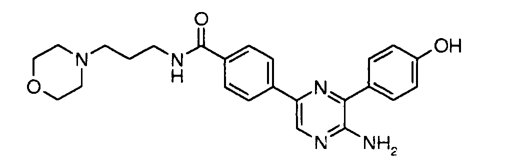

Amine: N-(3-aminopropyl)morpholine (9.5 mg). Purified by reversed phase preparative HPLC using XTerra Prep MS C18 5 µm 19 x 50mm, flow 25 ml/min, 50 mM pH 10 NH₄HCO₃ / ACN, fractions collected based on UV-signal (254 nm). Yield: 2.3 mg (16%). Yellow solid. HPLC 100% (system A. 10-97% MeCN over 3 minutes. MS (electronspray) M+H+ m/z 434.4.

### Example 66

### 4-[5-amino-6-(4-hydroxyphenyl)pyrazin-2-yl]-N-cyclopentylbenzamide

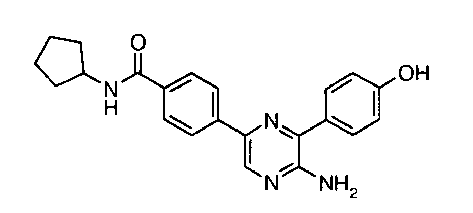

Amine: cyclopentylamine (5.6 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 6.7 mg (54%). Light yellow solid. HPLC 100% (system A. 10-97% MeCN over 3 minutes. MS (electronspray) M+H+ m/z 375.4.

### Example 67

### 4-[5-amino-6-(4-hydroxyphenyl)pyrazin-2-yl]-N-isopropylbenzamide

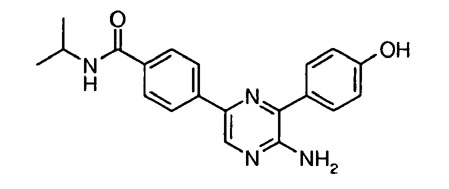

Amine: isopropylamine (3.9 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 6.7 mg (58%). Yellow solid. HPLC 100% (system A. 10-97% MeCN over 3 minutes. MS (electronspray) M+H+ m/z 349.4. 1H NMR (500 MHz, DMSO-D6) δ ppm 1.18 (d, *J*=6.70 Hz, 6 H) 4.07 - 4.16 (m, 1 H) 6.25 (s, 2 H) 6.90 (d, *J*=8.53 Hz, 2 H) 7.64 (d, *J*=8.53 Hz, 2 H) 7.90 (d, *J*=8.53 Hz, 2 H) 8.04 (d, *J*=7.92 Hz, 2 H) 8.17 (d, *J*=7.92 Hz, 1 H) 8.55 (s, 1 H) 9.70 (s, 1 H).

### Example 68

### 4-(3-amino-6-{4-[(4-methylpiperazin-1-yl)carbonyl]phenyl}pyrazin-2-yl)phenol

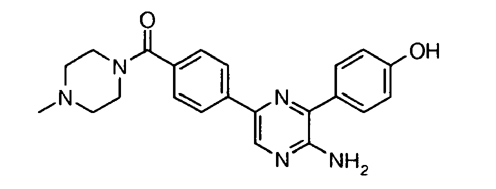

Amine: 1-methylpiperazine (6.6 mg). Purified by reversed phase preparative HPLC using XTerra Prep MS C18 5 µm 19 x 50mm, flow 25 ml/min, 50 mM pH 10 NH₄HCO₃ / ACN, fractions collected based on UV-signal (254 nm). Yield: 8.1 mg (63%). Yellow solid. HPLC 100% (system A. 10-97% MeCN over 3 minutes.MS (electronspray) M+H+ m/z 390.4.

### Example 69

### 4-[5-amino-6-(4-hydroxyphenyl)pyrazin-2-yl]-N-ethyl-N-(2-hydroxyethyl)benzamide

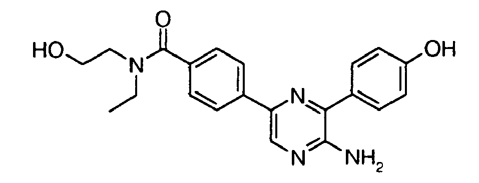

Amine: 2-(ethylamino)ethanol (5.9 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 0.9 mg (7%). Light yellow solid. HPLC 95% (system A. 10-97% MeCN over 3 minutes. MS (electronspray) M+H+ m/z 379.4.

### Example 70

### 4-[3-amino-6-(4-{[4-(hydroxymethyl)piperidin-1-yl]carbonyl}phenyl)pyrazin-2-yl]phenol

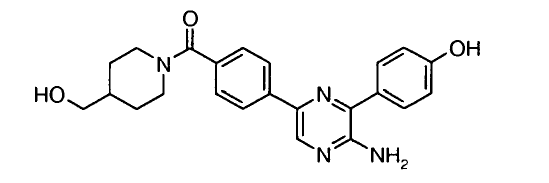

Amine: 4-piperidinemethanol (7.6 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 5.6 mg (45%). Light yellow solid. HPLC 95% (system A. 10-97% MeCN over 3 minutes .MS (electronspray) M+H+ m/z 375.4

### Example 71

### R-4-[5-amino-6-(4-hydroxyphenyl)pyrazin-2-yl]-N-[1-(hydroxymethyl)-2-methylpropyl]benzamide

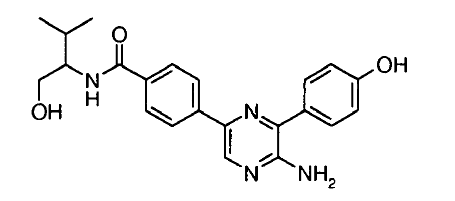

Amine: R-2-amino-3-methyl-1-butanol (6.8 mg). Purified by reversed phase preparative HPLC using XTerra Prep MS C18 5 µm 19 x 50 mm, flow 25 ml/min, 50 mM pH10 NH₄HCO₃ / ACN, fractions collected based on UV-signal (254 nm). Yield: 7.1 mg (55%). Yellow solid. HPLC 99% (system A. 10-97% MeCN over 3 minutes. MS (electronspray) M+H+ m/z 393.4.

### Example 72

### 4-{3-amino-6-[4-({4-[3-(dimethylamino)propyl]piperazin-1-yl}carbonyl)phenyl]pyrazin-2-yl}phenol

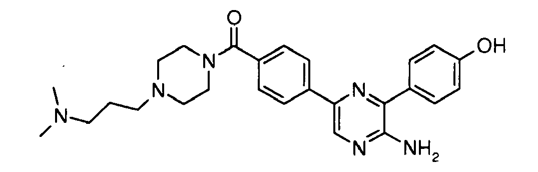

Amine: 1-(3-dimethylaminopropyl)-piperazine (11.3 mg). Purified by reversed phase preparative HPLC using XTerra Prep MS C 18 5 µm 19 x 50 mm, flow 25 ml/min, 50 mM pH10 NH₄HCO₃ / ACN, fractions collected based on UV-signal (254 nm). Yield: 6.2 mg (41%). Yellow solid. HPLC 100% (system A. 10-97% MeCN over 3 minutes. MS (electronspray) M+H+ m/z 461.4.

### Example 73

### 4-[3-amino-6-(4-{[4-(2-furoyl)piperazin-1-yl]carbonyl}phenyl)pyrazin-2-yl]phenol

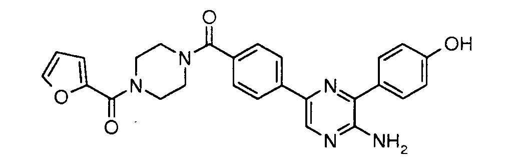

Amine: 1-(2-furoyl)-piperazine (11.9 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm).

Yield: 8.2 mg (53%). Off-white solid. HPLC 99% (system A. 10-97% MeCN over 3 minutes. MS (electronspray) M+H+ m/z 470.4.
1H NMR (500 MHz, DMSO-D6) δ ppm 3.42 - 3.87 (m, 8 H) 6.25 (s, 2 H) 6.63 (s, 1 H) 6.90 (d, *J*=7.92 Hz, 2 H) 7.02 (d, *J*=3.05 Hz, 1 H) 7.51 (d, *J*=8.53 Hz, 2 H) 7.63 - 7.69 (m, *J*=8.53 Hz, 2 H) 7.81 - 7.87 (m, 1 H) 8.06 (d, *J*=7.92 Hz, 2 H) 8.54 (s, 1 H) 9.70 (s, 1 H).

### Example 74

### 4-(3-amino-6-{4-[(4-cyclohexylpiperazin-1-yl)carbonyl]phenyl}pyrazin-2-yl)phenol

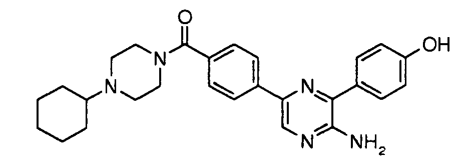

Amine: 1-cyclohexylpiperazine (11.1 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 7.6 mg (50%). Yellow solid. HPLC 99% (system A. 10-97% MeCN over 3 minutes. MS (electronspray) M+H+ m/z 458.4.

### Example 75

### 4-[5-amino-6-(4-hydroxyphenyl)pyrazin-2-yl]-N-benzyl-N-[2-(dimethylamino)ethyl]benzamide

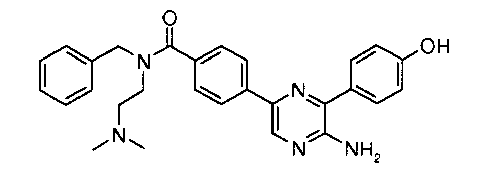

Amine: N-benzyl-N,N-dimethylethylendiamine (11.8 mg). Purified by reversed phase preparative HPLC using XTerra Prep MS C18 5 µm 19 x 50 mm, flow 25 ml/min, 50 mM pH10 NH₄HCO₃ / ACN, fractions collected based on UV-signal (254 nm). Yield: 7.6 mg (51 %). Yellow solid. HPLC 94% (system A. 10-97% MeCN over 3 minutes. MS (electronspray) M+H+ m/z 468.4.

### Example 76

### 4-[5-amino-6-(4-hydroxyphenyl)pyrazin-2-yl]-N,N-dimethylbenzamide

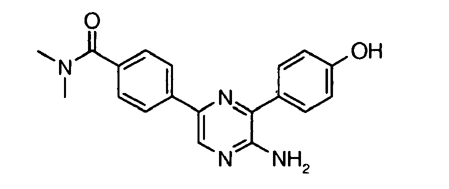

Amine: Dimethylamine hydrochloride (5.4 mg). Purified by reversed phase preparative HPLC using XTerra Prep MS C18 5 µm 19 x 50mm, flow 25 ml/min, 50 mM pH10 NH₄HCO₃ / ACN, fractions collected based on UV-signal (254 nm). Yield: 9.5 mg (86%). Light yellow solid. HPLC 100% (system A. 10-97% MeCN over 3 minutes. MS (electronspray) M+H+ m/z 335.4.
1H NMR (500 MHz, DMSO-D6) δ ppm 2.97 (s, 6 H) 6.22 (s, 2 H) 6.90 (d, *J*=8.53 Hz, 2 H) 7.46 (d, *J*=8.53 Hz, 2 H) 7.65 (d, *J*=8.53 Hz, 2 H) 8.03 (d, *J*=7.92 Hz, 2 H) 8.52 (s, 1 H) 9.69 (s, 1 H).

### Example 77

### 4-{6-[4-(morpholin-4-ylcarbonyl)phenyl]pyrazin-2-yl}phenol

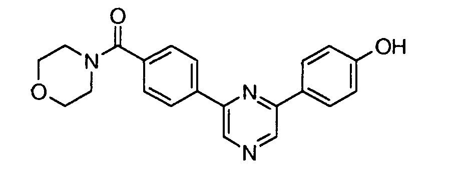

4-(6-chloropyrazin-2-yl)phenol (200 mg, 0.97 mmol), 4-(Morpholine-4-carbonyl)phenyl]boronic acid, (300 mg, 1.27 mmol), tetrakis(triphenylphosphine)paliadium (20 mg, 0.017), NaHCO₃ (200 mg, 2.38 mmol) and water (1 ml) were suspended in DME (3 ml) and heated to 130°C for 10 minutes in the microwave. The mixture was diluted with dichloromethane, filtered and transferred to a separation funnel. The organic phase was washed with water (1x), brine (1x), dried (MgSO₄) and evaporated. The crude product was purified by suspending it with dichloromethane (sparingly soluble) and filtered. The grey powder was washed with dichloromethane (2x) and dried in vacuo. Yield 118 mg (33%). Grey solid. HPLC 96% Rt=1.74 (system A. 10-97% MeCN over 3 minutes). HPLC 95% Rt=1.62 (system B. 10-97% MeCN over 3 minutes). MS (electronspray; [M+H]+) m/z 362.4.
1H NMR (400 MHz, DMSO-D6) δ ppm 3.49 - 3.75 (m, 6 H) 6.93 (d, *J*=8.78 Hz, 2 H) 7.58 (d, *J*=8.28 Hz, 2 H) 8.13 (d, *J*=8.78 Hz, 2 H) 8.30 (d, *J*=8.53 Hz, 2 H) 9.13 (s, 2 H) 9.95 (s, 1 H).

### Intermediate 6

### 5-bromopyrazin-2-amine

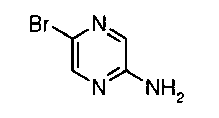

NBS (45 g, 253 mmol) was added in portions to a suspention of 2-aminopyrazine (25 g, 263 mmol) in dichloromethane (500 ml) over a period of 2h. The mixture was filtered and evaporated. The residue was suspended in dichloromethane (60 ml) and stirred for 10 minutes before hexane (60 ml) was added. The mixture was stirred vigorously for 15 minutes and filtrated. The yellow powder was washed with CH₂Cl₂/hexane 1:1 (3x). The solid was dissolved in diethylether and washed with water (3x), dried (mgso4) and evaporated. yield: 15.0 g (33%). Light yellow solid. HPLC 95% Rt=1.04 (system A. 10-97% MeCN over 3 minutes). HPLC 95% Rt=0.78 (system B. 10-97% MeCN over 3 minutes). MS (elecronspray; [M+H}+ 174.4. 1H NMR (400 MHz, CHLOROFORM-D) δ ppm 4.60 (s, 2 H) 7.76 (d, *J*=1.51 Hz, 1 H) 8.08 (d, *J*=1.51 Hz, 1 H).

### Intermediate 7

### 5-[4-(morpholin-4-ylcarbonyl)phenyl]pyrazin-2-amine

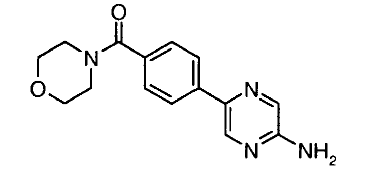

5-bromo-2-aminopyrazine (100 mg, 0.57 mmol), 4-(morpholine-4-carbonyl)phenyl]boronic acid-pinacol ester, (220 mg, 0.69 mmol), tetrakis(triphenylphosphine)palladium (10 mg, 0.0086), NaHCO₃ (70 mg, 0.833 mmol) and water (2 ml) were suspended in DME (1 ml) and heated to 140°C for 10 minutes in the microwave. The mixture was evaporated and the residue was partitioned between water and dichloromethane and extracted with dichloromethane (2x). The combined organics were washed with brine and evaporated. The crude product was purified by flashchromatography using 2.5%-5% MeOH in dichloromethane as the eluent.
Yield 90 mg (55%). Light yellow solid. HPLC 100% (system A. 10-97% MeCN over 3 minutes). HPLC 100% (system B. 10-97% MeCN over 3 minutes).
MS (electronspray) M+H+ m/z 285.1
1H NMR (400 MHz, CHLOROFORM-D) δ ppm 3.49 - 3.87 (m, 8 H) 4.66 (s, 2 H) 7.46 - 7.51 (m, 2 H) 7.90 - 7.95 (m, 2 H) 8.06 (d, *J*=1.51 Hz, 1 H) 8.46 (d, *J*=1.51 Hz, 1 H)

### Intermediate 8

### 3-bromo-5-[4-(morpholin-4-ylcarbonyl)phenyl]pyrazin-2-amine

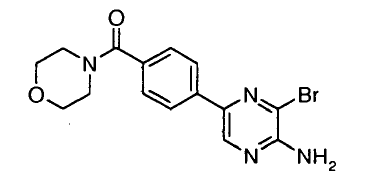

A solution of NBS (0.95 g, 5.3 mmol) in DMF (30 ml) was added dropwise to a solution of 5-[4-(morpholin-4-ylcarbonyl)phenyl]pyrazin-2-amine (1.4 g, 4.9 mmol) in DMF (55 ml) at room temperature. The mixture was diluted with 110 ml of water and stirred for 1 hour (precepitation). The red solid was filtered and washed with diethylether (2x) and redissolved in dichloromethane (300 ml). The organic phase was extracted with a mixture of brine and 1N Na₂CO₃ 1:1, dried (MgSO₄) and evaporated. The crude product was suspended in dichloromethane (20 ml) and 100 ml diethylether was added. The powder was filtered and washed with diethylether (2x) and dried in vacuo.
Yield 0.95 g (53%). Yellow solid. HPLC 98% (system A. 10-97% MeCN over 3 minutes). HPLC 98% (system B. 10-97% MeCN over 3 minutes). MS (electronspray; [M+H]+) m/z 363.4.
1H NMR (400 MHz, DMSO-D6) δ ppm 3.38 - 3.79 (m, 8 H) 6.97 (s, 2 H) 7.47 (d, *J*=8.53 Hz, 2 H) 7.95 (d, *J*=8.53 Hz, 2 H) 8.64 (s, 1 H).

### General procedure for the synthesis of Examples 78 and 79:

3-bromo-5-[4-(morpholin-4-ylcarbonyl)phenyl]pyrazin-2-amine (15 mg, 0.041 mmol), the requisite boronic acid, (0.050 mmol), tetrakis(triphenylphosphine)palladium (3 mg, 0.0026 mmol), NaHCO₃ (10 mg, 0.12 mmol) and water (0.5 ml) were suspended in DME (1 ml) and heated to 120°C for 10 minutes in the microwave.

### Example 78

### 4-{3-amino-6-[4-(morpholin-4-ylcarbonyl)phenyl]pyrazin-2-yl}-2-methoxyphenol

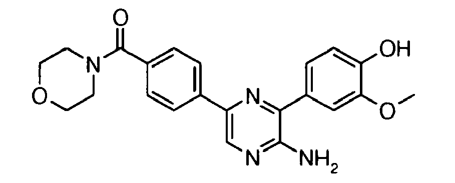

Boronic acid: 2-methoxy-4-(4,4,5,5-tetramethyl-1,3,2-dioxaborolan-2-yl)phenol (12.5 mg). The crude product was purified by XTerra Prep MS C18 5 µm 19 x 50mm, flow 25 ml/min, 50 mM pH10 NH₄HCO₃ / ACN, fractions collected based on UV-signal (254 nm). Yield 1.7 mg (10%). Brown solid. HPLC purity 95%. MS (electronspray) M+H+ m/z 407.4.

### Example 79

### 3-(1-benzofuran-2-yl)-5-[4-(morpholin-4-ylcarbonyl)phenyl]pyrazin-2-amine

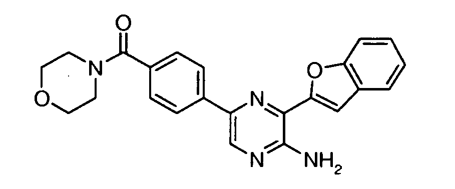

Boronic acid: benzo[b]furan-2-boronic acid (8.1 mg). The crude product was purified by XTerra Prep MS C 18 5 µm 19 x 50mm, flow 25 ml/min, 50 mM pH10 NH₄HCO₃ / ACN, fractions collected based on UV-signal (254 nm). Yield 3.2 mg (19%). Yellow solid. HPLC purity 100%. MS (electronspray) M+H+ m/z 401.4.

### General procedure for the synthesis of Examples 80, 81 and 82:

4-(2-amino-5-bromopyrazin-3-yl)phenol (10 mg, 0.038 mmol), requisite boronic acid, (0.050 mmol), tetrakis(triphenylphosphine)palladium (2 mg, 0.0026 mmol), NaHCO₃ (10 mg, 0.12 mmol) and water (0.5 ml) were suspended in DME (1 ml) and heated to 120°C for 15 minutes in the microwave. The mixtures were evaporated and dissolved in MeOH water (9:1) (1.5 ml), filtered and purified as described below.

### Example 80

### 4-{3-amino-6-[3-chloro-4-(morpholin-4-ylcarbonyl)phenyl]pyrazin-2-yl}phenol

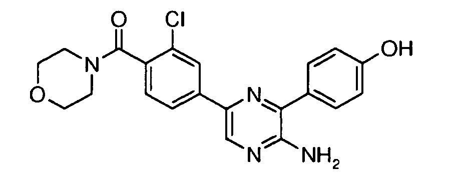

Boronic acid: 3-chloro-4-(morpholine-4-carbonyl)phenyl]boronic acid (13.47 mg, 0.05 mmol). Purified by reversed phase preparative HPLC using ACE Prep 5 C8 5 µm 50 x 21.2 mm, flow 25 ml/min, pH 7, 1% NH₄OAc / ACN, fractions collected based on UV-signal (254 nm). Yield: 2.0 mg (13%). Light yellow solid. HPLC 99% (system A. 10-97% MeCN over 3 minutes). HPLC 99% (system B. 10-97% MeCN over 3 minutes). MS (electronspray; [M+H]+) m/z 411.4.

### Example 81

### 4-{3-amino-6-[4-(morpholin-4-ylsulphonyl)phenyl]pyrazin-2-yl}phenol

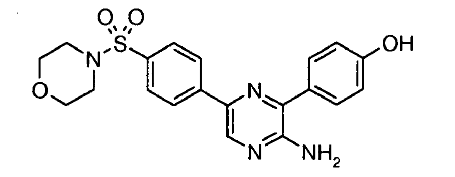

Boronic acid: 4-(N-Morpholinylsulphonamidophenyl)boronic acid (13.6 mg, 0.05 mmol). Purified by reversed phase preparative HPLC using ACE Prep 5 C8 5 µm 50 x 21.2 mm, flow 25 ml/min, pH7, 1% NH₄OAc / ACN, fractions collected based on UV-signal (254 nm). Yield: 2.3 mg (15%). Light yellow solid. HPLC 98% (system A. 10-97% MeCN over 3 minutes). HPLC 98% (system B. 10-97% MeCN over 3 minutes). MS (electronspray; [M+H]+) m/z 413.4.

### Example 82

### 4-{3-amino-6-[4-(thiomorpholin-4-ylsulphonyl)phenyl]pyrazin-2-yl}phenol

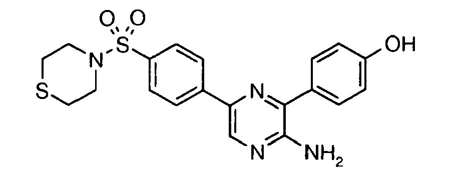

Boronic acid: 4-(N-thiomorpholinylsulphonamidophenyl)boronic acid (14.4 mg, 0.05 mmol). Purified by reversed phase preparative HPLC using ACE Prep 5 C8 5 µm 50 x 21.2 mm, flow 25 ml/min, pH 7, 1% NH₄OAc / ACN, fractions collected based on UV-signal (254 nm). Yield: 0.8 mg (5%). Light yellow solid. HPLC 90% (system A. 10-97% MeCN over 3 minutes). HPLC 90% (system B. 10-97% MeCN over 3 minutes). MS (electronspray; [M+H]+) m/z 429.4.

### General procedure for the synthesis of Examples 83, 84 and 85:

A stock solution of the 4-(6-chloropyrazin-2-yl)phenol was made by dissolving 210 mg in 14 ml DME and 1 ml was added to each vial (15 mg, 0.073 mmol). A stock solution was made by dissolving 260 mg NaHCO₃ in 6.76 ml H2O and 0.5 ml (0.24 mmol) was added to each vial. followed by the requisite boronic acid (0.1 mmol) and tetrakis(triphenylphosphine)palladium (3 mg, 0.0026 mmol) The mixtures were heated 130°C for 600 s, evaporated and dissolved in MeOH water (9:1) (1.5 ml), filtered and purified as described below.

### Example 83

### 4-{6-[4-(morpholin-4-ylsulphonyl)phenyl]pyrazin-2-yl}phenol

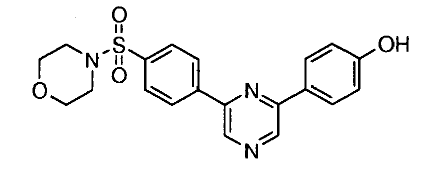

Boronic acid: 4-(N-Morpholinylsulphonamidophenyl)boronic acid (27 mg, 0.1 mmol).
Purified by reversed phase preparative HPLC using ACE Prep UV C 18 5 µm 19 x 50 mm, flow 25 ml/min, 50 mM pH 7 NH₄OAc / ACN, fraction collected based on UV-signal (254 nm). Yield 4.0 mg (14%). White solid. MS (electronspray) M+H+ m/z 398.4.

### Example 84

### 4-{6-[3-chloro-4-(morpholin-4-ylcarbonyl)phenyl]pyrazin-2-yl}phenol

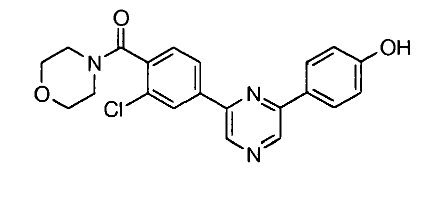

Boronic acid: N-Morpholinyl 2-chloro-4-boronobenzamide (26.9 mg, 0.1 mmol). Purified by reversed phase preparative HPLC using ACE Prep UV C 18 5 µm 19 x 50 mm, flow 25 ml/min, 50 mM pH 7 NH₄OAc / ACN, fractions collected based on UV-signal (254 nm). Yield 7.1 mg (25%). White solid. MS (electronspray) M+H+ m/z 396.6.

### Example 85

### 4-[6-(4-hydroxyphenyl)pyrazin-2-yl]benzamide

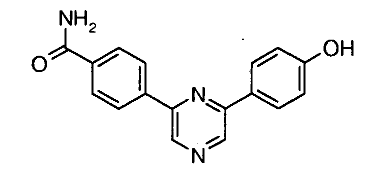

Boronic acid: 4-Aminocarbonylphenylboronic acid (16.5 mg, 0.1 mmol). Purified by reversed phase preparative HPLC using ACE Prep UV C 18 5 µm 19 x 50mm, flow 25 ml/min, 50 mM pH 7 NH₄OAc / ACN, fractions collected based on UV-signal (254 nm). Yield 0.7 mg (3%). White solid. MS (electronspray) M+H+ m/z 292.4.

### Example 86

### 4-{3-amino-6-[4-(morpholin-4-ylcarbonyl)phenyl]pyrazin-2-yl}-2-fluorophenol

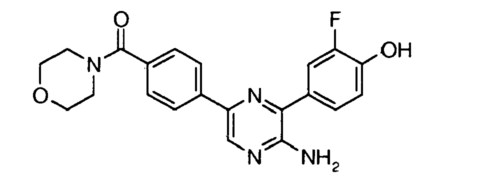

3-bromo-5-[4-(morpholin-4-ylcarbonyl)phenyl]pyrazin-2-amine (Intermediate 8) (10 mg, 0.028 mmol), 3-fluoro-4-hydroxyboronic acid (7.8 mg, 0.050 mmol), tetrakis(triphenylphosphine)palladium (3 mg, 0.0026 mmol), NaHCO₃ (10 mg, 0.12 mmol) and water (0.5 ml) were suspended in DME (1 ml) and heated to 120°C for 10 minutes in the microwave. The mixtures were evaporated and dissolved in MeOH water (9: 1) (1.5 ml), filtered and purified by reversed phase preparative HPLC using ACE Prep UV C18 5 µm 19 x 50mm, flow 25 ml/min, 50 mM pH 7 NH₄OAc / ACN, fractions collected based on UV-signal (254 nm). Yield 3.2 mg (29%). Yellow solid. MS (electronspray) M+H+ m/z 395.4. 1H NMR (400 MHz, CHLOROFORM-D) δ ppm 2.09 (s, 1 H) 3.38 - 3.91 (m, 8 H) 5.14 (s, 2 H) 7.12 (t, *J*=8.53 Hz, 1 H) 7.47 - 7.54 (m, 3 H) 7.58 (dd, *J*=11.29, 2.01 Hz, 1 H) 7.97 - 8.01 (m, 2 H) 8.39 (s, 1 H).

### General procedure for the synthesis of Examples 87 and 88:

4-[4-(6-chloropyrazin-2-yl)benzoyl]morpholine (10 mg, 0.033 mmol), the requisite boronic acid, (0.050 mmol), tetrakis(triphenylphosphine)palladium (3 mg, 0.0026 mmol), NaHCO₃ (10 mg, 0.12 mmol) and water (0.2 ml) were suspended in DME (1 ml) and heated to 120°C for 10 minutes in the microwave. The mixtures were evaporated and dissolved in MeOH water (9:1) (1.5 ml), filtered and purified as described below.

### Example 87

### 3-{6-[4-(morpholin-4-ylcarbonyl)phenyl]pyrazin-2-yl}phenol

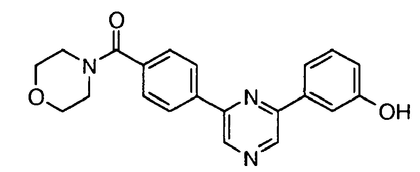

Boronic acid: 3-hydroxyphenylboronic acid (6.9 mg, 0.050 mmol). Purified by reversed phase preparative HPLC using ACE Prep UV C18 5 µm 19 x 50 mm, flow 25 ml/min, 25 mM pH10 NH₄CO₃ / ACN, fractions collected based on UV-signal (254 nm). Yield 3.6 mg (30%). White solid. HPLC 95% Rt=1.79 (system A. 10-97% MeCN over 3 minutes). HPLC 95% Rt=1.67(system B. 10-97% MeCN over 3 minutes). MS (electronspray) M+H+ m/z 362.4.
1H NMR (500 MHz, DMSO-D6) δ ppm 3.41 - 3.77 (m, 9 H) 6.93 (d, *J*=7.31 Hz, 1 H) 7.34 - 7.40 (m, 1 H) 7.61 (d, *J*=7.92 Hz, 2 H) 7.65 - 7.70 (m, 2 H) 8.32 (d, *J*=8.53 Hz, 2 H) 9.16 (s, 1 H) 9.23 (s, 1 H).

### Example 88

### (2-hydroxy-5-{6-[4-(morpholin-4-ylcarbonyl)phenyl]pyrazin-2-yl}phenyl)(phenyl)methanone

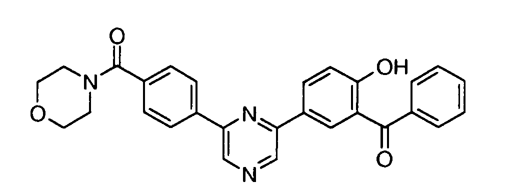

Boronic acid: [2-hydroxy-5-(4,4,5,5-tetramethyl-1,3,2-dioxaborolan-2-yl)phenyl](phenyl)methanone (16.2 mg, 0.05 mmol). Purified by reversed phase preparative HPLC using ACE Prep UV C18 5 µm 19x50mm, flow 25 ml/min, 25 mM pH10 NH₄CO₃ / ACN, fractions collected based on UV-signal (254 nm). Yield 5.4 mg (35%). White solid. HPLC 95% Rt=2.36 (system A. 10-97% MeCN over 3 minutes). HPLC 95% Rt=2.39 (system B. 10-97% MeCN over 3 minutes). MS (electronspray) M+H+ m/z 466.4.
1H NMR (500 MHz, DMSO-D6) δ ppm 3.42 - 3.77 (m, 9 H) 7.15 (d, *J*=8.53 Hz, 1 H) 7.53 - 7.60 (m, 4 H) 7.68 (t, *J*=7.31 Hz, 1 H) 7.81 (d, *J*=7.31 Hz, 2 H) 8.25 - 8.31 (m, *J*=7.92 Hz, 3 H) 8.35 (d, *J*=9.14 Hz, 1 H) 9.18 (d, *J*=6.70 Hz, 2 H).

### Intermediate 9

### 3-(1-benzofuran-2-yl)-5-bromopyrazin-2-amine

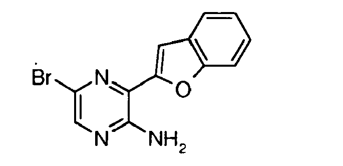

3,5-dibromo-2-aminopyrazine (100 mg, 0.395 mmol), benzo[b]furan-2-boronic acid, (72.9 mg, 0.45 mmol), tetrakis(triphenylphosphine)palladium (5 mg, 0.0043 mmol), NaHCO₃ (126 mg, 1.5 mmol) and water (1 ml) were suspended in DME (1 ml) and heated to 120°C for 10 minutes in the microwave. The crude product was evaporated, dissolved in MeOH and purified by preparative HPLC. Purified by reversed phase preparative HPLC using ACE Prep UV C 18 5 µm 19 x 50mm, flow 25 ml/min, 25 mM pH10 NH₄CO₃ / ACN, fractions collected based on UV-signal (254 nm). Yield 7.2 mg (15%). Light yellow solid. HPLC 95% Rt=1.5 (system A. 10-97% MeCN over 3 minutes). HPLC 95% Rt=2.31 (system B. 10-97% MeCN over 3 minutes). MS (electronspray) M+H+ m/z 291.4.
1H NMR (400 MHz, CHLOROFORM-D) δ ppm 5.74 (s, 2 H) 7.27 - 7.40 (m, 2 H) 7.51 - 7.56 (m, 2 H) 7.65 (d, *J*=7.53 Hz, 1 H) 8.06 (s, 1 H).

### Example 89

### 3-(1-benzofuran-2-yl)-5-{4-[(4-methylpiperazin-1-yl)carbonyl]phenyl}pyrazin-2-amine

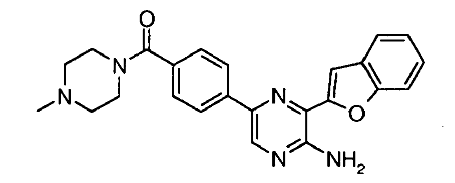

3-(1-benzofuran-2-yl)-5-bromopyrazin-2-amine (7.2 mg, 0.025 mmol), 1-methyl-4-[4-(4,4,5,5-tetramethyl-1,3,2-dioxaborolan-2-yl)benzoyl]piperazine, (16.5 mg, 0.05 mmol), tetrakis(triphenylphosphine)palladium (2 mg, 0.0017 mmol), NaHCO₃ (8.4 mg, 0.1 mmol) and water (0.1 ml) were suspended in DME (1 ml) and heated to 120°C for 10 minutes in the microwave. Additional Pd was added (1 mg) and the mixture heated at 120°C for 5 minutes. The mixture was evaporated, dissolved in MeOH and filtered. Purified by reversed phase preparative HPLC using ACE Prep UV C18 5 µm 19 x 50mm, flow 25 ml/min, 25 mM pH 10 NH₄CO₃ / ACN, fractions collected based on UV-signal (254 nm). Yield 2.2 mg (21%). Light yellow solid. HPLC 99% Rt=1.80 (system A. 10-97% MeCN over 3 minutes). HPLC 99% Rt=1.61 (system B. 10-97% MeCN over 3 minutes). MS (electronspray) M+H+ m/z 413.4.
1H NMR (400 MHz, CHLOROFORM-D) δ ppm 2.33 (s, 3 H) 2.36 - 2.55 (m, *J*=39.15 Hz, 4 H) 3.44 - 3.57 (m, 2 H) 3.76 - 3.90 (m, 2 H) 5.85 (s, 2 H) 7.28 - 7.40 (m, 2 H) 7.49 - 7.59 (m, 3 H) 7.63 - 7.70 (m, 2 H) 8.04 (d, *J*=8.53 Hz, 2 H) 8.50 (s, 1 H).

### Intermidiate 10

### Sodium 4-[5-(1H-Indol-2-yl)pyridin-3-yl]benzoate

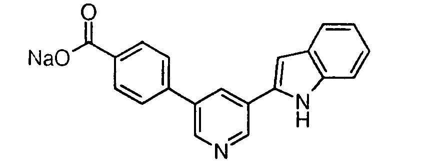

*Step i*) A mixture of 3,5-dibromo pyridine (250 mg, 1.05 mmol 300 mg) *N*-Boc-indol-2-yl boronic acid (300 mg, 1.15 mmol), palladium, tetrakis(triphenylphosphine) (6 mg), sodium bicarbonate (113 mg, 3.6 mmol) in DME (3.5 ml) and water (1 ml) was heated in a microwave reactor at 120°C for 900 s to effect the Suzuki coupling. The material was further heated in the microwave to 150°C for 300 s, and subsequently to 180°C for a further 300 s to effect the removal of the BOC-group. Organic phase from the two phase reaction mixture was removed and reduced in vacuo before purification of the crude material by preparative HPLC to give 2-(5-bromopyridin-3-yl)-1*H*-indole trifluoroacetate 210 mg, 52%.
*Step ii*) A mixture of 2-(5-bromopyridin-3-yl)-1*H*-indole trifluoroacetate (100 mg, 0.26 mmol), 4-carboxy-phenylboronic acid (47 mg, 0.28 mmol), NaHCO₃ (84 mg, 1 mmol) and palladium tetrakis(triphenylphosphine) (6 mg. 0.005 mmol) in DME (3.5 mL) and water (1 mL) were heated to 120°C in the microwave for 600 s. The reaction mixture was diluted with methanol and filtered through celite to remove insoluble salt before concentrating in vacuo. The material was used without further purification and thus exisited as Na+ salt.

### Example 90

### 2-{5-[4-(morpholin-4-ylcarbonyl)phenyl]pyridin-3-yl}-1H-indole trifluoroacetate

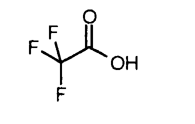

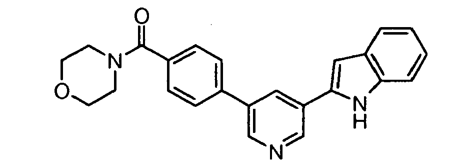

The title compound was synthesised using general procedure 3 starting from crude sodium 4-[5-(1*H*-Indol-2-yl)pyridin-3-yl]benzoate (Intermediate 10) (10 mg 0.03 mmol), morpholine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (3.7 mg, 25%). HPLC 98% R_{T}=1.95 (System A 10-97% MeCN over 3 minutes).
MS (electrospray) m/z (M+H+) 384.

### Example 91

### 2-(5-{4-[(4-methylpiperazin-1-yl)carbonyl]phenyl}pyridin-3-yl)-1H-indole trifluoroacetate

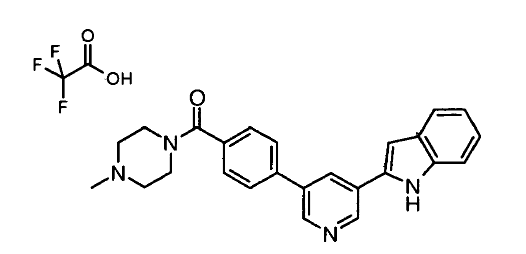

The title compound was synthesised using general procedure 3 starting from crude sodium 4-[5-(1*H*-Indol-2-yl)pyridin-3-yl] benzoate (Intermediate 10) (10 mg 0.03 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.4 mg, 29%).
HPLC 99% R_{T}=1.96 (System A 10-97% MeCN over 3 minutes).
MS (electrospray) m/z ½(M+2H+ ) 199 (100%), (M+H+) 397 (80%).

### Example 92

### N-[2-(diethylamino)ethyl]-4-[5-(1H-indol-2-yl)pyridin-3-yl]benzamide trifluoroacetate

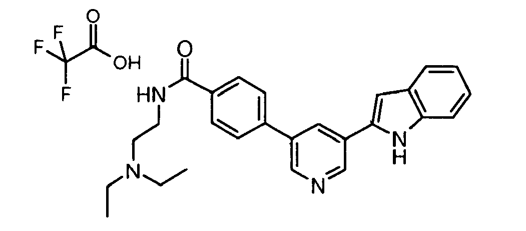

The title compound was synthesised from crude sodium 4-[5-(1*H*-Indol-2-yl)pyridin-3-yl] benzoate (Intermediate 10) (10 mg 0.03 mmol), *N,N-*diethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.5 mg, 29%). HPLC 97% R_{T}=2.09 (System A 10-97% MeCN over 3 minutes).
MS (electrospray) m/z ½(M+2H+ ) 207, (M+H+) 413.

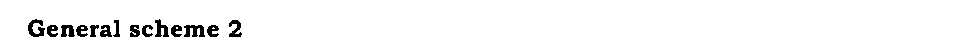

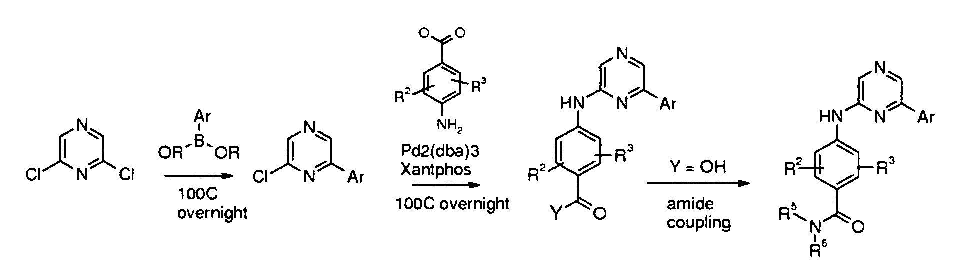

### Example 93

### N-{2-(Diethylamino)ethyl]-4-{[6-(4-hydroxyphenyl)pyrazin-2-yl]amino}benzamide

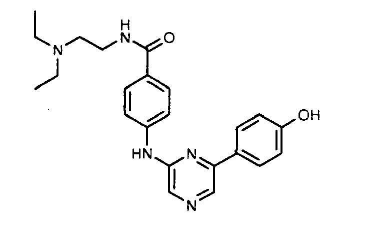

To a test tube were added 4-(6-chloropyrazin-2-yl)phenol (19 mg, 0.0920 mmol), 4-amino-*N*-[2-(diethylamino)ethyl]benzamide (23 mg, 0.101 mmol), Pd₂(dba)₃ (3 mg, 0.00368 mmol), Xantphos (4 mg, 0.00736 mmol), Na*^{t}*BuO (12 mg, 0.129 mmol) and 1,4-dioxane (3 ml). The reaction was heated in a Stemblock at 100°C over weekend, then purified by preparative HPLC. Yield 5%, HPLC purity = 98%, m/z = 312 (M+H)+.
Yield 17%, HPLC purity = 100%, m/z = 406 (M+H)+.

### Intermediate 11

### 4-{[6-(4-Hydroxyphenyl)pyrazin-2-yl]amino}benzoic acid

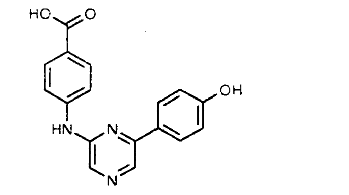

The title compound was prepared according to the same procedure as described for Example 93. Yield 84%, HPLC purity = 75%, m/z = 308 (M+H)+.

### Example 94

### N-[2-(Dimethylamino)ethyl]-4-{[6-(4-hydroxyphenyl)pyrazin-2-yl]amino}benzamide

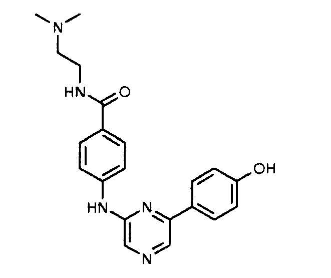

To a tube containing 4-amino-*N*-[2-(dimethylamino)ethyl]benzamide (28 mg, 0.137 mmol) were added 4-[6-(4-hydroxyphenyl)pyrazin-2-yl]benzoic acid (20 mg, 0.0684 mmol in 2 ml dry THF) and triethylamine (38 ml, 0.274 mmol) . After a few minutes HOBT (19 mg, 0.137 mmol) and EDC (26 mg, 0.137 mmol) were added to the solution and it was stirred at R.T. overnight. Purified by preparative HPLC. Yield 13%, HPLC purity = 100%, m/z = 378 (M+H)+.

### Example 95

### 4-{[6-(4-Hydroxyphenyl)pyrazin-2-yl]aminol}-N-(2-pyrrolidin-1-ylethyl)benzamide

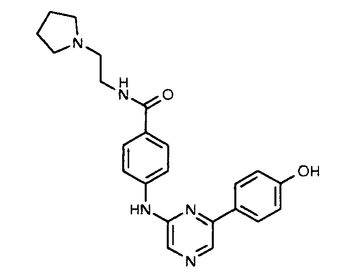

The title compound was prepared according to the same procedure as described for Example 94. Yield 36%, HPLC purity = 100%, m/z = 404 (M+H)⁺.

### Example 96

### 4-{[6-(4-Hydroayphenyl)pyrazin-2-yl]amino}-N-(pyridin-2-ylmethyl)benzamide

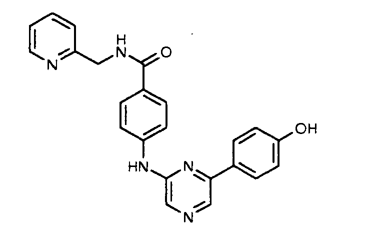

The title compound was prepared according to the same procedure as described for Example 94. Yield 37%, HPLC purity = 96%, m/z = 398 (M+H)+.

### Example 97

### 4-{[6-(4-Hydroxyphenyl)pyrazin-2-yl]amino}-N-[2-(1H-imidazol-4-yl)ethyl]benzamide

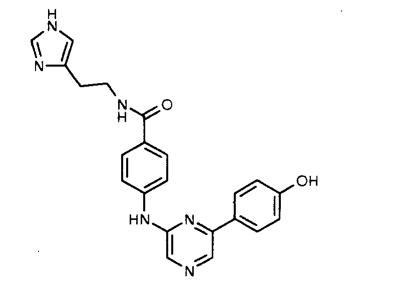

The title compound was prepared according to the same procedure as described for Example 94. Yield 3%, HPLC purity = 98%, m/z = 401 (M+H)+.

### General procedure 1: Synthesis of intermediates 4-{[6-(4-hydroxyphenyl)pyrazin-2-yl]amino}-2-benzoic acids and 3-{[6-(4-hydroxyphenyl)pyrazin-2-yl]amino}-2-benzoic acids

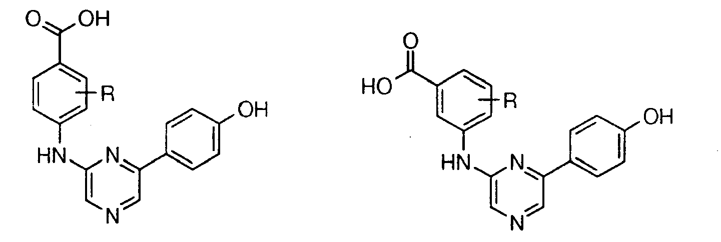

*A typical experimental procedure:* A mixture of 4-(6-chloropyrazin-2-yl)phenol (100 mg, 0.84 mmol), the amino benzoic acid (1 mmol), Pd₂(dba)₃ (10 mg), Xantphos (15 mg), NaO*t*Bu (200 mg, 2.03 mmol) and dioxane (10 ml) was heated in a sealed tube under a nitrogen atmosphere at 130°C. The reaction mixture was allowed to cool and then diluted with dioxane (5 ml) and water (5 ml). The solution was adjusted to pH=7 to produce a precipitate which was isolated by centrifuging the suspension in a Whatman filter vial, subsequently washing the residue with methanol and drying to give the product in >90% purity. Materials subsequently used without further purification.

### Intermediate 12

### 4-{[6-(4-Hydroxyphenyl)pyrazin-2-yl]amino}-2-methoxybenzoic acid

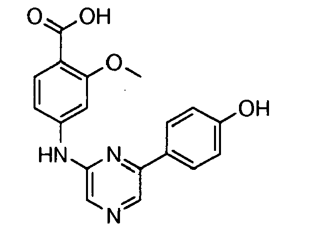

The title compound was synthesised using general procedure 1 starting from 4-(6-chloropyrazin-2-yl)phenol (300 mg, 1.45 mmol), 4-amino 2-methoxy benzoic acid (267 mg, 1.60 mmol), Pd₂(dba)₃ (30 mg, 0.0363 mmol), Xantphos (40 mg, 0.0725 mmol), Na*t*BuO (200 mg, 2.03 mmol) and dioxane (20 ml). Crude material subsequently used without further purification.

### Intermediate 13

### 5-chloro-4-{[6-(4-hydrosyphenyl)pyrazin-2-yl]amino}-2-methoxybenzoic acid

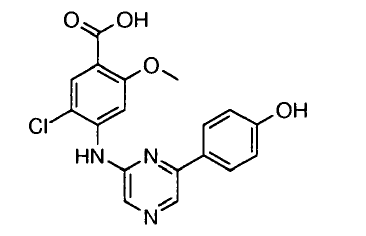

The title compound was synthesised using general procedure 1 starting from 4-(6-chloropyrazin-2-yl)phenol (300 mg, 1.45 mmol), 4-amino 2-methoay 5-chloro benzoic acid (322 mg, 1.60 mmol), Pd₂(dba)₃ (30 mg, 0.0363 mmol), Xantphos (40 mg, 0.0725 mmol), Na*t*BuO (200 mg, 2.03 mmol) and dioxane (20 ml). Crude material subsequently used without further purification.

### Intermediate 14

### 4-1[6-(4-hydroxyphenyl)pyrazin-2-yl]aminol-3-methoxybenzoic acid

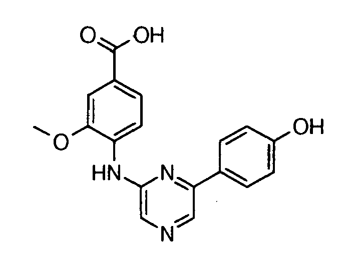

The title compound was synthesised using general procedure 1 starting from 4-(6-chloropyrazin-2-yl)phenol (300 mg, 1.45 mmol), 4-amino 3-methoxy benzoic acid (267 mg, 1.60 mmol), Pd₂(dba)₃ (30 mg, 0.0363 mmol), Xantphos (40 mg, 0.0725 mmol), Na*t*BuO (200 mg, 2.03 mmol) and dioxane (20 ml). Crude material subsequently used without further purification.

### Intermediate 15

### 3-chloro-4-{[6-(4-hydroxyphenyl)pyrazin-2-yl]amino}benzoic acid

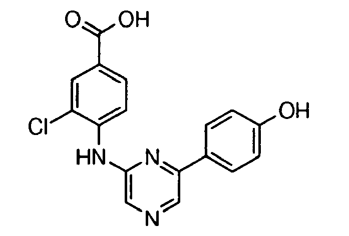

The title compound was synthesised using general procedure 1 starting from 4-(6-chloropyrazin-2-yl)phenol (300 mg, 1.45 mmol), 4-amino 3-chloro benzoic acid (274 mg, 1.60 mmol), Pd₂(dba)₃ (30 mg, 0.0363 mmol), Xantphos (40 mg, 0.0725 mmol), Na*t*BuO (200 mg, 2.03 mmol) and dioxane (20 ml). Crude material subsequently used without further purification.

### Intermediate 16

### 3-{[6-(4-hydroxyphenyl)pyrazin-2-yl]amino}benzoic acid

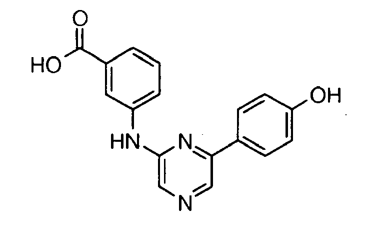

The title compound was synthesised using general procedure 1 starting from 4-(6-chloropyrazin-2-yl)phenol (300 mg, 1.45 mmol), 3-amino benzoic acid (220 mg, 1.60 mmol), Pd₂(dba)₃ (30 mg, 0.0363 mmol), Xantphos (40 mg, 0.0725 mmol), Na*t*BuO (200 mg, 2.03 mmol) and dioxane (20 ml). Crude material subsequently used without further purification.

### Intermediate 17

### 4-chloro-3-{[6-(4-hydroxyphenyl)pyrazin-2-yl]amino}benzoic acid

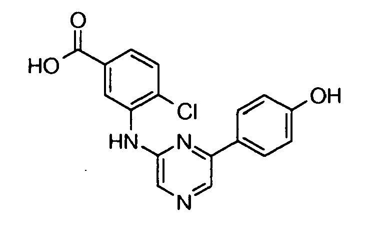

The title compound was synthesised using general procedure 1 starting from 4-(6-chloropyrazin-2-yl)phenol (100 mg, 0.84 mmol), 3-amino 4-chloro benzoic acid (171 mg, 1 mmol), Pd₂(dba)₃ (10 mg), Xantphos (15 mg), Na*t*BuO (200 mg, 2.03 mmol) and dioxane (10 ml). Crude material subsequently used without further purification.

### Intermediate 18

### 3-{[6-(4-hydroxyphenyl)pyrazin-2-yl]amino}-4-methoxybenzoic acid

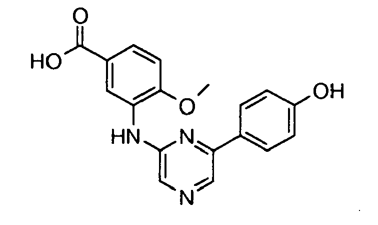

The title compound was synthesised using general procedure 1 starting from 4-(6-chloropyrazin-2-yl)phenol (100 mg, 0.84 mmol), 3-amino 4-methoxy benzoic acid (168mg, I mmol). Pd₂(dba)₃ (10 mg), Xantphos (15 mg), Na*t*BuO (200 mg, 2.03 mmol) and dioxane (10 ml). Crude material subsequently used without further purification.

### Intermediate 19

### 3-{[6-(4-hydroxyphenyl)pyrazin-2-yl]amino}-4-methylbenzoic acid

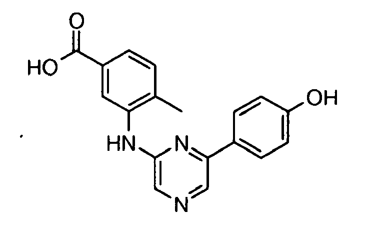

The title compound was synthesised using general procedure 1 starting from 4-(6-chloropyrazin-2-yl)phenol (100 mg, 0.84 mmol), 3-amino 4-methyl benzoic acid (151 mg, 1 mmol), Pd₂(dba)₃ (10 mg), Xantphos (15 mg), Na*t*BuO (200 mg, 2.03 mmol) and dioxane (10 ml). Crude material subsequently used without further purification.

### General procedure 2: Parallel amide couplings using 4-{[6-(4-hydroxyphenyl)pyrazin-2-yl]aminol-2-benzoic acids and 3-{[6-(4-hydroxyphenyl)pyrazin-2-yl]aminol-2-benzoic acids

The requisite benzoic acids (1 eq.) were treated with triethyl amine (3.0 eq.) and *O*-(benzotriazol-1-yl)-N,N,N',N'-tetramethyluronium tetrafluoroborate (TBTU) (1.2 eq.), dissolved in DMF (around 5 ml/mmol of starting material) and treated with the requisite amines (1.25 eq.). The mixtures were allowed to stand at room temp overnight. Additional equivalents of the reagents were added to incomplete reactions and the mixtures heated at 60°C overnight to drive conversions to completion. The products were subsequently purified using preperative HPLC (high throughput).

### Example 98

### N-[3-(dimethylamino)propyl]-4-{[6-(4-hydroxyphenyl)pyrazin-2-yl]amino}-2-methoxybenzamide

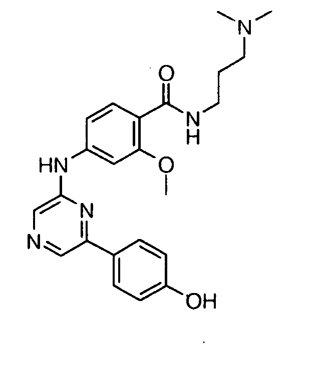

The title compound was prepared using general procedure 2, starting from 4-{[6-(4-hydroxyphenyl)pyrazin-2-yl]amino}-2-methoxybenzoic acid (Intermediate 12) (20 mg, 0.059 mmol), *N*,*N*-dimethylpropane-1,3-diamine (10 µl), triethylamine (25 µl, 0.177 mmol) and TBTU (23 mg, 0.071 mmol). The crude material was purified using preparative HPLC to give an off-white solid, (10.5 mg).
1H NMR (500 MHz, DMSO-D6) δ ppm 1.60 - 1.71 (m, 2 H) 2.11 - 2.18 (m, 6 H) 2.28 (t, *J*=6.70 Hz, 2 H) 3.97 (s, 3 H) 6.91 (d, *J*=8.53 Hz, 2 H) 7.23 (d, *J*=8.53 Hz, 1 H) 7.85 (d, *J*=8.53 Hz, 1 H) 7.92 - 8.02 (m, 3 H) 8.09 - 8.18 (m, 2 H) 8.49 (s, 1 H) 9.77 - 9.84 (m, 2 H) (1xCH₂ obscured by solvent peak surpression). HPLC 99% R_{T}=1.92 (5-100% MeCN over 3.2 minutes).
MS (electrospray) M+H+ m/z 421.

### Example 99

### 4-{[6-(4-hydroxyphenyl)pyrazin-2-yl]amino}-2-methoxy-N-methyl-N-(1-methylpyrrolidin-3-yl)benzamide

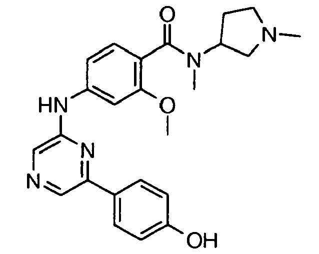

The title comopound was prepard using general procedure 2, starting from 4-{[6-(4-hydroxyphenyl)pyrazin-2-yl]amino}-2-methoxybenzoic acid (Intermediate 12) (20 mg, 0.059 mmol), *N*,1-dimethylpyrrolidin-3-amine (10 µ), triethylamine (25 µl, 0.177 mmol) and TBTU (23 mg, 0.071 mmol). The crude material was purified using preparative HPLC to give a yellow solid, (12.3 mg).
HPLC 96% R_{T}=1.87 (5-100% MeCN over 3.2 minutes).
MS (electrospray) M+H+ m/z 433.

### Example 100

### N-[2-(diethylamino)ethyl]-4-{[6-(4-hydroxyphenyl)pyrazin-2-yl]amino}-2-methoxybenzamide

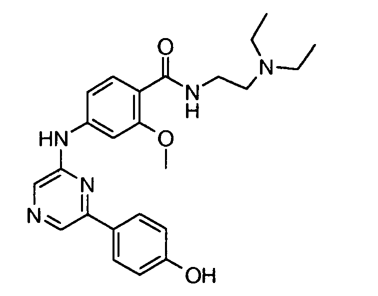

The title comopound was prepard using general procedure 2, starting from 4-{[6-(4-hydroxyphenyl)pyrazin-2-yl]amino}-2-methoxybenzoic acid (Intermediate 12) (20 mg, 0.059 mmol), *N*,*N*-diethylethane-1,2-diamine (10 µ), triethylamine (25 µl, 0.177 mmol) and TBTU (23 mg, 0.071 mmol). The crude material was purified using preparative HPLC to give a yellow solid, (12.2 mg).
HPLC 99% R_{T}=1.99 (5-100% MeCN over 3.2 minutes).
MS (electrospray) M+H+ m/z 435.

### Example 101

### 4-{[6-(4-hydroxyphenyl)pyrazin-2-yl]amino}-2-methoxy-N-(pyridin-2-ylmethyl)benzamide

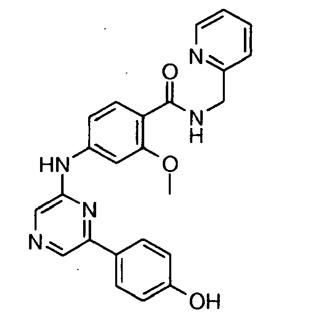

The title comopound was prepard using general procedure 2, starting from 4-{[6-(4-hydroxyphenyl)pyrazin-2-yl]amino}-2-methoxybenzoic acid (Intermediate 12) (20 mg, 0.059 mmol), 1-pyridin-2-ylmethanamine (10 µl), triethylamine (25 µl, 0.177 mmol) and TBTU (23 mg, 0.071 mmol). The crude material was purified using preparative HPLC to give a beige solid, (1.6 mg).
HPLC 94% R_{T}=2.11 (5-100% MeCN over 3.2 minutes).
MS (electrospray) M+H+ m/z 427.

### Example 102

### 4-{[6-(4-hydroxyphenyl)pyrazin-2-yl]amino}-2-methoxy-N-(2-pyrrolidin-1-ylethyl)benzamide

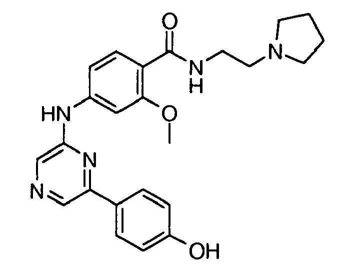

The title comopound was prepard using general procedure 2, starting from 4-{[6-(4-hydroxyphenyl)pyrazin-2-yl]amino}-2-methoxybenzoic acid (Intermediate 12) (20 mg, 0.059 mmol), 2-pyrrolidin-1-ylethanamine (10 µl), triethylamine (25 µl, 0.177 mmol) and TBTU (23 mg, 0.071 mmol). The crude material was purified using preparative HPLC to give a yellow solid, (10.3 mg).
HPLC 99% R_{T}=1.96 (5-100% MeCN over 3.2 minutes).
MS (electrospray) M+H+ m/z 433.

### Example 103

### 5-chloro-N-[2-(dimethylamino)ethyl]-4-{[6-(4-hydroxyphenyl)pyrazin-2-yl]amino}-2-methoxybenzamide

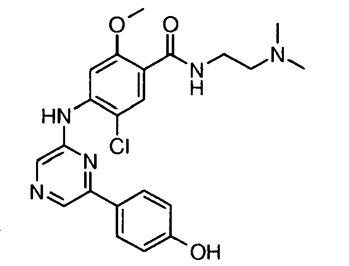

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*-dimethylethane-1,2-diamine (10 µl), triethylamine (22 µl, 0.16 mmol) and TBTU (23 mg, 0.071 mmol). The crude material was purified using preparative HPLC to give a yellow solid, (2.6 mg). HPLC 100% R_{T}=2.09 (5-100% MeCN over 3.2 minutes).
MS (electrospray) M+H+ m/z 441.

### Example 104

### 5-chloro-N-[3-(dimethylamino)propyl]-4-{[6-(4-hydroxyphenyl)pyrazin-2-yl]amino}-2-methoxybenzamide

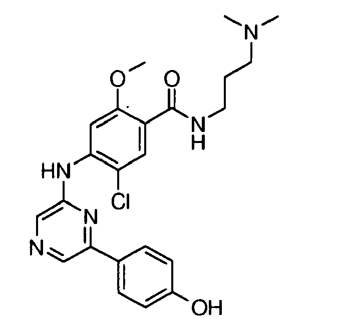

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*-dimethylpropane-1,3-diamine (10 µl), triethylamine (22 µl, 0.16 mmol) and TBTU (23 mg, 0.071 mmol). The crude material was purified using preparative HPLC to give a yellow solid, (3.1 mg). HPLC 100% R_{T}=2.09 (5-100% MeCN over 3.2 minutes).
MS (electrospray) M+H+ m/z 455.

### Example 105

### 5-chloro-4-{[6-(4-hydroxyphenyl)pyrazin-2-yl]amino}-2-methoxy-N-methyl-N-(1-methylpyrrolidin-3-yl)benzamide

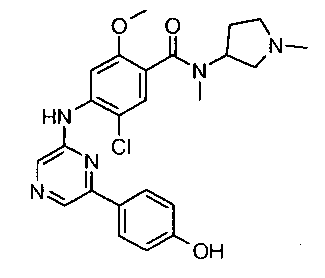

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,1-dimethylpyrrolidin-3-amine (10 µl), triethylamine (22 µl, 0.16 mmol) and TBTU (23mg, 0.071 mmol). The crude material was purified using preparative HPLC to give a light brown solid, (4.2mg). HPLC 97% R_{T}=2.04 (5-100% MeCN over 3.2 minutes).
MS (electrospray) M+H+ m/z 467.

### Example 106

### 5-chloro-N-[2-(diethylamino)ethyl]-4-{[6-(4-hydroxyphenyl)pyrazin-2-yl]amino}-2-methoxybenzamide

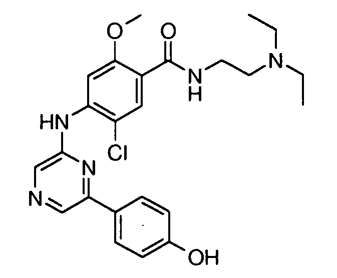

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*-diethylethane-1,2-diamine (10 µl), triethylamine (22 µl, 0.16 mmol) and TBTU (23 mg, 0.071 mmol). The crude material was purified using preparative HPLC to give a light brown solid, (3.4 mg).
HPLC 97% R_{T}=2.16 (5-100% MeCN over 3.2 minutes).
MS (electrospray) M+H+ m/z 469.

### Example 107

### 5-chloro-4-{[6-(4-hydroxyphenyl)pyrazin-2-yl]amino}-2-methoxy-N-(2-pyrrolidin-1-ylethyl)benzamide

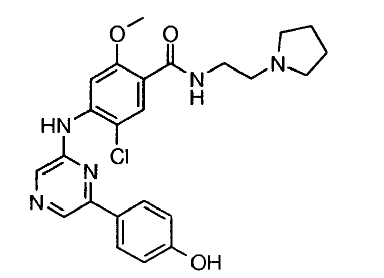

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 2-pyrrolidin-1-ylethanamine (10 µl), triethylamine (22 µl, 0.16 mmol) and TBTU (23 mg, 0.071 mmol). The crude material was purified using preparative HPLC to give an off-white solid, (3.8 mg).
HPLC 99% R_{T}=2.16 (5-100% MeCN over 3.2 minutes).

MS (electrospray) M+H+ m/z 467.

### Example 108

### N-[2-(dimethylamino)ethyl]-4-{[6-(4-hydroxyphenyl)pyrazin-2-yl]amino}-3-methoxybenzamide

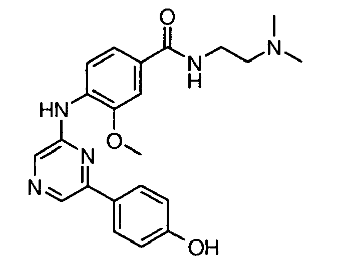

The title comopound was prepard using general procedure 2, starting from 4-{[6-(4-hydroxyphenyl)pyrazin-2-yl]amino}-3-methoxybenzoic acid (Intermediate 14) (20 mg, 0.059 mmol), *N*,*N*-dimethylethane-1,2-diamine (10 µl), triethylamine (25 µl, 0.177 mmol) and TBTU (23 mg, 0.071 mmol). The crude material was purified using preparative HPLC to give a yellow solid, (5.8 mg).
HPLC 95% R_{T}=1.92 (5-100% MeCN over 3.2 minutes).
MS (electrospray) M+H+ m/z 407.

### Example 109

### 4-[6-({2-methoxy-4-[(4-methylpiperazin-1-yl)carbonyl]phenyl}amino)pyrazin-2-yl]phenol

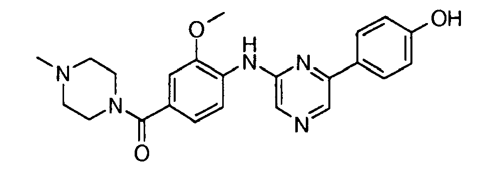

The title comopound was prepard using general procedure 2, starting from 4-{[6-(4-hydroxyphenyl)pyrazin-2-yl]amino}-3-methoxybenzoic acid (Intermediate 14) (20 mg, 0.059 mmol), 1-methylpiperazine (10 µl), triethylamine (25 µl, 0.177 mmol) and TBTU (23 mg, 0.071 mmol). The crude material was purified using preparative HPLC to give a white solid, (7.3 mg).
HPLC 99% R_{T}=1.97 (5-100% MeCN over 3.2 minutes).
MS (electrospray) M+H+ m/z 419.

### Example 110

### N-[3-(dimethylamino)propyl]4-{[6-(4-hydroxyphenyl)pyrazin-2-yl]amino}-3-methoxybenzamide

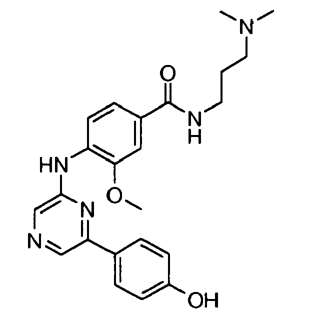

The title comopound was prepard using general procedure 2, starting from 4-{[6-(4-hydroxyphenyl)pyrazin-2-yl]amino}-3-methoxybenzoic acid (Intermediate 14) (20 mg, 0.059 mmol), *N*,*N*-dimethylpropane-1,3-diamine (10 µl), triethylamine (25 µl, 0.177 mmol) and TBTU (23 mg, 0.071 mmol). The crude material was purified using preparative HPLC to give a yellow solid, (6.4 mg).
HPLC 97% R_{T}=1.93 (5-100% MeCN over 3.2 minutes).
MS (electrospray) M+H+ m/z 421.

### Example 111

### 4-{[6-(4-hydroxyphenyl)pyrazin-2-yl]amino}-3-methoxy-N-methyl-N-(1-methylpyrrolidin-3-yl)benzamide

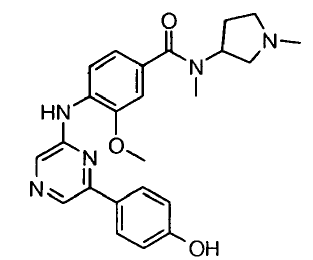

The title comopound was prepard using general procedure 2, starting from 4-{[6-(4-hydroxyphenyl)pyrazin-2-yl]amino}-3-methoxybenzoic acid (Intermediate 14) (20 mg, 0.059 mmol), *N*,1-dimethylpyrrolidin-3-amine (10 µl), triethylamine (25 µl, 0.177 mmol) and TBTU (23 mg, 0.071 mmol). The crude material was purified using preparative HPLC to give a yellow solid, (8.9 mg).
HPLC 95% R_{T}=1.96 (5-100% MeCN over 3.2 minutes).
MS (electrospray) M+H+ m/z 433.

### Example 112

### N-[2-(diethylamino)ethyl]-4-{[6-(4-hydroxyphenyl)pyrazin-2-yl]amino}-3-methoxybenzamide

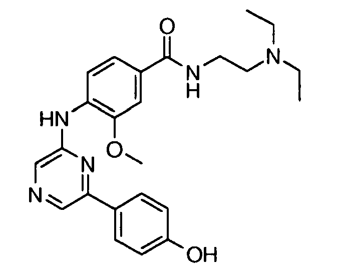

The title comopound was prepard using general procedure 2, starting from 4-{[6-(4-Hydroxyphenyl)pyrazin-2-yl]amino}-3-methoxybenzoic acid (Intermediate 14) (20 mg, 0.059 mmol), *N*,*N*-diethylethane-1,2-diamine (10 µl), triethylamine (25 µl, 0.177 mmol) and TBTU (23 mg, 0.071 mmol). The crude material was purified using preparative HPLC to give a yellow solid, (7.4 mg).
HPLC 99% R_{T}=1.99 (5-100% MeCN over 3.2 minutes).
MS (electrospray) M+H+ m/z 435.

### Example 113

### 4-{[6-(4-hydroxyohenyl)oyrazin-2-yl]amino}-3-methoxy-N-(pyridin-2-ylmethyl)benzamide

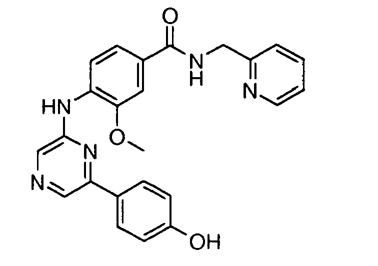

The title comopound was prepard using general procedure 2, starting from 4-{[6-(4-Hydroxyphenyl)pyrazin-2-yl]amino}-3-methoxybenzoic acid (Intermediate 14) (20 mg, 0.059 mmol), 1-pyridin-2-ylmethanamine (10 µl), triethylamine (25 µl, 0.177 mmol) and TBTU (23 mg, 0.071 mmol). The crude material was purified using preparative HPLC to give a yellow solid, (5.0 mg).
HPLC 96% R_{T}=2.10 (5-100% MeCN over 3.2 minutes).
MS (electrospray) M+H+ m/z 427.

### Example 114

### 4-{[6-(4-hydroxyphenyl)pyrazin-2-yl]amino}-3-methoxy-N-(2-pyrrolidin-1-ylethyl)benzamide

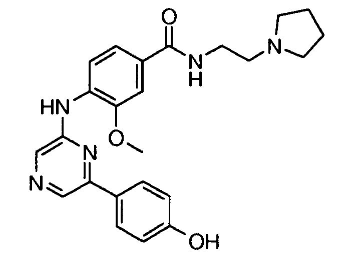

The title comopound was prepard using general procedure 2, starting from 4-{[6-(4-hydroxyphenyl)pyrazin-2-yl]amino}-3-methoxybenzoic acid (Intermediate 14) (20mg, 0.059mmol), 2-pyrrolidin-1-ylethanamine (10µl), triethylamine (25µl, 0.177mmol) and TBTU (23mg, 0.071mmol). The crude material was purified using preparative HPLC to give a yellow solid, (7.0mg).
HPLC 91% R_{T}=1.95 (5-100% MeCN over 3.2 minutes).
MS (electrospray) M+H+ m/z 433.

### Example 115

### 4-[6-({2-chloro-4-[(4-methylpiperazin-1-yl)carbonyl]phenyl}amino)pyrazin-2-yl]phenol

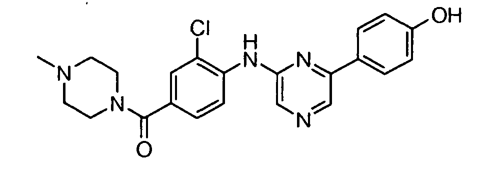

The title comopound was prepard using general procedure 2, starting from 3-chloro-4-{[6-(4-hydroxyphenyl)pyrazin-2-yl]amino}benzoic acid (Intermediate 15) (20 mg, 0.59 mmol), 1-methylpiperazine (10 µl), triethylamine (24 µl, 0.18 mmol) and TBTU (23 mg, 0.071 mmol), The crude material was purified using preparative HPLC to give an off-white solid, (8.1 mg).
1H NMR (500 MHz, DMSO-D6) δ ppm 2.21 (s, 3 H) 3.43 - 3.63 (m, 4 H) 6.87 (d, *J*=8.53 Hz, 2 H) 7.41 (d, *J*=8.53 Hz, 1 H) 7.53 (s, 1 H) 7.88 (d, *J*=8.53 Hz, 2 H) 8.28 (d, *J*=7.92 Hz, 1 H) 8.35 (s, 1 H) 8.53 (s, 1 H) 8.95 (s, 1 H) 9.80 (s, 1 H) (2xCH₂ obscured by solvent peak surpression).
HPLC 99% R_{T}=2.04 (5-100% MeCN over 3.2 minutes).
MS (electrospray) M+H+ m/z 423.

### Example 116

### 3-chloro-4-{[6-(4-hydroxyphenyl)pyrazin-2-yl]amino}-N-methyl-N-(1-methylpyrrolidin-3-yl)benzamide

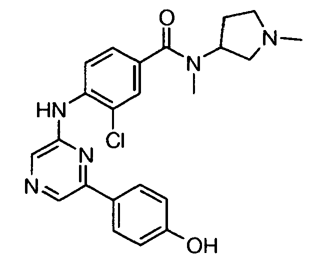

The title comopound was prepard using general procedure 2, starting from 3-chloro-4-{[6-(4-hydroxyphenyl)pyrazin-2-yl]amino}benzoic acid (Intermediate 15) (20 mg, 0.59 mmol), *N*,1-dimethylpyrrolidin-3-amine (10 µl), triethylamine (24 µl, 0.18 mmol) and TBTU (23 mg, 0.071 mmol). The crude material was purified using preparative HPLC to give a light-yellow solid, (8.9 mg).
HPLC 91% R_{T}=2.01 (5-100% MeCN over 3.2 minutes).
MS (electrospray) M+H+ m/z 437.

### Example 117

### 3-chloro-4-{[6-(4-hydroxyphenyl)pyrazin-2-yl]amino}-N-(2-pyrrolidin-1-ylethyl)benzamide

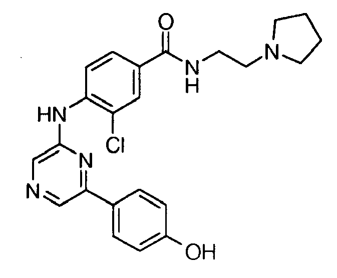

The title comopound was prepard using general procedure 2, starting from 3-chloro-4-{[6-(4-hydroxyphenyl)pyrazin-2-yl]amino}benzoic acid (Intermediate 15) (20 mg, 0.59 mmol), 2-pyrrolidin-1-ylethanamine (10 µl), triethylamine (24 µl, 0.18 mmol) and TBTU (23 mg, 0.071 mmol). The crude material was purified using preparative HPLC to give a yellow solid, (4.4 mg).
HPLC 90% R_{T}=2.00 (5-100% MeCN over 3.2 minutes).
MS (electrospray) M+H+ m/z 437.

### Example 118

### N-[2-(dimethylamino)ethyl]-3-{[6-(4-hydroxyphenyl)pyrazin-2-yl]amino}benzamide

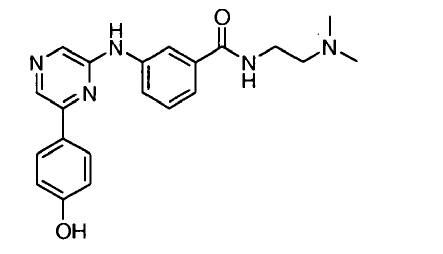

The title comopound was prepard using general procedure 2, starting from 3-{[6-(4-hydroxyphenyl)pyrazin-2-yl]amino}benzoic acid (Intermediate 16) (20 mg, 0.065 mmol), *N*,*N*-dimethylethane-1,2-diamine (10 µl), triethylamine (27 µl, 0.20 mmol) and TBTU (23 mg, 0.071 mmol). The crude material was purified using preparative HPLC to give a yellow solid, (4.4 mg).
1H NMR (500 MHz, DMSO-D6) δ ppm 2.18 (s, 6 H) 6.89 (d, *J*=8.53 Hz, 2 H) 7.34 - 7.46 (m, 2 H) 7.90 (d, *J*=6.70 Hz, 1 H) 7.99 (d, *J*=8.53 Hz, 2 H) 8.09 (s, 1 H) 8.26 (t, *J*=5.18 Hz, 1 H) 8.33 (s, 1 H) 8.45 (s, 1 H) 9.61 (s, 1 H) (2xCH₂ obscured by solvent peak surpression).
HPLC 98% R_{T}=1.86 (5-100% MeCN over 3.2 minutes).
MS (electrospray) M+H+ m/z 377.

### Example 119

### 4-[6-({3-[(4-methylpiperazin-1-yl)carbonyl]phenyl}amino)pyrazin-2-yl]phenol

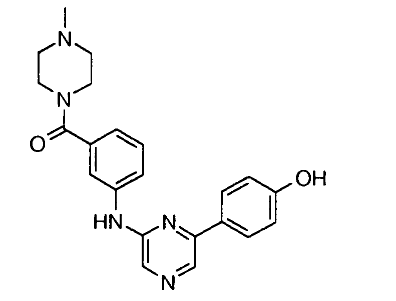

The title comopound was prepard using general procedure 2, starting from 3-{[6-(4-hydroxyphenyl)pyrazin-2-yl]amino}benzoic acid (Intermediate 16) (20 mg, 0.065 mmol), 1-methylpiperazine (10 µl), triethylamine (27 µl, 0.20 mmol) and TBTU (23 mg, 0.071 mmol). The crude material was purified using preparative HPLC to give an off-white solid, (6.4 mg).
HPLC 95% R_{T}=1.92 (5-100% MeCN over 3.2 minutes).
MS (electrospray) M+H+ m/z 389.

### Example 120

### N-[3-(dimethylamino)propyl]-3-{[6-(4-hydroxyphenyl)pyrazin-2-yl]amino}benzamide

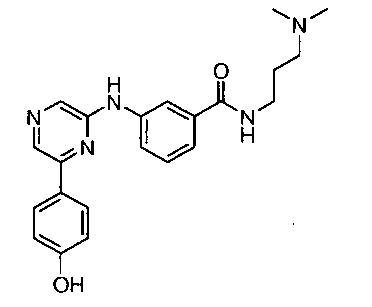

The title comopound was prepard using general procedure 2, starting from 3-{[6-(4-hydroxyphenyl)pyrazin-2-yl]amino}benzoic acid (Intermediate 16) (20 mg, 0.065 mmol), *N*,*N*-dimethylpropane-1,3-diamine (10 µl), triethylamine (27 µl, 0.20 mmol) and TBTU (23 mg, 0.071 mmol). The crude material was purified using preparative HPLC to give a light-yellow solid, (5.2 mg).
HPLC 97% R_{T}=1.87 (5-100% MeCN over 3.2 minutes).
MS (electrospray) M+H+ m/z 391.

### Example 121

### 3-{[6-(4-hydroxyphenyl)pyrazin-2-yl]amino}-N-methyl-N-(1-methylpyrrolidin-3-yl)benzamide

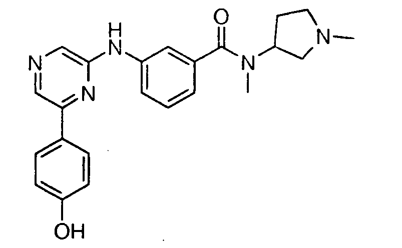

The title comopound was prepard using general procedure 2, starting from 3-{[6-(4-hydroxyphenyl)pyrazin-2-yl]amino}benzoic acid (Intermediate 16) (20 mg, 0.065 mmol), *N*,1-dimethylpyrrolidin-3-amine (10 µl), triethylamine (27 µl, 0.20 mmol) and TBTU (23 mg, 0.071 mmol). The crude material was purified using preparative HPLC to give a white solid, (5.9 mg).
HPLC 92% R_{T}=1.94 (5-100% MeCN over 3.2 minutes).
MS (electrospray) M+H+ m/z 403.

### Example 122

### N-[2-(diethylamino)ethyl]-3-{[6-(4-hydroxyphenyl)pyrazin-2-yl]amino}benzamide

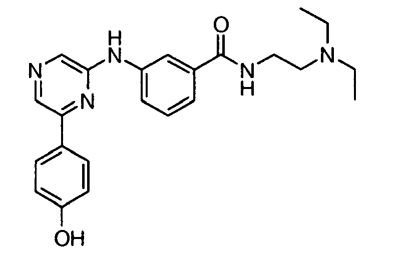

The title comopound was prepard using general procedure 2, starting from 3-{[6-(4-hydroxyphenyl)pyrazin-2-yl]amino}benzoic acid (Intermediate 16) (20 mg, 0.065 mmol), *N*,*N*-diethylethane-1,2-diamine (10 µl), triethylamine (27 µl, 0.20 mmol) and TBTU (23 mg, 0.071 mmol). The crude material was purified using preparative HPLC to give a light-yellow solid, (6.2 mg).
HPLC 90% R_{T}=1.93 (5-100% MeCN over 3.2 minutes).
MS (electrospray) M+H+ m/z 405.

### Example 123

### 5-chloro-4-{[6-(4-hydroxyphenyl)pyrazin-2-yl]amino}-2-methoxy-N-(2-morpholin-4-lyethyl)benzamide

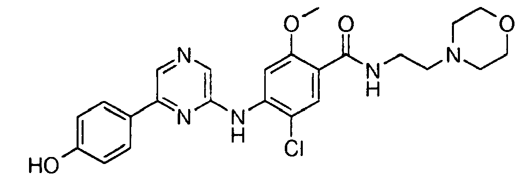

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 2-morpholin-4-ylethanamine (15 mg), triethylamine (22 µl, 0.16 mmol) and TBTU (21 mg, 0.065 mmol). The crude material was purified using preparative HPLC to give a light-yellow solid (4.5 mg).
1H NMR (500 MHz, DMSO-D6) δ ppm 3.43 (m, 2 H) 3.62 (m, 4 H) 3.96 (s, 3 H) 6.89 (d, *J*=8.53 Hz, 2 H) 7.93 (s, 1 H) 7.96 (d, *J*=8.53 Hz, 2 H) 8.30 (t, *J*=5.18 Hz, 1 H) 8.38 (s, 1 H) 8.48 (s, 1 H) 8.48 (s, 1 H) 8.59 (s, 1 H) 8.96 (s, 1 H) 9.83 (s, 1 H) (3xCH₂ obscured by solvent peak surpression).
HPLC 99% R_{T}=2.17 (10-90% MeCN over 3 minutes).
MS (electrospray) M+H+ m/z 483.

### Example 124

### 5-chloro-4-{[6-(4-hydroxyphenyl)pyrazin-2-yl]aminol-N-{2-[(2-hydroxypropyl)amino]ethyl}-2-methoxybenzamide

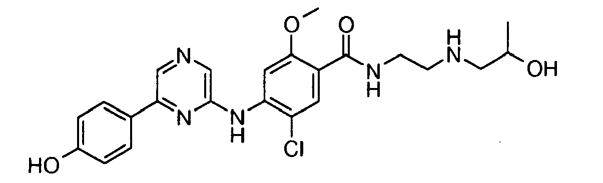

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 1-[(2-aminoethyl)amino]propan-2-ol (15 mg), triethylamine (22 µl, 0.16 mmol) and TBTU (21 mg, 0.065 mmol). The crude material was purified using preparative HPLC to give a light-yellow solid (4.1 mg).
HPLC 100% R_{T}=2.07 (5-100% MeCN over 3.2 minutes).
MS (electrospray) M+H+ m/z 471.

### Example 125

### 4-[6-({2-chloro-5-methoxy-4-[(4-methyl-1,4-diazepan-1-yl)carbonyl]phenyl}amino)pyrazin-2-yl]phenol

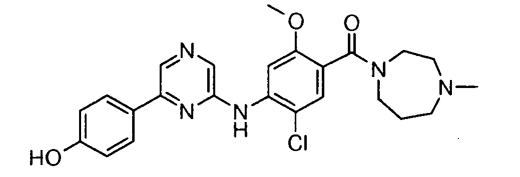

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 1-methyl-1,4-diazepane (15 mg), triethylamine (22 µl, 0.16 mmol) and TBTU (21 mg, 0.065 mmol). The crude material was purified using preparative HPLC to give a light-yellow solid (11.8 mg). HPLC 100% R_{T}=2.07 (5-100% MeCN over 3.2 minutes).
MS (electrospray) M+H+ m/z 467.

### Example 126

### 5-chloro-N-[2-(dimethylamino)ethyl]-4-{[6-(4-hydroxyphenyl)pyrazin-2-yl]amino}-2-methoxy-N-methylbenzamide

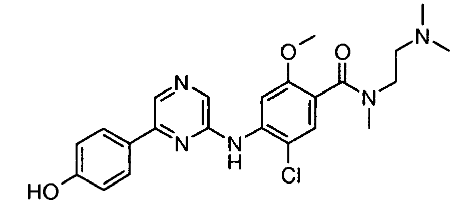

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*,*N*-trimethylethane-1,2-diamine (15 mg), triethylamine (22 µl, 0.16 mmol) and TBTU (21 mg, 0.065 mmol). The crude material was purified using preparative HPLC to give a white solid (13.5 mg).
HPLC 74% R_{T}=2.08 (10-90% MeCN over 3 minutes).
MS (electrospray) M+H+ m/z 455.

### Example 127

### 4-(6-{[2-chloro-4-({2-[(dimethylamino)methyl]piperidin-1-yl}carbonyl)-5-methoxyphenyl]amino}pyrazin-2-yl)phenol

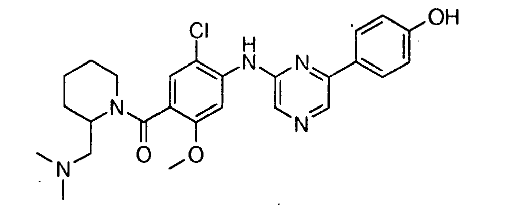

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*-dimethyl-1-piperidin-2-ylmethanamine (15 mg), triethylamine (22 µl, 0.16 mmol) and TBTU (21 mg, 0.065 mmol). The crude material was purified using preparative HPLC to give a light-brown solid (12.6 mg).
HPLC 100% R_{T}=2.22 (5-100% MeCN over 3.2 minutes).
MS (electrospray) M+H+ m/z 495.

### Example 128

### N-(1-benzylpiperidin-4-yl)-5-chloro-4-{[6-(4-hydroxyphenyl)pyrazin-2-yl]amino}-2-methoxybenzamide

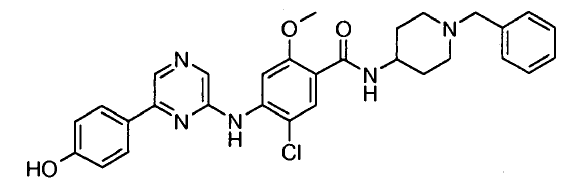

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 1-benzylpiperidin-4-amine (15 mg), triethylamine (22 µl, 0.16 mmol) and TBTU (21 mg, 0.065 mmol). The crude material was purified using preparative HPLC to give a light-yellow solid (13.5 mg).
1H NMR (500 MHz, DMSO-D6) δ ppm 1.49 - 1.65 (m, *J*=10.36, 10.36, 10.36 Hz, 2 H) 1.76 - 1.89 (m, *J*=10.36 Hz, 2 H) 2.07 - 2.19 (m, *J*=10.66, 10.66 Hz, 2 H) 2.71 - 2.78 (m, 2 H) 3.48 (s, 2 H) 3.78 - 3.88 (m, 1 H) 3.91 (s, 3 H) 6.89 (d, *J*=8.53 Hz, 2 H) 7.21 - 7.36 (m, 5 H) 7.83 (s, 1 H) 7.93 (d, *J*=7.92 Hz, 1 H) 7.96 (d, *J*=8.53 Hz, 2 H) 8.33 (s, 1 H) 8.47 (s, 1 H) 8.59 (s, 1 H) 8.95 (s, 1 H) 9.84 (s, 1 H) (Some peaks may be obscured by solvent surpression).
HPLC 98% R_{T}=2.49 (5-100% MeCN over 3.2 minutes).
MS (electrospray) M+H+ m/z 543.

### Example 129

### 5-chloro-N-[3-(dimethylamino)-2,2-dimethylpropyl]-4-{[6-(4-hydroxyphenyl)pyrazin-2-yl]amino}-2-methoxybenzamide

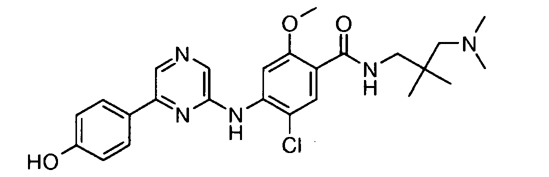

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*,2,2-tetramethylpropane-1,3-diamine (15 mg), triethylamine (22 µl, 0.16 mmol) and TBTU (21 mg, 0.065 mmol). The crude material was purified using preparative HPLC to give a light-yellow solid (10.8 mg).
1H NMR (500 MHz, DMSO-D6) δ ppm 2.17 (s, 2 H) 2.27 (s, 6 H) 3.95 (s, 3 H) 6.89 (d, *J*=7.92 Hz, 2 H) 7.90 (s, 1 H) 7.96 (d, *J*=8.53 Hz, 2 H) 8.28 - 8.35 (m, *J*=5.18, 5.18 Hz, 1 H) 8.37 (s, 1 H) 8.48 (s, 1 H) 8.59 (s, 1 H) 8.96 (s, 1 H) 9.82 (s, 1 H) (1xCH2 obscured by solvent peak surpression).
HPLC 93% R_{T}=2.26 (5-100% MeCN over 3.2 minutes).
MS (electrospray) M+H+ m/z 483.

### Example 130

### 5-chloro-4-{[6-(4-hydroxyphenyl)pyrazin-2-yl]amino}-2-methoxy-N-(3-morpholin-4-ylpropyl)benzamide

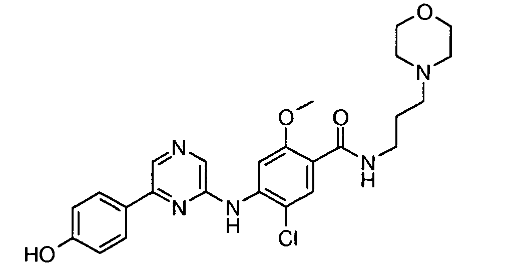

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 3-morpholin-4-ylpropan-1-amine (15 mg), triethylamine (22 µl, 0.16 mmol) and TBTU (21 mg, 0.065 mmol). The crude material was purified using preparative HPLC to give a white solid (10.7 mg). HPLC 52% R_{T}=2.16 (5-100% MeCN over 3.2 minutes). Contains 48% dimethyl amide
MS (electrospray) M+H+ m/z 497.

### Example 131

### 5-chloro-N-[3-(dimethylamino)propyl]-4-{[6-(4-hydroxyphenyl)pyrazin-2-yl]amino}-2-methoxy-N-methylbenzamide

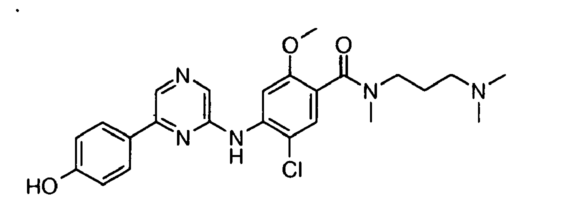

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N*,*N*,*N*'-trimethylpropane-1,3-diamine (15 mg), triethylamine (22 µl, 0.16 mmol) and TBTU (21 mg, 0.065 mmol). The crude material was purified using preparative HPLC to give a white solid (12.7 mg).
HPLC 72% E_{T}=2.10 (5-100% MeCN over 3.2 minutes). Contains 28% dimethyl amide.
MS (electrospray) M+H+ m/z 469.

### Example 132

### 5-chloro-4-{[6-(4-hydroxyphenyl)pyrazin-2-yl]amino}-2-methoxy-N-(2,2,2-trifluoroethyl)benzamide

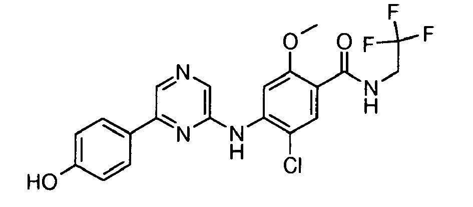

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 2,2,2-trifluoroethanamine (15 mg), triethylamine (22 µl, 0.16 mmol) and TBTU (21 mg, 0.065 mmol). The crude material was purified using preparative HPLC to give a white solid (13.0 mg). 1H NMR (500 MHz, DMSO-D6) δ ppm 3.94 (s, 3 H) 4.15 (m, 2 H) 6.89 (d, *J*=8.53 Hz, 2 H) 7.88 (s, 1 H) 7.97 (d, *J*=8.53 Hz, 2 H) 8.40 (s, 1 H) 8.50 (s, 1 H) 8.52 - 8.59 (m, *J*=6.40, 6.40 Hz, 1 H) 8.61 (s, 1 H) 9.00 (s, 1 H) 9.84 (s, 1 H). HPLC 98% R_{T}=2.47 (5-100% MeCN over 3.2 minutes).
MS (electrospray) M+H+ m/z 452.

### Example 133

### 4-(6-{[2-chloro-4-({4-[2-(dimethylamino)ethyl]piperazin-1-yl}carbonyl)-5-methoxyphenyl]amino}pyrazin-2-yl)phenol

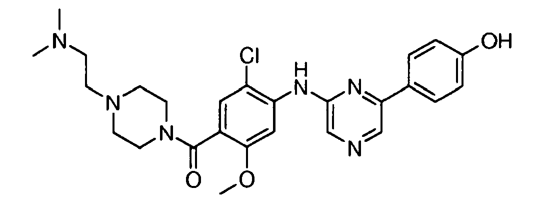

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), *N,N*-dimethyl-2-piperazin-1-ylethanamine (15 mg), triethylamine (22 µl, 0.16 mmol) and TBTU (21 mg, 0.065 mmol). The crude material was purified using preparative HPLC to give a light-yellow solid (11.9 mg).
HPLC 96% R_{T}=2.05 (5-100% MeCN over 3.2 minutes).
MS (electrospray) M+H+ m/z 510.

### Example 134

### 5-chloro-4-{[6-(4-hydroxyphenyl)pyrazin-2-yl]amino}-2-methoxy-N-(tetrahydrofuran-2-ylmethyl)benzamide

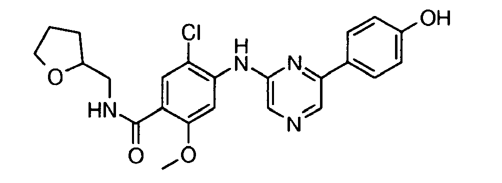

The title comopound was prepard using general procedure 2, starting from 5-chloro-4-{[6-(4-hydroxyphenyl)pyrazin-2-yl]amino}-2-methoxybenzoic acid (Intermediate 13) (20 mg, 0.54 mmol), 1-(tetrahydrofuran-2-yl)methanamine (15 mg), triethylamine (22 µl, 0.16 mmol) and TBTU (21 mg, 0.065 mmol). The crude material was purified using preparative HPLC to give a white solid (7.4 mg).
HPLC 98% R_{T}=2.30 (5-100% MeCN over 3.2 minutes).
MS (electrospray) M+H+ m/z 454.

### General procedure 3: Parallel amide couplings using 3-{[6-(4-hydroxyphenyl)pyrazin-2-yl]amino}-2-benzoic acids (EDC/HOBt method)

The starting amino benzoic acids (10 mg, about 0.03 mmol) were weighed out into 1.5 ml vials and HOBt (6 mg, 0.044 mmol) and EDC (8.6 mg, 0.44 mmol) added. The mixtures were diluted with 0.5 ml THF and treated with triethylamine (14 µl, 0.1 mmol) before treatment with the requisite amine (10 µl). The mixtures were shaken at room temperature overnight. Additional equivalents of the reagents were added to incomplete reactions and the mixtures heated at 60°C overnight to drive conversions to completion. The products were subsequently purified using preperative HPLC, products isolated as TFA salts.

### Example 135

### 4-chloro-N-[2-(dimethylamino)ethyl]-3-{[6-(4-hydroxyphenyl)pyrazin-2-yl]amino}benzamide trifluoroacetate (salt)

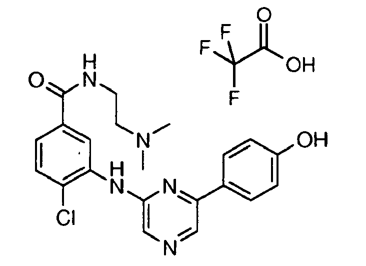

The title compound was synthesised using general procedure 3 starting from 4-chloro-3-{[6-(4-hydroxyphenyl)pyrazin-2-yl]amino}benzoic acid (Intermediate 17) (10 mg 0.029 mmol), *N,N*-dimethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (6.7 mg).
HPLC 100% R_{T}=1.48 (10-97% MeCN over 3 minutes).
MS (electrospray) m/z ½(M+2H+)207 (70%), M+H+ 412 (100%).

### Example 136

### 4-[6-({2-chloro-5-[(4-methylpiperazin-1-yl)carbonyl]phenyl}amino)pyrazin-2-yl]phenol trifluoroacetate (salt)

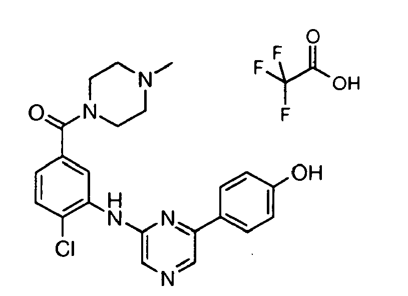

The title compound was synthesised using general procedure 3 starting from 4-chloro-3-{[6-(4-hydroxyphenyl)pyrazin-2-yl]amino}benzoic acid (Intermediate 17) (10 mg 0.029 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (8.3 mg).
1H NMR (400 MHz, METHANOL-D4) δ ppm 2.81 (s, 3 H) 2.90 - 3.16 (m, 4 H) 3.18 - 3.48 (m, 4 H) 6.82 - 7.00 (m, 2 H) 7.20 (dd, *J*=8.30, 1.95 Hz, 1 H) 7.50 - 7.68 (m, *J*=8.06 Hz, 1 H) 7.77 - 7.95 (m, 2 H) 8.18 - 8.27 (m, 1 H) 8.39 - 8.44 (m, 1 H) 8.47 (d, *J*=1.95 Hz, 1 H).
HPLC 100% R_{T}=1.54 (10-97% MeCN over 3 minutes).
MS (electrospray) m/z ½(M+2H+ ) 213 (100%), M+H+ 424 (90%).

### Example 137

### 4-chloro-3-{[6-(4-hydroxyphenyl)pyrazin-2-yl]amino}-N-methyl-N-(1-methylpyrrolidin-3-yl)benzamide trifluoroacetate (salt)

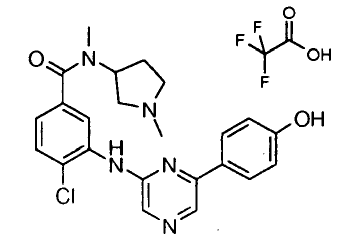

The title compound was synthesised using general procedure 3 starting from 4-chloro-3-{[6-(4-hydroxyphenyl)pyrazin-2-yl]amino}benzoic acid (Intermediate 17) (10 mg 0.029 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.9 mg).
HPLC 100% R_{T}=1.63 (10-97% MeCN over 3 minutes).

MS (electrospray) m/z ½(M+2H+ ) 220 (100%), M+H+ 438 (95%).

### Example 138

### 4-chloro-N-[2-(diethylamino)ethyl]-3-{[6-(4-hydroxyphenyl)pyrazin-2-yl]amino}benzamide trifluoroacetate (salt)

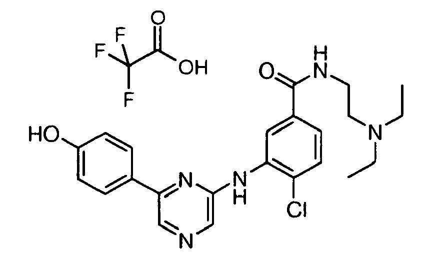

The title compound was synthesised using general procedure 3 starting from 4-chloro-3-{[6-(4-hydroxyphenyl)pyrazin-2-yl]amino}benzoic acid (Intermediate 17) (10 mg 0.029 mmol), *N,N*-diethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (7.2 mg).
HPLC 97% R_{T}=1.67 (10-97% MeCN over 3 minutes).
MS (electrospray) m/z ½(M+2H+ ) 221 (60%), M+H+ 440 (100%).

### Example 139

### N-[2-(dimethylamino)ethyl]-3-{[6-(4-hydroxyphenyl)pyrazin-2-yl]amino}-4-methoxybenzamide trifluoroacetate (salt)

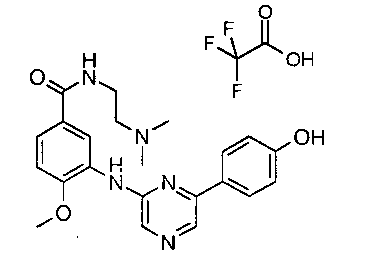

The title compound was synthesised using general procedure 3 starting from 3-{[6-(4-hydroxyphenyl)pyrazin-2-yl]amino}-4-methoxybenzoic acid (Intermediate 18) (10 mg 0.029 mmol), *N,N*-dimethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give give a yellow gum (5.1 mg).

HPLC 100% R_{T}=1.48 (10-97% MeCN over 3 minutes).
MS (electrospray) m/z ½(M+2H+ ) 205 (40%), M+H+ 408 (100%).

### Example 140

### 4-[6-({2-methoxy-5-[(4-methylpiperazin-1-yl)carbonyl]phenyl}amino)pyrazin-2-yl]phenol trifluoroacetate (salt)

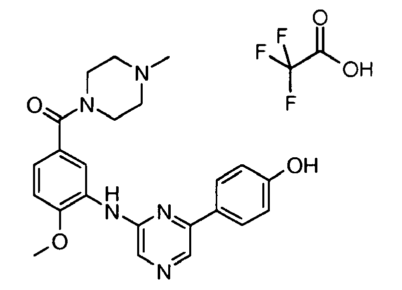

The title compound was synthesised using general procedure 3 starting from 3-{[6-(4-hydroxyphenyl)pyrazin-2-yl]amino}-4-methoxybenzoic acid (Intermediate 18) (10 mg 0.029 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give give a yellow gum (6.8 mg).
HPLC 100% R_{T}=1.43 (10-97% MeCN over 3 minutes).
MS (electrospray) m/z ½(M+2H+ ) 211 (100%), M+H+ 420 (90%).

### Example 141

### N-[3-(dimethylamino)propyl]-3-{[6-(4-hydroxyphenyl)pyrazin-2-yl]amino}-4-methoxybenzamide trifluoroacetate (salt)

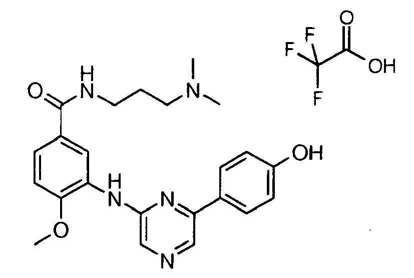

The title compound was synthesised using general procedure 3 starting from 3-{[6-(4-hydroxyphenyl)pyrazin-2-yl]amino}-4-methoxybenzoic acid (Intermediate 18) (10 mg 0.029 mmol), *N,N*-dimethylpropane-1,3-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (8.2 mg).
HPLC 100% R_{T}=1.49 (10-97% MeCN over 3 minutes).
MS (electrospray) m/z ½(M+2H+ ) 212 (40%), M+H+ 422 (100%).

### Example 142

### 3-{[6-(4-hydroayphenyl)pyrazin-2-yl]amino}-4-methoxy-N-methyl-N-(1-methylpyrrolidin-3-yl)benzamide trifluoroacetate (salt)

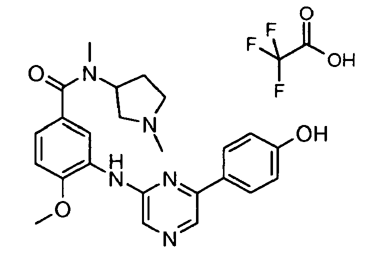

The title compound was synthesised using general procedure 3 starting from 3-{[6-(4-hydroxyphenyl)pyrazin-2-yl]amino}-4-methoxybenzoic acid (Intermediate 18) (10 mg 0.029 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.9 mg).
HPLC 98% R_{T}=1.53 (10-97% MeCN over 3 minutes).
MS (electrospray) m/z ½(M+2H+ ) 218 (90%), M+H+ 434 (100%).

### Example 143

### N-[2-(diethylamino)ethyl]-3-{[6-(4-hydroxyphenyl)pyrazin-2-yl]amino}-4-methoxybenzamide trifluoroacetate (salt)

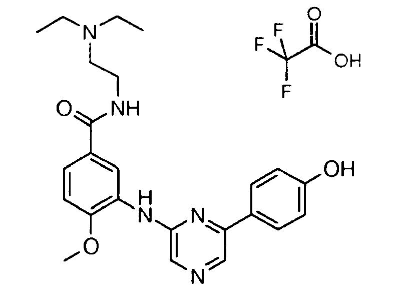

The title compound was synthesised using general procedure 3 starting from 3-{[6-(4-hydroxyphenyl)pyrazin-2-yl]amino}-4-methoxybenzoic acid (Intermediate 18) (10 mg 0.029 mmol), *N,N*-diethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.3 mg).
HPLC 98% R_{T}=1.58 (10-97% MeCN over 3 minutes).
MS (electrospray) m/z ½(M+2H+ ) 219 (45%), M+H+ 436 (100%).

### Example 144

### 4-[6-({2-methyl-5-[(4-methylpiperazin-1-yl)carbonyl]phenyl}amino)pyrazin-2-yl]phenol trifluoroacetate (salt)

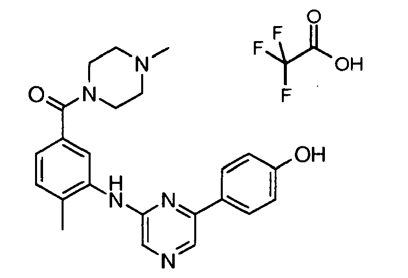

The title compound was synthesised using general procedure 3 starting from 3-{[6-(4-hydroxyphenyl)pyrazin-2-yl]amino}-4-methylbenzoic acid (Intermediate 19) (10 mg 0.031 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (6.3 mg).
HPLC 100% R_{T}=1.44 (10-97% MeCN over 3 minutes).
MS (electrospray) m/z ½(M+2H+ ) 203 (100%), M+H+ 404 (80%), 2M+H+ 807 (90%), 2M+Na+ 829 (25%).

### Example 145

### {[6-(4-hydroxyphenyl)pyrazin-2-yl]amino}-N,4-dimethyl-N-(1-methylpyrrolidin-3-yl)benzamide trifluoroacetate (salt)

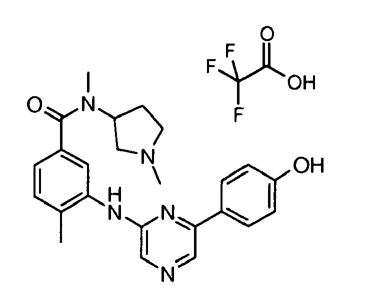

The title compound was synthesised using general procedure 3 starting from 3-{[6-(4-hydroxyphenyl)pyrazin-2-yl]amino}-4-methylbenzoic acid (Intermediate 19) (10 mg 0.031 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (5.3 mg).
HPLC 96% R_{T}=1.52 (10-97% MeCN over 3 minutes).
MS (electrospray) m/z ½(M+2H+ ) 210 (80%), M+H+ 418 (100%).

### Intermediate 20

### 3-[(6-chloropyrazin-2-yl)amino]-4-methoxybenzoic acid

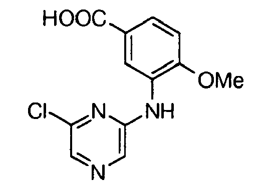

A mixture of 2,6-dichloropyrazine (200 mg, 1.35 mmol), 3-amino 4-methoxy benzoic acid (251 mg, 1.5 mmol), Pd₂(dba)₃ (10 mg), Xantphos (20 mg) and NaOtBu (288 mg, 3 mmol) was suspended in dioxane (10 ml) and stirred under nitrogen at135°C in a sealed tube for 3h. The resulting suspension was partitioned between ethyl acetate and water. The aqueous phase was neutralised with a few drops of conc. hydrochloric acid to produce a precipitate. Back extraction of the mixture with ethyl acetate followed by drying and concentration of the organic phase gave the title product a brown solid, used without further purification.

### Intermediate 21

### 4-[(6-chloropyrazin-2-yl)amino]-3-methoxybenzoic acid

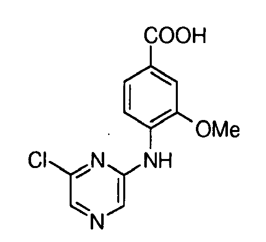

A mixture of 2,6-dichloropyrazine (200 mg, 1.35 mmol), 4-amino 3-methoxy benzoic acid (251 mg, 1.5 mmol), Pd₂(dba)₃ (10 mg), Xantphos (20 mg) and NaOtBu (288 mg, 3 mmol) was suspended in dioxane (10 ml) and stirred under nitrogen at135°C in a sealed tube for 3h. The resulting suspension was partitioned between ethyl acetate and water. The aqueous phase was neutralised with a few drops of conc. hydrochloric acid to produce a precipitate. Back extraction of the mixture with ethyl acetate followed by drying and concentration of the organic phase gave the title product a brown solid (289 mg, 77%, >90% purity).

### Intermediate 22

### 4-{[6-(1H-indol-2-yl)pyrazin-2-yl]amino}-3-methoxybenzoic acid

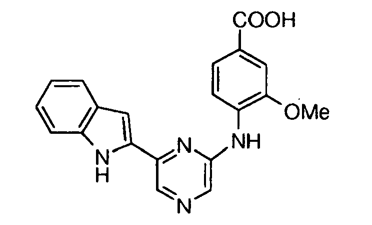

A mixture of crude 4-[(6-chloropyrazin-2-yl)amino]-3-methoxybenzoic acid (Intermediate 21) (125 mg, 0.45 mmol), *N*-Boc-indol-2-yl boronic acid (155 mg, 0.6 mmol), palladium tetrakis(triphenylphosphine) (10 mg), sodium bicarbonate (150 mg) in DME (3.5 ml) and water (1 ml) was heated in a microwave reactor at 120°C for 900 s to effect the Suzuki coupling. The reaction mixture was subsequently reheated to 150°C for 300 s in order to effect the deprotection step. The reaction mixture was partitioned between ethyl acetate and K₂CO₃ (2x20ml) and the pH of the combined aqueous phases adjusted to pH 7. The aqueous phase was extracted with ethyl acetate (2 x 25 ml) and the combined organic phases dried (MgSO₄) and filtered. The solution was concentrated *in vacuo* and the crude (55 mg, 90%+ pure) used without further purification.

### Intermediate 23

### 3-{[6-(1-benzofuran-2-yl)pyrazin-2-yl]amino}-4-methoxybenzoic acid

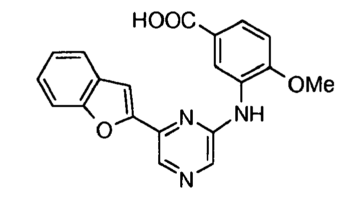

A mixture of crude 3-[(6-chloropyrazin-2-yl)amino]-4-methoxybenzoic acid (Intermediate 20) (125 mg, 0.45 mmol), 1-benzofuran 2-yl boronic acid (97 mg, 0.6 mmol), palladium tetrakis(triphenylphosphine) (10 mg), sodium bicarbonate (150 mg) in DME (3.5 ml) and water (1 ml) was heated in a microwave reactor at 120°C for 900 s. The reaction mixture was partitioned between ethyl acetate and K₂CO₃ (2 x 20 ml) and the pH of the combined aqueous phases adjusted to pH 7. The aqueous phase was extracted with ethyl acetate (2 x 25 ml) and the combined organic phases dried (MgSO₄) and filtered. The solution was concentrated *in vacuo* and the crude (44 mg, 90%+ pure) used without further purification.

### Intermediate 24

### 4-{[6-(1-benzofuran-2-yl)pyrazin-2-yl]amino}-3-methoxybenzoic acid

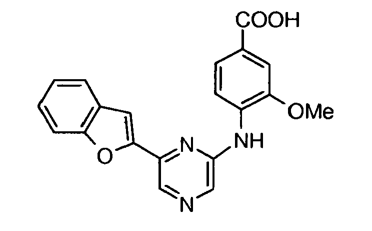

A mixture of crude 4-[(6-chloropyrazin-2-yl)amino]-3-methoxybenzoic acid (Intermediate 21) (125 mg, 0.45 mmol), 1-benzofuran 2-yl boronic acid (97 mg, 0.6 mmol), palladium tetrakis(triphenylphosphine) (10 mg), sodium bicarbonate (150 mg) in DME (3.5 ml) and water (1 ml) was heated in a microwave reactor at 120°C for 900 s. The reaction mixture was partitioned between ethyl acetate and K₂CO₃ (2 x 20 ml) and the pH of the combined aqueous phases adjusted to pH 7. The aqueous phase was extracted with ethyl acetate (2 x 25 ml) and the combined organic phases dried (MgSO₄) and filtered. The solution was concentrated *in vacuo* and the crude (36 mg, 90%+ pure) used without further purification.

### Example 146

### 6-(1H-indol-2-yl)-N-{2-methoxy-4-[(4-methylpiperazin-1-yl)carbonyl]phenyl}pyrazin-2-amine trifluoroacetate

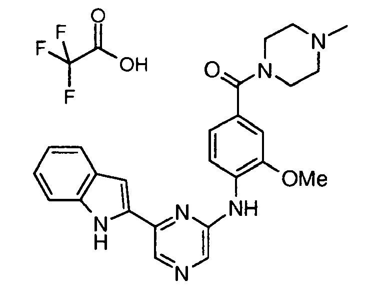

The title compound was synthesised using general procedure 3 starting from 4-{[6-(1H-indol-2-yl)pyrazin-2-yl]amino}-3-methoxybenzoic acid (Intermediate 22) (10 mg 0.03 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (8.6 mg, 56%).
HPLC 97% R_{T}=1.79 (System A 10-97% MeCN over 3 minutes); 98% R_{T}=1.61 (System B 10-97% MeCN over 3 minutes).
MS (electrospray) m/z ½(M+2H+ ) 222, (M+H+) 443, (2M+H+) 885.

### Example 147

### 4-{[6-(1H-indol-2-yl)pyrazin-2-yl]amino}-3-methoxy-N-methyl-N-(1-methylpyrrolidin-3-yl)benzamide trifluoroacetate

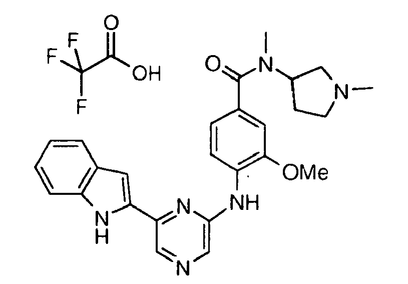

The title compound was synthesised using general procedure 3 starting from 4-{[6-(1H-indol-2-yl)pyrazin-2-yl]amino}-3-methoxybenzoic acid, (Intermediate 22) (10 mg 0.03 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (6.6 mg, 41%).
HPLC 95% R_{T}=1.87 (System A 10-97% MeCN over 3 minutes); 96% R_{T}=1.69 (System B 10-97% MeCN over 3 minutes).
MS (electrospray) m/z ½(M+2H+ ) 229, M+H+ 457 (100%).

### Example 148

### 4-{[6-(1-Benzofuran-2-yl)pyrazin-2-yl]amino}-3-methoxy-N-methyl-N-(1-methylpyrrolidin-3-yl)benzamide trifluoroacetate

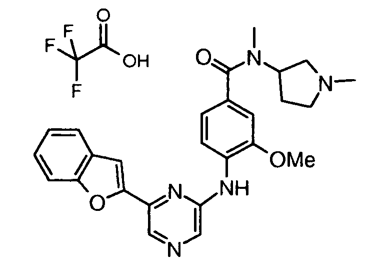

The title compound was synthesised using general procedure 3 starting from 4-{[6-(1-benzofuran-2-yl)pyrazin-2-yl]amino}-3-methoxybenzoic acid (Intermediate 24) (10 mg 0.03 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4. 2mg, 27%).
HPLC 90% R_{T}=1.99 (System A 10-97% MeCN over 3 minutes); 90% R_{T}=1.80 (System B 10-97% MeCN over 3 minutes).
MS (electrospray) m/z ½(M+2H+ ) 230 (70%), (M+H+) 458 (100%).

### Example 149

### 3-{[6-(1-Benzofuran-2-yl)pyrazin-2-yl]amino}-4-methoxy-N-methyl-N-(1-methylpyrrolidin-3-yl)benzamide trifluoroacetate

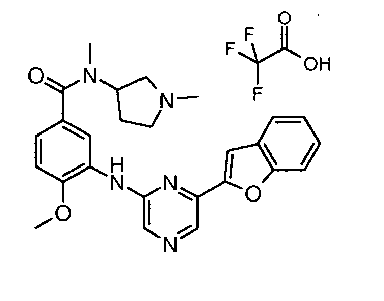

The title compound was synthesised using general procedure 3 starting from crude 3-{[6-(1-benzofuran-2-yl)pyrazin-2-yl]amino}-4-methoxybenzoic acid (Intermediate 23) (10 mg 0.03 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (3.7 mg, 23%).
HPLC 96% R_{T}=1.88 (System A 10-97% MeCN over 3 minutes); 97% R_{T}=1.70 (System B 10-97% MeCN over 3 minutes).
MS (electrospray) m/z ½(M+2H+ ) 230, (M+H+) 458.

### Example 150

### 6-(1H-indol-2-yl)-N-[2-methoxy-4-(morpholin-4-ylcarbonyl)phenyl]pyrazin-2-amine trifluoroacetate

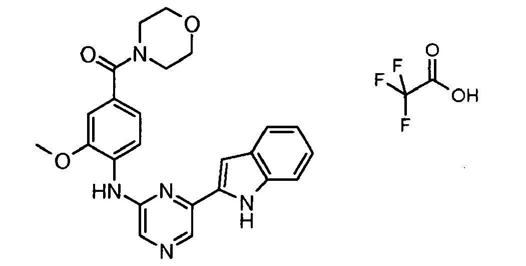

The title compound was synthesised using general procedure 3 starting from 4-{[6-(1H-indol-2-yl)pyrazin-2-yl]amino}-3-methoxybenzoic acid, (Intermediate 22) (10 mg 0.03 mmol), morpholine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.0 mg, 27%).
HPLC 96% R_{T}=2.19 (System A 10-97% MeCN over 3 minutes).
MS (electrospray) m/z (M+H+) 430.

### Example 151

### N-[2-(diethylamino)ethyl]-4-{[6-(1H-indol-2-yl)pyrazin-2-yl]amino}-3-methoxybenzamide trifluoroacetate

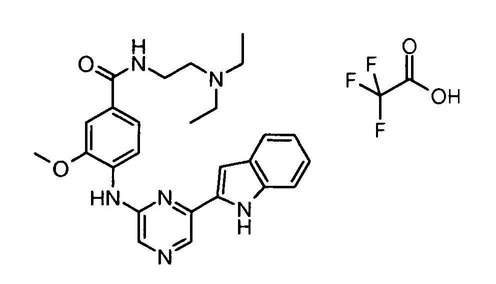

The title compound was synthesised using general procedure 3 starting from 4-{[6-(1H-indol-2-yl)pyrazin-2-yl]amino}-3-methoxybenzoic acid, (Intermediate 22) (10 mg 0.03 mmol), *N,N*-diethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (7.6 mg, 48%).
HPLC 99% R_{T}=1.95 (System A 10-97% MeCN over 3 minutes.
MS (electrospray) m/z ½(M+2H+ ) 230 (40%), (M+H+) 459.

### Example 152

### N-[3-(dimethylamino)propyl]-4-{[6-(1H-indol-2-yl)pyrazin-2-yl]amino}-3-methoxybenzamide trifluoroacetate

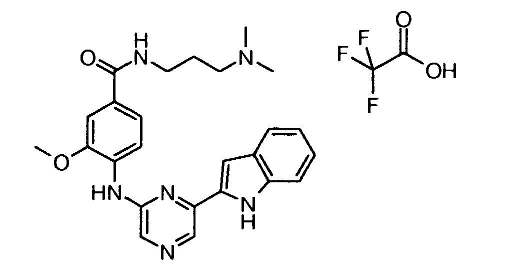

The title compound was synthesised using general procedure 3 starting from 4-{[6-(1H-indol-2-yl)pyrazin-2-yl]amino}-3-methoxybenzoic acid, (Intermediate 22) (10 mg 0.03 mmol), *N,N*-dimethylpropane-1,3-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (6.7 mg, 43%).
HPLC 99% R_{T}=1.97 (System A 10-97% MeCN over 3 minutes).
MS (electrospray) m/z ½(M+2H+) 223 (35%), (M+H+) 445.

### Example 153

### N-(4-{[3-(dimethylamino)pyrrolidin-1-yl]carbonyl}-2-methoxyphenyl)-6-(1H-indol-2-yl)pyrazin-2-amine

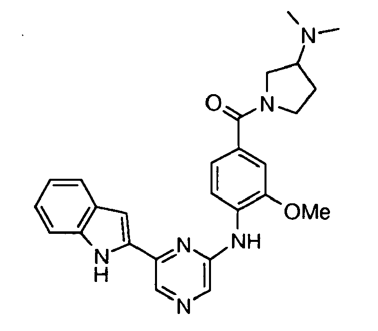

1.6 g (11.0 mmol) of 2,6-dichloropyrazine was dissolved in 150 mL dioxane and to the solution were 2.0 g (12.0 mmol) of 4-amino-3-methoxybenzoic acid, 2.1 g (22.0 mmol) of NaOtBu, 160 mg Xantphos and 80 mg Pd₂dba₃ added. The mixture was heated at 135°C overnight and then partioned between water and EtOAc, and the aques phase was treated with a few drops of conc HCl and then extracted with EtOAc several times. The collected organic phases after acidification were combined, filtrated and concentrated to give 4-[(6-chloropyrazin-2-yl)amino]-3-methoxybenzoic acid as a yellow solid, amount 2.5 g.

2.5 g (9.0 mmol) of 4-[(6-chloropyrazin-2-yl)amino]-3-methoxybenzoic acid was dissolved in 150 mL DME, and 2.6 g (10.0 mmol) 1-Boc-indole-2-boronic acid, 960 mg (31.0 mmol) NaHCO₃ and 125 mg of Pd(tetrakis) were added as well as 25 mL water. Stir at 100°C for 5 hrs. The mixture was portioned between water and EtOAC and the organic phase was collected, and then concentrated. Finally redissolved in 50:50 MeOH/ACN to give a brown precipite, 4-({6-[1-(tert-butoxycarbonyl)-1H-indol-2-yl]pyrazin-2-yl}amino)-3-methoxybenzoic acid, amount 4.0 g.

1.91 g (4.2 mmol) of 4-({6-[1-(tert-butoxycarbonyl)-1H-indol-2-yl]pyrazin-2-yl}amino)-3-methoxybenzoic acid was dissolved in 100 mL DMF and 568 mg (5.0 mmol) of 3-(Dimethylamino)pyrrolidine, 2.0 mg (6.3 mmol) TBTU and 505 mg (5.0 mmol) TEA were added. The mixture was stirred at room temperature overnight. To the solution was water and DCM added and the organic phase was collected and concentrated to a brown gum, which was then deprotected by 10 vol% conc HCl in MeOH heated at 75°C for 30 mins. Finally purified by preparative HPLC giving the product as a brown gum, amount 1.01 g (yield 52%)
HPLC (system A) 99 %, RT 1.851 min (10-97 % MeCN over 3 min)
HPLC (system B) 97 %, RT 1.643 min (10-97 % MeCN over 3 min)
MS (ESI⁺) for C₂₆H₂₈N₆O₂ m/z 457 (M+H⁺)
¹HNMR (400MHz, MeOD) ppm 1.89-1.95 (m, 2H); 2.06 (s, 6H); 2.25-2.29 (m, 2H); 2.33-2.38 (m, 2H); 3.30-3.35 (m, 4H); 7.05-7.10 (m, 1H); 7.16-7.21 (m, 2H); 7.23-7.27 (m, 1H); 7.29-7.33 (m, 1H); 7.49-7.53 (m, 1H); 7.62 (d, *J*=8.03, 1H); 8.18 (s, 1H); 8.48 (s, 1H); 8.59-8.64 (m, 1H)

### Intermdiate 25

### 4-{[6-(5-fluoro-1H-indol-2-yl)pyrazin-2-yl]amino}-3-methoxybenzoic acid

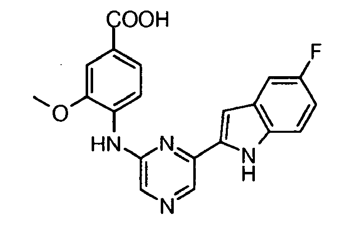

A mixture of crude 4-[(6-chloropyrazin-2-yl)amino]-3-methoxybenzoic acid (Intermediate 21) (125mg, 0.45mmol), *N*-Boc-5-fluoro-indol-2-yl boronic acid (155 mg, 0.6 mmol), palladium tetrakis(triphenylphosphine) (10 mg), sodium bicarbonate (150 mg) in DME (3.5 ml) and water (1 ml) was heated in a microwave reactor at 120°C for 900 s to effect the Suzuki coupling. The reaction mixture was subsequently reheated to 160°C for 600 s in order to effect the deprotection step. The reaction mixture was partitioned between ethyl acetate and K₂CO₃ (2 x 20 ml) and the pH of the combined aqueous phases adjusted to pH 7. The aqueous phase was extracted with ethyl acetate (2 x 25 ml) and the combined organic phases dried (MgSO₄) and filtered. The solution was concentrated *in vacuo* and the crude used without further purification.

### Example 154

### 6-(5-fluoro-1H-indol-2-yl)-N-{2-methoxy-4-[(4-methylpiperazin-1-yl)carbonyl]phenyl}pyrazin-2-amine trifluoroacetate

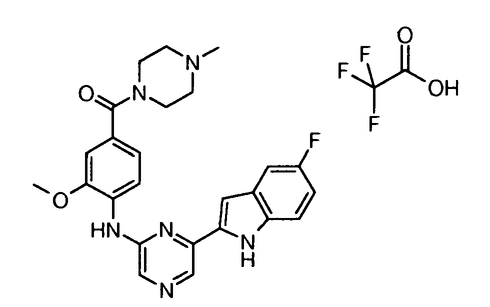

The title compound was synthesised using general procedure 3 starting from 4-{[6-(5-fluoro-1*H*-indol-2-yl)pyrazin-2-yl]amino}-3-methoxybenzoic acid (Intermediate 25) (10 mg 0.03 mmol), 1-methylpiperazine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (5.0 mg, 33%).
HPLC 98% R_{T}=1.97 (System A 10-97% MeCN over 3 minutes).
MS (electrospray) m/z ½(M+2H+) 231 (100%), (M+H+) 461 (90%).

### Example 155

### 4-{[6-(5-fluoro-1H-indol-2-yl)pyrazin-2-yl]amino}-3-methoxy-N-methyl-N-(1-methylpyrrolidin-3-yl)benzamide trifluoroacetate

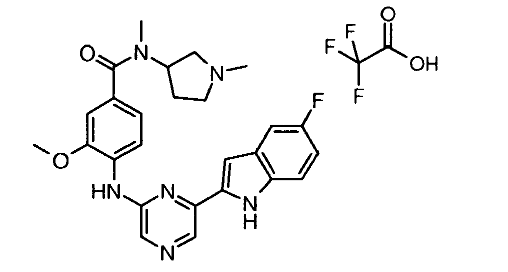

The title compound was synthesised using general procedure 3 starting from 4-{[6-(5-fluoro-1*H*-indol-2-yl)pyrazin-2-yl]amino}-3-methoxybenzoic acid (Intermediate 25) (10 mg 0.03 mmol), *N*,1-dimethylpyrrolidin-3-amine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (3.5 mg, 23%).
HPLC 99% R_{T}=2.06 (System A 10-97% MeCN over 3 minutes).
MS (electrospray) m/z ½(M+2H+) 238 (80%), (M+H+) 475.

### Example 156

### N-[2-(diethylamino)ethyl]-4-{[6-(5-fluoro-1H-indol-2-yl)pyrazin-2-yl]amino}-3-methoxvbenzamide trifluoroacetate

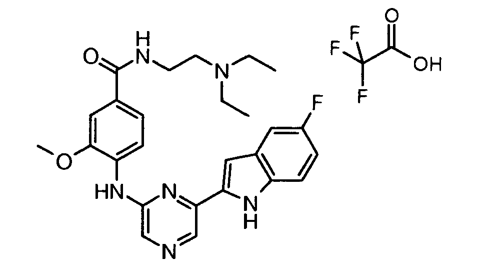

The title compound was synthesised using general procedure 3 starting from 4-{[6-(5-fluoro-1*H*-indol-2-yl)pyrazin-2-yl]amino}-3-methoxybenzoic acid (Intermediate 25) (10 mg 0.03 mmol), *N,N*-diethylethane-1,2-diamine (10 µl), HOBt (6 mg, 0.044 mmol), EDC (8.6 mg, 0.44 mmol) and triethylamine (14 µl, 0.1 mmol) in 0.5 ml THF. The crude material was purified using preparative HPLC to give a yellow gum (4.4 mg, 28%).
HPLC 97% R_{T}=2.17 (System A 10-97% MeCN over 3 minutes).
MS (electrospray) m/z ½(M+2H+ ) 239 (40%), (M+H+) 477.

### Intermediate 26

### tert-butyl 4-[(6-chloropyridin-3-yl)carbonyl]piperazine-1-carboxylate

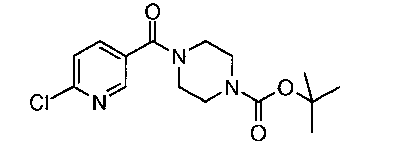

To *N*-Boc-piperazine (7.8 mmol) was added 10 mL 5M NaOH and the mixture was stirred for 5 min. 6-Chloronicotinoyl chloride (9.1 mmol) dissolved in 1 mL dry THF was added and the reaction was stirred for 5 min. The precipitate was collected by filtration. The solid was dissolved in 25 mL DCM and dried over MgSO4. The solvent was removed *in vacuo* to give the title compound (1.78 g, 70%) 1H NMR (400 MHz, CHLOROFORM-D) δ ppm 1.46 (s, 9 H) 3.30 - 3.82 (m, 8 H) 7.40 (dd, *J*=8.16, 0.63 Hz, 1 H) 7.72 (dd, *J*=8.16, 2.38 Hz, 1 H) 8.43 (dd, *J*=2.38, 0.63 Hz, 1 H).

### Intermediate 27

### tert-butyl 4-[(6-chloropyridin-3-yl)carbonyl]piperazine-1-carboxylate

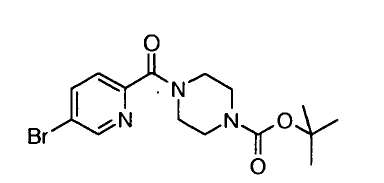

To 5-bromo-2-pyridinecarboxylic acid (6.1 mmol) was added SOCl₂ (5 ml) and the reaction was refluxed for 1 hour. The SOCl₂ was removed under vacuum. The residual acid chloride was suspended in 3 mL dry THF and was added to a mixture of *N*-Boc-piperazine (5.4 mmol) and 10 mL 5M NaOH under stirring. After 5 min, 40 mL H₂O was added and after another 5 min the precipitate was collected by filtration. The solid was dissolved in 25 mL DCM and dried over MgSO4. The solvent was removed *in vacuo* to give the title compound (1.18 g, 67%). 1H NMR (400 MHz, CHLOROFORM-D) □ ppm 1.46 (s, 9 H) 3.42 - 3.79 (m, 8 H) 7.60 (d, *J*=8.28 Hz, 1 H) 7.93 (dd, *J*=8.28, 2.26 Hz, 1 H) 8.62 (d, *J*=2.01 Hz, 1 H).

### Intermediate 28

### tert-butyl 4-[(4-bromophenyl)carbonyl]piperazine-1-carboxylate

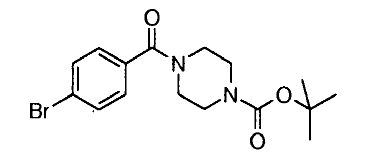

To *N*-Boc-piperazine (7.8 mmol) was added 10 mL 5 M NaOH (aq) and the mixture was stirred for 5 min. 4-Bromobenzoyl chloride (9.1 mmol) dissolved in 1 mL dry THF was added and the reaction was stirred for 5 min and the precipitate was collected by filtration. The solid was dissolved in 25 mL DCM and dried over MgSO₄. The solvent was removed in *vacuo* to give the title compound (0.93 g, 32%).

### Intermediate 29

### tert-butyl 2-(6-aminopyrazin-2-yl)-5-fluoro-1H-indole-1-carboxylate

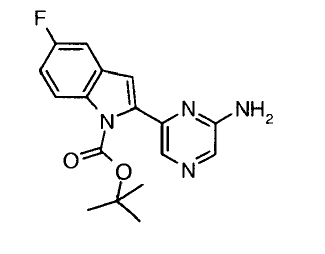

To 2-amino-6-chloropyrazine (3.1 mmol), 1-Boc-5-fluoroindole-2-boronic acid (3.1 mmol), Pd(PPh3)₄, (100 mg, 0.0865 mmol) and K₂CO₃ (7.2 mmol) were added 7 mL MeCN and 3 mL H₂O and the mixture was heated in a sealed tube at 80 °C for 1 h. The water layer was separated and the organic phase was dried over MgSO₄. Filtration and removal of the solvent gave 1.05 g of the title compound.

### Intermediate 30

### tert-butyl 2-(5-aminopyridin-3-yl)-5-fluoro-1H-indole-1-carboxylate

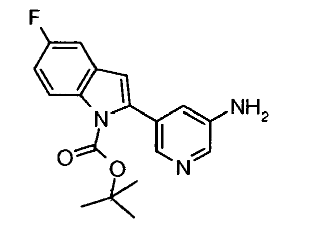

To 3-amino-5-bromo-pyridine (2.9 mmol), 1-Boc-5-fluoroindole-2-boronic acid (2.9 mmol), Pd(PPh₃)₄ (100 mg, 0.0865 mmol) and K₂CO₃ (7.2 mmol) were added 7 mL MeCN and 3 mL H₂O and the mixture was heated in a sealed tube at 80 °C for 1 h. The water layer was separated and the organic phase was dried over MgSO₄. Filtration and removal of the solvent gave 1.11 g of the title compound.

### Intermediate 31

### tert-butyl 2-(5-aminopyridin-3-yl)-1H-indole-1-carboxylate

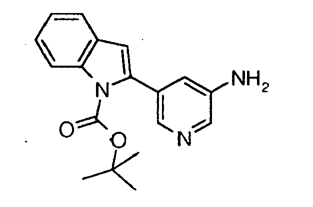

To 3-amino-5-bromo-pyridine (2.2 mmol), 1-Boc-indole-2-boronic acid (2.2 mmol), Pd(PPh₃)₄ (70 mg, 0.061 mmol) and K₂CO₃ (7.2 mmol) was added 7 mL MeCN and 3 mL H₂O and the mixture was heated in a sealed tube at 80 °C for 1 h. The water layer was separated and the organic phase was dried over MgSO₄. Filtration and removal of the solvent gave 0.80 g of the title compound.

### Example 157

### 6-(5-fluoro-1H-indol-2-yl)-N-[6-(piperazin-1-ylcarbonyl)pyridin-3-yl]pyrazin-2-amine bis(trifluoroacetate)

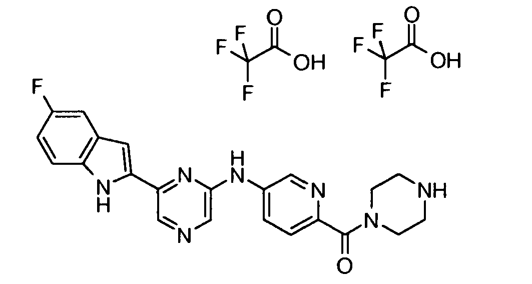

Pd(OAc)₂ (3 mg, 0.01 mmol) and (±)-BINAP (8 mg, 0.01 mmol) were dissolved in 5 mL dry toluene. The solution was added to a mixture of Intermediate 29 (50 mg, 0.15 mmol), Intermediate 27 (60 mg, 0.16 mmol) and K₂CO₃ (700 mg, 5.06 mmol) in 10 mL dry toluene. The reaction was heated at 120 °C in a sealed tube. After 3 h the mixture was filtrated and the solvent was removed *in vacuo.* The crude material was dissolved in a mixture of 0.5 mL DCM and 0.5 mL TFA. After 1 h, the solvent was removed *in vacuo* and about half of the residue was purified using preparative HPLC system A.
Yield 7 mg (14%) 1H NMR (400 MHz, MeOD) d ppm 3.36 - 3.42 (m, 4 H) 4.02 - 4.10 (m, 4 H) 6.94 - 7.04 (m, 1 H) 7.20 (s, 1 H) 7.29 (dd, *J*=9.54, 2.51 Hz, 1 H) 7.47 (dd, *J*=8.91, 4.39 Hz, 1 H) 7.87 (s, 1 H) 8.14 (s, 1 H) 8.58 (s, 1 H) 8.62 (dd, *J*=8.53, 2.01 Hz, 1 H) 9.03 (s, 1 H). HPLC 100%, *t*_{R} = 1.75 min (System A), 100%, *t*_{R} = 1.36 min (System B). MS(electronspray) M+H+ m/z 418

### General procedure 4 for the synthesis of Examples 158-160.

Pd(OAc)₂ (5 mg, 0.02 mmol) and (±)-BINAP (15 mg, 0.024 mmol) was dissolved in 2 mL dry toluene. The solution was added to a mixture of Intermediate 30 (50 mg, 0.15 mmol), the requisite aryl- or heteroaryl halide (0.15 mmol) and K₂CO₃ (500 mg, 3.6 mmol) and another 5 mL of dry toluene was added. The mixture was heated at 120 °C in a sealed tube for 1 h. The reaction mixture was filtrated and the solvent was removed under vacuum. The residue was dissolved in a mixture of 0.5 mL DCM and 0.5 mL TFA. After 1 h the solvent was removed *in vacuo* and about half of the crude material was purified using preparative HPLC system E.

### Example 158

### N-[5-(5-fluoro-1H-indol-2-yl)pyridin-3-yl]-5-(piperazin-1-ylcarbonyl)pyridin-2-amine

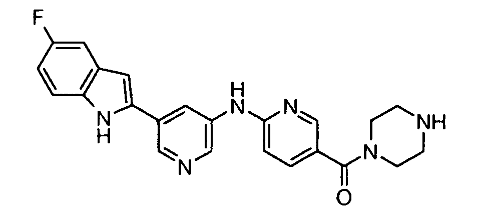

The title compound was made from Intermediate 30 and Intermediate 26 according to general procedure 4. Yield 5 mg. 1H NMR (400 MHz, MeOD) □ ppm 2.87 (s, 4 H) 3.58 - 3.70 (m, 4 H) 6.87 - 6.95 (m, 3 H) 7.23 (dd, *J*=9.54, 2.51 Hz, 1 H) 7.38 (dd, *J*=9.03, 4.52 Hz, 1 H) 7.70 (dd, *J*=8.53, 2.51 Hz, 1 H) 8.37 (d, *J*=2.26 Hz, 1 H) 8.55 (s, 1 H) 8.69 (s, 1 H) 8.74 (t, *J*=2.13 Hz, 1 H) HPLC 100%, *t*_{R} = 1.48 min (System A), 96%, *t*_{R} = 1.27 min (System B). MS(electronspray) (M+H)+ m/z 417

### Example 159

### 5-(5-fluoro-1H-indol-2-yl)-N-[6-(piperazin-1-ylcarbonyl)pyridin-3-yl]pyridin-3-amine

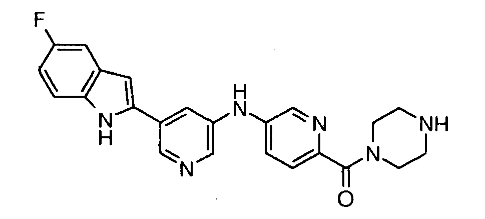

The title compound was made from Intermediate 30 and Intermediate 27 according to general procedure 4. Yield 11 mg. 1H NMR (400 MHz, MeOD) □ ppm 2.78 - 2.95 (m, 4 H) 3.57 - 3.79 (m, 4 H) 6.87 - 6.94 (m, 2 H) 7.21 (dd, *J*=9.66, 2.38 Hz, 1 H) 7.35 (dd, *J*=8.91, 4.39 Hz, 1 H) 7.58 (d, *J*=8.53 Hz, 1 H) 7.67 - 7.72 (m, *J*=8.53, 2.76 Hz, 1 H) 7.97 - 8.00 (m, 1 H) 8.28 (d, *J*=2.26 Hz, 1 H) 8.38 (d, *J*=2.51 Hz, 1 H) 8.57 (d, *J*=1.51 Hz, 1 H). HPLC 100%, *t*_{R} = 1.43 min (System A), 100%, *t*_{R} = 1.23 min (System B). MS(electronspray) (M+H)+ m/z 417

### Example 160

### 5-(5-fluoro-1H-indol-2-yl)-N-[4-(piperazin-1-ylcarbonyl)phenyl]pyridin-3-amine

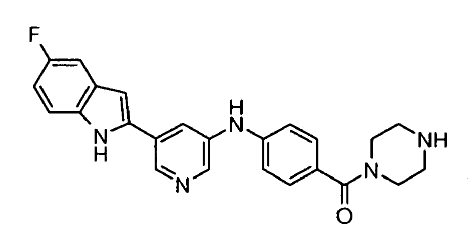

The title compound was made from Intermediate 30 and Intermediate 28 according to general procedure A. Yield 8 mg (25%). 1 H NMR (400 MHz, MeOD) d ppm 2.79 - 2.90 (m, 4 H) 3.54 - 3.71 (m, 4 H) 6.86 - 6.93 (m, 2 H) 7.18 - 7.25 (m, 3 H) 7.35 (dd, *J*=8.78, 4.52 Hz, 1 H) 7.37 - 7.43 (m, 2 H) 7.94 - 7.98 (m, 1 H) 8.25 (d, *J*=2.26 Hz, 1 H) 8.50 (d, *J*=1.76 Hz, 1 H). HPLC 100%, *t*_{R} = 1.48 min (System A), 96%, *t*_{R} = 1.27 min (System B). MS(electronspray) (M+H)+ m/z 416

### Intermediate 32

### 6-chloro-N-[2-methoxy-4-(piperazin-1-ylcarbonyl)phenyl]pyrazin-2-amine

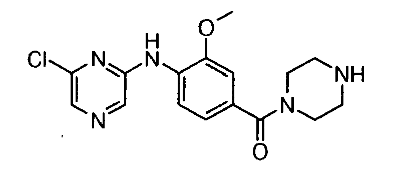

6-chloro-N-(4-{4-(boc-piperazinyl)carbonyl}-2-methoxyphenyl)pyrazin-2-amine (which was synthesized using the same general procedure as Intermediate 45) (100 mg, 0.223 mmol) was dissolved in 1 mL DCM. 1 mL TFA was added and the reaction mixture was stirred in a sealed tube for 90 min. The solvent was removed *in vacuo* and the product was dissolved in 20 mL DCM and washed with NaHCO3 (aq). The solution was dried (MgSO₄) and the solvent was removed in *vacuo* to give 30 mg (39%) of the title compound.

### Example 161

### 6-(6-methoxy-1H-indol-2-yl)-N-[2-methoxy-4-(piperazin-1-ylcarbonyl)phenyl]pyrazin-2-amine

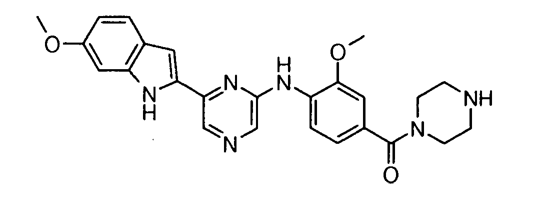

To Intermediate 34 (15 mg, 0.052 mmol), Intermediate 32 (15 mg, 0.043 mmol), Pd(PPh₃)₄ (15 mg, 0.013 mmol) and K₂CO₃ (50 mg, 0.36 mmol) were added 0.5 mL MeCN and 0.2 mL H₂O. The reaction was heated in the micro oven for 5 min at 120 °C. The aqueaous layer was removed with a pipette and the remaining mixture was heated for 5 min at 160 °C. The crude reaction mixture was filtrated and the residue purified using preparative HPLC system E to give the title compound (7 mg, 29%). 1H NMR (400 MHz, MeOD) □ ppm 2.82 - 2.91 (m, 4 H) 3.58 - 3.72 (m, 4 H) 3.85 (s, 3 H) 3.98 (s, 3 H) 6.72 (dd, *J*=8.53, 2.26 Hz, 1 H) 7.01 (d, *J*=2.26 Hz, 1 H) 7.09 (d, *J*=1.00 Hz, 1 H) 7.11 (d, *J*=2.01 Hz, 1 H) 7.15 (dd, *J*=8.28, 1.76 Hz, 1 H) 7.46 (d, *J*=8.78 Hz, 1 H) 8.13 (s, 1 H) 8.41 (s, 1 H) 8.63 (d, *J*=8.03 Hz, 1 H). HPLC 100%, *t*_{R} = 1.79 min (System A), 100%, *t*_{R} = 1.60 min (System B). MS(electronspray) (M+H)+ m/z 459.

### Example 162

### N-(4-{[(3R)-3-(dimethylamino)pyrrolidin-1-yl]carbonyl}phenyl)-6-(1H-indol-2-yl)pyrazin-2-amine trifluoroacetate

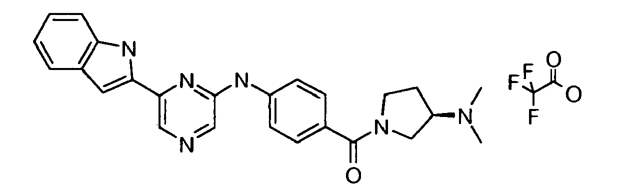

2,6-Dichloropyrazine (0.40 g, 2.7 mmol), 4-aminobenzoic acid (0.41 g, 3.0 mmol), NaO*^{t}*Bu (0.52 g, 5.3 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 4-[(6-chloropyrazin-2-yl)amino]benzoic acid. Half of the obtained crude 4-[(6-chloropyrazin-2-yl)amino]benzoic acid (theoretically containing 335 mg, 1.34 mmol) was dissolved in DME/water. [1-(tert-Butoxycarbonyl)-H-indol-2-yl]boronic acid (350 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 4-{[6-(H-indol-2-yl)pyrazin-2-yl]amino}benzoic acid (30 mg). C 19H 14N402 m/z 331 (M+H)+.
To 4-{[6-(H-indol-2-yl)pyrazin-2-yl]amino}benzoic acid (10 mg, 0.03 mmol) in dry DMF (1.5 mL) were added NEt₃ (13 µL, 0.09 mmol), 2,4,6-tripropyl-1,3,5,2,4,6-trioxatriphosphorinane-2,4,6-trioxide (50% in EtOAc, 54 µL, 0.09 mmol) and (3*R*)-*N,N*-dimethylpyrrolidin-3-amine (10 mg, 0.09 mmol). The mixture was stirred at rt over night. 1 drop of water was added and the mixture was concentrated in *vacuo.* Purification was performed by preparative HPLC (ACE C8, 0.1% TFA, MeCN) to give the title compound as a brown gum (4 mg). HPLC 99% (System A), 99% (System B). 1H NMR (400 MHz, MeOD) □ ppm 2.13 - 2.27 (m, 1 H) 2.42 - 2.57 (m, 1 H) 3.16 - 3.24 (m, 1 H) 3.73 - 3.93 (m, 3 H) 4.05 - 4.13 (m, 1 H) 7.02 - 7.08 (m, 1 H) 7.14 - 7.21 (m, 2 H) 7.46 - 7.51 (m, 1 H) 7.58 - 7.62 (m, 1 H) 7.62 - 7.67 (m, 2 H) 7.92 - 7.99 (m, 2 H) 8.05 (s, 1 H) 8.48 (s, 1 H).
MS (ESI+) calcd for C₂₅H₂₆N₆O 426.2168, found 426.2160.

### Example 163

### N-(4-{[(3S)-3-(dimethylamino)pyrrolidin-1-yl]carbonyl}phenyl)-6-(1H-indol-2-yl)pyrazin-2-amine trifluoroacetate

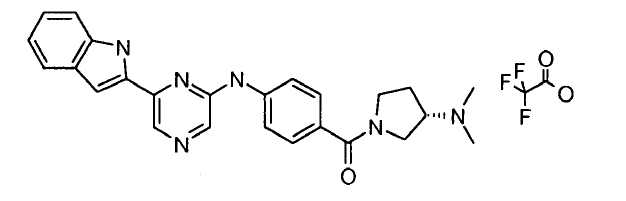

2,6-Dichloropyrazine (0.40 g, 2.7 mmol), 4-aminobenzoic acid (0.41 g, 3.0 mmol), NaOtBu (0.52 g, 5.3 mmol), Xantphos (45 mg, 0.08 mmol) and Pd2(dba)3 (25 mg, 0.027 mmol) were stirred in dry dioxane (10 mL) for XX min?. The reaction mixture was refluxed at 135 °C for 4 hours, then allowed to attain rt. The mixture was concentrated under reduced pressure to give crude 4-[(6-chloropyrazin-2-yl)amino]benzoic acid. Half of the obtained crude 4-[(6-chloropyrazin-2-yl)amino]benzoic acid (theoretically containing 335 mg, 1.34 mmol) was dissolved in DME/water. [1-(tert-Butoxycarbonyl)-H-indol-2-yl]boronic acid (350 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 4-{[6-(H-indol-2-yl)pyrazin-2-yl]amino}benzoic acid (30 mg). C19H14N4O2 m/z 331 (M+H)+. To 4-{[6-(H-indol-2-yl)pyrazin-2-yl]amino}benzoic acid (10 mg, 0.03 mmol) in dry DMF (1.5 mL) were added NEt3 (13 µL, 0.09 mmol), 2,4,6-tripropyl-1,3,5,2,4,6-trioxatriphosphorinane-2,4,6-trioxide (50% in EtOAc, 54 µL, 0.09 mmol) and (3*S*)-N,N-dimethylpyrrolidin-3-amine (10 mg, 0.09 mmol). The mixture was stirred at rt over night. 1 drop of water was added and the mixture was concentrated *in vacuo.* Purification was performed by preparative HPLC (ACE C8, 0.1% TFA, MeCN) to give the title compound as a brown gum (4 mg).
HPLC 99%(System A), 99%(System B). 1H NMR (400 MHz, MeOD) □ppm 2.14 - 2.26 (m, 1 H) 2.45 - 2.54 (m, 1 H) 3.16 - 3.26 (m, 1 H) 3.71 - 3.97 (m, 3 H) 4.04 - 4.16 (m, 1 H) 7.02 - 7.08 (m, 1 H) 7.15 - 7.21 (m, 2 H) 7.46 - 7.51 (m, 1 H) 7.58 - 7.62 (m, 1 H) 7.62 - 7.70 (m, 2 H) 7.96 (d, J=8.53 Hz, 2 H) 8.03 - 8.07 (m, 1 H) 8.48 (s, 1 H).
MS (ESI+) calcd for C₂₅H₂₆N₆O 426.2168, found 426.2160.

### Example 164

### N-[2-(diethylamino)ethyl]-2-fluoro-4-{[6-(1H-indol-2-yl)pyrazin-2-yl]amino}benzamide trifluoroacetate

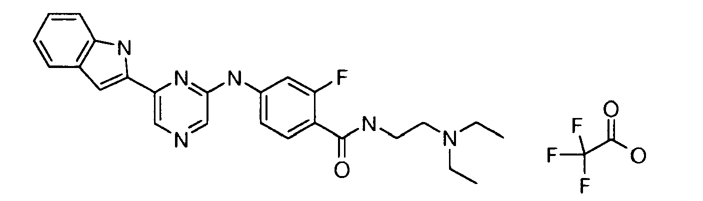

2,6-Dichloropyrazine (0.40 g, 2.7 mmol), 4-amino-2-fluorobenzoic acid (0.46 g, 3.0 mmol), NaO*^{t}*Bu (0.52 g, 5.3 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 4-[(6-chloropyrazin-2-yl)amino]-2-fluorobenzoic acid. Half of the obtained crude 4-[(6-chloropyrazin-2-yl)amino]-2-fluorobenzoic acid (theoretically containing 342 mg, 1.28 mmol) was dissolved in DME/water. [1-(tert-Butoxycarbonyl)-H-indol-2-yl]boronic acid (350 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration in *vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1 % TFA-MeCN) to give 2-fluoro-4-{[6-(1H-indol-2-yl)pyrazin-2-yl]amino}benzoic acid (36 mg). MS (ESI+) for C19H13FN4O2 m/z 349 (M+H)+.
To 2-fluoro-4-{[6-(1H-indol-2-yl)pyrazin-2-yl]amino}benzoic acid (13 mg, 0.04 mmol) in dry DMF (1.5 mL) were added NEt3 (16 µL, 0.1 mmol), 2,4,6-tripropyl-1,3,5,2,4,6-trioxatriphosphorinane-2,4,6-trioxide (50% in EtOAc, 89 µL, 0.15 mmol) and *N,N*-diethylethane-1,2-diamine (16 mg, 0.11 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1% TFA, MeCN) to give the title compound as a brown gum (4 mg). HPLC 95%(System A), 95%(System B).
MS (ESI+) calcd for C₂₅H₂₇FN₆O 446.223, found 446.2224.

### Example 165

### N-[3-chloro-4-(piperazin-1-ylcarbonyl)phenyl]-6-(1H-indol-2-yl)pyrazin-2-amine trifluoroacetate

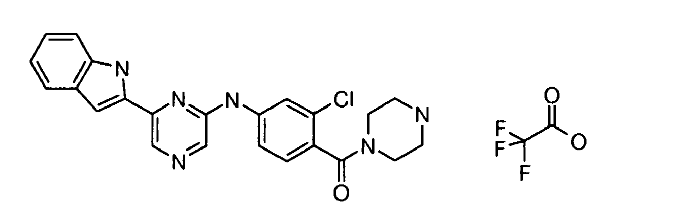

2,6-Dichloropyrazine (0.40 g, 2.7 mmol), 4-amino-2-chlorobenzoic acid (0.51 g, 3.0 mmol), NaOtBu (0.52 g, 5.3 mmol), Xantphos (45 mg, 0.08 mmol) and Pd₂(dba)₃ (25 mg, 0.027 mmol) were stirred in dry dioxane (10 mL) . The reaction mixture was refluxed at 135 °C for 4 hours, then allowed to attain rt. The mixture was concentrated under reduced pressure to give crude 2-chloro-4-[(6-chloropyrazin-2-yl)amino]benzoic acid. Half of the obtained crude 2-chloro-4-[(6-chloropyrazin-2-yl)amino]benzoic acid (theoretically containing 381 mg, 1.34 mmol) was dissolved in DME/water. [1-(tert-Butoxycarbonyl)-H-indol-2-yl]boronic acid (350 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 2-chloro-4-{[6-(1H-indol-2-yl)pyrazin-2-yl]amino}benzoic acid (22 mg). MS (ESI+) for C19H13ClN4O2 m/z 365 (M+H)+.
To 2-chloro-4-{[6-(1H-indol-2-yl)pyrazin-2-yl]amino}benzoic acid (11 mg, 0.03 mmol) in dry DMF (1.5 mL) were added NEt3 (16 µL, 0.11 mmol), 2,4,6-tripropyl-1,3,5,2,4,6-trioxatriphosphorinane-2,4,6-trioxide (50% in EtOAc, 89 µL, 0.15 mmol) and piperazine (78 mg, 0.91 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1% TFA, MeCN) to give the title compound (1 mg).
HPLC 95%(System A), 98%(System B).
MS (ESI+) calcd for C₂₃H₂₁ClN₆O 432.1465, found 432.1456.

### Example 166

### N-[4-({4-[3-(dimethylamino)propyl]piperazin-1-yl}carbonyl)-2-methylphenyl]-6-(5-fluoro-1H-indol-2-yl)pyrazin-2-amine trifluoroacetate

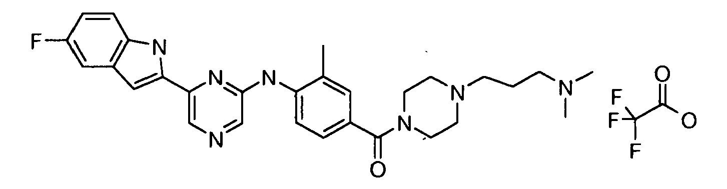

2,6-dichloropyrazine (0.40 g, 2.7 mmol), 4-amino-3-methylbenzoic acid (0.45 g, 3.0 mmol), NaOtBu (0.52 g, 5.3 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 4-[(6-chloropyrazin-2-yl)amino]-3-methylbenzoic acid. Half of the obtained crude 4-[(6-chloropyrazin-2-yl)amino]-3-methylbenzoic acid (theoretically containing 335 mg, 1.34 mmol) was dissolved in DME/water. [1-(tert-Butoxycarbonyl)-H-indol-2-yl]boronic acid (355 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 4-{[6-(5-fluoro-1H-indol-2-yl)pyrazin-2-yl]amino}-3-methylbenzoic acid (45 mg). MS (ESI+) for C20H15FN4O2 m/z 363 (M+H)+.
To 4-{[6-(5-fluoro-1H-indol-2-yl)pyrazin-2-yl]amino}-3-methylbenzoic acid (15 mg, 0.04 mmol) in dry DMF (1.5 mL) were added NEt₃ (17 µL, 0.12 mmol), 2,4,6-tripropyl-1,3,5,2,4,6-trioxatriphosphorinane-2,4,6-trioxide (50% in EtOAc, 99 µL, 0.17 mmol) and *N,N*-dimethyl-3-piperazin-1-ylpropan-1-amine (21 mg, 0.12 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1 % TFA, MeCN) to give the title compound (12 mg). HPLC 99%(System A), 99%(System B).
MS (ESI+) calcd for C₂₉H₃₄FN₇O 515.2809, found 515.2800.

### Example 167

### N-[2-chloro-5-methoxy-4-(piperazin-1-ylcarbonyl)phenyl]-6-(5-fluoro-1H-indol-2-yl)pyrazin-2-amine trifluoroacetate

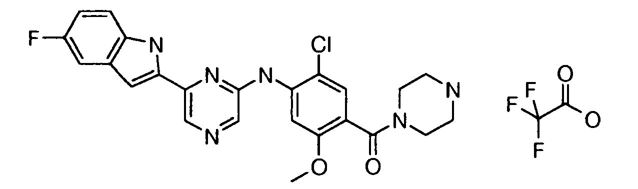

2,6-dichloropyrazine (0.40 g, 2.7 mmol), 4-amino-5-chloro-2-methoxybenzoic acid (0.60 g, 3.0 mmol), NaOtBu (0.52 g, 5.3 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 5-chloro-4-[(6-chloropyrazin-2-yl)amino]-2-methoxybenzoic acid. Half of the obtained crude 5-chloro-4-[(6-chloropyrazin-2-yl)amino]-2-methoxybenzoic acid (theoretically containing 421 mg, 1.34 mmol) was dissolved in DME/water. [1-(tert-Butoxycarbonyl)-H-indol-2-yl]boronic acid (350 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 5-chloro-4-{[6-(5-fluoro-1H-indol-2-yl)pyrazin-2-yl]amino}-2-methoxybenzoic acid (26 mg).
To 5-chloro-4-{[6-(5-fluoro-1H-indol-2-yl)pyrazin-2-yl]amino}-2-methoxybenzoic acid (13 mg, 0.03 mmol) in dry DMF (1.5 mL) were added NEt3 (14 µL, 0.09 mmol), 2,4,6-tripropyl-1,3,5,2,4,6-trioxatriphosphorinane-2,4,6-trioxide (50% in EtOAc, 75 µL, 0.13 mmol) and piperazine (81 mg, 0.95 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1% TFA, MeCN) to give the title compound (2 mg). MS (ESI+) for C24H22ClFN6O2 m/z 481 (M+H)+.
HPLC 97%(System A), 98%(System B).
MS (ESI+) calcd for C₂₄H₂₂ClFN₆O₂ 480.1477, found 480.1469.

### Example 168

### 3-chloro-N-[2-(diethylamino)ethyl]-4-{[6-(5-fluoro-1H-indol-2-yl)pyrazin-2-yl]amino}benzamide trifluoroacetate

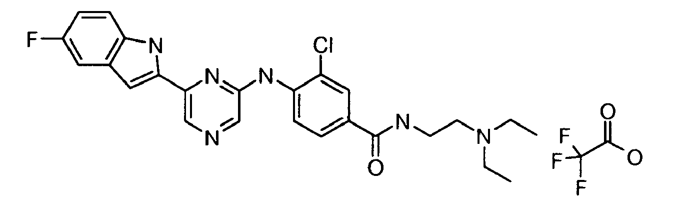

2,6-dichloropyrazine (0.35 g, 2.3 mmol), 4-amino-3-chlorobenzoic acid (0.35 g, 2.0 mmol), NaOtBu (0.44 g, 4.6 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 3-chloro-4-[(6-chloropyrazin-2-yl)amino]benzoic acid. Half of the obtained crude 3-chloro-4-[(6-chloropyrazin-2-yl)amino]benzoic acid (theoretically containing 335 mg, 1.34 mmol) was dissolved in DME/water. [1-(tert-Butoxycarbonyl)-H-indol-2-yl]boronic acid (381 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 3-chloro-4-{[6-(5-fluoro-1H-indol-2-yl)pyrazin-2-yl]amino}benzoic acid (30 mg).

To 3-chloro-4-{[6-(5-fluoro-1H-indol-2-yl)pyrazin-2-yl]amino}benzoic acid (15 mg, 0.04 mmol) in dry DMF (1.5 mL) were added NEt3 (14 µL, 0.09 mmol), 2,4,6-tripropyl-1,3,5,2,4,6-trioxatriphosphorinane-2,4,6-trioxide (50% in EtOAc, 75 µL, 0.13 mmol) and N,N-diethylethane-1,2-diamine (14 mg, 0.83 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1% TFA, MeCN) to give the title compound (1 mg).
HPLC 95%(System A), 95%(System B).
MS (ESI+) calcd for C₂₅H₂₆ClFN₆O 480.1841, found 480.1838.

### Example 169

### N-[2-chloro-4-(piperazin-1-ylcarbonyl)phenyl]-6-(5-fluoro-1H-indol-2-yl)pyrazin-2-amine trifluoroacetate

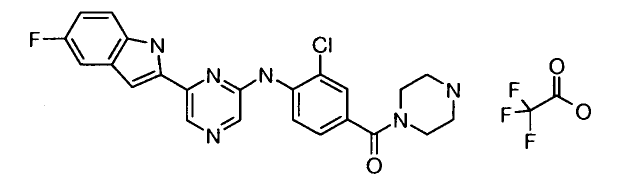

2,6-dichloropyrazine (0.35 g, 2.3 mmol), 4-amino-3-chlorobenzoic acid (0.35 g, 2.0 mmol), NaOtBu (0.44 g, 4.6 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 3-chloro-4-[(6-chloropyrazin-2-yl)amino]benzoic acid
. Half of the obtained crude 3-chloro-4-[(6-chloropyrazin-2-yl)amino]benzoic acid
(theoretically containing 335 mg, 1.34 mmol) was dissolved in DME/water. [1-(tert-Butoxycarbonyl)-H-indol-2-yl]boronic acid (381 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 3-chloro-4-{[6-(5-fluoro-1H-indol-2-yl)pyrazin-2-yl]amino}benzoic acid (30 mg).
To 3-chloro-4-{[6-(5-fluoro-1H-indol-2-yl)pyrazin-2-yl]amino}benzoic acid (15 mg, 0.04 mmol) in dry DMF (1.5 mL) were added NEt3 (14 µL, 0.09 mmol), 2,4,6-tripropyl-1,3,5,2,4,6-trioxatriphosphorinane-2,4,6-trioxide (50% in EtOAc, 75 µL, 0.13 mmol) and piperazine (101 mg, 1.2 mmol). The mixture was stirred at rt over night. TBTU (2 eq) was added and the reaction mixture was stirred over night. 1 drop of water was added and the mixture was concentrated in vacuo. Purification was performed by preparative HPLC (ACE C8, 0.1 % TFA, MeCN) to give the title compound (1 mg).
HPLC 99%(System A), 99%(System B).
MS (ESI+) calcd for C₂₃H₂₀ClFN₆O 450.1371, found 450.1373.

### Example 170

### N-[2-chloro-4-({f4-[3-(dimethylamino)propyl]piperazin-1-yl}carbonyl)phenyl]-6-(1H-indol-2-yl)pyrazin-2-amine trifluoroacetate

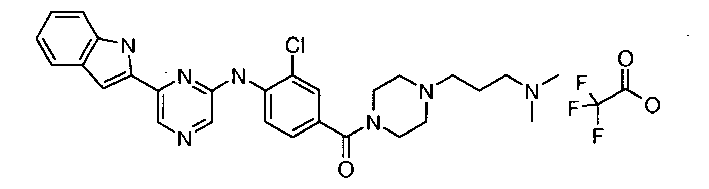

2,6-Dichloropyrazine (0.35 g, 2.3 mmol), 4-amino-3-chlorobenzoic acid (0.35 g, 2.0 mmol), NaOtBu (0.44 g, 4.6 mmol), Xantphos (45 mg, 0.08 mmol) and Pd₂(dba)₃ (25 mg, 0.027 mmol) were stirred in dry dioxane (10 mL). The reaction mixture was refluxed at 135 °C for 4 hours, then allowed to attain rt. The mixture was concentrated under reduced pressure to give crude 3-chloro-4-[(6-chloropyrazin-2-yl)amino]benzoic acid. Half of the obtained crude 3-chloro-4-[(6-chloropyrazin-2-yl)amino]benzoic acid (theoretically containing 335 mg, 1.34 mmol) was dissolved in DME/water. [1-(tert-Butoxycarbonyl)-H-indol-2-yl]boronic acid (381 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo.* The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1% TFA-MeCN) to give 3-chloro-4-{[6-(1H-indol-2-yl)pyrazin-2-yl]amino}benzoic acid (45 mg).
To 3-chloro-4-{[6-(1H-indol-2-yl)pyrazin-2-yl]amino}benzoic acid (15 mg, 0.04 mmol) in dry DMF (1.5 mL) were added NEt3 (17 µL, 0.12 mmol), 2,4,6-tripropyl-1,3,5,2,4,6-trioxatriphosphorinane-2,4,6-trioxide (50% in EtOAc, 97 µL, 0.16 mmol) and N,N-dimethyl-3-piperazin-1-ylpropan-1-amine (21 mg, 0.12 mmol). The mixture was stirred at rt over night. TBTU (2 eq) was added with continous stirring over night. 1 drop of water was added and the mixture was concentrated in vacuo. Purification was performed by preparative HPLC (ACE C8, 0.1% TFA, MeCN) to give the title compound (9 mg). HPLC 99% (System A), (System B). MS (ESI+) calcd for C₂₈H₃₂ClN₇O 517.2357, found 517.2353.

### Example 171

### N-[2-chloro-4-(piperazin-1-ylcarbonyl)phenyl]-6-(1H-indol-2-yl)pyrazin-2-amine trifluoroacetate

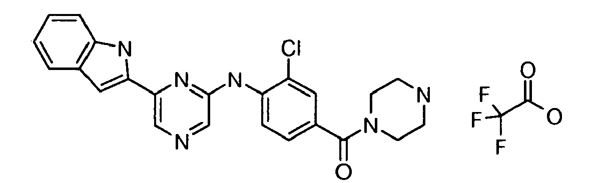

2,6-Dichloropyrazine (0.35 g, 2.3 mmol), 4-amino-3-chlorobenzoic acid (0.35 g, 2.0 mmol), NaOtBu (0.44 g, 4.6 mmol), Xantphos (45 mg, 0.08 mmol) and Pd₂(dba)₃ (25 mg, 0.027 mmol) were stirred in dry dioxane (10 mL) . The reaction mixture was refluxed at 135 °C for 4 hours, then allowed to attain rt. The mixture was concentrated under reduced pressure to give crude 3-chloro-4-[(6-chloropyrazin-2-yl)amino]benzoic acid. Half of the obtained crude 3-chloro-4-[(6-chloropyrazin-2-yl)amino]benzoic acid (theoretically containing 335 mg, 1.34 mmol) was dissolved in DME/water. [1-(tert-Butoxycarbonyl)-H-indol-2-yl]boronic acid (381 mg, 1.34 mmol), NaHCO₃ (394 mg, 4.69 mmol) and Pd(PPh₃)₄ (31 mg, 0.03 mmol) were added. The reaction mixture was stirred at 100 °C for 7 h and at rt for 8 hours. MeOH (5mL) was added followed by filtration and concentration *in vacuo*. The residue was dissolved in EtOAc (15 mL) and 1 M aq NaOH (20 mL). The phases were separated, and the aqueous layer was extracted with EtOAc (15 mL). The water phase was acidified with 1 M HCl (aq) and extracted with EtOAc (10 mL). The combined organic phases were concentrated and the remaining material was dissolved in DCM/TFA (4:1, 10 mL) and stirred for 5 h at rt. The solvent was evaporated, and the residue was purified by preparative HPLC (YMC ODS-AQ, 0.1 % TFA-MeCN) to give 3-chloro-4-{[6-(1H-indol-2-yl)pyrazin-2-yl]amino}benzoic acid (45 mg).
To 3-chloro-4-{[6-(1H-indol-2-yl)pyrazin-2-yl]amino}benzoic acid (15 mg, 0.04 mmol) in dry DMF (1.5 mL) were added NEt3 (17 µL, 0.12 mmol), 2,4,6-tripropyl-1,3,5,2,4,6-trioxatriphosphorinane-2,4,6-trioxide (50% in EtOAc, 97 µL, 0.16 mmol) and piperazine (106 mg, 1.23 mmol). The mixture was stirred at rt over night. TBTU (2 eq) was added with continous stirring over night. 1 drop of water was added and the mixture was concentrated in vacuo. Purification was performed by preparative HPLC (ACE C8, 0.1 % TFA, MeCN) to give the title compound (5 mg). HPLC 98%(System A), 98%(System B).
MS (ESI+) calcd for C₂₃H₂₁ClN₆O 432.1465, found 432.1458.

### Example 172

### 5-(1H-indol-2-yl)-N-{4-[(4-methylpiperazin-1-yl)carbonyl]phenyl}pyridin-3-amine

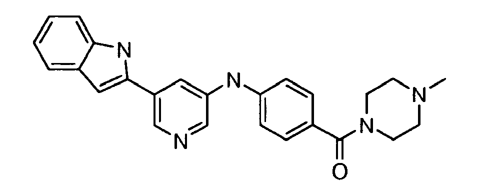

3,5-Dibromopyridine (249 mg, 1.05 mmol), [1-(tert-butoxycarbonyl)-H-indol-2-yl]boronic acid (302 mg, 1.16 mmol), Pd(PPh₃)₄ (61 mg, 0.05 mmol) and NaHCO₃ (309 mg, 3.68 mmol) in DME/water (3.5:1, 4.5 mL) was irradiated with microwaves at 120 °C for 5 minutes, followed by 180 °C for 12 minutes. The reaction mixture was extracted with DCM (2x10 mL) and water (10 mL). The organic layers were combined, dried (Na₂SO₄), filtered and concentrated. The residue was purified by preparative HPLC (YMC ODS-AQ, 0.1% TFA-MeCN) to give 2-(5-bromopyridin-3-yl)-1H-indole. 2-(5-bromopyridin-3-yl)-1 H-indole (10 mg, 0.04 mmol), {4-[(4-methylpiperazin-1-yl)carbonyl]phenyl}amine (12 mg, 0.06 mmol), Pd(OAc)₂ (2 mg, 0.01 mmol), NaOtBu (12 mg, 0.13 mmol) and Xantphos (9 mg, 0.018 mmol) in toluene/tBuOH (5:1, 1mL) was irradiated with micro waves at 160 °C for 20 minutes. Additional Pd(OAc)₂ (5 mg, 0.02 mmol) was added and the resulting mixture was irradiated with micro waves for a further 20 min at 160 °C. The crude mixture was extracted with EtOAc and 1M aq NaOH and the product precipitated and was filtered off. The crude product was purified by preparative HPLC (ACE C8, 0.1% TFA, MeCN) followed by (XTerra C18, 50mM NH4HCO3 pH 10, MeCN). This gave the title compound as a white solid (0.7 mg). MS (ESI+) for C25H25N5O m/z 412 (M+H)+.
HPLC 100%(System A), 96%(System B).

### General procedure for the synthesis of intermediates 33-42.

The indole (1 equiv, -1.5 - 6 mmol) was dissolved in 2 mL dry DCM and 1M di-tert-butyl dicarbonate (1.2 equiv) in DCM and 1M DMAP (1.2 eq) in DCM was added and the reaction mixture was stirred for 20 min. The mixture was washed with 1M HCl, dried (MgSO₄) and the solvent was removed *in vacuo* to give the requisite N-t-BOC indole derivative.
The N-t-BOC indole (1 equiv) was dissolved in THF (∼5-10 mL) and triisopropyl borate (1.5 equiv) was added. The reaction mixture was kept under N₂, cooled in an ice bath, and 2M LDA (1.2 equiv) was added (over a time period of 10 min.). After 60 min, 0.2 mL 2M LDA was added and the reaction mixture was left at r.t. for 10 min after which time 20 mL 2M HCl was added and the reaction stirred for a further 2 min. The mixture was extracted with 15 mL Et₂O and dried over MgSO₄. The solvent was removed *in vacuo* until a small amount was left. The products were precipitated by adding water. Otherwise, the crude ether solution was used directly in the next step.

### Intermediate 33

### [1-(tert-Butoxycarbonyl)-4-methoxy-H-indol-2-yl]boronic acid

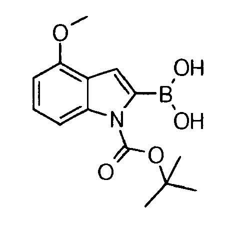

The title compound was made from 4-methoxyindole (821 mg, 5.60 mmol) according to the general procedure described above. Yield 751 mg (46%).

### Intermediate 34

### [1-(tert-Butoxycarbonyl)-6-methoxy-H-indol-2-yl]boronic acid

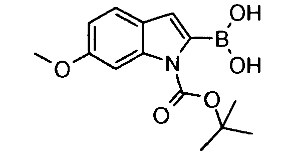

The title compound was made from 6-methoxyindole (233 mg, 1.60 mmol) according to the general procedure described above. Yield 51 mg (11%).

### Intermediate 35

### [1-(tert-Butoxycarbonyl)-7-fluoro-H-indol-2-yl]boronic acid

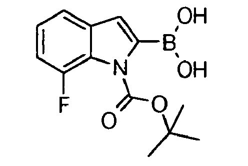

The title compound was made from 7-fluoroindole (122 mg, 0.90 mmol) according to the general procedure described above.

### Intermediate 36

### [1-(tert-Butoxycarbonyl)-5-chloro-H-indol-2-yl]boronic acid

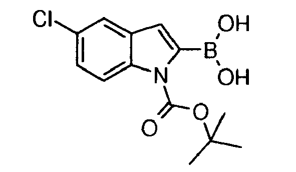

The title compound was made from 5-chloroindole (1.02 g, 6.70 mmol) according to the general procedure described above. Yield 731 mg (37%).

### Intermediate 37

### [1-(tert-Butoxycarbonyl)-6-chloro-H-indol-2-yl]boronic acid

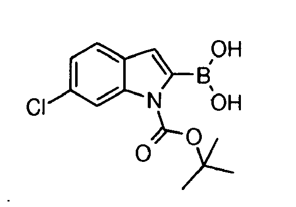

The title compound was made from 6-chloroindole (921 mg, 6.10 mmol) according to the general procedure described above. Yield 489 mg (27%).

### Intermediate 38

### [1-(tert-Butoxycarbonyl)-7-chloro-H-indol-2-yl]boronic acid

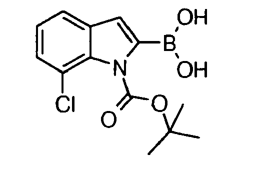

The title compound was made from 7-chloroindole (909 mg, 6.00 mmol) according to the general procedure described above. Yield 265 mg (15%).

### Intermediate 39

### [1-(tert-Butoxycarbonyl)-7-methyl-H-indol-2-yl]boronic acid

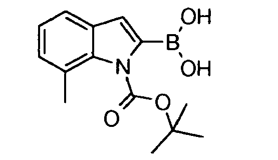

The title compound was made from 7-methylindole (816 mg, 6.20 mmol) according to the general procedure described above.

### Intermediate 40

### [1-(tert-Butoxycarbonyl)-7-methoxy-H-indol-2-yl]boronic acid

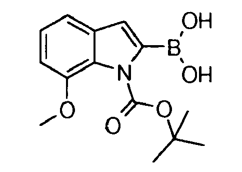

The title compound was made from 7-methoxyindole (740 mg, 5.00 mmol) according to the general procedure described above.

### Intermediate 41

### [1-(tert-Butoxycarbonyl)-4-fluoro-H-indol-2-yl]boronic acid

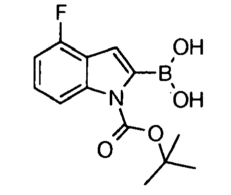

The title compound was made from 4-fluoroindole (784 mg, 5.80 mmol) according to the general procedure described above. Yield 325 mg (20%).

### Intermediate 42

### [1-(tert-Butoxycarbonyl)-6-fluoro-H-indol-2-yl]boronic acid

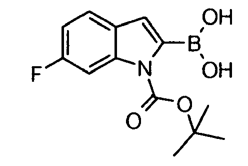

The title compound was made from 6-fluoroindole (1.04 g, 7.70 mmol) according to the general procedure described above. Yield 575 mg (27%).

### Example 173

### 6-(4-fluoro-1H-indol-2-yl)-N-[2-methoxy-4-(piperazin-1-ylcarbonyl)phenyl]pyrazin-2-amine trifluoroacetate

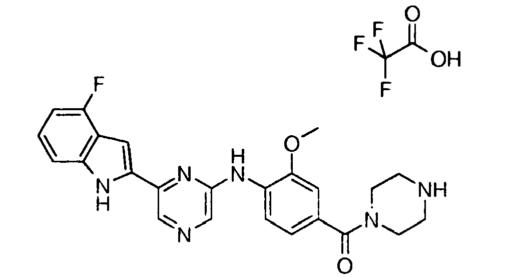

To Intermediate 41 (30 mg, 0.11 mmol), Intermediate 32 (30 mg, 0.086 mmol), Pd(PPh₃)₄ (15 mg, 0.013 mmol) and K₂CO₃ (50 mg, 0.36 mmol) were added 0.5 mL MeCN and 0.2 mL H₂O. The reaction was heated by microwaves at 120 °C for 5 min. The water phase was removed with a pipette and the reaction mixture was heated for 5 min at 160 °C. The crude material was filtrated and purified using preparative HPLC system A. Yield 12 mg (20%) 1 H NMR (400 MHz, MeOD) □ ppm 3.31 - 3.35 (m, 4 H) 3.89 - 3.97 (m, 4 H) 4.00 (s, 3 H) 6.73 (dd, *J*=10.54, 7.78 Hz, 1 H) 7.09 - 7.16 (m, 1 H) 7.18 - 7.24 (m, 3 H) 7.29 (d, *J*=8.03 Hz, 1 H) 8.26 (s, 1 H) 8.51 (s, 1 H) 8.68 (d, *J*=8.28 Hz, 1 H). HPLC 100%, *t*_{R} = 1.86 min (System A), 100%, *t*_{R} = 1.67 min (System B). MS (electronspray) M+H+ m/z 447.

### Example 174

### 4-{[6-(5-chloro-1H-indol-2-yl)pyrazin-2-yl]amino}-3-methoxy-N-methyl-N-(1-methylpyrrolidin-3-yl)benzamide trifluoroacetate

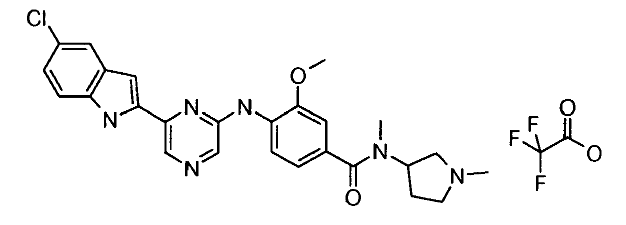

A mixture of 2,6-dichloropyrazine (3.30 g, 22.2 mmol), 4-amino-3-methoxybenzoic acid (4.07 g, 24.4 mmol), NaOtBu (4.47 g, 46.5 mmol), Xantphos (390 mg, 0.7 mmol) and Pd2(dba)3 (203 mg, 0.2 mmol) was stirred in dry dioxane (200 mL). The reaction mixture was refluxed at 135 °C for 4 hours, then allowed to attain rt and stored over night. The resulting mixture was concentrated under reduced pressure and the residue was dissolved in a 125 mL EtOAc and 150 mL 1 M aq HCl. The phases were separated, and the aqueous layer was extracted with EtOAc (2x 125 mL). The combined organic phases were dried (Na₂SO₄), filtered and concentrated to give a redbrown solid (5.24 g) of 4-[(6-chloropyrazin-2-yl)amino]-3-methoxybenzoic acid. The material was used without further purification. MS (ESI+) for C₁₂H₁₀ClN₃O₃ m/z 280 (M+H)+. To a solution of 4-[(6-chloropyrazin-2-yl)amino]-3-methoxybenzoic acid (1.40g, 5.00 mmol) in dry MeCN (50 mL) were added NEt₃ (1.39 mL, 10.0 mmol), 2,4,6-tripropyl-1,3,5,2,4,6-trioxatriphosphorinane-2,4,6-trioxide (50% in EtOAc, 5.96 mL, 10.0 mmol) and *N*,1-dimethylpyrrolidin-3-amine (857 mg, 7.51 mmol). The mixture was heated at 60 °C for 100 min. The mixture was concentrated *in vacuo* and residue was dissolved in EtOAc (100 mL) and 1 M aq NaOH (150 mL). The phases were separated, and the aqueous layer was extracted with EtOAc (2x100 mL). The combined organic phases were dried (Na₂SO₄) filtered and concentrated to give 1.8 g of a dark brown gum. Purification was performed by flash chromatography (DCM/NEt3/MeOH, 98:1:1). This gave 4-[(6-chloropyrazin-2-yl)amino]-3-methoxy-N-methyl-N-(1-methylpyrrolidin-3-yl)benzamide as a light brown solid (930 mg). MS (ESI+) for C₁₈H₂₂ClN₅O₂ m/z 376 (M+H)+. 4-[(6-A mixture of chloropyrazin-2-yl)amino]-3-methoxy-N-methyl-N-(1-methylpyrrolidin-3-yl)benzamide (20 mg, 0.05 mmol), [1-(tert-butoxycarbonyl)-5-chloro-1H-indol-2-yl]boronic acid (17 mg, 0.06 mmol), NaHCO₃ (16 mg, 0.19 mmol) and Pd(PPh₃)₄ (3 mg, 0.003 mmol) in DME/water (3.5:1, 1 mL) were irradiated in a microwave oven at 120 °C for 10 min. Additional [1-(tert-butoxycarbonyl)-5-chloro-1H-indol-2-yl]boronic acid (1 eq), NaHCO3 (3 eq) and Pd(PPh₃)₄ (5 mg) were added and reacted at 120 °C for 10 min. The reaction mixture was extracted with EtOAc (2x3 mL) and washed with 1M aqueous NaOH (3 mL). The organic layers were combined and concentrated in vacuo. The crude material was purified by preparative HPLC (ACE C8, 0.1 % TFA, MeCN) to give the title compound as a brown gum (2 mg). HPLC 99% (System A), 99% (System B).
MS (ESI+) calcd for C₂₆H₂₇ClN₆O₂ 490.1884, found 490.1880.

### Example 175

### 4-{[6-(6-chloro-1H-indol-2-yl)pyrazin-2-yl]amino}-3-methoxy-N-methyl-N-(1-methylpyrrolidin-3-yl|)benzamide trifluoroacetate

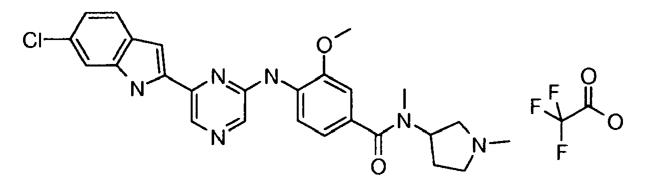

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-6-chloro-1H-indol-2-yl]boronic acid (17 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated in vacuo. The residue was purified by preparative HPLC (ACE C8, 0.1 % TFA, MeCN) to give the title compound as a brown gum (5 mg). HPLC 98% (System A), 97% (System B).
MS (ESI+) calcd for C₂₆H₂₇ClN₆O₂ 490.1884, found 490.1880.

### Example 176

### 3-methoxy-4-{[6-(4-methoxy-1H-indol-2-yl)pyrazin-2-yl]amino}-N-methyl-N-(1-methylpyrrolidin-3-yl)benzamide trifluoroacetate

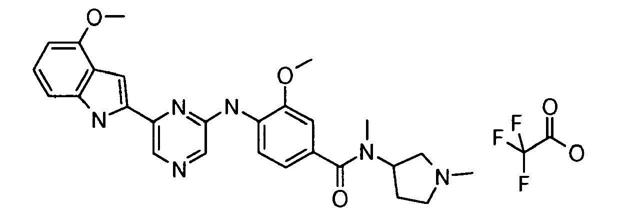

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-4-methoxy-1H-indol-2-yl]boronic acid (17 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated *in vacuo.* The residue was purified by preparative HPLC (ACE C8, 0.1% TFA, MeCN) to give the title compound as a brown gum (8 mg).
HPLC 100% (System A), 100% (System B).
MS (ESI+) calcd for C₂₇H₃₀N₆O₃ 486.2379, found 486.2374.

### Example 177

### 3-methoxy-N-methyl-4-{[6-(5-methyl-1H-indol-2-yl)pyrazin-2-yl]amino|}-N-(1-methylpyrrolidin-3-yl)benzamide trifluoroacetate

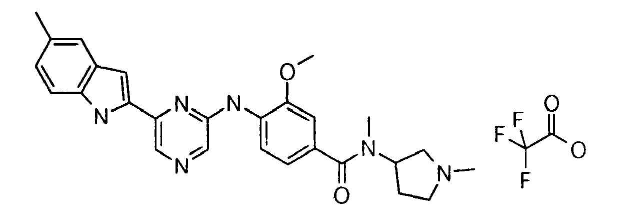

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-5-methyl-1H-indol-2-yl]boronic acid (16 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated in vacuo. The residue was purified by preparative HPLC (ACE C8, 0.1% TFA, MeCN) to give the title compound as a brown gum (9 mg).
HPLC 98% (System A), 96% (System B).
MS (ESI+) calcd for C₂₇H₃₀N₆O₂ 470.2430, found 470.2427.

### Example 178

### 3-methoxy-N-methyl-4-{[6-(6-methyl-1H-indol-2-yl)pyrazin-2-yl]amino}-N-(1-methylpyrrolidin-3-yl)benzamide trifluoroacetate

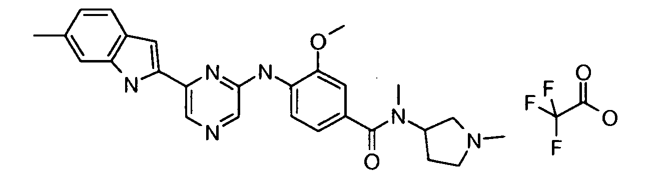

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-6-methyl-1H-indol-2-yl]boronic acid (16 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated in vacuo. The residue was purified by preparative HPLC (ACE C8, 0.1% TFA, MeCN) to give the title compound as a brown gum (9 mg).
HPLC 97% (System A), 99% (System B).
MS (ESI+) calcd for C₂₇H₃₀N₆O₂ 470.2430, found 470.2426.

### Example 179

### 4-{[6-(5-cyano-1H-indol-2-yl)pyrazin-2-yl]amino}-3-methoxy-N-methyl-N-(1-methylpyrrolidin-3-yl)benzamide trifluoroacetate

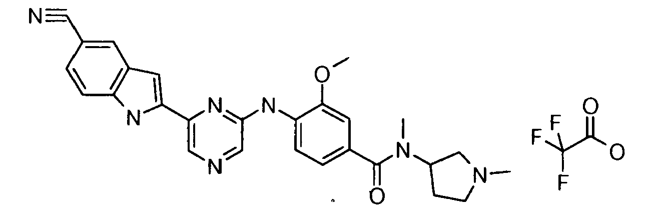

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-5-cyano-1H-indol-2-yl]boronic acid (17 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated in vacuo. The residue was purified by preparative HPLC (ACE C8, 0.1% TFA, MeCN) to give the title compound as a brown gum (3 mg).
HPLC 96% (System A), 96% (System B).
MS (ESI+) calcd for C₂₇H₂₇N₇O₂ 481.2226, found 481.2219.

### Example 180

### 4-{[6-(7-fluoro-1H-indol-2-yl)pyrazin-2-yl]amino}-3-methoxy-N-methyl-N-(1-methylpyrrolidin-3-yl)benzamide trifluoroacetate

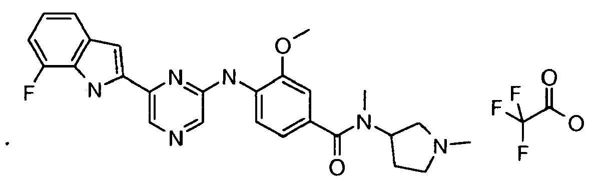

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-7-fluoro- H-indol-2-yl]boronic acid (16 mg, 0.06 mmol), NaHCO₃ (16 mg, 0.19 mmol) and Pd(PPh₃)₄ (3 mg, 0.003 mmol) in DME/water (3.5:1; 1 mL) was irradiated in a microwave oven at 120 °C for 10 min. The reaction mixture was extracted with EtOAc (2x3 mL) and washed with 1M aq NaOH (3 mL). The organic layers were combined and concentrated in vacuo. The residue was purified by preparative HPLC (ACE C8, 0.1% TFA, MeCN) to give the title compound as a brown gum (6 mg).
HPLC 94% (System A), 92% (System B).
MS (ESI+) calcd for C₂₆H₂₇FN₆O₂ 474.2180, found 474.2174.

### Example 181

### 4-{[6-(4-fluoro-1H-indol-2-yl)pyrazin-2-yl]amino}-3-methoxy-N-methyl-N-(1-methylpyrrolidin-3-yl)benzamide trifluoroacetate

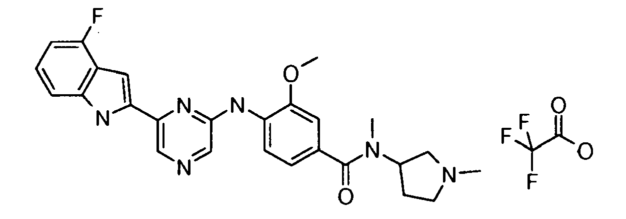

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-4-fluoro-1H-indol-2-yl]boronic acid (16 mg, 0.06 mmol), Na₂CO₃ (17 mg, 0.18 mmol) and Pd(PPh₃)₄ (3 mg, 0.003 mmol) in MeCN/water (3:1; 1.3 mL) was irradiated in a microwave oven at 120 °C for 10 min. The reaction mixture was extracted with EtOAc (2x3 mL) and 1M aq NaOH (3 mL). The organic layers were combined and concentrated in vacuo. The residue was purified by preparative HPLC (ACE C8, 0.1 % TFA, MeCN) to give the title compound as a brown gum (5 mg).
HPLC 97%(System A), 95%(System B).
MS (ESI+) calcd for C₂₆H₂₇FN₆O₂ 474.2180, found 474.2174.

### Example 182

### 4-{[6-(6-fluoro-1H-indol-2-yl)pyrazin-2-yl]amino}-3-methoxy-N-methyl-N-(1-methylpyrrolidin-3-yl)benzamide trifluoroacetate

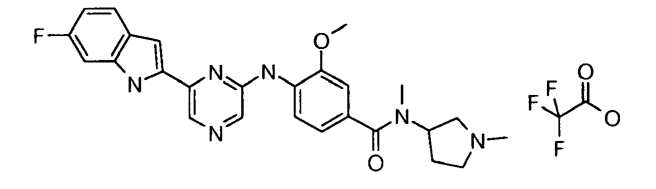

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (20 mg, 0.05 mmol), [1-(tert-butoxycarbonyl)-6-fluoro- H-indol-2-yl]boronic acid (16 mg, 0.06 mmol), Na₂CO₃ (17 mg, 0.18 mmol) and Pd(PPh₃)₄ (3 mg, 0.003 mmol) in MeCN/water (3:1; 1.3 mL) was irradiated in a microwave oven at 120 °C for 10 minutes. The reaction mixture was extracted with EtOAc (2x3 mL) and 1M aq NaOH (3 mL). The organic layers were combined and concentrated in vacuo. The residue was purified by preparative HPLC (ACE C8, 0.1% TFA, MeCN) to give the title compound as a brown gum (5 mg). HPLC 98% (System A), 90% (System B).
MS (ESI+) calcd for C₂₆H₂₇FN₆O₂ 474.2180, found 474.2174.

### Example 183

### 3-methoxy-4-{[6-(5-methoxy-1H-indol-2-yl)pyrazin-2-yl]amino}-N-methyl-N-(1-methylpyrrolidin-3-yl)benzamide trifluoroacetate

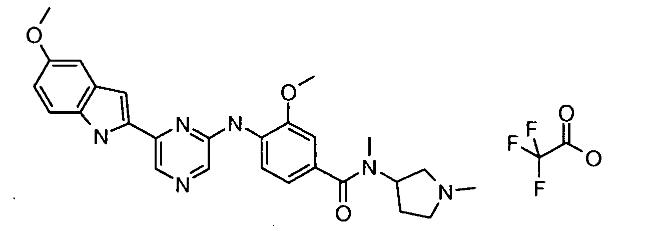

The title compound was synthesisized according to the same general procedure as described in Example 174. A mixture of 4-[(6-chloropyrazin-2-yl)amino]-3-methoxy-N-methyl-N-(1-methylpyrrolidin-3-yl)benzamide (15 mg, 0.04 mmol), [1-(tert-butoxycarbonyl)-5-methoxy-1H-indol-2-yl]boronic acid (13 mg, 0.04 mmol), NaHCO₃ (12 mg, 0.14 mmol) and Pd(PPh₃)₄ (2 mg, 0.002 mmol) in DME/water (3.5:1; 1 mL) was irradiated in a microwave oven at 120 °C for 10 min. The reaction mixture was extracted with EtOAc (2x10 mL) and washed with 1M aq NaOH (10 mL). The organic layers were combined and concentrated in vacuo. The residue was purified by preparative HPLC (ACE C8, 0.1 % TFA, MeCN) to give the title compound as a brown gum (2 mg). HPLC 98% (System A), 98% (System B).
MS (ESI+) calcd for C₂₇H₃₀N₆O₃ 486.2379, found 486.2375.

### Intermediate 43

### tert-Butyl 5-nitro-1H-indole-1-carboxylate

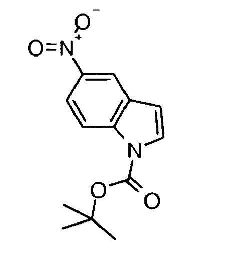

4-DMAP (414.4 mg, 3.4 mmol) and di-tert-butyl dicarbonate (740.3 mg, 3.4 mmol) were added to 5-nitro-1*H-*indole (500.0 mg, 3.1 mmol) in DCM (5 mL), stirred for 1 h at ambient temperature, 2 M HCL was added followed by extraction with DCM, organic phase was collected and the solvent was removed under reduced pressure. Yield 724.0 mg (89%). HPLC 100%. LC-MS 263 (M+H)⁺.

### Example 184

### N-(4-{[(3S)-3-(dimethylamino)pyrrolidin-1-yl]carbonyl}-2-methoxyphenyl)-6-(5-nitro-1H-indol-2-yl)pyrazin-2-amine

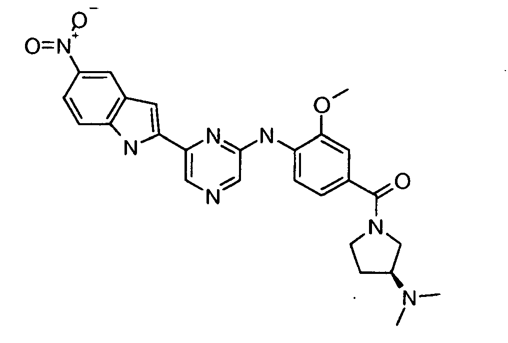

LDA (0.8 µL, 3.3 mmol) was added dropwise to a suspension of *tert*-butyl 5-nitro-1*H-*indole-1-carboxylate (724.0 mg, 2.8 mmol) and triisopropyl borate (0.8 µL, 3.3 mmol) in dry THF (15 mL) at 0 °C under nitrogen atmosphere. The reaction was stirred at 0 °C for 2 h. Additional portions of LDA (0.8 µL, 3.3 mmol) and triisopropyl borate (0.8 µL, 3.3 mmol) were added. The resulting mixture was stirred at 0 °C for 1 h. More LDA 2 x (0.8 µL**,** 3.3 mmol) was added. The reaction was quenched with 2M HCl, basified with saturated NaHCO₃, extraction with DCM, dried over MgSO₄ and the solvent was removed to give [1-(tert-butoxycarbonyl)-5-nitro-1 H-indol-2-yl]boronic acid. 6-Chloro-*N*-(4-{[(3S)-3-(dimethylamino)pyrrolidin-1-yl]carbonyl}-2-methoxyphenyl)pyrazin-2-amine (56.0 mg, 0.15 mmol), [1-(tert-butoxycarbonyl)-5-nitro-1H-indol-2-yl]boronic acid (43.5 mg, 0.14 mmol), NaHCO₃ (60.4 mg, 0.52 mmol) and Pd(PPh₃)₄ (1.7 mg, 0.0002 mmol) in DME (3 mL) and water (1 mL) were heated in a microwave oven at 120 °C for 10 min. More [1-(tert-butoxycarbonyl)-5-nitro-1H-indol-2-yl]boronic acid (724.0 mg, 2.8 mmol) and Pd(PPh₃)₄ (1.7 mg, 0.0002 mmol) were added followed by heating in a microwave oven for a further 10 min at 120 °C. The reaction mixture was filtered, solvent was removed under reduced pressure. A small amount of the obtained material was purified using preparative HPLC system B followed by system C to give 1.4 mg of the title compound. HPLC 99%. MS (ESI+) calcd for C₂₆H₂₇N₇O₄ 501.2125, found 501.2118.

### Intermediate 44

### 6-{[6-(1H-indol-2-yl)pyrazin-2-yl]amino}nicotinic acid

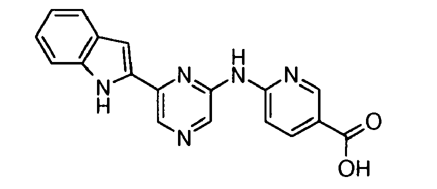

[1-(tert-butoxycarbonyl)-1H-indol-2-yl]boronic acid (2.22 g, 8.49 mmol), 6-chloropyrazin-2-amine (1.00 g, 7.72 mmol), K₂CO₃ (2.67 g, 19.3 mmol) and Pd(PPh₃)₄ (180 mg, 0.15 mmol) were mixed in MeCN/water (3.5:1) and stirred at 85 °C for 20 hours. The reaction mixture was concentrated and the residue was extracted with EtOAc (2x 40 mL) and water (50 mL). The organic layers were combined, dried (Na₂SO₄) filtered and concentrated to give 2.5 g of a brown solid of tert-butyl 2-(6-aminopyrazin-2-yl)-1H-indole-1-carboxylate. The material was used in the next step without further purification. To tert-butyl 2-(6-aminopyrazin-2-yl)-1H-indole-1-carboxylate (1.1 g, 3.5 mmol) in dry toluene (10 mL) were added methyl 6-chloronicotinate (0.67 g, 3.90 mmol), K₂CO₃ (7.35 g, 53.2 mmol), (±)-BINAP (0.11 g, 0.18 mmol) and Pd(OAc)₂ (40 mg, 0.18 mmol). The mixture was refluxed for 50 minutes. The solvent was evaporated and the residue was extracted with EtOAc (2x100 mL) and washed with water/brine (1:1; 100 mL). The organic layers were combined, dried (Na₂SO₄) filtered and concentrated to give 2 g of crude product. Purification was performed by flash chromatography (1% NEt₃ in DCM-> 1% NEt₃, 1% MeOH in DCM). This gave tert-butyl 2-(6-{[5-(methoxycarbonyl)pyridin-2-yl]amino}pyrazin-2-yl)-1H-indole-1-carboxylate (620 mg). The material was divided into four microwave tubes (155 mg, 0.35 mmol in each) and dissolved in MeOH. 2M aq NaOH (0.35 mL, 0.70 mmol) was added and the reaction mixture was irradiated in a microwave oven at 110 °C for 15 minutes. 6M aq HCl (0.12 mL, 0.70 mmol) was added. The reactions were combined and concentrated in vacuo to give the crude title compound as a brown solid (720 mg). MS (ESI+) for C18H13N5O2 m/z 332 (M+H)+.

### Example 185

### 6-(1H-indol-2-yl)-N-{5-[(4-methyl-1,4-diazepan-1-yl)carbonyl]pyridin-2-yl}pyrazin-2-amine

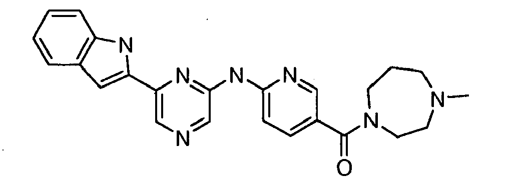

To the crude 6-{[6-(1H-indol-2-yl)pyrazin-2-yl]amino}nicotinic acid (Intermediate 44)
(30 mg) in MeCN (1.5 mL) were added dry NEt₃ (29 µL, 0.21 mmol) and 2,4,6-tripropyl-1,3,5,2,4,6-trioxatriphosphorinane-2,4,6-trioxide (50% in EtOAc, 0.12 mL, 0.21 mmol). The mixture was stirred for 3 minutes after which 1-methyl-1,4-diazepane (16 mg, 0.14 mmol) was added, and the reaction mixture was heated at 60 °C for 1 h. The material was purified by preparative HPLC (XTerra C18, 50 mM NH4HCO3 pH 10, MeCN). The title compound was obtained as a brown solid (8 mg).
HPLC 100%(System A), 100%(System B).
1H NMR (400 MHz, MeOD) □ppm 1.86 - 2.05 (m, 2 H) 2.32 - 2.46 (m, 3 H) 2.60 - 2.74 (m, 3 H) 2.77 - 2.87 (m, 1 H) 3.59 - 3.71 (m, 2 H) 3.71 - 3.86 (m, 2 H) 7.01 - 7.08 (m, 1 H) 7.14 - 7.22 (m, 2 H) 7.47 (d, J=8.28 Hz, 1 H) 7.60 (d, J=7.78 Hz, 1 H) 7.74 - 7.80 (m, 1 H) 7.81 - 7.87 (m, 1 H) 8.36 - 8.44 (m, 1 H) 8.61 (s, 1 H) 8.91 (s, 1 H)
.MS (ESI+) calcd for C₂₄H₂₅N₇O 427.2121, found 427.2122.

### Example 186

### N-{5-[(4-ethylpiperazin-1-yl)carbonyl]pyridin-2-yl}-6-(1H-indol-2-yl)pyrazin-2-amine

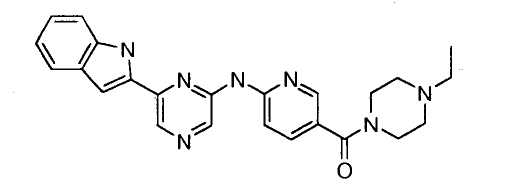

To the crude 6-{[6-(1H-indol-2-yl)pyrazin-2-yl]amino}nicotinic acid (Intermediate 44) (30 mg) in MeCN (1.5 mL) were added dry NEt₃ (29 µL, 0.21 mmol) and 2,4,6-tripropyl-1,3,5,2,4,6-trioxatriphosphorinane-2,4,6-trioxide (50% in EtOAc, 0.12 mL, 0.21 mmol). The mixture was stirred for 3 minutes after which 1-ethylpiperazine (16 mg, 0.14 mmol) was added, and the reaction mixture was heated at 60 °C for 1 h. The material was purified by preparative HPLC (XTerra C 18, 50 mM NH4HCO3 pH 10, MeCN). The title compound was obtained as a brown solid (10 mg). HPLC 97%(System A), 99%(System B).
MS (ESI+) calcd for C₂₄H₂₅N₇O 427.2121, found 427.2123.

### Example 187

### 6-(1H-indol-2-yl)-N-[5-(morpholin-4-ylcarbonyl)pyridin-2-yl]pyrazin-2-amine

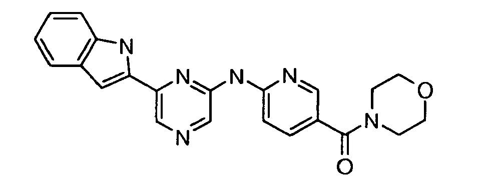

To the crude 6-{[6-(1H-indol-2-yl)pyrazin-2-yl]amino}nicotinic acid (Intermediate 44) (30 mg) in MeCN (1.5 mL) were added dry NEt3 (29 µL, 0.21 mmol) and 2,4,6-tripropyl-1,3,5;2,4,6-trioxatriphosphorinane-2,4,6-trioxide (50% in EtOAc, 0.12 mL, 0.21 mmol). The mixture was stirred for 3 minutes after which morpholine (12 mg, 0.14 mmol) was added, and the reaction mixture was heated at 60 °C for 1 h. The material was purified by preparative HPLC (XTerra C18, 50 mM NH4HCO3 pH 10, MeCN). The title compound was obtained as a brown solid (3 mg).
HPLC 99%(System A), 99%(System B).
MS (ESI+) calcd for C₂₂H₂₀N₆O₂ 400.1648, found 400.1657.

### Example 188

### N-{4-[(4-methylpiperazin-1-yl)carbonyl]phenyl}-6-(1H-pyrrolo[2,3-c]pyridin-2-yl)pyrazin-2-amine

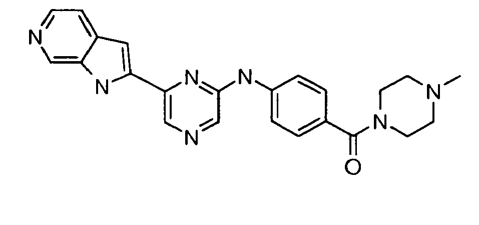

To 1*H*-Pyrrolo[2,3-c]pyridine (500 mg, 4.2 mmol) in dry DCM (2 mL) were added DMAP (569 mg, 4.7 mmol, in 2 mL dry DCM ) and di-tert-butyl carbonate (1.02 g, 4.7 mmol, in 2 mL dry DCM). The mixture was stirred for 1 hour 15 minutes and subsequently extracted with 1M aq HCl (20 mL) and DCM (3x 20 mL). The organic layers were combined, dried (Na2SO4), filtered and concentrated to give 675 mg of white solid, tert-butyl 1H-pyrrolo[2,3-c]pyridine-1-carboxylate. MS (ESI+) for C12H14N2O2 m/z 219 (M+H)+. The material was suspended in dry THF (10 mL) and triisopropyl borate (0.85 mL, 3.71 mmol) was added and the mixture was cooled on ice. LDA (1.86 mL, 3.71 mmol, 1.8M) was added dropwise over 40 minutes. Additional LDA (total 2.9 mL, 5.2 mmol) and triisopropyl borate (0.5 mL, 2.2 mmol) were added in portions over 6 hours to complete the reaction. An aliquot of 0.9 mL was added to a mixture of 6-chloro-*N*-{4-[(4-methylpiperazin-1-yl)carbonyl]phenyl}pyrazine-2-amine (synthesized according to the method described for Intermediate 45) (40 mg, 0.12 mmol), K₂CO₃ (25 mg, 0.30 mmol) and Pd(PPh₃)₄ (7 mg, 0.06 mmol) in MeCN/water (7:3, 4 mL). The mixture was irradiated in a microwave oven at 120 °C for 10 minutes. The solvent was evaporated and the residue was extracted with DCM (2x 10 mL) and saturated aq Na₂CO₃ (10 mL). The organic layers were combined, dried (Na₂SO₄), filtered and concentrated. The material was purified by preparative HPLC (XTerra C 18, 50 mM NH₄HCO₃ pH 10, MeCN). The title compound was obtained as a light yellow solid (6 mg).
HPLC 98% (System A), 99% (System B).
MS (ESI+) calcd for C₂₃H₂₃N₇O 413.1964, found 413.1966.

### Example 189

### 2-[6-({4-[(4-methylpiperazin-1-yl)carbonyl]phenyl}amino)pyrazin-2-yl]-1H. indol-4-ol trifluoroacetate (salt)

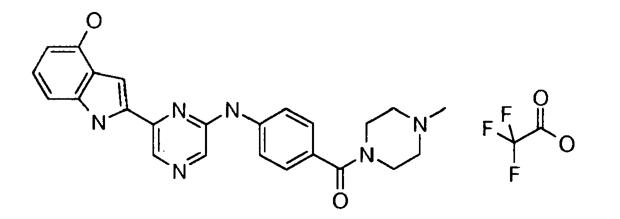

[4-(Benzyloxy)-1-(tert-butoxycarbonyl)- H-indol-2-yl]boronic acid (121 mg, 0.33 mmol) was added to a mixture of 6-chloro-*N*-{4-[(4-methylpiperazin-1-yl)carbonyl]phenyl}pyrazine-2-amine (synthesized according to the method described for Intermediate 45) (100 mg, 0.30 mmol), K₂CO₃ (104 mg, 0.75 mmol) and Pd(PPh₃)₄ (17 mg, 0.015 mmol) in MeCN/water (7:3, 4 mL). The mixture was stirred at 85 °C for 2 hours, subsequently filtered and concentrated. The residue was extracted by EtOAc (2x 20 mL) and saturated aq Na₂CO₃ (20 mL). The organic layers were combined, dried (Na₂SO₄), filtered and concentrated to give a brown solid. 75% of the crude material was dissolved in MeOH (7 mL) and Pd/C (5%, 200 mg) was added. The mixture was stirred under hydrogen gas (balloon) over night. The reaction mixture was filtered and concentrated. The residue wasdissolved in DCM/TFA (7:1, 8 mL) and stirred at 50 °C for 3 hours, subsequently extracted with saturated aq Na₂CO₃ and DCM (x2). The organic layers were combined and concentrated. Purification by preparative HPLC (ACE C8, 0.1% TFA, MeCN) gave the title compound (24 mg) as a yellow gum.
HPLC 98% (System A), 98% (System B).
.MS (ESI+) calcd for C₂₄H₂₄N₆O₂ 428.1961, found 428.1962.

### Intermediate 45

### 6-chloro-N-(4-{[(3R)-3-(dimethylamino)pyrrolidin-1-yl]carbonyl}-2-methoxyphenyl)pyrazin-2-amine

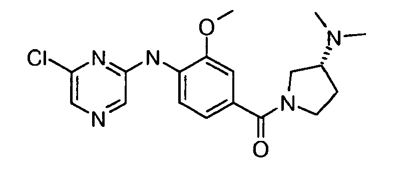

2,6-Dichloropyrazine (2.00 g, 13.4 mmol), 4-amino-3-methoxybenzoic acid (2.47 g, 14.8 mmol), NaOtBu (2.71 g, 28.2 mmol), Xantphos (233 mg, 0.40 mmol) and Pd₂(dba)₃ (123 mg, 0.13 mmol) were stirred in dry dioxane (100 mL). The reaction mixture was refluxed at 135 °C over night. The mixture was extracted with EtOAc (3x 100 mL) and 1M aq HCl (125 mL). The organic layers were combined, dried (Na₂SO₄) filtered and concentrated under reduced pressure to give 3.22 g of a brown solid. The material was used in the next step without further purification. Half of the material was suspended in dry MeCN (50 mL) and NEt₃ (1.58 mL, 11.4 mmol), 2,4,6-tripropyl-1,3,5,2,4,6-trioxatriphosphorinane-2,4,6-trioxide (50% in EtOAc, 6.81 mL, 11.4 mmol) and (3R)-N,N-dimethylpyrrolidin-3-amine (0.98 g, 8.58 mmol). The mixture was stirred at 60 °C over night. Water (2 mL) was added and the mixture was concentrated in vacuo. The material was extracted with EtOAc (3x 100 mL) and saturated aq Na₂CO₃/water (1:1, 150 mL). The organic layers were combined, dried (Na₂SO₄), filtered and concentrated to give 1.6 g of a brown solid. Purification was performed by flash column chromatography (2% NEt3, 1.5% MeOH in DCM). This gave the title comound as a beige solid (582 mg). MS (ESI+) for C18H22ClNSO2 m/z 376 (M+H)+.
HPLC 88% (System A).

### Example 190

### N-(4-{[(3R)-3-(dimethylamino)pyrrolidin-1-yl]carbonyl}-2-methoxyphenyl)-6-(1H-pyrrolo[3,2-c]pyridin-2-yl)pyrazin-2-amine

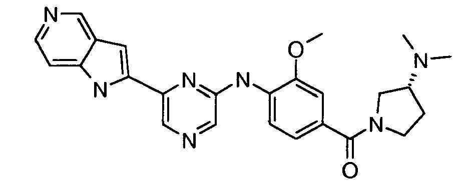

To 1H-pyrrolo[3,2-c]pyridine (500 mg, 4.2 mmol) in dry DCM (2 mL) were added DMAP (569 mg, 4.7 mmol, in 2 mL dry DCM ) and di-tert-butyl carbonate (1.02 g, 4.7 mmol, in 2 mL dry DCM). The mixture was stirred over night and extracted with 1M aq HCl (20 mL) and DCM (3x 20 mL). The organic layers were combined, dried (Na₂SO₄), filtered and concentrated to give 333 mg of a white solid, tert-butyl 1H-pyrrolo[3,2-c]pyridine-1-carboxylate. MS (ESI+) for C12H14N2O2 m/z 219 (M+H)⁺. Part of the material (163 mg, 0.75 mmol) was suspended in dry THF (5 mL) and triisopropyl borate (0.21 mL, 0.90 mmol) was added and the mixture was cooled on ice. 1.8 M LDA (0.50 mL, 0.90 mmol) was added dropwise over 20 minutes. Additional LDA (0.85 mL, 1.52 mmol, 2 additions) was added over 45 minutes to complete the reaction. An aliquot of 0.9 mL was added to a mixture of 6-chloro-N-(4-{[(3R)-3-(dimethylamino)pyrrolidin-1-yl]carbonyl}-2-methoxyphenyl)pyrazin-2-amine (20 mg, 0.05 mmol), K₂CO₃ (11 mg, 0.13 mmol) and Pd(PPh₃)₄ (6 mg, 0.05 mmol) in MeCN/water (7:3, 4 mL). The mixture was irradiated in a microwave oven at 120 °C for 10 minutes. This was performed twice, using a total of 40 mg of 6-chloro-N-(4-{[(3R)-3-(dimethylamino)pyrrolidin-1-yl]carbonyl}-2-methoxyphenyl)pyrazin-2-amine. The reactions were combined, the solvent was evaporated and the residue was extracted with DCM (2x 10 mL) and saturated aq Na₂CO₃ (10 mL). The organic layers were combined, dried (Na₂SO₄) filtered and concentrated. The material was purified twice by preparative HPLC (XTerra C 18, 50 mM NH₄HCO₃ pH 10, MeCN). The title compound was obtained as a light yellow solid (7 mg).
HPLC 99%(System A), 100%(System B).
1H NMR (400 MHz, MeOD), □ppm 1.75 - 1.95 (m, 1 H) 2.12 - 2.24 (m, 1 H) 2.25 (s, 3 H) 2.34 (s, 3 H) 2.75 - 2.96 (m, 1 H) 3.41 - 3.54 (m, 1 H) 3.57 - 3.91 (m, 3 H) 3.98 (s, 3 H) 7.22 (d, J=1.76 Hz, 1 H) 7.25 - 7.31 (m, 1 H) 7.33 (s, 1 H) 7.48 - 7.52 (m, 1 H) 8.17 (d, J=5.77 Hz, 1 H) 8.27 (s, 1 H) 8.51 (s, 1 H) 8.58 - 8.68 (m, 1 H) 8.84 (d, J=1.00 Hz, 1 H).
MS (ESI+) calcd for C₂₅H₂₇N₇O₂ 457.2226, found 457.2222.

### Intermediate 46

### 6-chloro-N-{5-[(4-methyl-1,4-diazepan-1-yl)carbonyl]pyridin-2-yl}pyrazin-2-amine

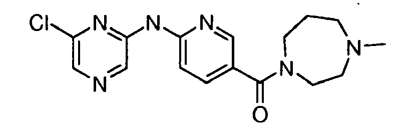

6-Chloronicotinoyl chloride (1.00 g, 5.68 mmol) in dry DCM (4 mL) was added dropwise to an ice cold solution of 1-methyl-1,4-diazepane (648 mg, 5.68 mmol) and NEt₃ (1.18 mL, 8.52 mmol) in dry DCM (16 mL). The mixture was allowed to attain rt and stirred for 2 hours. Saturated aq Na₂CO_{3/}wate (1:1, 40 mL) was added and the mixture was extracted with DCM (2x 30 mL). The organic layers were combined, dried (Na2SO4), filtered and concentrated to 1.33 g of a light brown oil. MS (ESI+) for C12H16ClN3O m/z 254 (M+H)⁺. Part of the material (294 mg, 1.16 mmol) was added to 6-chloropyrazin-2-amine (150 mg, 1.16 mmol), palladiumacetate (13 mg, 0.06 mmol), (±)-BINAP (36 mg, 0.06) and K₂CO₃ (2.40 g, 17.4 mmol) in dry toluene (12 mL) and stirred at 120 °C for 1.5 hours. The solvent was evaporated and the crude material was extracted with water/brine (1:1; 50 mL) and EtOAc (2x 50 mL). The organic layers were combined and concentrated. The title compound was obtained after purification by preparative HPLC (XTerra C 18, 50 mM NH₄HCO₃ pH 10, MeCN), as a light brown solid (55 mg). MS (ESI+) for C16H19ClN6O m/z 347 (M+H)+.
HPLC 92%(System A), 85%(System B).

### Example 191

### N-{5-[(4-methyl-1,4-diazepan-1-yl)carbonyl]pyridin-2-yl}-6-(1H-pyrrolo[3,2-c]pyridin-2-yl)pyrazin-2-amine bis(trifluoroacetate)

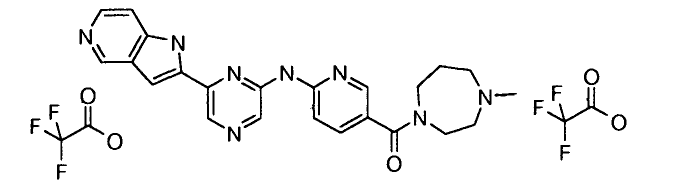

To an ice cold suspension of tert-butyl 1H-pyrrolo[3,2-c]pyridine-1-carboxylate (165 mg, 0.76 mmol) and triisopropyl borate (0.21 mL, 0.90 mmol) in dry THF (4 mL) was added LDA (0.50 mL, 0.90 mmol, 1.8M) dropwise over 20 minutes. The mixture was stirred for 30 minutes. Additional LDA (total 0.70 mL, 1.42 mmol) and triisopropyl borate (0.10 mL, 0.60 mmol), were added in portions over 4 hours to complete the reaction. An aliquot of 0.6 mL was added to a mixture of 6-chloro-N-{5-[(4-methyl-1,4-diazepan-1-yl)carbonyl]pyridin-2-yl}pyrazin-2-amine (20 mg, 0.06 mmol), K₂CO₃ (12 mg, 0.14 mmol) and Pd(PPh₃)₄ (7 mg, 0.06 mmol) in MeCN/water (7:3; 4 mL). The mixture was irradiated in a microwave oven at 120 °C for 15 minutes. This was performed three times, using a total of 50 mg of 6-chloro-N-{5-[(4-methyl-1,4-diazepan-1-yl)carbonyl]pyridin-2-yl}pyrazin-2-amine. The reactions were combined, the solvent was evaporated and the residue was extracted with DCM (2x 20 mL) and saturated aq Na₂CO₃ (20 mL). The organic layers were combined and concentrated. The material was purified by preparative HPLC (XTerra C18, 50 mM NH₄HCO₃ pH 10, MeCN). The title compound was obtained as a light yellow solid (2 mg).
HPLC 96% (System B), 98% (System A).
MS (ESI+) calcd for C₂₃H₂₄N₈O 428.2073, found 428.2062.

### Example 192

### 4-{[6-(1H-indol-2-yl)pyrazin-2-yl]amino}benzamide acetate

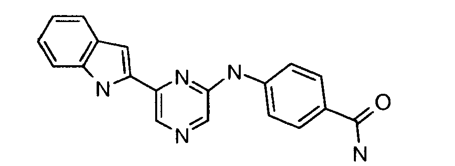

2-(6-Chloropyrazin-2-yl)-1*H*-indole (16.7 mg, 0.07 mmol), 4-aminobenzamide (24.5 mg, 0.18 mmol), sodium *tert*-butoxide (16.7 mg, 0.17 mmol), xantphos (1.0 mg, 0.002 mol) and bis(dibenzylideneacetone)palladium (0.5 mg, 0.001 mol) in dioxane (8 mL) were heated at 135°C for 16h, let to ambient temperature, filtered and the solvent was removed. The crude was dissolved in methanol (1 mL) and DMSO (0.5 mL) and and purified using preparative HPLC system E. Yield 5.4 mg (22%). HPLC 100%, R_{T}: 1.96 (10-97% MeCN over 3min).1H NMR (400 MHz, MeOD) □ ppm 6.92 - 7.00 (m, 1 H) 7.04 - 7.13 (m, 2 H) 7.36 - 7.43 (m, 1 H) 7.51 (d, *J*=8.03 Hz, 1 H) 7.81 - 7.84 (m, 4 H) 7.95 (s, 1 H) 8.39 (s, 1 H). MS 330 (M+H)+.

### Example 193

### 6-(1H-indol-2-yl)-N-[4-(morpholin-4-ylcarbonyl)phenyl]pyrazin-2-amine acetate

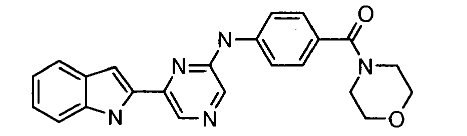

2-(6-Chloropyrazin-2-yl)-1*H*-indole (16.7 mg, 0.07 mmol), 4-(morpholin-4-ylcarbonyl)aniline (37.1 mg, 0.18 mmol), sodium *tert*-butoxide (16.7 mg, 0.17 mmol), xantphos (1.0 mg, 0.002 mol) and bis(dibenzylideneacetone)palladium (0.5 mg, 0.001 mol) in dioxane (8 mL) were heated at 135°C for 16h, let to ambient temperature, filtered and the solvent was removed. The crude was dissolved in methanol (1 mL) and DMSO (0.5 mL) and purified using preparative HPLC system E. Yield 2.0 mg (7%). HPLC 90%, R_{T}: 2.38 (5-100% MeCN over 3min). MS 399.2 (M+H)⁺.

### Example 194

### 4-{[6-(1H-indol-2-yl)pyrazin-2-yl]amino}-3-methoxy-N-[(3R)-pyrrolidin-3-yl]benzamide dihydrochloride

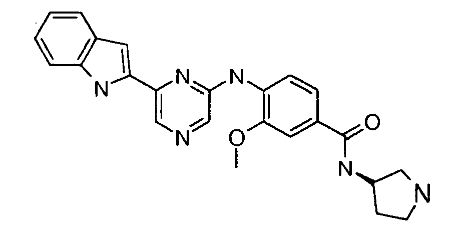

4-({6-[1-(tert-Butoxycarbonyl)-1H-indol-2-yl]pyrazin-2-yl}aamino)-3-methoxybenzoic acid (20.0 mg, 0.04 mmol), *tert*-butyl (3R)-3-aminopyrrolidine-1-carboxylate (13.5 mg, 0.07 mmol), TBTU (18.1 mg, 0.06 mmol) and TEA (7.5 µL, 0.06 mmol) in DMF (1 mL) were shaken at ambient temperature for 48h and then the temperature was raised to 140°C for 6h, let to ambient temperature and purified using preparative HPLC system A. Methanol (2 mL) and 2M HCl in ether (0.08 mol) were added to the crude and the mixture was shaken at ambient temperature for 3h and solvent was removed under reduced pressure. Yield 1.9 mg (11%). HPLC 97%, R_{T}: 1.862 (10-97% MeCN over 3min). 1H NMR (400 MHz, MeOD) □ ppm 2.17 (d, *J*=7.03 Hz, 1 H) 2.26 - 2.42 (m, 1 H) 3.27 - 3.44 (m, 2 H) 3.46 - 3.65 (m, 2 H) 3.95 (s, 3 H) 4.50 - 4.66 (m, 1 H) 6.87 - 7.07 (m, 1 H) 7.08 - 7.23 (m, 1 H) 7.25 (s, 1 H) 7.42 (d, *J*=8.28 Hz, 1 H) 7.47 - 7.72 (m, 3 H) 8.12 (s, 1 H) 8.39 - 8.53 (m, 2 H). MS 429 (M+H)+.

### Example 195

### 4-{[6-(1H-indol-2-yl)pyrazin-2-yl]amino}-3-methoxy-N-[(3S)-pyrrolidin-3-yl]benzamide dihydrochloride

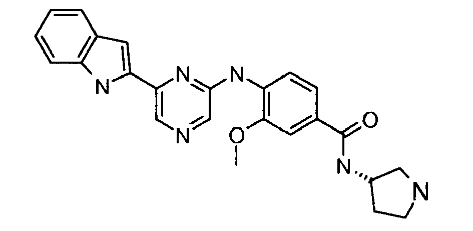

4-({6-[1-(*Tert*-butoxycarbonyl)-1H-indol-2-yl]pyrazin-2-yl}amino)-3-methoxybenzoic acid (20.0 mg, 0.04 mmol), *tert*-butyl (3S)-3-aminopyrrolidine-1-carboxylate (13.5 mg, 0.07 mmol), TBTU (18.1 mg, 0.06 mmol) and TEA (7.5 µL, 0.06 mmol) in DMF (1 mL) were shaken at ambient temperature for 48h and then the temperature was raised to 140°C for 6h, let to ambient temperature and purified using preparative HPLC system A. Methanol (2 mL) and 2M HCl in ether (0.08 mol) were added to the residue and the mixture was shaken at ambient temperature for 3h and solvent was removed under reduced pressure. Yield 9.5 mg (52%). HPLC 97%, R_{T}: 1.855 (10-97% MeCN over 3min). 1H NMR (400 MHz, MeOD) □ ppm 2.24 - 2.37 (m, 1 H) 2.39 - 2.53 (m, 1 H) 3.42 - 3.55 (m, 2 H) 3.60 - 3.71 (m, 2 H) 4.06 (s, 3 H) 4.69 - 4.78 (m, 1 H) 7.11 (t, J=7.53 Hz, 1 H) 7.24 - 7.32 (m, 1 H) 7.39 (s, 1 H) 7.54 (d, *J*=8.28 Hz, 1 H) 7.61 - 7.67 (m, 1 H) 7.68 (d, *J*=1.76 Hz, 1 H) 7.76 (dd, *J*=8.41, 1.88 Hz, 1 H) 8.20 (s, 1 H) 8.49 (d, *J*=8.28 Hz, 1 H) 8.54 (s, 1 H). MS 429 (M+H)+.

### Example 196

### N-(4-{[(3S)-3-aminopyrrolidin-1-yl]carbonyl}-2-methoxyphenyl)-6-(1H-indol-2-yl)pyrazin-2-amine dihydrochloride

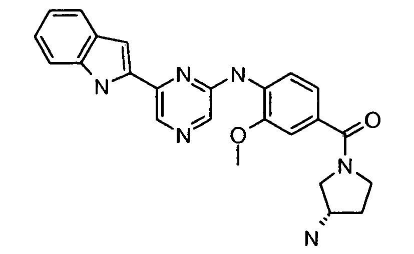

4-({6-[1-(tert-Butoxycarbonyl)-1 H-indol-2-yl]pyrazin-2-yl}amino)-3-methoxybenzoic acid (20.0 mg, 0.04 mmol), *tert*-butyl (3S)-pyrrolidin-3-ylcarbamate (13.5 mg, 0.07 mmol), TBTU (18.1 mg, 0.06 mmol) and TEA (7.5 µL, 0.06 mmol) in DMF (1 mL) were shaken at ambient temperature for 48h and then the temperature was raised 140°C for 6h, let to ambient temperature and purified using preparative HPLC system A. Methanol (2 mL) and 2M HCl in ether (0.08 mol) were added to the crude and the mixture was shaken at ambient temperature for 3h and solvent was removed under reduced pressure. Yield 5.4 mg (29%). HPLC 90%, R_{T}: 1.781 (10-97% MeCN over 3min). 1H NMR (400 MHz, MeOD) □ ppm 2.08 (s, 1 H) 2.27 - 2.46 (m, 1 H) 3.61 - 4.03 (m, 8 H) 7.00 (t, *J*=7.53 Hz, 1 H) 7.13 - 7.20 (m, 1 H) 7.28 (d, *J*=8.53 Hz, 3 H) 7.43 (d, *J*=8.28 Hz, 1 H) 7.54 (d, *J*=8.03 Hz, 1 H) 8.08 (s, 1 H) 8.37 (d, *J*=7.53 Hz, 1 H) 8.43 (s, 1 H). MS 429 (M+H)⁺.

### Example 197

### N-(4-{[(3R)-3-aminopyrrolidin-1-yl]carbonyl}-2-methoxyphenyl)-6-(1H-indol-2-yl)pyrazin-2-amine dihydrochloride

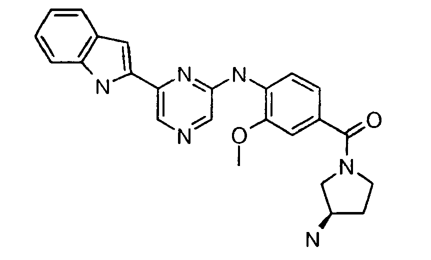

4-({6-[1-(tert-Butoxycarbonyl)-1H-indol-2-yl]pyrazin-2-yl}amino)-3-methoxybenzoic acid (20.0 mg, 0.04 mmol), *tert*-butyl (3R)-pyrrolidin-3-ylcarbamate (13.5 mg, 0.07 mmol), TBTU (18.1 mg, 0.06 mmol) and TEA (7.5 µL, 0.06 mmol) in DMF (1 mL) were shaken at ambient temperature for 48h and then the temperature was raised 140°C for 6h, let to ambient temperature and purified using preparative HPLC system A. Methanol (2 mL) and 2M HCl in ether (0.08 mol) were added and the crude mixture was shaken at ambient temperature for 3h after which the solvent was removed under reduced pressure. Yield 4.2 mg (23%). HPLC 90%, R_{T}: 1.776 (10-97% MeCN over 3min). 1H NMR (400 MHz, MeOD) 0 ppm 2.19 (s, 1 H) 2.47 (s, 1 H) 3.71 - 4.15 (m, 8 H) 7.12 (t, *J*=7.53 Hz, 1 H) 7.25 - 7.32 (m, 1 H) 7.41 (s, 3 H) 7.55 (d, *J*=8.28 Hz, 1 H) 7.67 (d, *J*=8.03 Hz, 1 H) 8.21 (s, 1 H) 8.48 (s, 1 H) 8.56 (s, 1 H). MS 429 (M+H)+.

### Example 198

### N-(2-hydroxyethyl)-4-{[6-(1H-indol-2-yl)pyrazin-2-yl]amino}-3-methoxybenzamide

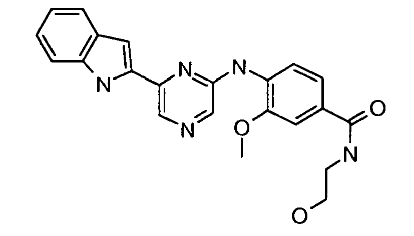

4-({6-[1-(tert-Butoxycarbonyl)-1H-indol-2-yl]pyrazin-2-yl}amino)-3-methoxybenzoic acid (20.0 mg, 0.04 mmol), 2-aminoethanol (4.3 µL, 0.07 mmol), TBTU (18.1 mg, 0.06 mmol) and TEA (7.5 µL, 0.06 mmol) in DMF (1 mL) were shaken at ambient temperature for 48h and then the temperature was raised 140°C for 6h, let to ambient temperature and purified using preparative HPLC system A followed by system E. Yield 0.4 mg (2%). HPLC 100%, R_{T}: 1.906 (10-97% MeCN over 3min). MS 404 (M+H)⁺.

### Example 199

### 6-(1H-indol-2-yl)-N-(2-methoxy-4-{[(2S)-2-(pyrrolidin-1-ylmethyl)pyrrolidin-1-yl]carbonyl}phenyl)pyrazin-2-amine trifluoroacetate

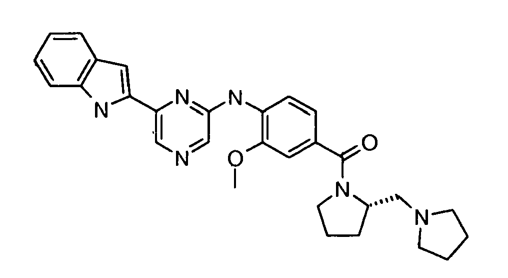

4-({6-[1-(tert-Butoxycarbonyl)-1H-indol-2-yl]pyrazin-2-yl}amino)-3-methoxybenzoic acid (20.0 mg, 0.04 mmol), 1-[(2S)-pyrrolidin-2-ylmethyl]pyrrolidine (11.1 mg, 0.07 mmol), TBTU (18.1 mg, 0.06 mmol) and TEA (7.5 µL, 0.06 mmol) in DMF (1 mL) were shaken at ambient temperature for 48h after which the temperature was raised 140°C for 6h. Purification using preparative HPLC system A gave 4.6 mg of the title compound (22%). HPLC 90%, R_{T}: 2.063 (10-97% MeCN over 3min). 1H NMR (400 MHz, MeOD) □ ppm 1.65 - 1.84 (m, 2 H) 1.85 - 2.16 (m, 6 H) 2.19 - 2.33 (m, 1 H) 3.05 - 3.18 (m, 2 H) 3.53 (dd, *J*=13.30, 8.28 Hz, 1 H) 3.60 - 3.70 (m, 3 H) 3.86 - 4.03 (m, 5 H) 4.49 (dd, *J*=7.65, 3.14 Hz, 1 H) 6.96 (t, *J*=7.53 Hz, 1 H) 7.06 - 7.15 (m, 2 H) 7.21 (d, *J*=1.76 Hz, 1 H) 7.28 (dd, *J*=8.28, 1.76 Hz, 1 H) 7.38 (d, *J*=8.03 Hz, 1 H) 7.51 (d, *J*=8.03 Hz, 1 H) 8.10 - 8.16 (m, 1 H) 8.40 (s, 1 H) 8.58 (d, *J*=8.28 Hz, 1 H). MS 497 (M+H)+.

### Example 200

### N-[4-(2,7-diazaspiro[4.5]dec-2-ylcarbonyl)-2-methoxyphenyl]-6-(1H-indol-2-yl)pyrazin-2-amine dihydrochloride

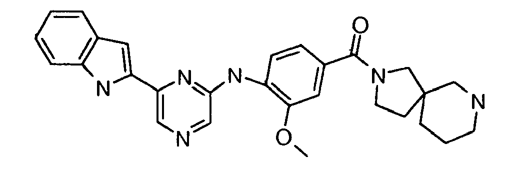

4-({6-[1-(tert-Butoxycarbonyl)-1H-indol-2-yl]pyrazin-2-yl}amino)-3-methoxybenzoic acid (20.0 mg, 0.04 mmol), *tert*-butyl 2,7-diazaspiro[4.5]decane-7-carboxylate (11.1 mg, 0.07 mmol), TBTU (18.1 mg, 0.06 mmol) and TEA (7.5 µL, 0.06 mmol) in DMF (1 mL) were shaken at ambient temperature for 48h and then the temperature was raised 140°C for 6h, let to ambient temperature and purified using preparative HPLC system A. Methanol (2 mL) and 2M HCl in ether (0.08 mol) were added to the crude mixture and was shaken at ambient temperature for 3h after which solvent was removed under reduced pressure. Yield 4.2 mg (20%). HPLC 90%, R_{T}: 1.883 (10-97% MeCN over 3min). 1H NMR (400 MHz, MeOD) □ ppm 1.63 - 2.18 (m, 8 H) 3.06 - 3.26 (m, 2 H) 3.45 - 3.85 (m, 4 H) 4.04 (s, 3 H) 7.04 - 7.80 (m, 7 H) 8.22 (s, 1 H) 8.46 (s, 1 H) 8.58 (s, 1 H). MS 483 (M+H)⁺.

### Example 201

### 4-{[6-(1H-indol-2-yl)pyrazin-2-yl]amino}-3-methoxy-N-piperidin-3-ylbenzamide trifluoroacetate

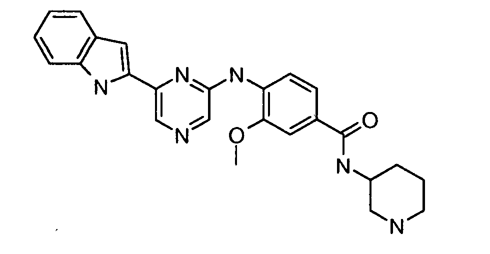

4-({6-[1-(tert-Butoxycarbonyl)-1H-indol-2-yl]pyrazin-2-yl}amino)-3-methoxybenzoic acid (20.0 mg, 0.04 mmol), *tert*-butyl 3-aminopiperidine-1-carboxylate (14.5 mg, 0.07 mmol), TBTU (18.1 mg, 0.06 mmol) and TEA (7.5 µL, 0.06 mmol) in DMF (1 mL) were shaken at ambient temperature for 48h after which the temperature was raised to 140°C for 6h, let to ambient temperature and purified using preparative HPLC system A. Methanol (2 mL) and 2M HCl in ether (0.08 mol) were added to the crude mixture and was shaken at ambient temperature for 3h after which the solvent was removed under reduced pressure. Yield 3.6 mg (19%). HPLC 98%, R_{T}: 1.946 (10-97% MeCN over 3min). 1H NMR (400 MHz, MeOD) □ ppm 2.60 (s, 1 H) 2.76 (s, 1 H) 2.85 - 2.92 (m, 1 H) 3.03 (q, *J*=2.01 Hz, 1 H) 3.30 - 3.37 (m, 1 H) 3.78 (s, 2 H) 3.91 (s, 3 H) 4.20 (s, 2 H) 6.91 - 7.02 (m, 1 H) 7.06 - 7.22 (m, 4 H) 7.40 (d, *J*=8.28 Hz, 1 H) 7.53 (d, *J*=8.03 Hz, 1 H) 8.09 (s, 1 H) 8.37 - 8.45 (m, 1 H) 8.50 (d, *J*=8.28 Hz, 1 H). MS 443 (M+H)⁺.

### Example 202

### 2-[4-(4-{[6-(1H-indol-2-yl)pyrazin-2-yl]amino}-3-methoxybenzoyl)piperazin-1-yl]ethanol

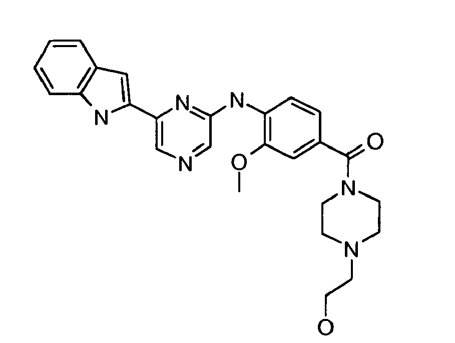

4-({6-[1-(tert-Butoxycarbonyl)-1H-indol-2-yl]pyrazin-2-yl}amino)-3-methoxybenzoic acid (20.0 mg, 0.04 mmol), 2-piperazin-1-ylethanol (8.9 µL, 0.07 mmol), TBTU (18.1 mg, 0.06 mmol) and TEA (7.5 µL, 0.06 mmol) in DMF (1 mL) were shaken at ambient temperature for 48h and then the temperature was raised 140°C for 6h, let to ambient temperature and purified using preparative HPLC system A followed by system E. Yield 0.5 mg (2%). HPLC 100%, R_{T}: 1.753 (10-97% MeCN over 3min). MS 473 (M+H)⁺.

### Example 203

### 6-(1H-indol-2-yl)-N-(2-methoxy-4-{[4-(2-methoxyethyl)piperazin-1-yl]carbonyl}phenyl)pyrazin-2-amine

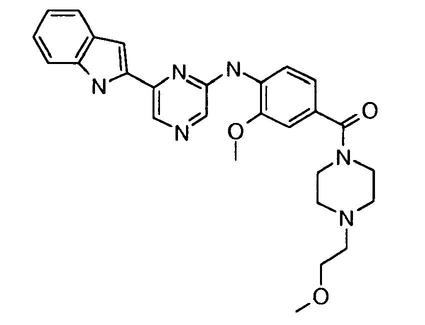

4-({6-[1-(tert-B-utoxycarbonyl)-1H-indol-2-yl]pyrazin-2-yl}amino)-3-methoxybenzoic acid (20.0 mg, 0.04 mmol), 1-(2-methoxyethyl)piperazine (10.4 mg, 0.07 mmol), TBTU (18.1 mg, 0.06 mmol) and TEA (7.5 µL, 0.06 mmol) in DMF (1 mL) were shaken at ambient temperature for 48h after which the temperature was raised to 140°C for 6h, let to ambient temperature and purified using preparative HPLC system A followed by system E. Yield 0.4 mg (2%). HPLC 100%, R_{T}: 1.854 (10-97% MeCN over 3min). MS 487 (M+H)+.

### Example 204

### N-(4-{[(3R)-3-aminopyrrolidin-1-yl]carbonyl}-2-methoxyphenyl)-6-(5-fluoro-1H-indol-2-yl)pyrazin-2-amine dihydrochloride

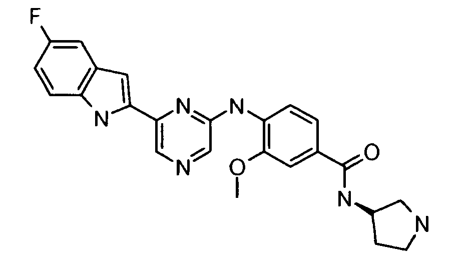

4-({6-[1-(tert-Butoxycarbonyl)-5-fluoro-1H-indol-2-yl]pyrazin-2-yl}amino)-3-methoxybenzoic acid (20.8 mg, 0.04 mmol), and *tert*-butyl (3R)-pyrrolidin-3-ylcarbamate (13.5 mg, 0.07 mmol), TBTU (18.1 mg, 0.06 mmol) and TEA (7.5 µL, 0.06 mmol) in DMF (1 mL) were shaken at ambient temperature for 48h after which the temperature was raised to 140°C for 6h, let to ambient temperature and purified using preparative HPLC system A followed by system E. Methanol (2 mL) and 2M HCl in ether (0.08 mol) were added to the crude and the mixture was shaken at ambient temperature for 3h and solvent was removed under reduced pressure. Yield 1.1 mg (6%). HPLC 100%, R_{T}: 1.832 (10-97% MeCN over 3min). MS 447 (M+H)⁺.

### Example 205

### 6-(5-fluoro-1H-indol-2-yl)-N-(2-methoxy-4-{[(3R)-3-methylpiperazin-1-yl]carbonyl}phenyl)pyrazin-2-amine

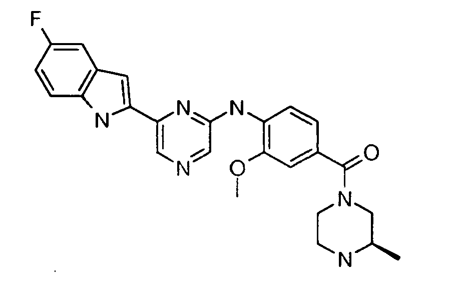

4-({6-[1-(tert-Butoxycarbonyl)-5-fluoro-1H-indol-2-yl]pyrazin-2-yl}amino)-3-methoxybenzoic acid (20.8 mg, 0.04 mmol), (2*R*)-2-methylpiperazine (5.7 mg, 0.06 mmol), TBTU (18.1 mg, 0.06 mmol) and TEA (7.5 µL, 0.06 mmol) in DMF (1 mL) were shaken at ambient temperature for 16h after which the temperature was raised to 140°C for 5h, let to ambient temperature and purified using preparative HPLC system E. Yield 2.0 mg (10%). HPLC 91%, R_{T}: 1.889 (10-97% MeCN over 3min). 1H NMR (400 MHz, MeOD) □ ppm 1.08 (s 3 H) 2.82 (s, 3 H) 3.09 (s, 2 H) 3.72 - 3.97 (m, 1 H) 4.01 (s, 3 H) 4.49 (s, 1 H) 6.91 - 7.06 (m, 1 H) 7.09 - 7.23 (m, 3 H) 7.29 (dd, *J*=9.54, 2.51 Hz, 1 H) 7.47 (dd, *J*=8.78, 4.52 Hz, 1 H) 8.23 (s, 1 H) 8.45 - 8.53 (m, 1 H) 8.67 (d, *J*=8.28 Hz, 1 H). MS 461 (M+H)⁺.

### Example 206

### 6-(5-fluoro-1H-indol-2-yl)-N-(2-methoxy-4-{[(3S)-3-methylpiperazin-1-yl]carbonyl}phenyl)pyrazin-2-amine

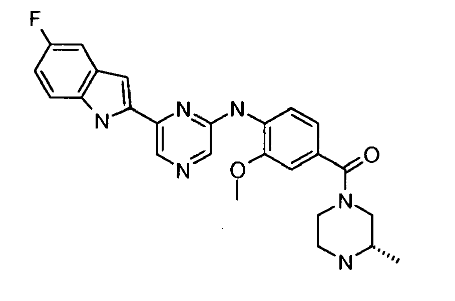

4-({6-[1-(tert-Butoxycarbonyl)-5-fluoro-1H-indol-2-yl]pyrazin-2-yl}amino)-3-methoxybenzoic acid (20.8 mg, 0.04 mmol), (2S)-2-methylpiperazine (5.7 mg, 0.06 mmol), TBTU (18.1 mg, 0.06 mmol) and TEA (7.5 µL, 0.06 mmol) in DMF (1 mL) were shaken at ambient temperature for 16h after which the temperature was raised to 140°C for 5h, let to ambient temperature and purified using preparative HPLC system E. Yield 1.5 mg (8%). HPLC 92%, R_{T}: 1.888 (10-97% MeCN over 3min). 1H NMR (400 MHz, MeOD) □ ppm 0.99 (s, 3 H) 2.71 (d, J=4.52 Hz, 2 H) 2.81 - 3.10 (m, 2 H) 3.78 (s, 1 H) 3.85 - 3.95 (m, 3 H) 4.39 (s, 1 H) 6.78 - 6.92 (m, 1 H) 6.97 - 7.11 (m, 3 H) 7.17 (dd, *J*=9.54, 2.51 Hz, 1 H) 7.35 (dd, *J*=8.78, 4.52 Hz, 1 H) 8.12 (s, 1 H) 8.37 (s, 1 H) 8.55 (d, *J*=8.03 Hz, 1 H). MS 461 (M+H)⁺.

### Example 207

### N-(4-{[(3R,5S)-3,5-dimethylpiperazin-1-yl]carbonyl}-2-methoxyphenyl)-6-(1H-indol-2-yl)pyrazin-2-amine

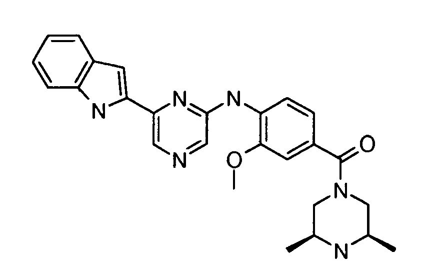

4-({6-[1-(tert-Butoxycarbonyl)-1H-indol-2-yl]pyrazin-2-yl}amino)-3-methoxybenzoic acid (20.0 mg, 0.04 mmol), (2*R*,6*S*)-2,6-dimethylpiperazine (24.8 mg, 0.22 mmol), TBTU (69.7 mg, 0.22 mmol) and TEA (29.0 µL, 0.22 mmol) in DMF (1 mL) were shaken at ambient temperature for 6h, heated at 140°C for 16h and purified using preparative HPLC system E. Yield 6.7 mg (34%). HPLC 100%, R_{T}: 1.871 (10-97% MeCN over 3min). 1H NMR (400 MHz, MeOD) □ ppm 1.11 (s, 6 H) 2.50 (s, 1 H) 2.73 - 3.02 (m, 3 H) 3.72 - 3.92 (m, 1 H) 3.99 (s, 3 H) 4.55 (s, 1 H) 7.00 - 7.32 (m, 5 H) 7.50 (d, *J*=8.28 Hz, 1 H) 7.62 (d, *J*=7.78 Hz, 1 H) 8.14 - 8.24 (m, 1 H) 8.43 - 8.54 (m, 1 H) 8.61 - 8.72 (m, 1 H). MS 457 (M+H)⁺.

### Example 208

### N-(1,2-diethylpyrazolidin-4-yl)-4-{[6-(1H-indol-2-yl)pyrazin-2-yl]amino}-3-methoxybenzamide

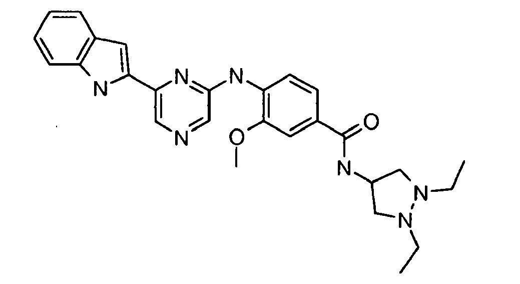

4-({6-[1-(tert-Butoxycarbonyl)-1H-indol-2-yl]pyrazin-2-yl}amino)-3-methoxybenzoic acid (20.0 mg, 0.04 mmol), 1,2-diethylpyrazolidin-4-amine (30.8 mg, 0.22 mmol), TBTU (69.7 mg, 0.22 mmol) and TEA (29.0 µL, 0.22 mmol) in DMF (1 mL) were shaken at ambient temperature for 6h, heated at 140°C for 16h and purified using preparative HPLC system E. Yield 10.3 mg (49%). HPLC 100%, R_{T}: 2.025 (10-97% MeCN over 3min). 1H NMR (400 MHz, MeOD) □ ppm 1.14 (t, *J*=7.28 Hz, 6 H) 2.76 (q, *J*=7.1 1 Hz, 4 H) 2.92 (dd, *J*=11.04, 6.53 Hz, 2 H) 3.34 - 3.40 (m, 2 H) 4.02 (s, 3 H) 4.74 - 4.82 (m, 1 H) 7.03 - 7.11 (m, 1 H) 7.14 - 7.25 (m, 2 H) 7.49 (d, *J*=8.28 Hz, 1 H) 7.51 - 7.55 (m, *J*=2.01 Hz, 1 H) 7.57 - 7.66 (m, 2 H) 8.20 (s, 1 H) 8.47 (s, 1 H) 8.66 (d, *J*=8.53 Hz, 1 H). MS 486 (M+H)⁺.

### Example 209

### N-[2-(dimethylamino)ethyl]-4-{[6-(1H-indol-2-yl)pyrazin-2-yl]amino}-3-methoxy-N-methylbenzamide

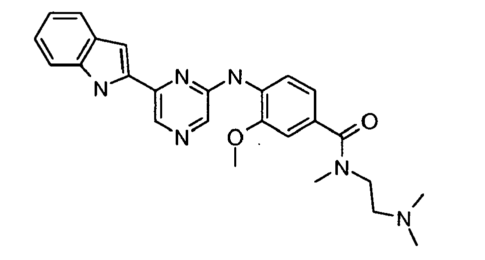

4-({6-[1-(tert-Butoxycarbonyl)-1H-indol-2-yl]pyrazin-2-yl}amino)-3-methoxybenzoic acid (20.0 mg, 0.04 mmol), *N*,*N-*dimethylethane-1,2-diamine (23.9 mg, 0.22 mmol), TBTU (69.7 mg, 0.22 mmol) and TEA (29.0 µL, 0.22 mmol) in DMF (1 mL) were shaken at ambient temperature for 6h, heated at 140°C for 16h and purified using preparative HPLC system E. Yield 6.8 mg (36%). HPLC 100%, R_{T}: 1.886 (10-97% MeCN over 3min).
1H NMR (400 MHz, MeOD) □ ppm 2.08 - 2.51 (m, 6 H) 2.67 (d, *J*=3.76 Hz, 2 H) 3.14 (s, 3 H) 3.52 - 3.80 (m, 2 H) 4.00 (s, 3 H) 7.03 - 7.12 (m, 1 H) 7.12 - 7.26 (m, 4 H) 7.50 (d, *J*=8.28 Hz, 1 H) 7.62 (d, *J*=8.03 Hz, 1 H) 8.19 (s, 1 H) 8.48 (s, 1 H) 8.64 (d, *J*=8.28 Hz, 1 H). LC-MS 445 (M+H)⁺.

### Example 210

### tert-butyl [1-(4-{[6-(5-fluoro-1H-indol-2-yl)pyrazin-2-yl]amino}-3-methoxybenzoyl)azetidin-3-yl]carbamate

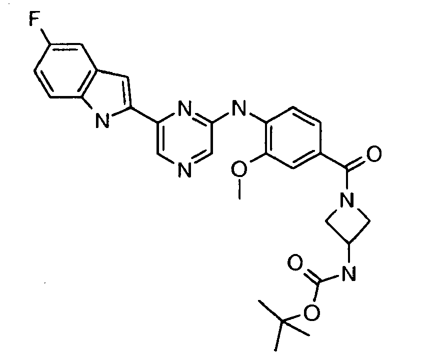

4-({6-[1-(tert-Butoxycarbonyl)-5-fluoro-1 H-indol-2-yl]pyrazin-2-yl}amino)-3-methoxybenzoic acid (100.0 mg, 0.21 mmol), *tert*-butyl azetidin-3-ylcarbamate (47.0 mg, 0.27 mmol), TBTU (87.0 mg, 0.27 mmol) and TEA (38.0 µL, 0.27 mmol) in DMF (2 mL) were stirred at ambient temperature for 3h, heated at 140°C for 3h, let to ambient temperature and purified using preparative HPLC system E. Yield 23.5 mg (21%). HPLC 97%, R_{T}: 2.452 (10-97% MeCN over 3min). 1H NMR (400 MHz, MeOD) □ ppm 1.36 (s, 9 H) 3.88 (s, 3 H) 3.91 - 3.99 (m, *J*=3.01 Hz, 1 H) 4.22 (s, 1 H) 4.35 (s, 2 H) 4.59 (s, 1 H) 6.80 - 6.91 (m, 1 H) 7.02 (s, 1 H) 7.15 (dd, *J*=9.66, 2.38 Hz, 1 H) 7.19 - 7.29 (m, 2 H) 7.34 (dd, *J*=8.91, 4.39 Hz, 1 H) 8.09 (s, 1 H) 8.34 (s, 1 H) 8.55 (d, *J*=8.28 Hz, 1 H). MS 533 (M+H)⁺.

### Example 211

### tert-butyl 3-[(4-{[6-(5-fluoro-1H-indol-2-yl)pyrazin-2-yl]amino}-3-methoxybenzoyl)amino]azetidine-1-carboxylate

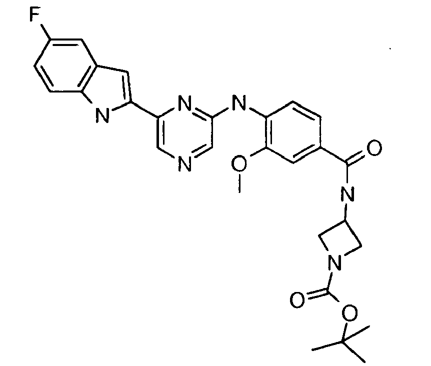

4-({6-[1-(tert-Butoxycarbonyl)-5-fluoro-1H-indol-2-yl]pyrazin-2-yl}amino)-3-methoxybenzoic acid (100.0 mg, 0.21 mmol), *tert*-butyl 3-aminoazetidine-1-carboxylate (47.0 mg, 0.27 mmol), TBTU (87.0 mg, 0.27 mmol) and TEA (38.0 µL, 0.27 mmol) in DMF (2 mL) were stirred at ambient temperature for 3h, heated at 140°C for 3h, let to ambient temperature and the product was purified using preparative HPLC system E. Yield 45.5 mg (41%). HPLC 99%, R_{T}: 2.004 (10-97% MeCN over 3min). 1H NMR (400 MHz, MeOD) □ ppm 1.48 (s, 9 H) 3.99 (t, 5 H) 4.29 (t, *J*=8.41 Hz, 2 H) 4.72 - 4.81 (m, 1 H) 6.91 - 7.00 (m, 1 H) 7.09 (s, 1 H) 7.24 (dd, *J*=9.66, 2.38 Hz, 1 H) 7.42 (dd, *J*=9.03, 4.52 Hz, 1 H) 7.51 (d, *J*=2.01 Hz, 1 H) 7.59 (dd, *J*=8.41, 1.88 Hz, 1 H) 8.17 (s, 1 H) 8.42 (s, 1 H) 8.62 (d, *J*=8.53 Hz, 1 H). MS 533 (M+H)⁺.

### Example 212

### N-{4-[(3-aminoazetidin-1-yl)carbonyl]-2-methoxyphenyl}-6-(5-fluoro-1H-indol-2-yl)pyrazin-2-amine hydrochloride

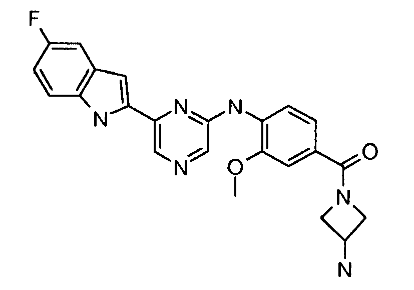

Methanol (4 mL) and 2M HCI (1 mL) were added to *tert*-butyl [1-(4-{[6-(5-fluoro-1H-indol-2-yl)pyrazin-2-yl]amino}-3-methoxybenzoyl)azetidin-3-yl]carbamate and the mixture was heated at 60°C for 2h, let to ambient temperature and the solvent was removed under reduced pressure. Yield 3.8 mg. HPLC 100%, R_{T}: 1.890 (10-97% MeCN over 3min). 1H NMR (400 MHz, MeOD) □ ppm 3.96 - 4.12 (m, 4 H) 4.25 (s, 2 H) 4.55 (s, 2 H) 7.04 (s, 1 H) 7.21 - 7.80 (m, 7 H) 8.26 (s, 1 H) 8.53 (s, 2 H). MS 433 (M+H)⁺.

### Example 213

### N-azetidin-3-yl-4-{[6-(5-fluoro-1H-indol-2-yl)pyrazin-2-yl]amino}-3-methoxybenzamide trifluoroacetate

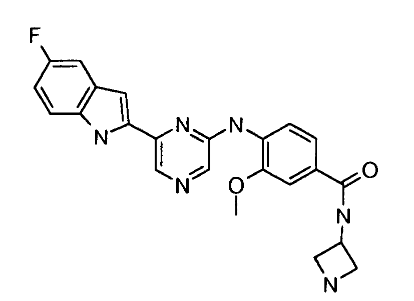

Methanol (4 mL) and 2M HCl (1 mL) were added to *tert*-butyl 3-[(4-{[6-(5-fluoro-1H-indol-2-yl)pyrazin-2-yl]amino}-3-methoxybenzoyl)amino]azetidine-1-carboxylate after which the mixture was heated at 60°C for 2h, let to ambient temperature and the solvent was removed under reduced pressure. Purification by preparative HPLC system A gave 3.9 mg of the title compound. HPLC 88%, R_{T}: 1.714 (10-97% MeCN over 3min). 1H NMR (400 MHz, MeOD) □ ppm 3.88 (s, 1 H) 3.90 - 3.97 (m, 4 H) 4.24 - 4.31 (m, 3 H) 6.81 - 6.92 (m, 1 H) 7.04 - 7.10 (m, 1 H) 7.17 (dd, *J*=9.66, 2.38 Hz, 1 H) 7.34 (dd, *J*=8.91, 4.39 Hz, 1 H) 7.47 (d, *J*=2.01 Hz, 1 H) 7.51 - 7.57 (m, 1 H) 8.18 (s, 1 H) 8.42 (s, 1 H) 8.58 - 8.62 (m, 1 H). MS 433 (M+H)⁺.

### Example 214

### 6-(1H-indol-2-yl)-N-{4-[(4-isopropylpiperazin-1-yl)carbonyl]-2-methoxyphenyl}pyrazin-2-amine

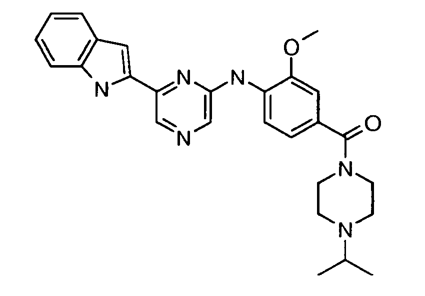

4-({1H-Indol-2-yl]pyrazin-2-yl}amino)-3-methoxybenzoic acid (15.1 mg, 0.04 mmol), 1-isopropylpiperazine (16.1 mg, 0.13 mmol), TBTU (40.3 mg, 0.13 mmol) and TEA (17.0 µL, 0.13 mmol) in DMF (1 mL) were shaken at ambient temperature for 5h and purified using preparative HPLC system E. Yield 4.5 mg (24%). HPLC 100%, R_{T}: 1.902 (10-97% MeCN over 3min). 1H NMR (400 MHz, MeOD) □ ppm 1.12 (d, *J*=6.53 Hz, 6 H) 2.63 (s, 4 H) 2.72 - 2.82 (m, 1 H) 3.57 - 3.86 (m, 4 H) 4.00 (s, 3 H) 7.03 - 7.10 (m, 1 H) 7.13 (d, *J*=1.76 Hz, 1 H) 7.15 - 7.24 (m, 3 H) 7.51 (d, *J*=8.28 Hz, 1 H) 7.62 (d, *J*=8.03 Hz, 1 H) 8.20 (s, 1 H) 8.48 (s, 1 H) 8.67 (d, *J*=8.28 Hz, 1 H). MS 471 (M+H)⁺.

### Example 215

### N-{4-[(4-ethylpiperazin-1-yl)carbonyl]-2-methosyphenyl}-6-(1H-indol-2-yl)pyrazin-2-amine

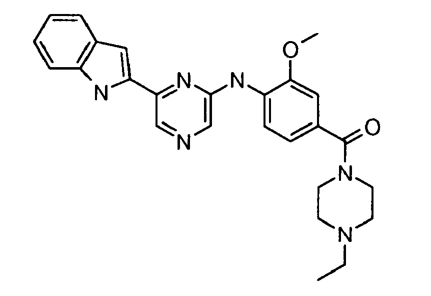

4-({1H-Indol-2-yl]pyrazin-2-yl}amino)-3-methoxybenzoic acid (15.1 mg, 0.04 mmol), 1-ethylpiperazine (14.3 mg, 0.13 mmol), TBTU (40.3 mg, 0.13 mmol) and TEA (17.0 µL, 0.13 mmol) in DMF (1 mL) were shaken at ambient temperature for 5h and the product was purified using preparative HPLC system E. Yield 5.2 mg (28%). HPLC 100%, R_{T}: 1.865 (10-97% MeCN over 3min). 1H NMR (400 MHz, MeOD) □ ppm 1.15 (t, *J*=7.28 Hz, 3 H) 2.39 - 2.71 (m, 6 H) 3.74 (s, 4 H) 4.00 (s, 3 H) 7.07 (t, *J*=7.40 Hz, 1 H) 7.13 (s, 1 H) 7.15 - 7.26 (m, 2 H) 7.50 (d, *J*=8.28 Hz, 1 H) 7.62 (d, *J*=7.78 Hz, 1 H) 8.20 (s, 1 H) 8.48 (s, 1 H) 8.67 (d, *J*=8.28 Hz, 1 H). MS 457 (M+H)⁺.

### Example 216

### 6-(1H-indol-2-yl)-N-{2-methoxy-4-[(4-methyl-1,4-diazepan-1-yl)carbonyl]phenyl}pyrazin-2-amine

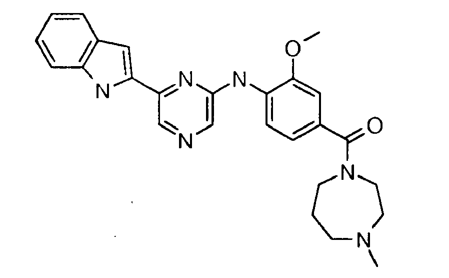

4-({1H-Indol-2-yl]pyrazin-2-yl}amino)-3-methoxybenzoic acid (15.1 mg, 0.04 mmol), 1-methyl-1,4-diazepane (14.3 mg, 0.13 mmol), TBTU (40.3 mg, 0.13 mmol) and TEA (17.0 µL, 0.13 mmol) in DMF (1 mL) were shaken at ambient temperature for 5h and purified using preparative HPLC system E. Yield 6.2 mg (34%). HPLC 100%, R_{T}: 1.845 (10-97% MeCN over 3min). 1H NMR (400 MHz, MeOD) □ ppm 2.05 (s, 2 H) 2.40 - 2.69 (m, 3 H) 2.88 (s, 3 H) 3.06 (s, 1 H) 3.63 - 3.91 (m, 4 H) 4.00 (s, 3 H) 7.07 (t, *J*=7.40 Hz, 1 H) 7.13 - 7.28 (m, 4 H) 7.50 (d, *J*=8.03 Hz, 1 H) 7.62 (d, *J*=7.28 Hz, 1 H) 8.20 (s, 1 H) 8.48 (s, 1 H) 8.66 (d, *J*=8.28 Hz, 1 H). MS 457 (M+H)⁺.

### Intermediate 47

### tert-Butyl methyl 4-{[5-(1H-indol-2-yl)pyridin-3-yl]amino}-3-methoxybenzoate-1-carboxylate

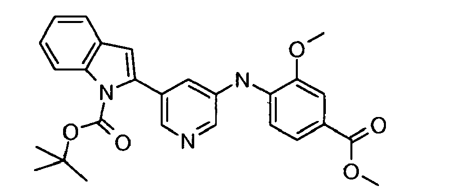

*tert-Butyl* 2-(5-aminopyridin-3-yl)-1*H*-indole-1-carboxylate (350.0 mg, 1.13 mmol), methyl 3-methoxy-4-{[(trifluoromethyl)sulfonyl]oxy}benzoate (528.0 mg, 1.68 mol), Xanthpos (26.5 mg, 0.05 mol), potassium *tert*-butoxide (342.8 mg, 3.05 mmol) and Pd₂dba₃ (14.0 mg, 0.02 mmol) in dioxane (50 mL) were refluxed for 16h, let to ambient temperature, filtered through a pad of celite and the solvent was removed under reduced pressure. The product was purified using preparative HPLC system D. Yield 85.7 mg (12%). HPLC 96%, R_{T}: 2.380 (10-97% MeCN over 3min). MS 474 (M+H)⁺.

### Intermediate 48

### 4-{[5-(1H-Indol-2-yl)pyridin-3-yl]amino}-3-methoxybenzoic acid

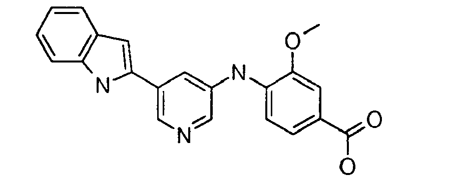

THF (8 mL) and 2 M HCl (8 mL) were added to *tert*-butyl methyl 4-{[5-(1*H-*indol-2-yl)pyridin-3-yl]amino}-3-methoxybenzoate-1-carboxylate (85.7 mg, 0.18 mmol) and the reaction was refluxed for 1h. The organic phase was separated and the solvent was removed under reduced pressure. Ethanol (8 mL) and 5 M NaOH (8 mL) were added to the crude mixture which was refluxed for 1h. To the reaction was added etylacetate and 2 M HCl and the mixture was extracted three times. The organic phases were combined, dried with MgSO₄ and the solvent was removed under reduced pressure. Yield 43.2 mg (67%).HPLC 70%, R_{T}: 1.748 (10-97% MeCN over 3min). MS 360 (M+H)⁺.

### Example 217

### 5-(1H-indol-2-yl)-N-{2-methoxy-4-[(4-methylpiperazin-1-yl)carbonyl]phenyl}pyridin-3-amine trifluoroacetate

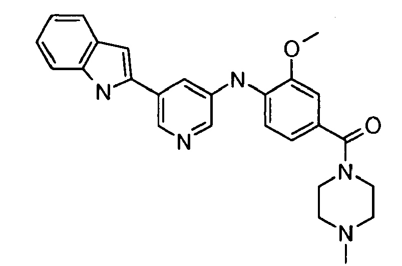

4-{[5-(1*H*-Indol-2-yl)pyridin-3-yl]amino}-3-methoxybenzoic acid (10.0 mg, 0.03 mol), TBTU (44.7 mg, 0.14 mmol), TEA (19.0 µL, 0.14 mmol) and 1-methylpiperazine (14.0 mg, 0.14 mmol) in DMF (1 mL) were shaken at ambient temperature for 3h and purified using preparative HPLC system A. Yield 4.9 mg (40%). HPLC 100%, R_{T}: 1.512 (10-97% MeCN over 3min). MS (electronspray) M+H+ m/z calcd 441.2165 found 441.2162.

### Example 218

### N-(4-{[(3R)-3-(dimethylamino)pyrrolidin-1-yl]carbonyl}-2-methoxyphenyl)-5-(1H-indol-2-yl)pyridin-3-amine trifluoroacetate

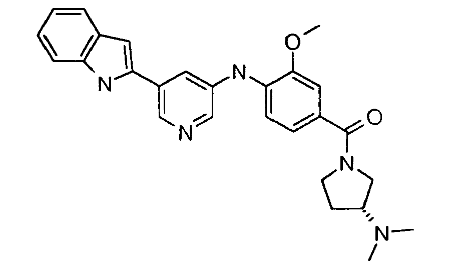

4-{[5-(1*H*-Indol-2-yl)pyridin-3-yl]amino}-3-methoxybenzoic acid (10.0 mg, 0.03 mol), TBTU (44.7 mg, 0.14 mmol), TEA (19.0 µL, 0.14 mmol) and (3*R*)-*N*,*N-*dimethylpyrrolidin-3-amine (15.9 mg, 0.14 mmol) in DMF (1 mL) were shaken at ambient temperature for 3h and purified using preparative HPLC system A.

Yield 4.4 mg (48%). HPLC 100%, R_{T}: 1.523 (10-97% MeCN over 3min). MS 456 (M+H)⁺. MS (electronspray) M+H+ m/z calcd 455.2321 found 455.2316.

### Example 219

### N-(4-{[(3S)-3-(dimethylamino)pyrrolidin-1-yl]carbonyl}-2-methoxyphenyl)-5-(1H-indol-2-yl)pyridin-3-amine trifluoroacetate

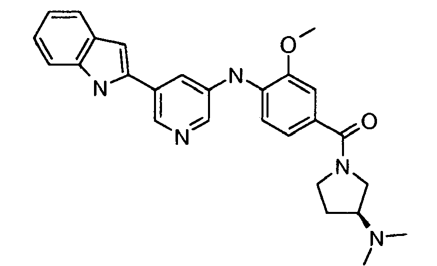

4-{[5-(1*H*-Indol-2-yl)pyridin-3-yl]amino}-3-methoxybenzoic acid (10.0 mg, 0.03 mol), TBTU (44.7 mg, 0.14 mmol), TEA (19.0 µL, 0.14 mmol) and (3*S*)-*N*,*N-*dimethylpyrrolidin-3-amine (15.9 mg, 0.14 mmol) in DMF (1 mL) were shaken at ambient temperature for 3h and purified using preparative HPLC system A. Yield 5.0 mg (39%). HPLC 100%, R_{T}: 1.513 (10-97% MeCN over 3min). MS 456 (M+H)⁺. MS (electronspray) M+H+ m/z calcd 455.2321 found 455.2310.

### Intermediate 49

### Methyl 4-{[5-(5-fluoro-1H-indol-2-yl)pyridin-3-yl]amino}benzoate

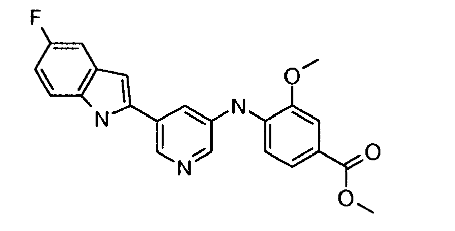

5-(5-fluoro-1*H*-indol-2-yl)pyridin-3-amine (500.0 mg. 2.20 mmol), methyl 3-methoxy-4-{[(trifluoromethyl)sulfonyl]oxy}benzoate (520.5 mg, 2.42 mol), xanthpos (38.2 mg, 0.07 mol), potassium *tert*-butoxide (493.8 mg, 4.40 mmol) and Pd₂dba₃ (20.2 mg, 0.02 mmol) in dioxane (50 mL) were refluxed for 3h, filtered through a pad of celite and the solvent was removed under reduced pressure. The product was purified using preparative HPLC system D. Yield 89.7 mg (11%). HPLC 100%, R_{T}: 2.025 (10-97% MeCN over 3min). MS 362 (M+H)⁺.

### Intermediate 50

### 4-{[5-(5-Fluoro-1H-indol-2-yl)pyridin-3-yl]amino}benzoic acid

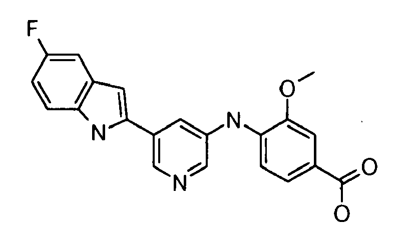

THF (8 mL) and 2 M HCl (8 mL) were added to methyl 4-{[5-(5-fluoro-1*H*-indol-2-yl)pyridin-3-yl]amino}benzoate (85.7 mg, 0.24 mmol) and the mixture was refluxed for 1h. HCl (2M) 10 mL was added and the mixture was extracted by etylacetate were (three times). The organic phases were combined, dried with MgSO₄ and the solvent was removed under reduced pressure. Yield: 60 mg (72%). HPLC 100%, R_{T}: 1.806 (10-97% MeCN over 3min).

### Example 220

### 5-(5-fluoro-1H-indol-2-yl)-N-{4-[(4-methyl-1,4-diazepan-1-yl)carbonyl]phenyl}pyridin-3-amine trifluoroacetate

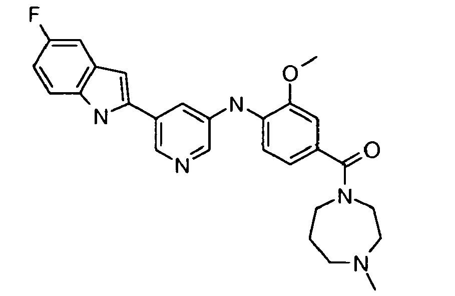

4-{[5-(5-Fluoro-1*H*-indol-2-yl)pyridin-3-yl]amino}benzoic acid 10.0 mg (0.03 mmol), TBTU (46.2 mg, 0.14 mmol), TEA (19.0 µL, 0.14 mmol) and 1-methyl-1,4-diazepane (16.4 mg, 0.14 mmol) in DMF (1 mL) were shaken at ambient temperature for 3h and purified using preparative HPLC system A. Yield 2.7 mg (21%). HPLC 100%, R_{T}: 1.54 (10-97% MeCN over 3min). MS 444 (M+H)⁺. MS (electronspray) M+H+ m/z calcd 443.2121 found 443.2120.

### Example 221

### N-(4-{[(3R)-3-(dimethylamino)pyrrolidin-1-yl]carbonyl}-2-methoxyphenyl)-6-(6-methoxy-1H-indol-2-yl)pyrazin-2-amine

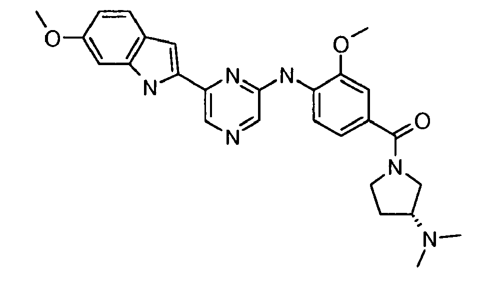

4-({6-[1-(tert-Butoxycarbonyl)-6-methoxy-1*H*-indol-2-yl]pyrazin-2-yl}amino)-3-methoxybenzoic acid (8.0 mg, 0.02 mmol), TBTU (26.2 mg, 0.08 mmol), TEA (11 µL, 0.08 mmol) and (3*R*)-N,N-dimethylpyrrolidin-3-amine (9.3 mg, 0.08 mmol) in DMF (1 mL) were stirred at 140 □C for 16h. The solvent was removed under reduced pressure. Purification by preperative HPLC system E gave 1.3 mg of the titled compound (16%). HPLC 100%, R_{T}: 1.77 (10-97% MeCN over 3min). 1H NMR (400 MHz, MeOD) □ ppm 1.82 - 1.93 (m, 1 H) 2.14 - 2.24 (m, 1 H) 2.28 (s, 3 H) 2.37 (s, 3 H) 2.80 - 2.99 (m, 2 H) 3.48 - 3.52 (m, 1 H) 3.60 - 3.84 (m, 2 H) 3.87 (s, 3 H) 4.01 (s, 3 H) 7.02 - 7.07 (m, 1 H) 7.12 (s, 1 H) 7.16-7.21 (m, 1 H) 7.23 - 7.27 (m, 1 H) 7.32 (d, *J*=8.03 Hz, 1 H) 7.50 (t, *J*=8.78 Hz, 1 H) 8.19 (d, *J*=22.84 Hz, 1 H) 8.47 (d, *J*=25.60 Hz, 1 H) 8.67 (d, *J*=7.78 Hz, 1 H). MS 487 (M+H)⁺.

### General procedure for the synthesis of Examples 222-224.

To 4-bromo-3-hydroxybenzoic acid (1g, 4.6mmol) in DCM was added DMF and oxalyl chloride (409uL, 4.6mmol) at 0°C. After 10 min., N-methylpiperazine (1.02mL, 9.2mmol) was added and the reaction was stirred at ambient temperature for 30 min. The resulting 1-methylpiperazinylcarbonyl-4-bromo-3-hydroxybenzene was filtered off . Yield 586mg (42%). White solid.

Alkylations were performed on 0.17mmol scale with the appropriate alkyl halide (0.18mmol), K₂CO₃ (47mg, 0.34mmol) in DMF with a few drops of MeCN. The reaction was heated in the microwave oven at 150°C for 30 min. Water was added and the crude mixtures were extracted with EtOAc three times. The combined organic phases were concentrated and used in the next step without further purification to afford the title compounds in yields between 31-70 %.

### Buchwald coupling was performed according to the following:

A mixture of the arylbromides (20mg, 0.05mmol), (6-(5-fluoro-*tert*-butylindol-1-carboxylate-2-yl)pyrazin-2-yl)amine (17mg, 0.05mmol), tris(benzylideneacetone)dipalladium(0) (3mg, 0.003mmol), xantphos (6mg, 0.009mmol), KO*^{t}*Bu (18mg, 0.17mmol) in dry dioxane was warmed 120°C for 1.5h. Full Boc-deprotection was effected using TFA in DCM 1:4 (10mL) at 50°C for 1h. The solvent was removed and the crude products were purified using System E.

### Example 222

### 2-{2-{[6-(5-fluoro-1H-indol-2-yl)pyrazin-2-yl]amino}-5-[(4-methylpiperazin-1-yl)carbonyl]phenoxy}-N,N-dimethylacetamide

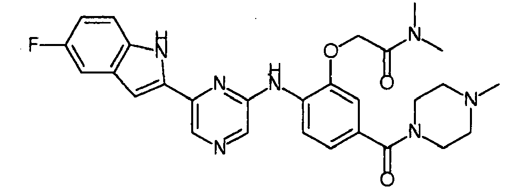

Alkyl halide: chloro-N,N-dimethylacetamide (19us, 0.18mmol). Yield 4mg (8%). Yellow solid. MS (electronspray) M+H+ m/z 531.2.

### Example 223

### N-{2-(2-ethoxyethoxy)-4-[(4-methylpiperazin-1-yl)carbonyl]phenyl}-6-(5-fluoro-1H-indol-2-yl)pyrazin-2-amine

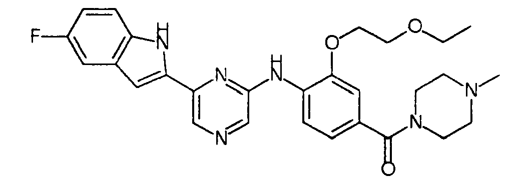

Alkyl halide: bromoethyl ethyl ether (21uL, 0.18mmol). Yield 7mg (19%). Yellow solid. MS (electronspray) M+H+ m/z 518.2.

### Example 224

### N-{2-[2-(dimethyrlamino)ethoxy]-4-[(4-methylpiperazin-1-yl)carbonyl]phenyl}-6-(5-fluoro-1H-indol-2-yl)pyrazin-2-amine

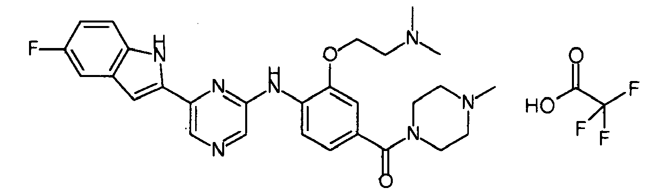

Alkyl halide: chloro-N,N-dimethylethylamine hydrochloride (104mg, 0.72mmol). The crude product was further purified using System D to give the product as the trifluoroacetate salt. Yield 16mg (16%). Yellow solid. MS (electronspray) M+H+ m/z 518.2.

### Example 225

### 2-{[6-(5-fluoro-1H-indol-2-yl)pyrazin-2-yl]amino}-5-(piperazin-1-ylcarbonyl)phenol

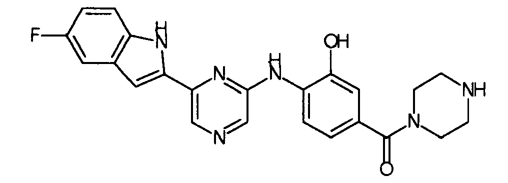

To a solution of 6-(5-fluoro-1H-indol-2-yl)-N-[2-methoxy-4-(piperazin-1-ylcarbonyl)phenyl]pyrazin-2-amine hydrochloride (which was synthesized using the same procedure as Example 206) (25mg, 0.052mmol) in 1,2-dichloroethane (4mL) was added BBr₃ (930uL, 1M in DCM, 0.93mmol) and the reaction was stirred at 40°C for 8 h and at ambient temperature for 5 days. After concentration of the solvent, purification was performed using system E. Yield 3mg (13%). Yellow solid. MS (electronspray) M+H+ m/z 432.2.

### Example 226

### 2-[6-({4-[(4-methylpiperazin-1-yl)carbonyl]phenyl}amino)pyrazin-2-yl]-1H-indole-5-carboxamide

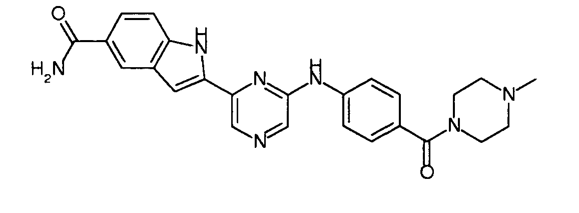

To 2-(6-chloropyrazinyl)-4-aminobenzoic acid (AF0951001) (1g, 4mmol) in DCM (200mL), and a few drops of DMF, oxalyl chloride (710uL, 0.8mmol) was added dropwise at 0°. After 10 min., methylpiperazine (1.6mL, 14.4mmol) was added and the mixture was allowed to stir overnight. The product was filtered off and the material was purified using System E to afford 269 mg of a beige solid.

A mixture of 1-boc-5-cyanoindole-2-boronic acid (233mg, 0.81mmol), 2-[6-({4-[(4-methylpiperazin-1-yl)carbonyl]phenyl}amino)pyrazin from above (269mg, 0.81mmol), Pd(PPh₃)₄ (46mg, 0.04mmol, 5mol%) and K₂CO₃ (280mg, 2mmol) in MeCN/H₂O 7:3 (10mL) was warmed in the microwave at 120°C for 10 min. The solvent was removed and the crude mixture was purified using System E to afford 105mg of a yellow solid.

To N-{4-[(4-methylpiperazin-1-yl)carbonyl]-phenyl}-6-(1H-indol-5-cyano-2-yl)pyrazin-2-amine from above (30mg, 0.069mmol) was added conc HCl (2mL) and the mixture was warmed at 50°C overnight. To the orange mixture was added water which was made basic with saturated NaHCO₃. The product was purified using System E to afford 3mg (10%) of a light yellow solid. HPLC 100 %, MS (electronspray) M+H+ m/z 456.1. MS (ESI+) Calcd for C25H25N7O2 455.2070 found 455.2081.

### Example 227

### 4-[6-({4-[(4-methylpiperazin-1-yl)carbonyl]phenyl}amino)pyrazin-2-yl]benzamide trifluoroacetate

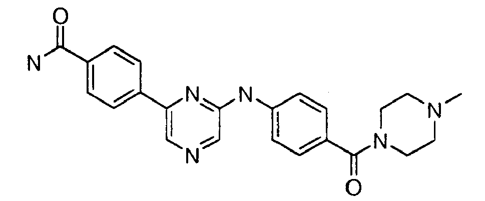

6-Chloro-*N*-{4-[(4-methylpiperazin-1-yl)carbonyl]phenyl}pyrazin-2-amine (10.0 mg, 0.03 mmol), NaHCO₃ (12.2 mg, 0.11 mmol) palladium tetrakis (0.4 mg, 0.0003 mmol) and [4-(aminocarbonyl)phenyl]boronic acid (4.9 mg, 0.03 mmol) in DME (3 mL) and water (1 mL) were heated in the microwave oven at 110 □C for 10 min, filter, solvent was removed under reduced pressure and purified using preparative HPLC system E. Yield 10.6 mg (85%). HPLC 98%. MS (ESI+) calcd for C₂₃H₂₄N₆O₂ 416.1961, found 416.1955.

### BIOLOGICAL METHODS

The ability of a compound of the invention to bind or act at MNK2 or MNK1 can be determined using *in vitro* and *in vivo* assays known in the art.

### In vitro MNK2a kinase assay (HTRF)

MNK2a inhibitor activity was determined using recombinant full length human MNK2a with an N-terminal GST-tag (glutathione-S-transferase; GST-MNK2a). The protein construct was expressed in Sf9 cells and purified using a Glutathione Sepharose 4 FF column followed by a gel filtration column.

The in-vitro kinase assay used for MNK2a activity was a homogeneous time resolved fluorescence (HTRF) assay. The assay uses a biotinylated 18 amino acids peptide sequence from the transcription factor CREB (biotin-AGAGKRREILSRRPSYRK purchased from NeoMPS, designated SEQ ID NO:5). The amount of phosporylated CREB-peptide was quantified by HTRF employing an europium (Eu³⁺) cryptate-conjugated phosphospecific antibody (CREB Ser 133 from Cisbio International) as donor and streptavidin labelled with XL665 (cross-linked allophycocyanin; StrepXLent from Cisbio International) as acceptor. The kinase reaction mixture consisted of 0.25 nM GST-MNK2a, 160 nM CREB peptide, 0.5 µM ATP and 0.05% Bovine Serum Albumine (BSA) in a buffer containing 50 mM HEPES pH 7.6, 0.25 mM MnCl₂, 1 mM dithiothreitol (DTT) and 0.001 % Tween 20. The kinase reaction mixture was incubated for 10 minutes at room temperature. The kinase reaction was terminated by addition of the Eu³⁺ cryptate-conjugated phosphospecific antibody (CREB Ser 133) containing 0.6 M potassium fluoride. The final concentration of 0.3 M potassium fluoride stops the reaction.

The detection step was performed by adding the streptavidin labelled XL665. The final concentrations of donor and acceptor were 0.2 nM and 42 nM, respectively. The buffer used for the detection reagents was 50 mM Hepes pH 7.0, 0.6 M potassium fluoride and 0.1 % BSA. The detection mixture was incubated for 1 hour at room temperature before analysis with a plate reader (Wallac Victor² V) for HTRF readout. The excitation wave length used was 340 nm, while the emission for the Eu³⁺cryptate and the acceptor XL665 was detected at 615 nm and 665 nm, respectively. The HTRF read-out is the ratio of the emission at 665 nm to the emission at 615 nm, since this ratio is independent of the optical characteristics of the media at the excitation wavelength.

For IC₅₀ determinations, test compounds were dissolved at 10 mM in 100% DMSO. The compounds were added in the kinase reaction mixture by 1:100 dilutions and typically assayed over an eleven point dilution range with each point in triplicate.

### Cell assay of MNK2a

### Analysis of the effect of MNK-inhibitors on phosphorylation of the downstream substrate eIF4E

According to the literature eIF4E is a unique substrate for MNK1 and MNK2. The purpose of the analysis was to over-express MNK2a together with eIF4E and subsequently incubate with substances of the invention. This would inhibit the MNK2a activity and thereby the phosphorylation of eIF4E.

### Experimental outline:

| | |
|---|---|
| Day 1 | HEK293 cells were plated in a T162cm2-flask. |
| Day 2 | Transient transfection with p-elF4E and p-MNK2a. |
| | |
| | Transient *batch transfection:* |
| | Mix FuGene6 (Roche cat.no. 11668-027) with Opti-MEM (Invitrogen cat.no. 31985-047); |
| | Incubated at RT, 5 min; |
| | DNA added; |
| | Mixed and incubated at RT, 30 min; |
| | The mixture was added to the cells (without removing the culture medium). Incubated at 37°C until the next day. |
| Day 3 | *Morning:* The cells were trypsinated and re-suspend in culture medium. Cells were plated in 2x12-well plates (Collagen-1 coated) |
| | *After lunch:* It was noted that the cells had attached. The medium was removed and add 2 ml/well of starvation medium (i.e. culture medium but with 0.5 % serum) was added. |
| Day 4 | After 20 hrs of starvation: The starvation medium was removed and 1 ml Opti-MEM with substance was added. The concentrations tested were 30, 10, 3, 1, 0.3, 0.1, 0.03, 0.001 and 0.003 µM. After incubation for 60 min, the cells were harvested. |
| | *Harvest of the cells:* |
| | The medium was carefully removed; |
| | Cells were washed once with ice-cold 1xPBS; |
| | 200 µl lysis buffer per well was added; the plates were frozen immediately in -70°C until further analysis. |
| | *Protein determination:* |
| | The cell lysates were thawed by putting the plates on ice, transferred to Eppendorf tubes and centrifuged at 1300 g, +4°C, 10 min. |
| | 12.5 µl of the supernatant was transferred directly to the plate for protein determination (->dil 1:2) and the rest of the supernatant to new pre-cold tubes and store in -70°C until assayed. |
| Day 5-6 | Western Blot analysis of cell lysates, according to standard methods, using a specific phospho-eIF4E antibody. |
| | To quantify the effect of the substance on the phosphorylation of eIF4E, developed x-ray film from the Western blot analysis was scanned using a densitometer and Image Gauge 4.0 software. The detected band at 28 kDa with 0 µM substance was set to 100%. The density of the band at 28 kDa with the different concentrations of the substance was then analysed. The substance concentrations were plotted against quantified value and the IC₅₀ was determined. Example 77 and Example 6 gave an IC₅₀ of 0.9 and 1.9 µM, respectively. |

### In vivo MNK2a kinase assay

The biological effect *in-vivo* can e.g. be assayed as follows.

### Effects of MNK-inhibitors on glucose homeostasis

### Animals

Male diet induced obese (DIO) mice (C57B1/6JB; Scanbur, Sweden) 3-8 months old are used in the studies. The animals are housed singly in cages at 23±1°C, 40-60 % humidity, 12/12-h light/dark cycle and have free access to water and high fat (60%) laboratory chow. The animals are conditioned for at least one week before start of study.

Animals are randomized into five groups (negative control, three different concentrations of compound and positive control). Body weight and food intake are measured the first and final day, respectively. Mice are dosed twice daily (orally, subcutaneously or intraperitonealy) five to fourteen days.

Day five or seven an oral glucose tolerance test (oGTT) or insulin tolerance test (ITT) is performed.

### Oral glucose tolerance test

Animals are fasted for 12 h (19-07). Animals are dosed as usually in the morning and about 30 min after the dosing the oGTT is performed. Blood for glucose and insulin measurements are taken by tail cut at time points 0, 30, 60 and 120 min after the glucose load (2 g/kg).

### Insulin tolerance test

Animals are fasted for 4 h (8-12). Blood for glucose measurements are taken by tail cut at time points 0, 30, 60 and 120 min after the intraperitoneal insulin injection.

### Endpoint

Animals are fasted for 4 h (08-12) the final day. Animals are anaesthetized with isoflurane and final blood samples for clinical chemistry are taken by orbital bleeding or from vena cava. Thereafter animals are euthanized by cervical dislocation and organs (fat, liver and muscle) are dissected, frozen in liquid nitrogen and stored in -70°C before analysis of i.e. glycogen, triglycerides, cholesterol. Blood is kept on ice before centrifuged and stored in -70°C before analysis is performed. Clinical chemistry might include glucose, insulin, triglycerides, free fatty acids, cholesterol, adiponectin, leptin, betahydroxy butyrate, leptin and cytokines.

### Statistical evaluation

The results are expressed as mean ± SD and ± SEM. Data are analysed by one-way ANOVA followed by Dunett's test or Kruskal-Walli's one-way ANOVA followed by Dunn's test. Significance is accepted at a p value less than 0.05.

### In vitro MNK1 kinase assay (HTRF)

The MNK1 activity for 13 compounds of the examples was tested as follows.

MNK1 inhibitor activity was determined using recombinant full length human MNK1 with an N-terminal GST-tag (glutathione-S-transferase; GST-MNK2A). The protein construct was expressed in Sf9 cells and purified using a Glutathione Sepharose 4 FF column. The *in-vitro* kinase assay used for MNK1 activity was a homogeneous time resolved fluorescence (HTRF) assay. The assay uses a biotinylated 18 amino acids peptide sequence from the transcription factor CREB (biotin-AGAGKRREILSRRPSYRK purchased from NeoMPS, designated SEQ ID NO:5). The amount of phosporylated CREB-peptide was quantified by HTRF employing an europium (Eu³⁺) cryptate-conjugated phosphospecific antibody (CREB Ser 133 from Cisbio International) as donor and streptavidin labelled with XL665 (cross-linked allophycocyanin; StrepXLent from Cisbio International) as acceptor. The kinase reaction mixture consisted of 0.63 nM GST-MNK1, 100 nM CREB peptide, 7.2 µM ATP and 0.05% Bovine Serum Albumine (BSA) in a buffer containing 50 mM HEPES pH 7.6, 0.25 mM MnCl₂, 1 mM dithiothreitol (DTT) and 0.001 % Tween 20. The kinase reaction mixture was incubated for 30 minutes at room temperature. The kinase reaction was terminated by addition of the Eu³⁺ cryptate-conjugated phosphospecific antibody (CREB Ser 133) containing 0.6 M potassium fluoride. The final concentration 0.3 M potassium fluoride stops the reaction. The detection step was performed by adding the streptavidin labelled XL665. The final concentrations of donor and acceptor were 0.2 nM and 42 nM, respectively. The buffer used for the detection reagents was 50 mM Hepes pH 7.0, 0.6 M potassium fluoride and 0.1% BSA. The detection mixture was incubated for 1 hour at room temperature before analysis with a plate reader (Wallac Victor² V) for HTRF readout. The excitation wave length used was 340 nm, while the emission for the Eu³⁺cryptate and the acceptor XL665 was detected at 615 nm and 665 nm, respectively. The HTRF read-out is the ratio of the emission at 665 nm and the emission at 615 nm, since this ratio is independent of the optical characteristics of the media at the excitation wavelength. For IC₅₀ determinations, test compounds were dissolved at 10 mM in 100% DMSO. The compounds were added in the kinase reaction mixture by 1:100 dilutions and typically assayed over an eleven point dilution range with each point in triplicate.

The results indicated that the MNK1 to MNK2a activity ratios for the compounds ranged from 11 to 0.1, *i*.*e*. about 10:1 to 1:10. The compounds of the present invention are thus believed to generally exhibit an MNK1 activity which is 1:20 to 20:1 of that for MNK2a.

### In vitro MNK2a and MNK2b kinase assay (radiometrically)

The dose response of the compounds for MNK2a and MNK2b can also be assayed radiometrically, such as described in the following.

### MNK assay and procedure for dose response assay

Substrate: **ACC2** peptide from Neosystem, part number SPO21928 Biotinyl-Arg-Val-Pro-Thr-Met-Arg-Pro-Ser-Met-Ser-Gly-Leu-His-NH₂, designated SEQ ID NO:6. Dissolve in sterile water to 4mM. Aliquot and store at-20°C.

Substrate: **³³P-ATP** from Perkin Elmer, product number NEG3302H001MC 1 mCi (=37 MBq)/100 µl (half life 25.4 days)

FlashPlate® PLUS Streptavidin 96-well coated microplate from Perkin ElmerSMP103A001PK.

### Assay

### 1. Assay Buffer:

60 mM HEPES-KOH, pH 7.4-7.5, 150 mM KC1, 15 mMMgCl₂, 15% Glycerol, 0.03% NP-40

### Before use add:

DTT to final conc. 3 mM (300 µl, 1 M DTT / 100 ml assay buffer). Add Complete mini (Roche's protease inhibitor cocktail tablet) - 1.5 tablets per 100 ml buffer (or 1 tablet in 10 ml buffer and then add 15 ml to 85 ml assay buffer).

### 2. Compounds for dose response assay:

60 µl compound/well 10 mM (usually) are serial diluted 1/3 in 100% DMSO. After transfer of 10 µl to a new plate, 190 µl buffer is added. Final assay concentration: 200 µM (for 10 mM compounds) and 2% DMSO.

### 3. Reaction solution:

**MNK2a assay:** Enzyme stock, MNK2a BB337, 50 µg/ml (0.6 µM) → dil 1/20
→ 12 nM in incubation.
   Enzyme stock, MNK2a BB304, 50 µg/ml (0.6 µM) → dil 1/20
→ 12 nM in incubation.

**MNK2b assay:** Enzyme stock, MNK2b, BB 170, 8 µM, dil 1/30 in assay buffer
→ 107 nM in incubation.

### 4. Substrate solution:

0.20 µCi ³³P-ATP/µl, 12 µM ATP and 20 µM ACC2 peptide
γ³³P-ATP (3000 Ci/mmol) 0.20 µCi/µl in Substrate solution
ATP ( 1 mM ATP dil to 12 µM → 2.4 µM in assay)
ACC2 (4 mM → dil to 20 µM → finally 4 µM in assay)

### 5. Incubation for reaction:

10 µl compound solution (transferred to a V-bottom Costar 96-plate with Quadra), is mixed with 10 µl reaction mixture. After centrifugation, add 5 µl substrate solution. Shake gentle for 2 hours. Add 200 µl H₂O (Multidrop) to terminate the reaction and continue shaking for a few minutes.

### 6. Incubation in FlashPlate:

Add 130 µl water to each well of the FlashPlate plate. Transfer 70 µl of the diluted reaction mix and incubate at room temperature with gentle shaking for 45 minutes.

### 7. Wash and count:

Remove the solution (pour to sink) and wash 3 times with 250 µl 1xPBS containing 0.02% Tween 20. Remove all washing buffer and seal the plate wells with film before reading in TopCount.

### Procedure for dose response assay

- From Compound Collection:
60 µl compound in 100% DMSO in 500 µl 96-well plate from Nunc. 10 mM (finally 200 µM in assay), 5 mM (100 µM in assay) or 2 mM (40 µM in assay) and 2% DMSO in final assay.

- Serial dilute 1/3 in 100% DMSO in the Nunc compound plate (Tecan or by hand).
- Transfer 10 µl (Quadra) to a Costar U-bottom Polypropylene plate (VWR 136410-3365).
- Add controls to row 12 (10µl according to assay protocol):

| | | |
|---|---|---|
| A - B, G - H | TOTB | 10 µl, 100% DMSO |
| C-D | QC₅₀ | 10 µl, 10 µM Staurosporine in DMSO |
| E-F | NSB (QC₁₀₀) | 10 µl, 1 mM Staurosporine in DMSO. |

- Add 190 µl assay buffer (Multidrop or by hand) - Mix.
- Transfer 10 µl (Quadra) to two Costar V-bottom Polypropylene plate (VWR 136410-3363).
- Add 10µl enzyme (MNK) manually.
- Centrifugate (700 rpm, 2 min, to get rid of air bubbles).
- Add 5µl Mastermix (ATP, ³³P-ATP, ACC2) cover with film.
- Centrifugate (700 rpm, 2 min, to get rid of air bubbles).
- Incubate at RT for 2h with agitation (= 400 rpm).
- Add 200µl H₂O (Multidrop) to the assay mixture (= STOP reaction) - Mix.
- Add 130µl H₂O (Multidrop) to the empty FlashPlate plates.
- Transfer 70µl of the "stopped" reaction mix to each FlashPlate plate.
- Incubate at RT for 45min with agitation (= 400 rpm).
- Empty plate (into sink).
- Wash 3 times with 230µl PBS, 0.02% Tween-20 (Multidrop or by hand).
- Finally - empty plate and cover with film (FlashPlate Cover).
- Read in Topcount (program 19).

## Claims

1. A compound of the formula I: 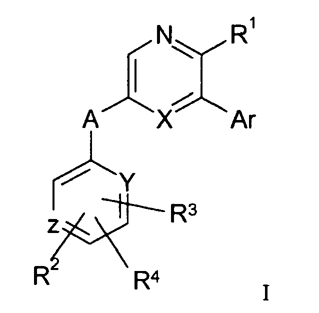 or a pharmaceutically acceptable salt, hydrates, geometrical isomers, racemates, tautomers, optical isomers, N-oxides and prodrug forms thereof, wherein:
X is N or CH;
Y and Z are each selected from N or CH;
A is a bond, -NH- or -N(C₁₋₆ alkyl)-;
R¹ is H or NH₂;
Ar is benzofuranyl, indolyl, hydroxyphenyl, thienyl, benzothiophenyl, pyrrolyl, aminocarbonylphenyl, or azaindolyl, each of which is unsubstituted or substituted with from 1-2 substituents independently selected from hydroxy, halogen, -CN, -NO₂, C₁-₆ alkyl, C₁₋₆ alkoxy, acyl, C₁₋₆ alkylsulphonyl, -C(O)NH₂, -NH-C(O)-R⁷, and arylsulphonyl;
R² and R³, are each, independently, located at a position ortho or meta with respect to A; and are each, independently, H, halogen, hydroxy, C₁-₆ alkyl, hydroxy-C₁₋₆ alkyl, carboxy-C₁₋₆ alkyl, C₁₋₆ alkoxy, C₁₋₆ alkoxy-C₁₋₆ alkoxy, di-C₁₋₆ alkylamino-C₁₋₆ alkoxy, or di-C₁₋₆ alkylaminocarbonyl-C₁₋₆ alkoxy;
R⁴ is located at a position para or meta with respect to A and is -C(O)NR⁵R⁶ or -S(O)₂NR⁵R⁶;
R⁵ is H, C₁₋₆ alkyl, or aryl-C₁₋₆ alkyl;
R⁶ is H, C₁₋₆ alkyl, halo-C₁₋₆ alkyl, di-C₁₋₆ alkylamino-C₁₋₆ alkyl, mono-C₁₋₆ alkylamino-C₁₋₆ alkyl, amino-C₁₋₆ alkyl, hydroxy-C₁₋₆ alkylanlino-C₁₋₆ alkyl, di(hydroxy-C₁₋₆ alkyl)amino-C₁₋₆ alkyl, hydroxy-C₁₋₆ alkyl, dihydroxy-C₃₋₆ alkyl, cycloalkyl, heterocyclyl, heterocyclyl-C₁₋₆ alkyl, or heteroaryl-C₁₋₆ alkyl, wherein the heterocyclyl or heteroaryl is unsubstituted or substituted with from 1-2 substituents independently selected from C₁₋₆ alkyl, C₁₋₆ alkyl-OC(O)-or aryl-C₁₋₆ alkyl, said C₁₋₆ alkyl-OC(O)- being attached to a ring N atom; or
R⁵ and R⁶ together with the nitrogen to which they are attached form a 4 to 7 membered heterocyclyl, wherein the 4 to 7 membered heterocyclyl optionally includes a second heteroatom ring member selected from N, S and O, and wherein the 4 to 7 membered heterocyclyl is optionally substituted with from 1-2 substituents independently selected from C₁₋₆ alkyl, hydroxy-C₁₋₆ alkyl, C₁₋₂-alkoxy-C₂₋₄-alkyl, oxo, di-C₁₋₆ alkylamino, mono-C₁₋₆ alkylamino, amino, di-C₁₋₆ alkylamino-C₁₋₆ alkyl, mono-C₁₋₆ alkylamino-C₁₋₆ alkyl, amino-C₁₋₆ alkyl, C₃₋₇ cycloalkyl, C₁₋₆ alkyl-OC(O)NH-, heterocyclyl-C₁₋₄ alkyl, and heteroaryl-carbonyl; or wherein two geminal substituents on said 4- to 7-membered heterocyclyl together form a second 5 to 6 membered heterocyclyl giving a spiro radical, which is optionally substituted;
R⁷ is H or C₁₋₆ alkyl;
with the provisos that:
when R¹ is NH₂, then A is a bond; and
when A is -NH- or -N-C₁₋₆-alkyl then R¹ is H.

2. A compound according to claim 1, of the formula II 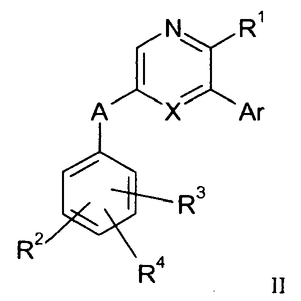 or pharmaceutically acceptable salts, hydrates, geometrical isomers, racemates, tautomers, optical isomers, N-oxides and prodrug forms thereof, wherein:
X is N or CH;
A is a bond or -NH-;
R¹ is H or NH₂;
Ar is benzofuranyl, indolyl, hydroxyphenyl, thienyl, benzothiophenyl, or pyrrolyl, each of which is unsubstituted or substituted with from 1-2 substituents independently selected from hydroxy, halogen, C₁₋₆ alkyl, C₁₋₆ alkoxy, acyl, C₁₋₆ alkylsulphonyl and arylsulphonyl;
R² and R³ are each, independently, located at a position that is ortho or meta with respect to A; and are each, independently, H, halogen, C₁₋₆ alkyl, hydroxy-C₁₋₆ alkyl, carboxy-C₁₋₆ alkyl, or C₁₋₆ alkoxy;
R⁴ is located at a position that is para or meta with respect to A and is - C(O)NR⁵R⁶ or -S(O)₂NR⁵R⁶;
R⁵ is H, C₁₋₆ alkyl, or aryl-C₁₋₆ alkyl;
R⁶ is H, C₁₋₆ alkyl, halo-C₁₋₆ alkyl, di-C₁₋₆ alkylamino-C₁₋₆ alkyl, mono-C₁₋₆ alkylamino-C₁₋₆ alkyl, amino-C₁₋₆ alkyl, hydroxy-C₁₋₆ alkylamino-C₁₋₆ alkyl, di(hydroxy-C₁₋₆ alkyl)amino-C₁₋₆ alkyl, hydroxy-C₁₋₆ alkyl, dihydroxy-C₃₋₆ alkyl, cycloalkyl, heterocyclyl, heterocyclyl-C₁₋₆ alkyl, or heteroaryl-C₁₋₆ alkyl, wherein the heterocyclyl or heteroaryl is unsubstituted or substituted with from 1-2 substituents independently selected from C₁₋₆ alkyl, C₁₋₆ alkyl-OC(O)-or aryl-C₁₋₆ alkyl, said C₁₋₆ alkyl-OC(O)- being attached to a ring N atom; or
R⁵ and R⁶ together with the nitrogen to which they are attached form a 4 to 7 membered heterocyclyl, wherein the 4 to 7 membered heterocyclyl optionally includes a second heteroatom ring member selected from N, S and O, and wherein the 4 to 7 membered heterocyclyl is optionally substituted with from 1-2 substituents independently selected from C₁₋₆ alkyl, hydroxy-C₁₋₆ alkyl, di-C₁₋₆ alkylamino, mono-C₁₋₆ alkylamino, amino, di-C₁₋₆ alkylamino-C₁₋₆ alkyl, mono-C₁₋₆ alkylamino-C₁₋₆ alkyl, amino-C₁₋₆ alkyl, C₃-₇ cycloalkyl, C₁₋₆ alkyl-OC(O)NH- and heteroaryl-carbonyl;
with the provisos that:
when R¹ is NH₂, then A is a bond; and
when A is -NH- then R¹ is H.

3. A compound according to claim 1 having the formula III 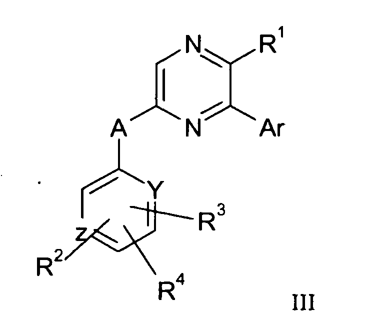 or pharmaceutically acceptable salts, hydrates, geometrical isomers, racemates, tautomers, optical isomers, N-oxides and prodrug forms thereof, wherein:
Y, Z, A, Ar, R¹, R², R³, R⁴, R⁵ and R⁶ are as defined in claim 1
with the provisos that:
when R¹ is NH₂, then A is a bond; and
when A is -NH- or -N-(C₁₋₆-alkyl)- then R¹ is H.

4. A compound according to claim 1 having the formula IV 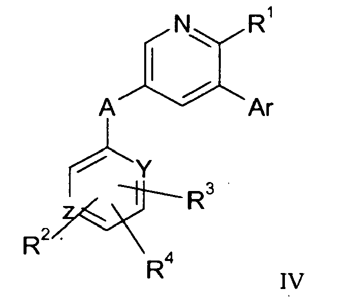 or pharmaceutically acceptable salts, hydrates, geometrical isomers, racemates, tautomers, optical isomers, N-oxides and prodrug forms thereof, wherein:
Y, Z, A, Ar, R¹, R², R³, R⁴, R⁵ and R⁶ are as defined in claim 1
with the provisos that:
when R¹ is NH₂, then A is a bond; and
when A is -NH- or -N-(C₁₋₆alkyl)- then R¹ is H.

5. A compound according to claim 1 wherein Y is N and Z is CH.

6. A compound according to claim 1 wherein Z is N and Y is CH.

7. A compound according to claim 1 wherein both Y and Z is CH.

8. The compound according to claim 1, wherein
R² and R³ are each, independently, located in a position that is ortho or meta with respect to A; and are each, independently, H, halogen, hydroxy, C₁₋₃ alkyl, C₁₋₃ alkoxy, C₁₋₃ alkoxy-C₁₋₃ alkoxy, di-C₁₋₆ alkylamino-C₁₋₆ alkoxy, or di-C₁₋₆ alkylaminocarbonyl-C₁₋₆ alkoxy;
R⁵ is H, C₁₋₃ alkyl, or aryl-C₁₋₃ alkyl;
R⁶ is H, C₁₋₃ alkyl, halo-C₁₋₃ alkyl, di-C₁₋₄ alkylamino-C₁₋₆ alkyl, mono-C₁₋₄ alkylamino-C₁₋₆ alkyl, amino-C₁₋₆ alkyl, hydroxy-C₁₋₃ alkylamino-C₁₋₅ alkyl, di(hydroxy-C₁₋₃ alkyl)amino-C₁₋₅ alkyl, hydroxy-C₁₋₅ alkyl, dihydroxy propyl, cycloalkyl, heterocyclyl, heterocyclyl-C₁₋₄ alkyl, or heteroaryl-C₁₋₃ alkyl, wherein the heterocyclyl or heteroaryl is unsubstituted or substituted with from 1-2 substituents independently selected from C₁₋₃ alkyl, C₁₋₅ alkyl-OC(O)-, or aryl-C₁₋₃ alkyl, said C₁₋₅ alkyl-OC(O)- being attached to a ring N atom,
or
R⁵ and R⁶ together with the nitrogen to which they are attached form a 4 to 7 membered heterocyclyl, wherein the 4 to 7 membered heterocyclyl optionally includes a second heteroatom ring member selected from N and O; and wherein the 4 to 7 membered heterocyclyl is unsubstituted or substituted with from 1-2 substituents independently selected from C₁₋₃ alkyl, hydroxy-C₁₋₃ alkyl, C₁₋₂-alkoxy-C₂₋₄-alkyl, di-C₁₋₃ alkylamino, mono-C₁₋₃ alkylamino, amino, di-C₁₋₃ alkylamino-C₁₋₄ alkyl, mono-C₁₋₃ alkylanllno-C₁₋₄ alkyl, amino-C₁₋₄ alkyl, C₅₋₆ cycloalkyl, C₁₋₆ alkyl-OC(O)NH-, heterocyclylmethyl, or heteroaryl-carbonyl, or wherein two geminal substituents on said 4- to 7-membered heterocyclyl together form a second 5 to 6 membered heterocyclyl giving a spiro radical;
with the provisos that:
when R¹ is NH₂, then A is a bond;
when A is -NH- or -N-C₁₋₆-alkyl then R¹ is H; and
when both R² and R³ are other than H, then both R² and R³ cannot be located at a position that is ortho with respect to A.

9. The compound of claim 8 wherein R⁶ is di-C₁₋₃ alkylamino-C₁₋₄ alkyl, mono-C₁₋₃ akylamino-C₁₋₄ alkyl, amino-C₁₋₄ alkyl, heterocyclyl, or heterocyclyl-C₁₋₃ alkyl, wherein the heterocyclyl is preferably 4, 5 or 6 membered containing 1 or 2 heteroatoms selected from O and N, wherein the heterocyclyl may be unsubstituted or substituted in one or two positions independently with C₁₋₃ alkyl, C₁₋₅ alkyl-OC(O)-, or aryl-C₁₋₃ alkyl, said C₁₋₅ alkyl-OC(O)- being attached to a ring N atom.

10. The compound according to claim 1 wherein R² is located at a position that is ortho with respect to A, R³ is located at a position that is meta with respect to A, and R⁴ is located at a position that is para with respect to A.

11. The compound oaccording to claim 1, wherein Ar is hydroxyphenyl, benzofuranyl, aminocarbonylphenyl or indolyl.

12. The compound of claim 11, wherein A is -NH-, and Ar is 2-benzofuranyl or 2-indolyl.

13. The compound according to claim 1, wherein Ar is azaindolyl.

14. The compound according to claim 13, wherein Ar is 5-, or 6-azaindol-2-yl.

15. The compound of claim 1, selected from the group consisting of:
4-{[6-(1H-indol-2-yl)pyrazin-2-yl]amino}-3-methoxy-N-methyl-N-(1-methylpyrrolidin-3-yl)benzamide;
4-[5-amino-6-(1-benzofuran-2-yl)pyrazin-2-yl]-N-[(2S)-2,3-dihydroxypropyl]benzamide;
5-{2-fluoro-4-[(4-methylpiperazin-1-yl)carbonyl]phenyl}-3-(1H-indol-2-yl)pyrazin-2-amine;
N-[2-(diethylamino)ethyl]-4-[5-(1H-indol-2-yl)pyridin-3-yl]benzamide;
3-(1-benzofuran-2-yl)-5-{2-fluoro-4-[(4-methylpiperazin-1-yl)carbonyl]phenyl}pyrazin-2-amine;
2-(5-{4-[(4-methylpiperazin-1-yl)carbonyl]phenyl}pyridin-3-yl)-1H-indole;
5-fluoro-2-{6-[4-(morpholin-4-ylcarbonyl)phenyl]pyrazin-2-yl}-1H-indole;
2-{5-[4-(morpholin-4-ylcarbonyl)phenyl]pyridin-3-yl}-1H-indole;
N-ethyl-N-(2-hydroxyethyl)-4-[6-(1H-indol-2-yl)pyrazin-2-yl]benzamide;
4-{[6-(1H-indol-2-yl)pyrazin-2-yl]amino}-3-methoxy-N-methyl-N-(1-methylpyrrolidin-3-yl)benzamide;
N-[2-(diethylamino)ethyl]-4-{[6-(5-fluoro-1H-indol-2-yl)pyrazin-2-yl]amino}-3-methoxybenzamide;
3-fluoro-4-[6-(6-fluoro-1H-indol-2-yl)pyrazin-2-yl]-N-methyl-N-(1-methylpyrrolidin-3-yl)benzamide;
4-{[6-(5-fluoro-1H-indol-2-yl)pyrazin-2-yl]amino}-3-methoxy-N-methyl-N-(1-methylpyrrolidin-3-yl)benzamide;
6-methyl-2-{6-[4-(morpholin-4-ylcarbonyl)phenyl]pyrazin-2-yl}-1H-indole;
2-{6-[4-(morpholin-4-ylcarbonyl)phenyl]pyrazin-2-yl}-1H-indole;
4-[5-amino-6-(1-benzofuran-2-yl)pyrazin-2-yl]-N-(2,3-dihydroxypropyl)benzamide;
6-(5-fluoro-1H-indol-2-yl)-N-{2-methoxy-4-[(4-methylpiperazin-1-yl)carbonyl]phenyl}pyrazin-2-amine;
3-(1H-indol-2-yl)-5-[4-(morpholin-4-ylcarbonyl)phenyl]pyrazin-2-amine;
N-[3-(dimethylamino)propyl]-4-{[6-(1H-indol-2-yl)pyrazin-2-yl]amino}-3-methoxybenzamide;
2-(6-{2-fluoro-4-[(4-methylpiperazin-1-yl)carbonyl]phenyl}pyrazin-2-yl)-1 H-indole;
tert-butyl 3-[({4-[6-(1H-indol-2-yl)pyrazin-2-yl]benzoyl}amino)methyl]piperidine-1-carboxylate;
2-{6-[2-fluoro-4-(morpholin-4-ylcarbonyl)phenyl]pyrazin-2-yl}-1H-indole;
3-{[6-(1-benzofuran-2-yl)pyrazin-2-yl]amino}-4-methoxy-N-methyl-N-(1-methylpyrrolidin-3-yl)benzamide;
5-[2-fluoro-4-(morpholin-4-ylcarbonyl)phenyl]-3-(1H-indol-2-yl)pyrazin-2-amine;
N-[2-(diethylamino)ethyl]-4-{[6-(1H-indol-2-yl)pyrazin-2-yl]amino}-3-methoxybenzamide;
tert-butyl (1-{4-[6-(1H-indol-2-yl)pyrazin-2-yl]benzoyl}pyrrolidin-3-yl)carbamate;
6-(1H-indol-2-yl)-N-[2-methoxy-4-(moipholin-4-ylcarbonyl)phenyl]pyrazin-2-amine;
5-fluoro-2-(6-{4-[(4-methylpiperazin-1-yl)carbonyl]phenyl}pyrazin-2-yl)-1H-indole;
4-[5-amino-6-(1-benzofuran-2-yl)pyrazin-2-yl]-N-[1-(hydroxymethyl)-2-methylpropyl]benzamide;
6-(1H-indol-2-yl)-N-[2-methoxy-4-(morpholin-4-ylcarbonyl)phenyl]pyrazin-2-amine;
tert-butyl (1-{4-[5-amino-6-(1-benzofuran-2-yl)pyrazin-2-yl]benzoyl}pyrrolidin-3-yl)carbamate;
3-(1-benzofuran-2-yl)-5-[4-(morpholin-4-ylcarbonyl)phenyl]pyrazin-2-amine;
3-(4-{4-[6-(1H-indol-2-yl)pyrazin-2-yl]benzoyl}piperazin-1-yl)-N,N-dimethylpropan-1-amine;
4-{[6-(1-benzofuran-2-yl)pyrazin-2-yl]amino}-3-methoxy-N-methyl-N-(1-methylpyrrolidin-3-yl)benzamide;
N-[(2S)-2,3-dihydroxypropyl]-4-[6-(1H-indol-2-yl)pyrazin-2-yl]benzamide;
N-[3-(dimethylamino)propyl]-4-[6-(1H-indol-2-yl)pyrazin-2-yl]-N-methylbenzamide;
N-(4-{[3-(dimethylamino)pyrrolidin-1-yl]carbonyl}-2-methoxyphenyl)-6-(1H-indol-2-yl)pyrazin-2-amine;
3-(1-benzofuran-2-yl)-5-{4-[(4-methylpiperazin-1-yl)carbonyl]phenyl}pyrazin-2-amine;
3-fluoro-5-[6-(6-fluoro-1H-indol-2-yl)pyrazin-2-yl]-N-methyl-N-(1-methylpyrrolidin-3-yl)benzamide;
6-(1H-indol-2-yl)-N-{2-methoxy-4-[(4-methylpiperazin-1-yl)carbonyl]phenyl}pyrazin-2-amine;
4-[5-amino-6-(1-benzofuran-2-yl)pyrazin-2-yl]-N-{3-[bis(2-hydroxyethyl)amino]propyl}benzamide;
N-(2,3-dihydroxypropyl)-4-[6-(1H-indol-2-yl)pyrazin-2-yl]benzamide;
2-(4-{3-fluoro-5-[6-(6-fluoro-1H-indol-2-yl)pyrazin-2-yl]benzoyl}piperazin-1-yl)-N,N-dimethylethanamine;
4-[5-amino-6-(1-benzofuran-2-yl)pyrazin-2-yl]-N-ethyl-N-(2-hydroxyethyl)benzamide;
3-chloro-N-[2-(diethylamino)ethyl]-4-{[6-(5-fluoro-1H-indol-2-yl)pyrazin-2-yl]amino}benzamide,
N-[2-chloro-4-(piperazin-1-ylcarbonyl)phenyl]-6-(5-fluoro-1H-indol-2-yl)pyrazin-2-amine,
N-[2-chloro-5-methoxy-4-(piperazin-1-ylcarbonyl)phenyl]-6-(5-fluoro-1H-indol-2-yl)pyrazin-2-amine,
4-{[6-(5-chloro-1H-indol-2-yl)pyrazin-2-yl]amino}-3-methoxy-N-methyl-N-(1-methylpyrrolidin-3-yl)benzamide,
N-[2-chloro-4-({4-[3-(dimethylamino)propyl]piperazin-1-yl}carbonyl)phenyl]-6-(1H-indol-2-yl)pyrazin-2-amine,
N-[2-chloro-4-(piperazin-1-ylcarbonyl)phenyl]-6-(1H-indol-2-yl)pyrazin-2-amine,
N-[4-({4-[3-(dimethylamino)propyl]piperazin-1-yl}carbonyl)-2-methylphenyl]-6-(5-fluoro-1H-indol-2-yl)pyrazin-2-amine,
N-[3-chloro-4-(piperazin-1-ylcarbonyl)phenyl]-6-(1H-indol-2-yl)pyrazin-2-amine,
4-{[6-(6-fluoro-1H-indol-2-yl)pyrazin-2-yl]amino}-3-methoxy-N-methyl-N-(1-methylpyrrolidin-3-yl)benzamide,
3-methoxy-4-{[6-(5-methoxy-1H-indol-2-yl)pyrazin-2-yl]amino}-N-methyl-N-(1-methylpyrrolidin-3-yl)benzamide,
4-{[6-(7-fluoro-1H-indol-2-yl)pyrazin-2-yl]amino}-3-methoxy-N-methyl-N-(1-methylpyrrolidin-3-yl)benzamide,
4-{[6-(4-fluoro-1H-indol-2-yl)pyrazin-2-yl]amino}-3-methoxy-N-methyl-N-(1-methylpyrrolidin-3-yl)benzamide,
2-[6-({4-[(4-methylpiperazin-1-yl)carbonyl]phenyl}amino)pyrazin-2-yl]-1H-indol-4-ol,
4-[[6-(5-fluoro-1H-indol-2-yl)pyrazin-2-yl](methyl)amino]-N-methyl-N-(1-methylpyrrolidin-3-yl)benzamide,
3-methoxy-4-{[6-(4-methoxy-1H-indol-2-yl)pyrazin-2-yl]amino}-N-methyl-N-(1-methylpyrrolidin-3-yl)benzamide,
4-{[6-(6-chloro-1H-indol-2-yl)pyrazin-2-yl]amino}-3-methoxy-N-methyl-N-(1-methylpyrrolidin-3-yl)benzamide,
4-{[6-(5-cyano-1H-indol-2-yl)pyrazin-2-yl]amino}-3-methoxy-N-methyl-N-(1-methylpyrrolidin-3-yl)benzamide,
3-methoxy-N-methyl-4-{[6-(5-methyl-1H-indol-2-yl)pyrazin-2-yl]amino}-N-(1-methylpyrrolidin-3-yl)benzamide,
3-methoxy-N-methyl-4-{[6-(6-methyl-1H-indol-2-yl)pyrazin-2-yl]amino}-N-(1-methylpyrrolidin-3-yl)benzamide,
N-[2-(diethylamino)ethyl]-2-fluoro-4-{[6-(1H-indol-2-yl)pyrazin-2-yl]amino}benzamide,
N-(4-{[(3R)-3-(dimethylamino)pyrrolidin-1-yl]carbonyl}phenyl)-6-(1H-indol-2-yl)pyrazin-2-amine,
N-(4-{[(3S)-3-(dimethylamino)pyrrolidin-1-yl]carbonyl}phenyl)-6-(1H-indol-2-yl)pyrazin-2-amine,
N-[4-({4-[3-(dimethylamino)propyl]piperazin-1-yl}carbonyl)-3-methoxyphenyl]-6-(1H-indol-2-yl)pyrazin-2-amine,
6-(6-methoxy-1H-indol-2-yl)-N-[2-methoxy-4-(piperazin-1-ylcarbonyl)phenyl]pyrazin-2-amine,
6-(4-fluoro-1H-indol-2-yl)-N-[2-methoxy-4-(piperazin-1-ylcarbonyl)phenyl]pyrazin-2-amine,
N-(4-{[3-(dimethylamino)pyrrolidin-1-yl]carbonyl}-2-methoxyphenyl)-6-(1H-indol-2-yl)pyrazin-2-amine,
tert-butyl [1-(4-{[6-(5-fluoro-1H-indol-2-yl)pyrazin-2-yl]amino}-3-methoxybenzoyl)azetidin-3-yl]carbamate,
N-[2-(dimethylamino)ethyl]-4-{[6-(1H-indol-2-yl)pyrazin-2-yl]amino}-3-methoxy-N-methylbenzamide,
N-azetidin-3-yl-4-{[6-(5-fluoro-1H-indol-2-yl)pyrazin-2-yl]amino}-3-methoxybenzamide,
N-{4-[(3-aminoazetidin-1-yl)carbonyl]-2-methoxyphenyl}-6-(5-fluoro-1H-indol-2-yl)pyrazin-2-amine,
tert-butyl 3-[(4-{[6-(5-fluoro-1H-indol-2-yl)pyrazin-2-yl]amino}-3-methoxybenzoyl)amino]azetidine-1-carboxylate,
N-(1,2-diethylpyrazolidin-4-yl)-4-{[6-(1H-indol-2-yl)pyrazin-2-yl]amino}-3-methoxybenzamide,
6-(5-fluoro-1H-indol-2-yl)-N-(2-methoxy-4-{[(3S)-3-methylpiperazin-1-yl]carbonyl}phenyl)pyrazin-2-amine,
6-(5-fluoro-1H-indol-2-yl)-N-(2-methoxy-4-{[(3R)-3-methylpiperazin-1-yl]carbonyl}phenyl)pyrazin-2-amine,
N-(4-{[(3R,5S)-3,5-dimethylpiperazin-1-yl]carbonyl}-2-methoxyphenyl)-6-(1H-indol-2-yl)pyrazin-2-amine,
N-{4-[(4-ethylpiperazin-1-yl)carbonyl]-2-methoxyphenyl}-6-(1H-indol-2-yl)pyrazin-2-amine,
N-(4-{[(3S)-3-(dimethylamino)pyrrolidin-1-yl]carbonyl}-2-methoxyphenyl)-6-(5-nitro-1H-indol-2-yl)pyrazin-2-amine,
6-(1H-indol-2-yl)-N-{4-[(4-isopropylpiperazin-1-yl)carbonyl]-2-methoxyphenyl}pyrazin-2-amine,
N-(4-{[(3R)-3-(dimethylamino)pyrrolidin-1-yl]carbonyl}-2-methoxyphenyl)-6-(6-methoxy-1H-indol-2-yl)pyrazin-2-amine,
6-(1H-indol-2-yl)-N-{2-methoxy-4-[(4-methyl-1,4-diazepan-1-yl)carbonyl]phenyl}pyrazin-2-amine,
N-[4-(2,7-diazaspiro[4.5]dec-2-ylcarbonyl)-2-methoxyphenyl]-6-(1H-indol-2-yl)pyrazin-2-amine,
6-(1H-indol-2-yl)-N-(2-methoxy-4-{[(2S)-2-(pyrrolidin-1-ylmethyl)pyrrolidin-1-yl]carbonyl}phenyl)pyrazin-2-amine,
N-(2-hydroxyethyl)-4-{[6-(1H-indol-2-yl)pyrazin-2-yl]amino}-3-methoxybenzamide,
6-(1H-indol-2-yl)-N-(2-methoxy-4-{[4-(2-methoxyethyl)piperazin-1-yl]carbonyl}phenyl)pyrazin-2-amine,
2-[4-(4-{[6-(1H-indol-2-yl)pyrazin-2-yl]amino}-3-methoxybenzoyl)piperazin-1-yl]ethanol,
4-{[6-(1H-indol-2-yl)pyrazin-2-yl]amino}-3-methoxy-N-piperidin-3-ylbenzamide,
N-(4-{[(3R)-3-aminopyrrolidin-1-yl]carbonyl}-2-methoxyphenyl)-6-(1H-indol-2-yl)pyrazin-2-amine,
4-{[6-(1H-indol-2-yl)pyrazin-2-yl]amino}benzamide acetate,
6-(1H-indol-2-yl)-N-[4-(morpholin-4-ylcarbonyl)phenyl]pyrazin-2-amine acetate,
N-(4-{[(3S)-3-aminopyrrolidin-1-yl]carbonyl}-2-methoxyphenyl)-6-(1H-indol-2-yl)pyrazin-2-amine,
4-{[6-(1H-indol-2-yl)pyrazin-2-yl]amino}-3-methoxy-N-[(3S)-pyrrolidin-3-yl]benzamide,
4-{[6-(1H-indol-2-yl)pyrazin-2-yl]amino}-3-methoxy-N-[(3R)-pyrrolidin-3-yl]benzamide,
N-(4-{[(3R)-3-aminopyrrolidin-1-yl]carbonyl}-2-methoxyphenyl)-6-(5-fluoro-1H-indol-2-yl)pyrazin-2-amine,
2-{[6-(5-fluoro-1H-indol-2-yl)pyrazin-2-yl]amino}-5-(piperazin-1-ylcarbonyl)phenol,
2-[6-({4-[(4-methylpiperazin-1-yl)carbonyl]phenyl}amino)pyrazin-2-yl]-1H-indole-5-carboxamide,
N-{2-[2-(dimethylamino)ethoxy]-4-[(4-methylpiperazin-1-yl)carbonyl]phenyl}-6-(5-fluoro-1H-indol-2-yl)pyrazin-2-amine,
2-{2-{[6-(5-fluoro-1H-indol-2-yl)pyrazin-2-yl]amino}-5-[(4-methylpiperazin-1-yl)carbonyl]phenox}-N,N-dimethylacetamide,
N-{2-(2-ethoxyethoxy)-4-[(4-methylpiperazin-1-yl)carbonyl]phenyl}-6-(5-fluoro-1H-indol-2-yl)pyrazin-2-amine,
N-{4-[(4-methylpiperazin-1-yl)carbonyl]phenyl}-6-(1H-pyrrolo[2,3-c]pyridin-2-yl)pyrazin-2-amine,
N-(4-{[(3R)-3-(dimethylamino)pyrrolidin-1-yl]carbonyl}-2-methoxyphenyl)-6-(1H-pyrrolo[3,2-c]pyridin-2-yl)pyrazin-2-amine,
N-{5-[(4-ethylpiperazin-1-yl)carbonyl]pyridin-2-yl}-6-(1H-indol-2-yl)pyrazin-2-amine,
6-(1H-indol-2-yl)-N-{5-[(4-methyl-1,4-diazepan-1-yl)carbonyl]pyridin-2-yl}pyrazin-2-amine,
6-(1H-indol-2-yl)-N-[5-(morpholin-4-ylcarbonyl)pyridin-2-yl]pyrazin-2-amine,
6-(5-fluoro-1H-indol-2-yl)-N-[6-(piperazin-1-ylcarbonyl)pyridin-3-yl]pyrazin-2-amine,
N-{5-[(4-methyl-1,4-diazepan-1-yl)carbonyl]pyridin-2-yl}-6-(1H-pyrrolo[3,2-c]pyridin-2-yl)pyrazin-2-amine,
N-[5-(5-fluoro-1H-indol-2-yl)pyridin-3-yl]-5-(piperazin-1-ylcarbonyl)pyridin-2-amine,
5-(1H-indol-2-yl)-N-{4-[(4-methylpiperazin-1-yl)carbonyl]phenyl}pyridin-3-amine,
5-(5-fluoro-1H-indol-2-yl)-N-[4-(piperazin-1-ylcarbonyl)phenyl]pyridin-3-amine,
5-(5-fluoro-1H-indol-2-yl)-N-{4-[(4-methyl-1,4-diazepan-1-yl)carbonyl]phenyl}pyridin-3-amine,
5-(5-fluoro-1H-indol-2-yl)-N-[6-(piperazin-1-ylcarbonyl)pyridin-3-yl]pyridin-3-amine, and
4-[6-({4-[(4-methylpiperazin-1-yl)carbonyl]phenyl}amino)pyrazin-2-yl]benzamide.

16. A compound according to any one of claims claims 1-15 for use in therapy.

17. The compound according to any one of the claims 1-15 for use in the treatment or prophylaxis of obesity, eating disorder, cachexia, diabetes mellitus, hypertension, coronary heart disease, hypercholesterolemia, dyslipidemia, hyperlipidemia, hyperglycemia, osteoarthritis, gallstones, sleep apnea, neurodegenerative disorders, cancer, inflammatory conditions and type 2 diabetes.

18. Use of a compound according to any one of the claims 1-15 for the manufacture of a medicament for use in the treatment or prophylaxis of a disorder related to undesired activity of MNK1 and/or MNK2 kinases, such as obesity, eating disorder, cachexia, diabetes mellitus, hypertension, coronary heart disease, hypercholesterolemia, dyslipidemia, hyperlipidemia, hyperglycemia, osteoarthritis, gallstones, sleep apnea, neurodegenerative disorders, cancer, inflammatory conditions and type 2 diabetes.

19. A pharmaceutical formulation comprising as an active ingredient a compound according to any one of the claims 1-15.

20. The pharmaceutical formulation of claim 19, further comprising a pharmaceutically acceptable diluent or carrier.

## Patentansprüche

1. Verbindung der Formel I: 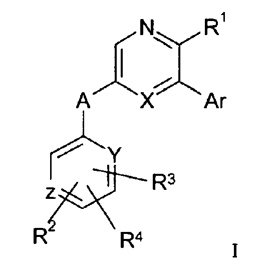 oder ein pharmazeutisch annehmbares Salz, Hydrate, geometrische Isomere, Racemate, Tautomere, optische Isomere, N-Oxide und Prodrugformen davon, wobei:
X N oder CH ist,
Y und Z jeweils aus N oder CH ausgewählt sind;
A eine Bindung, -NH- oder -N(C₁₋₆-Alkyl)- ist;
R¹ H oder NH₂ ist,
Ar Benzofuranyl, Indolyl, Hydroxyphenyl, Thienyl, Benzothiophenyl, Pyrrolyl, Aminocarbonylphenyl oder Azaindolyl ist, die jeweils unsubstituiert oder substituiert mit von 1-2 Substituenten sind, die unabhängig voneinander aus Hydroxy-, Halogen, -CN, -NO₂, C₁₋₆-Alkyl, C₁₋₆-Alkoxy, Acyl, C₁₋₆-Alkylsulfonyl, -C(O)NH2, -NH-C(O)-R⁷ und Arylsulfonyl ausgewählt sind;
R² und R³ jeweils unabhängig voneinander in ortho- oder meta-Position zum Rest A angeordnet sind; und jeweils unabhängig voneinander H, Halogen, Hydroxyl-, C₁₋₆-Alkyl, Hydroxy-C₁₋₆-alkyl, Carboxy-C₁₋₆-alkyl, C₁₋₆-Alkoxy, C₁₋₆-Alkoxy-C₁₋₆-alkoxy, Di-C₁₋₆-alkylamino-C₁₋₆-alkoxy oder Di-C₁₋₆-alkylaminocarbonyl-C₁₋₆-alkoxy sind;
R⁴ in para- oder meta-Position zum Rest A angeordnet und -C(O)NR⁵R⁶ oder - S(O)₂NR⁵R⁶ ist;
R⁵ H, C₁₋₆-Alkyl oder Aryl-C₁₋₆-alkyl ist;
R⁶ H, C₁₋₆-Alkyl, Halogen-C₁₋₆-alkyl, Di-C₁₋₆-alkylamino-C₁₋₆-alkyl, Mono-C₁₋₆-alkylamino-C₁₋₆-alkyl, Amino-C₁₋₆-alkyl, Hydroxy-C₁₋₆-alkylamino-C₁₋₆-alkyl, Di(Hydroxy-C₁₋₆-alkyl)amino-C₁₋₆-alkyl, Hydroxy-C₁₋₆-alkyl, Dihydroxy-C₃₋₆-alkyl, Cycloalkyl, Heterocyclyl, Heterocyclyl-C₁₋₆-alkyl oder Heteroaryl-C₁₋₆-alkyl ist, wobei das Heterocyclyl oder das Heteroaryl unsubstituiert oder substituiert mit von 1-2 Substituenten ist, die unabhängig voneinander aus C₁₋₆-Alkyl, C₁₋₆-Alkyl-OC(O)- oder Aryl-C₁₋₆-alkyl ausgewählt sind, wobei das genannte C₁₋₆-Alkyl-OC(O)-an ein N-Ringatom gebunden ist; oder
R⁵ und R⁶ zusammen mit dem Stickstoff, an den sie gebunden sind, ein 4- bis 7-gliedriges Heterocyclyl bilden, wobei das 4- bis 7-gliedriges Heterocyclyl optional ein zweites Heteroatom als Ringglied ausgewählt aus N, S und O enthält und wobei das 4- bis 7-gliedriges Heterocyclyl optional substituiert mit von 1-2 Substituenten ist, die unabhängig voneinander aus C₁₋₆-Alkyl, Hydroxy-C₁₋₆-alkyl, C₁₋₂-Alkoxy-C₂₋₄-alkyl, Oxo, Di-C₁₋₆-alkylamino, Mono-C₁₋₆-alkylamino, Amino, Di-C₁₋₆-alkylamino-C₁₋₆-alkyl, Mono-C₁₋₆-alkylamino-C₁₋₆-alkyl, Amino-C₁₋₆-alkyl, C₃₋₇-Cycloalkyl, C₁₋₆-Alkyl-OC(O)NH-, Heterocydyl-C₁₋₄-alkyl und Heteroaryl-carbonyl ausgewählt sind; oder wobei zwei geminale Substituenten auf dem genannten 4-bis 7-gliedrigen Heterocyclyl zusammen ein zweites 5- bis 6-gliedrigen Heterocyclyl bilden, wobei ein Spiro-Radikal entsteht, das optional substituiert ist;
R⁷ H oder C₁₋₆-Alkyl ist;
mit den Maßgaben, dass:
wenn R¹ NH₂ ist, dann ist A eine Bindung; und
wenn A -NH- oder -N-C₁₋₆-Alkyl ist, dann ist R¹ H.

2. Verbindung nach Anspruch 1 der Formel II: 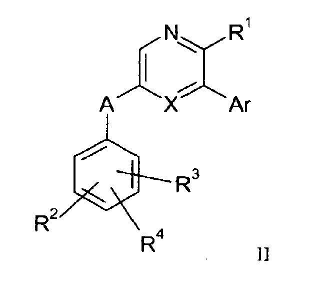 oder ein pharmazeutisch annehmbares Salz, Hydrate, geometrische Isomere, Racemate, Tautomere, optische Isomere, N-Oxide und Prodrugformen davon, wobei:
X N oder CH ist,
A eine Bindung oder -NH- ist;
R¹ H oder NH₂ ist,
Ar Benzofuranyl, Indolyl, Hydroxyphenyl, Thienyl, Benzothiophenyl oder Pyrrolyl ist, die jeweils unsubstituiert oder substituiert mit von 1-2 Substituenten sind, die unabhängig voneinander aus Hydroxy-, Halogen, C₁₋₆-Alkyl, C₁₋₆-Alkoxy, Acyl, C₁₋₆-Alkylsulfonyl und Arylsulfonyl ausgewählt sind;
R² und R³ jeweils unabhängig voneinander in ortho- oder meta-Position zum Rest A angeordnet sind; und jeweils unabhängig voneinander H, Halogen, C₁₋₆-Alkyl, Hydroxy-C₁₋₆-alkyl, Carboxy-C₁₋₆-alkyl oder C₁₋₆-Alkoxy sind;
R⁴ in para- oder meta-Position zum Rest A angeordnet und -C(O)NR⁵R⁶ oder - S(O)₂NR⁵R⁶ ist;
R⁵ H, C₁₋₆-Alkyl oder Aryl-C₁₋₆-alkyl ist;
R⁶ H, C₁₋₆-Alkyl, Halogen-C₁₋₆-alkyl, Di-C₁₋₆-alkylamino-C₁₋₆-alkyl, Mono-C₁₋₆-alkylamino-C₁₋₆-alkyl, Amino-C₁₋₆-alkyl, Hydroxy-C₁₋₆-alkylamino-C₁₋₆-alkyl, Di(Hydroxy-C₁₋₆-alkyl)amino-C₁₋₆-alkyl, Hydroxy-C₁₋₆-alkyl, Dihydroxy-C₃₋₆-alkyl, Cycloalkyl, Heterocyclyl, Heterocyclyl-C₁₋₆-alkyl oder Heteroaryl-C₁₋₆-alkyl ist, wobei das Heterocyclyl oder das Heteroaryl unsubstituiert oder substituiert mit von 1-2 Substituenten ist, die unabhängig voneinander aus C₁₋₆-Alkyl, C₁₋₆-Alkyl-OC(O)- oder Aryl-C₁₋₆-alkyl ausgewählt sind, wobei das genannte C₁₋₆-Alkyl-OC(O)-an ein N-Ringatom gebunden ist; oder
R⁵ und R⁶ zusammen mit dem Stickstoff, an den sie gebunden sind, ein 4- bis 7-gliedriges Heterocyclyl bilden, wobei das 4- bis 7-gliedriges Heterocyclyl optional ein zweites Heteroatom als Ringglied ausgewählt aus N, S und O enthält und wobei das 4- bis 7-gliedriges Heterocyclyl optional substituiert mit von 1-2 Substituenten ist, die unabhängig voneinander aus C₁₋₆-Alkyl, Hydroxy-C₁₋₆-alkyl, Di-C₁₋₆-alkylamino, Mono-C₁₋₆-alkylamino, Amino, Di-C₁₋₆-alkylamino-C₁₋₆-alkyl, Mono-C₁₋₆-alkylamino-C₁₋₆-alkyl, Amino-C₁₋₆-alkyl, C₃₋₇-Cycloalkyl, C₁₋₆-Alkyl-OC(O)NH und Heteroarylcarbonyl ausgewählt sind;
mit den Maßgaben, dass:
wenn R¹ NH₂ ist, dann ist A eine Bindung; und
wenn A -NH- ist, dann ist R¹ H.

3. Verbindung nach Anspruch 1 der Formel III 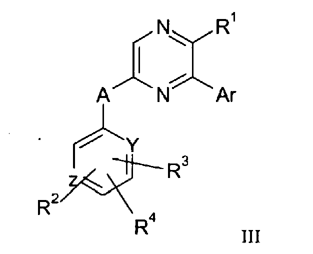 oder ein pharmazeutisch annehmbares Salz, Hydrate, geometrische Isomere, Racemate, Tautomere, optische Isomere, N-Oxide und Prodrugformen davon, wobei:
Y, Z, A, Ar, R¹, R², R³, R⁴, R⁵ und R⁶ wie in Anspruch 1 definiert sind,
mit den Maßgaben, dass:
wenn R¹ NH₂ ist, dann ist A eine Bindung, und
wenn A -NH- oder -N-(C₁₋₆-Alkyl)- ist, dann ist R¹ H.

4. Verbindung nach Anspruch 1 der Formel IV 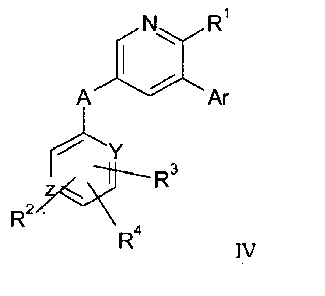 oder ein pharmazeutisch annehmbares Salz, Hydrate, geometrische Isomere, Racemate, Tautomere, optische Isomere, N-Oxide und Prodrugformen davon, wobei:
Y, Z, A, Ar, R¹, R², R³, R⁴, R⁵ und R⁶ wie in Anspruch 1 definiert sind,
mit den Maßgaben, dass:
wenn R¹ NH₂ ist, dann ist A eine Bindung, und
wenn A -NH- oder -N-(C₁₋₆-Alkyl)- ist, dann ist R¹ H.

5. Verbindung nach Anspruch 1, wobei Y N ist und Z CH ist.

6. Verbindung nach Anspruch 1, wobei Z N ist und Y CH ist.

7. Verbindung nach Anspruch 1, wobei beide Y und Z CH sind.

8. Verbindung nach Anspruch 1, wobei
R² und R³ jeweils unabhängig voneinander in ortho- oder meta-Position zum Rest A angeordnet sind; und jeweils unabhängig voneinander H, Halogen, Hydroxyl-, C₁₋₃-Alkyl, C_{1- 3}-Alkoxy, C₁₋₃-Alkoxy-C₁₋₃-alkoxy, Di-C₁₋₆-al kylamino-C₁₋₆-alkoxy oder Di-C₁₋₆-alkylaminocarbonyl-C₁₋₆-alkoxy sind;
R⁵ H, C₁₋₃-Alkyl oder Aryl-C₁₋₃-alkyl ist;
R⁶ H, C₁₋₃-Alkyl, Halogen-C₁₋₃-alkyl, Di-C₁₋₄-alkylamino-C₁₋₆-alkyl, Mono-C₁₋₄-alkylamino-C₁₋₆-alkyl, Amino-C₁₋₆-alkyl, Hydroxy-C₁₋₃-alkylamino-C₁₋₅-alkyl, Di(hydroxy-C₁₋₃-alkyl)amino-C₁₋₅-alkyl, Hydroxy-C₁₋₅-alkyl, Dihydroxypropyl, Cycloalkyl, Heterocyclyl, Heterocyclyl-C₁₋₄-alkyl oder Heteroaryl-C₁₋₃-alkyl ist, wobei das Heterocyclyl oder das Heteroaryl unsubstituiert oder substituiert mit von 1-2 Substituenten ist, die unabhängig voneinander aus C₁₋₃-Alkyl, C₁₋₅-Alkyl-OC(O)- oder Aryl-C₁₋₃-alkyl ausgewählt sind, wobei das genannte C₁₋₅-Alkyl-OC(O)-an ein N-Ringatom gebunden ist;
oder
R⁵ und R⁶ zusammen mit dem Stickstoff, an den sie gebunden sind, ein 4- bis 7-gliedriges Heterocyclyl bilden, wobei das 4- bis 7-gliedriges Heterocyclyl optional ein zweites Heteroatom als Ringglied ausgewählt aus N und O enthält und wobei das 4- bis 7-gliedriges Heterocyclyl unsubstituiert oder substituiert mit von 1-2 Substituenten ist, die unabhängig voneinander aus C₁₋₃-Alkyl, Hydroxy-C₁₋₃-alkyl, C₁₋₂-Alkoxy-C₂₋₄-alkyl, Di-C₁₋₃-alkylamino, Mono-C₁₋₃-alkylamino, Amino, Di-C₁₋₃-alkylamino-C₁₋₄-alkyl, Mono-C₁₋₃-alkylamino-C₁₋₄-alkyl, Amino-C₁₋₄-alkyl, C₅₋₆-Cycloalkyl, C₁₋₆-Alkyl-OC(O)NH-, Heterocyclylmethyl oder Heteroaryl-carbonyl ausgewählt sind, oder wobei zwei geminale Substituenten auf dem genannten 4-bis 7-gliedrigen Heterocyclyl zusammen ein zweites 5- bis 6-gliedrigen Heterocyclyl bilden, wobei ein Spiro-Radikal entsteht;
mit den Maßgaben, dass:
wenn R¹ NH₂ ist, dann ist A eine Bindung;
wenn A -NH- oder -N-C₁₋₆-Alkyl ist, dann ist R¹ H; und
wenn beide R² und R³ ungleich H sind, dann können beide R² und R³ nicht in ortho-Position zum Rest A angeordnet sein.

9. Verbindung nach Anspruch 8, wobei R⁶ Di-C₁₋₃-alkylamino-C₁₋₄-alkyl, Mono-C₁₋₃-alkylamino-C₁₋₄-alkyl, Amino-C₁₋₄-alkyl, Heterocyclyl oder Heterocyclyl-C₁₋₃-alkyl ist, wobei das Heterocyclyl vorzugsweise ein 4-, 5- oder 6-gliedriges Heterocyclyl ist, das 1 oder 2 Heteroatomen ausgewählt aus O und N enthält, wobei das Heterocyclyl unsubstituiert oder substituiert in einer oder zwei Positionen unabhängig voneinander mit C₁₋₃-Alkyl, C₁₋₅-Alkyl-OC(O)- oder Aryl-C₁₋₃-alkyl sein kann, wobei das genannte C₁₋₅-Alkyl-OC(O)- an ein N-Ringatom gebunden ist.

10. Verbindung nach Anspruch 1, wobei R² in ortho-Position zum Rest A angeordnet ist, R³ in meta-Position zum Rest A angeordnet ist und R⁴ in para-Position zum Rest A angeordnet ist.

11. Verbindung nach Anspruch 1, wobei Ar Hydroxyphenyl, Benzofuranyl, Aminocarbonylphenyl oder Indolyl ist.

12. Verbindung nach Anspruch 11, wobei A -NH- und Ar 2-Benzofuranyl oder 2-Indolyl sind.

13. Verbindung nach Anspruch 1, wobei Ar Azaindolyl ist.

14. Verbindung nach Anspruch 13, wobei Ar 5- oder 6-Azaindol-2-yl ist.

15. Verbindung nach Anspruch 1, ausgewählt aus der Gruppe, bestehend aus:
4-{[6-(1H-Indol-2-yl)pyrazin-2-yl]amino}-3-methoxy-N-methyl-N-(1-methylpyrrolidin-3-yl)benzamid;
4-[5-Amino-6-(1-benzofuran-2-yl)pyrazin-2-yl]-N-[(2S)-2,3-dihydroxypropyl]benzamid;
5-{2-Fluor-4-[(4-methylpiperazin-1-yl)carbonyl]phenyl}-3-(1H-indol-2-yl)pyrazin-2-amin;
N-[2-(Diethylamino)ethyl]-4-[5-(1H-indol-2-yl)pyridin-3-yl]benzamid;
3-(1-Benzofuran-2-yl)-5-{2-fluor-4-[(4-methylpiperazin-1-yl)carbonyl]phenyl}pyrazin-2-amin;
2-(5-{4-[(4-Methylpiperazin-1-yl)carbonyl]phenyl}pyridin-3-yl)-1H-indol;
5-Fluor-2-{6-[4-(morpholin-4-ylcarbonyl)phenyl]pyrazin-2-yl}-1H-indol;
2-{5-[4-(Morpholin-4-ylcarbonyl)phenyl]pyridin-3-yl}-1H-indol;
N-Ethyl-N-(2-hydroxyethyl)-4-[6-(1H-indol-2-yl)pyrazin-2-yl]benzamid;
4-{[6-(1H-Indol-2-yl)pyrazin-2-yl]amino}-3-methoxy-N-methyl-N-(1-methylpyrrolidin-3-yl)benzamid;
N-[2-(Diethylamino)ethyl]-4-{[6-(5-fluoro-1H-indol-2-yl)pyrazin-2-yl]amino}-3-methoxybenzamid;
3-Fluor-4-[6-(6-fluoro-1H-indol-2-yl)pyrazin-2-yl]-N-methyl-N-(1-methylpyrrolidin-3-yl)benzamid;
4-{[6-(5-Fluor-1H-indol-2-yl)pyrazin-2-yl]amino}-3-methoxy-N-methyl-N-(1-methylpyrrolidin-3-yl)benzamid;
6-Methyl-2-{6-[4-(morpholin-4-ylcarbonyl)phenyl]pyrazin-2-yl}-1H-indol;
2-{6-[4-(Morpholin-4-ylcarbonyl)phenyl]pyrazin-2-yl}-1H-indol;
4-[5-Amino-6-(1-benzofuran-2-yl)pyrazin-2-yl]-N-(2,3-dihydroxypropyl)benzamid;
6-(5-Fluor-1H-indol-2-yl)-N-{2-methoxy-4-[(4-methylpiperazin-1-yl)carbonyl]phenyl}pyrazin-2-amin;
3-(1H-Indol-2-yl)-5-[4-(morpholin-4-ylcarbonyl)phenyl]pyrazin-2-amin;
N-[3-(Dimethylamino)propyl]-4-{[6-(1H-indol-2-yl)pyrazin-2-yl]amino}-3-methoxybenzamid;
2-(6-{2-Fluor-4-[(4-methylpiperazin-1-yl)carbonyl]phenyl}yrazin-2-yl)-1H-indol;
tert-Butyl-3-[({4-[6-(1H-indol-2-yl)pyrazin-2-yl]benzoyl}amino)methyl]piperidine-1-carboxylat;
2-{6-[2-Fluor-4-(morpholin-4-ylcarbonyl)phenyl]pyrazin-2-yl}-1H-indol;
3-{[6-(1-Benzofuran-2-yl)pyrazin-2-yl]amino}-4-methoxy-N-methyl-N-(1-methylpyrrolidin-3-yl)benzamid;
5-[2-Fluor-4-(morpholin-4-ylcarbonyl)phenyl]-3-(1H-indol-2-yl)pyrazin-2-amin;
N-[2-(Diethylamino)ethyl]-4-{[6-(iH-indol-2-yl)pyrazin-2-yl]amino}-3-methoxybenzamid;
tert-Butyl-(1-{4-[6-(1H-indol-2-yl)pyrazin-2-yl]benzoyl}pyrrolidin-3-yl)carbamat;
6-(1H-Indol-2-yl)-N-[2-methoxy-4-(morpholin-4-ylcarbonyl)phenyl]pyrazin-2-amin;
5-Fluor-2-(6-{4-[(4-methylpiperazin-1-yl)carbonyl]phenyl}pyrazin-2-yl)-1H-indol;
4-[5-Amino-6-(1-benzofuran-2-yl)pyrazin-2-yl]-N-[1-(hydroxymethyl)-2-methylpropyl]benzamid;
6-(1H-Indol-2-yl)-N-[2-methoxy-4-(morpholin-4-ylcarbonyl)phenyl]pyrazin-2-amin;
tert-Butyl-(1-{4-[5-amino-6-(1-benzofuran-2-yl)pyrazin-2-yl]benzoyl}pyrrolidin-3-yl)carbamat;
3-(1-Benzofuran-2-yl)-5-[4-(morpholin-4-ylcarbonyl)phenyl]pyrazin-2-amin;
3-(4-{4-[6-(1H-Indol-2-yl)pyrazin-2-yl]benzoyl}piperazin-1-yl)-N,N-dimethylpropan-1-amin;
4-{[6-(1-Benzofuran-2-yl)pyrazin-2-yl]amino}-3-methoxy-N-methyl-N-(1-methylpyrrolidin-3-yl)benzamid;
N-[(2S)-2,3-Dihydroxypropyl]-4-[6-(1H-indol-2-yl)pyrazin-2-yl]benzamid;
N-[3-(Dimethylamino)propyl]-4-[6-(1H-indol-2-yl)pyrazin-2-yl]-N-methylbenzamid;
N-(4-{[3-(Dimethylamino)pyrrolidin-1-yl]carbonyl}-2-methoxyphenyl)-6-(1H-indol-2-yl)pyrazin-2-amin;
3-(1-Benzofuran-2-yl)-5-{4-[(4-methylpiperazin-1-yl)carbonyl]phenyl}pyrazin-2-amin;
3-Fluor-5-[6-(6-fluor-1H-indol-2-yl)pyrazin-2-yl]-N-methyl-N-(1-methylpyrrolidin-3-yl)benzamid;
6-(1H-Indol-2-yl)-N-{2-methoxy-4-[(4-methylpiperazin-1-yl)carbonyl]phenyl}pyrazin-2-amin;
4-[5-Amino-6-(1-benzofuran-2-yl)pyrazin-2-yl]-N-{3-[bis(2-hydroxyethyl)amino]propyl}benzamid;
N-(2,3-Dihydroxypropyl)-4-[6-(1H-indol-2-yl)pyrazin-2-yl]benzamid;
2-(4-{3-Fluor-5-[6-(6-fluor-1H-indol-2-yl)pyrazin-2-yl]benzoyl}piperazin-1-yl)-N,N-dimethylethanamin;
4-[5-Amino-6-(1-benzofuran-2-yl)pyrazin-2-yl]-N-ethyl-N-(2-hydroxyethyl)benzamid;
3-Chlor-N-[2-(diethylamino)ethyl]-4-{[6-(5-fluor-1H-indol-2-yl)pyrazin-2-yl]amino}benzamid;
N-[2-Chlor-4-(piperazin-1-ylcarbonyl)phenyl]-6-(5-fluor-1H-indol-2-yl)pyrazin-2-amin;
N-[2-Chlor-5-methoxy-4-(piperazin-1-ylcarbonyl)phenyl]-6-(5-fluoro-1H-indol-2-yl)pyrazin-2-amin;
4-{[6-(5-Chlor-1H-indol-2-yl)pyrazin-2-yl]amino}-3-methoxy-N-methyl-N-(1-methylpyrrolidin-3-yl)benzamid;
N-[2-Chlor-4-({4-[3-(dimethylamino)propyl]piperazin-1-yl}carbonyl)phenyl]-6-(1H-indol-2-yl)pyrazin-2-amin;
N-[2-Chlor-4-(piperazin-1-ylcarbonyl)phenyl]-6-(1H-indol-2-yl)pyrazin-2-amin;
N-[4-({4-[3-(Dimethylamino)propyl]piperazin-1-yl}carbonyl)-2-methylphenyl]-6-(5-fluor-1H-indol-2-yl)pyrazin-2-amin;
N-[3-Chlor-4-(piperazin-1-ylcarbonyl)phenyl]-6-(1H-indol-2-yl)pyrazin-2-amin;
4-{[6-(6-Fluor-1H-indol-2-yl)pyrazin-2-yl]amino}-3-methoxy-N-methyl-N-(1-methylpyrrolidin-3-yl)benzamid;
3-Methoxy-4-{[6-(5-methoxy-1H-indol-2-yl)pyrazin-2-yl]amino}-N-methyl-N-(1-methylpyrrolidin-3-yl)benzamid;
4-{[6-(7-Fluor-1H-indol-2-yl)pyrazin-2-yl]amino}-3-methoxy-N-methyl-N-(1-methylpyrrolidin-3-yl)benzamid;
4-{[6-(4-Fluor-1H-indol-2-yl)pyrazin-2-yl]amino}-3-methoxy-N-methyl-N-(1-methylpyrrolidin-3-yl)benzamid;
2-[6-({4-[(4-Methylpiperazin-1-yl)carbonyl]phenyl}amino)pyrazin-2-yl]-1H-indol-4-ol;
4-[[6-(5-Fluor-1H-indol-2-yl)pyrazin-2-yl](methyl)amino]-N-methyl-N-(1-methylpyrrolidin-3-yl)benzamid;
3-Methoxy-4-{[6-(4-methoxy-1H-indol-2-yl)pyrazin-2-yl]amino}-N-methyl-N-(1-methylpyrrolidin-3-yl)benzamid;
4-{[6-(6-Chlor-1H-indol-2-yl)pyrazin-2-yl]amino}-3-methoxy-N-methyl-N-(1-methylpyrrolidin-3-yl)benzamid;
4-{[6-(5-Cyano-1H-indol-2-yl)pyrazin-2-yl]amino}-3-methoxy-N-methyl-N-(1-methylpyrrolidin-3-yl)benzamid;
3-Methoxy-N-methyl-4-{[6-(5-methyl-1H-indol-2-yl)pyrazin-2-yl]amino}-N-(1-methylpyrrolidin-3-yl)benzamid;
3-Methoxy-N-methyl-4-{[6-(6-methyl-1H-indol-2-yl)pyrazin-2-yl]amino}-N-(1-methylpyrrolidin-3-yl)benzamid;
N-[2-(Diethylamino)ethyl]-2-fluor-4-{[6-(1H-indol-2-yl)pyrazin-2-yl]amino}benzamid;
N-(4-{[(3R)-3-(Dimethylamino)pyrrolidin-1-yl]carbonyl}phenyl)-6-(1H-indol-2-yl)pyrazin-2-amin;
N-(4-{[(3S)-3-(Dimethylamino)pyrrolidin-1-yl]carbonyl}phenyl)-6-(1H-indol-2-yl)pyrazin-2-amin;
N-[4-({4-[3-(Dimethylamino)propyl]piperazin-1-yl}carbonyl)-3-methoxyphenyl]-6-(1H-indol-2-yl)pyrazin-2-amin;
6-(6-Methoxy-1H-indol-2-yl)-N-[2-methoxy-4-(piperazin-1-ylcarbonyl)phenyl]pyrazin-2-amin;
6-(4-Fluor-1H-indol-2-yl)-N-[2-methoxy-4-(piperazin-1-ylcarbonyl)phenyl]pyrazin-2-amin; N-(4-{[3-(Dimethylamino)pyrrolidin-1-yl]carbonyl}-2-methoxyphenyl)-6-(1H-indol-2-yl)pyrazin-2-amin;
tert-Butyl-[1-(4-{[6-(5-fluor-1H-indol-2-yl)pyrazin-2-yl]amino}-3-methoxybenzoyl)azetidin-3-yl]carbamat;
N-[2-(Dimethylamino)ethyl]-4-{[6-(1H-indol-2-yl)pyrazin-2-yl]amino}-3-methoxy-N-methylbenzamid;
N-Azetidin-3-yl-4-{[6-(5-fluor-1H-indol-2-yl)pyrazin-2-yl]amino}-3-methoxybenzamid; N-{4-[(3-Aminoazetidin-1-yl)carbonyl]-2-methoxyphenyl}-6-(5-fluor-1H-indol-2-yl)pyrazin-2-amin;
tert-Butyl-3-[(4-{[6-(5-fluor-1H-indol-2-yl)pyrazin-2-yl]amino}-3-methoxybenzoyl)amino]azetidine-1-carboxylat;
N-(1,2-Diethylpyrazolidin-4-yl)-4-{[6-(1H-indol-2-yl)pyrazin-2-yl]amino}-3-methoxybenzamid;
6-(5-Fluor-1H-indol-2-yl)-N-(2-methoxy-4-{[(3S)-3-methylpiperazin-1-yl]carbonyl}phenyl)pyrazin-2-amin;
6-(5-Fluor-1H-indol-2-yl)-N-(2-methoxy-4-{[(3R)-3-methylpiperazin-1-yl]carbonyl}phenyl)pyrazin-2-amin;
N-(4-{[(3R,5S)-3,5-Dimethylpiperazin-1-yl]carbonyl}-2-methoxyphenyl)-6-(1H-indol-2-yl)pyrazin-2-amin;
N-{4-[(4-Ethylpiperazin-1-yl)carbonyl]-2-methoxyphenyl}-6-(1H-indol-2-yl)pyrazin-2-amin;
N-(4-{[(3S)-3-(Dimethylamino)pyrrolidin-1-yl]carbonyl}-2-methoxyphenyl)-6-(5-nitro-1H-indol-2-yl)pyrazin-2-amin;
6-(1H-Indol-2-yl)-N-{4-[(4-isopropylpiperazin-1-yl)carbonyl]-2-methoxyphenyl}pyrazin-2-amin;
N-(4-{[(3R)-3-(Dimethylamino)pyrrolidin-1-yl]carbonyl}-2-methoxyphenyl)-6-(6-methoxy-1H-indol-2-yl)pyrazin-2-amin;
6-(1H-Indol-2-yl)-N-{2-methoxy-4-[(4-methyl-1,4-diazepan-1-yl)carbonyl]phenyl}pyrazin-2-amin;
N-[4-(2,7-Diazaspiro[4.5]dec-2-ylcarbonyl)-2-methoxyphenyl]-6-(1H-indol-2-yl)pyrazin-2-amin;
6-(1H-Indol-2-yl)-N-(2-methoxy-4-{[(2S)-2-(pyrrolidin-1-ylmethyl)pyrrolidin-1-yl]carbonyl}phenyl)pyrazin-2-amin;
N-(2-Hydroxyethyl)-4-{[6-(1H-indol-2-yl)pyrazin-2-yl]amino}-3-methoxybenzamid;
6-(1H-Indol-2-yl)-N-(2-methoxy-4-{[4-(2-methoxyethyl)piperazin-1-yl]carbonyl}phenyl)pyrazin-2-amin;
2-[4-(4-{[6-(1H-Indol-2-yl)pyrazin-2-yl]amino}-3-methoxybenzoyl)piperazin-1-yl]ethanol;
4-{[6-(1H-Indol-2-yl)pyrazin-2-yl]amino}-3-methoxy-N-piperidin-3-ylbenzamid,
N-(4-{[(3R)-3-Aminopyrrolidin-1-yl]carbonyl}-2-methoxyphenyl)-6-(1H-indol-2-yl)pyrazin-2-amin;
4-{[6-(1H-Indol-2-yl)pyrazin-2-yl]amino}benzamidacetat;
6-(1H-Indol-2-yl)-N-[4-(morpholin-4-ylcarbonyl)phenyl]pyrazin-2-aminacetat;
N-(4-{[(3S)-3-Aminopyrrolidin-1-yl]carbonyl}-2-methoxyphenyl)-6-(1H-indol-2- yl)pyrazin-2-amin;
4-{[6-(1H-Indol-2-yl)pyrazin-2-yl]amino}-3-methoxy-N-[(3S)-pyrrolidin-3-yl]benzamid;
4-{[6-(1H-Indol-2-yl)pyrazin-2-yl]amino}-3-methoxy-N-[(3R)-pyrrolidin-3-yl]benzamid;
N-(4-{[(3R)-3-Aminopyrrolidin-1-yl]carbonyl}-2-methoxyphenyl)-6-(5-fluor-1H-indol-2-yl)pyrazin-2-amin;
2-{[6-(5-Fluor-1H-indol-2-yl)pyrazin-2-yl]amino}-5-(piperazin-1-ylcarbonyl)phenol;
2-[6-({4-[(4-Methylpiperazin-1-yl)carbonyl]phenyl}amino)pyrazin-2-yl]-1H-indol-5-carboxamid;
N-{2-[2-(Dimethylamino)ethoxy]-4-[(4-methylpiperazin-1-yl)carbonyl]phenyl}-6-(5-fluor-1H-indol-2-yl)pyrazin-2-amin;
2-{2-{[6-(5-Fluor-1H-indol-2-yl)pyrazin-2-yl]amino}-5-[(4-methylpiperazin-1-yl)carbonyl]phenoxy}-N,N-dimethylacetamid;
N-{2-(2-Ethoxyethoxy)-4-[(4-methylpiperazin-1-yl)carbonyl]phenyl}-6-(5-fluor-1H-indol-2-yl)pyrazin-2-amin;
N-{4-[(4-Methylpiperazin-1-yl)carbonyl]phenyl}-6-(1H-pyrrolo[2,3-c]pyridin-2-yl)pyrazin-2-amin;
N-(4-{[(3R)-3-(Dimethylamino)pyrrolidin-1-yl]carbonyl}-2-methoxyphenyl)-6-(1H-pyrrolo[3,2-c]pyridin-2-yl)pyrazin-2-amin;
N-{5-[(4-Ethylpiperazin-1-yl)carbonyl]pyridin-2-yl}-6-(1H-indol-2-yl)pyrazin-2-amin;
6-(1H-Indol-2-yl)-N-{5-[(4-methyl-1,4-diazepan-1-yl)carbonyl]pyridin-2-yl}pyrazin-2-amin;
6-(1H-Indol-2-yl)-N-[5-(morpholin-4-ylcarbonyl)pyridin-2-yl]pyrazin-2-amin;
6-(5-Fluor-1H-indol-2-yl)-N-[6-(piperazin-1-ylcarbonyl)pyridin-3-yl]pyrazin-2-amin;
N-{5-[(4-Methyl-1,4-diazepan-1-yl)carbonyl]pyridin-2-yl}-6-(1H-pyrrolo[3,2-c]pyridin-2-yl)pyrazin-2-amin;
N-[5-(5-Fluor-1H-indol-2-yl)pyridin-3-yl]-5-(piperazin-1-ylcarbonyl)pyridin-2-amin;
5-(1H-Indol-2-yl)-N-{4-[(4-methylpiperazin-1-yl)carbonyl]phenyl}pyridin-3-amin;
5-(5-Fluor-1H-indol-2-yl)-N-[4-(piperazin-1-ylcarbonyl)phenyl]pyridin-3-amin;
5-(5-Fluor-1H-indol-2-yl)-N-{4-[(4-methyl-1,4-diazepan-1-yl)carbonyl]phenyl}pyridin-3-amin;
5-(5-Fluor-1H-indol-2-yl)-N-[6-(piperazin-1-ylcarbonyl)pyridin-3-yl]pyridin-3-amin; und
4-[6-({4-[(4-Methylpiperazin-1-yl)carbonyl]phenyl}amino)pyrazin-2-yl]benzamid.

16. Verbindung nach einem der Ansprüche 1-15 zur Verwendung in der Therapie.

17. Verbindung nach einem der Ansprüche 1-15 zur Verwendung bei der Behandlung oder Prophylaxe von Übergewicht, Essstörungen, Kachexie, Diabetes mellitus, Bluthochdruck, koronare Herzkrankheit, Hypercholesterinämie, Dyslipidämie, Hyperlipidämie, Hyperglykämie, Osteoarthritis, Gallensteine, Schlafapnoe, neurodegenerative Erkrankungen, Krebs, entzündliche Erkrankungen und Typ-2-Diabetes.

18. Verwendung einer Verbindung nach einem der Ansprüche 1-15 zur Herstellung eines Arzneimittels zur Verwendung bei der Behandlung oder Prophylaxe einer Störung, die in Zusammenhang mit unerwünschter Aktivität von MNK1 und/oder MNK2 Kinasen steht, wie beispielsweise Übergewicht, Essstörungen, Kachexie, Diabetes mellitus, Bluthochdruck, koronare Herzkrankheit, Hypercholesterinämie, Dyslipidämie, Hyperlipidämie, Hyperglykämie, Osteoarthritis, Gallensteine, Schlafapnoe, neurodegenerative Erkrankungen, Krebs, entzündliche Erkrankungen und Typ-2-Diabetes.

19. Eine pharmazeutische Formulierung, umfassend als Wirkstoff eine Verbindung nach einem der Ansprüche 1-15.

20. Pharmazeutische Formulierung nach Anspruch 19, ferner umfassend ein pharmazeutisch verträgliches Verdünnungsmittel oder Träger.

## Revendications

1. Composé de formule I : 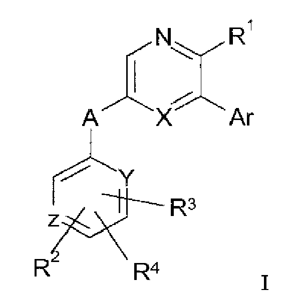 ou sel pharmaceutiquement acceptable, hydrates, isomères géométriques, racémates, tautomères, isomères optiques, N-oxydes et formes de promédicament de celui-ci, où :
X est N ou CH;
Y et Z sont choisis chacun parmi N et CH ;
A est une liaison, -NH- ou -N(C₁₋₆ alkyl)- ;
R¹ est H ou NH₂ ;
Ar est benzofuranyle, indolyle, hydroxyphényle, thiényle, benzothiophényle, pyrrolyle, aminocarbonylphényle ou azaindolyle, chacun desquels est non substitué ou substitué avec 1-2 substituants choisis indépendamment parmi hydroxy, halogène, -CN, -NO₂, C₁₋₆ alkyle, C₁₋₆ alcoxy, acyle, C₁₋₆ alkylsulfonyle, -C(O)NH₂, -NH-C(O)-R⁷ et arylsulfonyle ;
R² et R³ sont chacun, indépendamment, situés à une position ortho ou méta par rapport à A ; et sont chacun, indépendamment, H, halogène, hydroxy, C₁₋₆ alkyle, hydroxy-C₁₋₆ alkyle, carboxy-C₁₋₆ alkyle, C₁₋₆ alcoxy, C₁₋₆ alcoxy-C₁₋₆ alcoxy, di-C₁₋₆ alkylamino-C₁₋₆ alcoxy ou di-C₁₋₆ alkylaminocarbonyl-C₁₋₆ alcoxy ;
R⁴ est situé à une position para ou méta par rapport à A et est -C(O)NR⁵R⁶ ou -S(O)₂NR⁵R⁶ _{;}
R⁵ est H, C₁₋₆ alkyle ou aryl-C₁₋₆ alkyle ;
R⁶ est H, C₁₋₆ alkyle, halo-C₁₋₆ alkyle, di-C₁₋₆ alkylamino-C₁₋₆ alkyle, mono-C₁₋₆ alkylamino-C₁₋₆ alkyle, amino-C₁₋₆ alkyle, hydroxy-C₁₋₆ alkylamino-C₁₋₆ alkyle, di(hydroxy-C₁₋₆ alkyl)amino-C₁₋₆ alkyle, hydroxy-C₁₋₆ alkyle, dihydroxy-C₃₋₆ alkyle, cycloalkyle, hétérocyclyle, hétérocyclyl-C₁₋₆ alkyle ou hétéroaryl-C₁₋₆ alkyle, où l'hétérocyclyle ou hétéroaryle est non substitué ou substitué avec 1-2 substituants choisis indépendamment parmi C₁₋₆ alkyle, C₁₋₆ alkyl-OC(O)- et aryl-C₁₋₆ alkyle, ledit C₁₋₆ alkyl-OC(O)- étant lié à un atome N cyclique ; ou
R⁵ et R⁶ forment avec l'azote auquel ils sont liés un hétérocyclyle à 4 à 7 chaînons, où l'hétérocyclyle à 4 à 7 chaînons inclut éventuellement un second membre du cycle hétéroatome choisi parmi N, S et O, et où l'hétérocyclyle à 4 à 7 chaînons est éventuellement substitué avec 1-2 substituants choisis indépendamment parmi C₁₋₆ alkyle, hydroxy-C₁₋₆ alkyle, C₁₋₂ alcoxy-C₂₋₄ alkyle, oxo, di-C₁₋₆ alkylamino, mono-C₁₋₆ alkylamino, amino, di-C₁₋₆ alkylamino-C₁₋₆ alkyle, mono-C₁₋₆ alkylamino-C₁₋₆ alkyle, amino-C₁₋₆ alkyle, C₃₋₇ cycloalkyle, C₁₋₆ alkyl-OC(O)NH-, hétérocyclyl-C₁₋₄ alkyle et hétéroaryl-carbonyle ; ou bien où deux substituants géminés sur ledit hétérocyclyle à 4 à 7 chaînons forment ensemble un second hétérocyclyle à 5 à 6 chaînons donnant un radical spiro, qui est éventuellement substitué ;
R⁷ est H ou C₁₋₆ alkyle ;
avec les conditions que :
quand R¹ est NH₂, A est une liaison ; et
quand A est -NH- ou -N-C₁₋₆ alkyle, R¹ est H.

2. Composé selon la revendication 1 de formule II 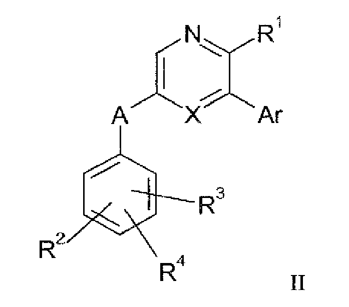 ou sels pharmaceutiquement acceptables, hydrates, isomères géométriques, racémates, tautomères, isomères optiques, N-oxydes et formes de promédicament de celui-ci, où :
X est N ou CH;
A est une liaison ou -NH- ;
R¹ est H ou NH₂ ;
Ar est benzofuranyle, indolyle, hydroxyphényle, thiényle, benzothiophényle ou pyrrolyle, chacun desquels est non substitué ou substitué avec 1-2 substituants choisis indépendamment parmi hydroxy, halogène, C₁₋₆ alkyle, C₁₋₆ alcoxy, acyle, C₁₋₆ alkylsulfonyle et arylsulfonyle ;
R² et R³ sont chacun, indépendamment, situés à une position qui est ortho ou méta par rapport à A ; et sont chacun, indépendamment, H, halogène, C₁₋₆ alkyle, hydroxy-C₁₋₆ alkyle, carboxy-C₁₋₆ alkyle ou C₁₋₆ alcoxy ;
R⁴ est situé à une position qui est para ou méta par rapport à A et est - C(O)NR⁵R⁶ ou -S(O)₂NR⁵R⁶ ;
R⁵ est H, C₁₋₆ alkyle ou aryl-C₁₋₆ alkyle ;
R⁶ est H, C₁₋₆ alkyle, halo-C₁₋₆ alkyle, di-C₁₋₆ alkylamino-C₁₋₆ alkyle, mono-C₁₋₆ alkylamino-C₁₋₆ alkyle, amino-C₁₋₆ alkyle, hydroxy-C₁₋₆ alkylamino-C₁₋₆ alkyle, di(hydroxy-C₁₋₆ alkyl)amino-C₁₋₆ alkyle, hydroxy-C₁₋₆ alkyle, dihydroxy-C₃₋₆ alkyle, cycloalkyle, hétérocyclyle, hétérocyclyl-C₁₋₆ alkyle ou hétéroaryl-C₁₋₆ alkyle, où l'hétérocyclyle ou hétéroaryle est non substitué ou substitué avec 1-2 substituants choisis indépendamment parmi C₁₋₆ alkyle, C₁₋₆ alkyl-OC(O)- et aryl-C₁₋₆ alkyle, ledit C₁₋₆ alkyl-OC(O)- étant lié à un atome N cyclique ; ou
R⁵ et R⁶ forment avec l'azote auquel ils sont liés un hétérocyclyle à 4 à 7 chaînons, où l'hétérocyclyle à 4 à 7 chaînons inclut éventuellement un second membre du cycle hétéroatome choisi parmi N, S et O, et où l'hétérocyclyle à 4 à 7 chaînons est éventuellement substitué avec 1-2 substituants choisis indépendamment parmi C₁₋₆ alkyle, hydroxy-C₁₋₆ alkyle, di-C₁₋₆ alkylamino, mono-C₁₋₆ alkylamino, amino, di-C₁₋₆ alkylamino-C₁₋₆ alkyle, mono-C₁₋₆ alkylamino-C₁₋₆ alkyle, amino-C₁₋₆ alkyle, C₃₋₇ cycloalkyle, C₁₋₆ alkyl-OC(O)NH- et hétéroaryl-carbonyle ;
avec les conditions que :
quand R¹ est NH₂, A est une liaison ; et
quand A est -NH-, R¹ est H.

3. Composé selon la revendication 1 ayant la formule III 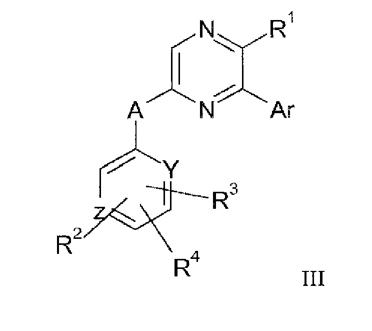 ou sels pharmaceutiquement acceptables, hydrates, isomères géométriques, racémates, tautomères, isomères optiques, N-oxydes et formes de promédicament de celui-ci, où :
Y, Z, A, Ar, R¹, R², R³, R⁴, R⁵ et R⁶ sont définis comme dans la revendication 1
avec les conditions que :
quand R¹ est NH₂, A est une liaison ; et
quand A est -NH- ou -N-(C₁₋₆ alkyl)-, R¹ est H.

4. Composé selon la revendication 1 ayant la formule IV 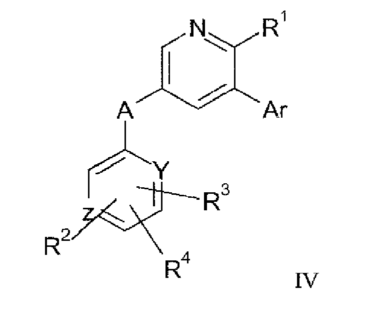 ou sels pharmaceutiquement acceptables, hydrates, isomères géométriques, racémates, tautomères, isomères optiques, N-oxydes et formes de promédicament de celui-ci, où :
Y, Z, A, Ar, R¹, R², R³, R⁴, R⁵ et R⁶ sont définis comme dans la revendication 1
avec les conditions que :
quand R¹ est NH₂, A est une liaison ; et
quand A est -NH- ou -N-(C₁₋₆ alkyl)-, R¹ est H.

5. Composé selon la revendication 1 où Y est N et Z est CH.

6. Composé selon la revendication 1 où Z est N et Y est CH.

7. Composé selon la revendication 1 où Y et Z sont l'un et l'autre CH.

8. Composé selon la revendication 1 où
R² et R³ sont chacun, indépendamment, situés à une position qui est ortho ou méta par rapport à A ; et sont chacun, indépendamment, H, halogène, hydroxy, C₁₋₃ alkyle, C₁₋₃ alcoxy, C₁₋₃ alcoxy-C₁₋₃ alcoxy, di- C₁₋₆ alkylamino-C₁₋₆ alcoxy ou di- C₁₋₆ alkylaminocarbonyl-C₁₋₆ alcoxy ;
R⁵ est H, C₁₋₃ alkyle ou aryl-C₁₋₃ alkyle ;
R⁶ est H, C₁₋₃ alkyle, halo-C₁₋₃ alkyle, di-C₁₋₄ alkylamino-C₁₋₆ alkyle, mono-C₁₋₄ alkylamino-C₁₋₆ alkyle, amino-C₁₋₆ alkyle, hydroxy-C₁₋₃ alkylamino-C₁₋₅ alkyle, di(hydroxy-C₁₋₃ alkyl)amino-C₁₋₅ alkyle, hydroxy-C₁₋₅ alkyle, dihydroxypropyle, cycloalkyle, hétérocyclyle, hétérocyclyl-C₁₋₄ alkyle ou hétéroaryl-C₁₋₃ alkyle, où l'hétérocyclyle ou hétéroaryle est non substitué ou substitué avec 1-2 substituants choisis indépendamment parmi C₁₋₃ alkyle, C₁₋₅ alkyl-OC(O)- et aryl-C₁₋₃ alkyle, ledit C₁₋₅ alkyl-OC(O)- étant lié à un atome N cyclique ;
ou
R⁵ et R⁶ forment avec l'azote auquel ils sont liés un hétérocyclyle à 4 à 7 chaînons, où l'hétérocyclyle à 4 à 7 chaînons inclut éventuellement un second membre du cycle hétéroatome choisi parmi N et O ; et où l'hétérocyclyle à 4 à 7 chaînons est non substitué ou substitué avec 1-2 substituants choisis indépendamment parmi C₁₋₃ alkyle, hydroxy-C₁₋₃ alkyle, C₁₋₂ alcoxy-C₂₋₄ alkyle, di-C₁₋₃ alkylamino, mono-C₁₋₃ alkylamino, amino, di-C₁₋₃ alkylamino-C₁₋₄ alkyle, mono-C₁₋₃ alkylamino-C₁₋₄ alkyle, amino-C₁₋₄ alkyle, C₅₋₆ cycloalkyle, C₁₋₆ alkyl-OC(O)NH-, hétérocyclylméthyle et hétéroaryl-carbonyle, ou bien où deux substituants géminés sur ledit hétérocyclyle à 4 à 7 chaînons forment ensemble un second hétérocyclyle à 5 à 6 chaînons donnant un radical spiro ;
avec les conditions que :
quand R¹ est NH₂, A est une liaison ;
quand A est -NH- ou -N-C₁₋₆ alkyle, R¹ est H ; et
quand R² et R³ sont l'un et l'autre différents de H, R² et R³ ne peuvent pas être situés à une position qui est ortho par rapport à A.

9. Composé selon la revendication 8 où R⁶ est di-C₁₋₃ alkylamino-C₁₋₄ alkyle, mono-C₁₋₃ alkylamino-C₁₋₄ alkyle, amino-C₁₋₄ alkyle, hétérocyclyle ou hétérocyclyl-C₁₋₃ alkyle où l'hétérocyclyle est de préférence à 4, 5 ou 6 chaînons contenant 1 ou 2 hétéroatomes choisis parmi O et N, où l'hétérocyclyle peut être non substitué ou substitué dans une ou deux positions indépendamment avec C₁₋₃ alkyle, C₁₋₅ alkyl-OC(O)- ou aryl-C₁₋₃ alkyle, ledit C₁₋₅ alkyl-OC(O)- étant lié à un atome N cyclique.

10. Composé selon la revendication 1 où R² est situé à une position qui est ortho par rapport à A, R³ est situé à une position qui est méta par rapport à A et R⁴ est situé à une position qui est para par rapport à A.

11. Composé selon la revendication 1 où Ar est hydroxyphényle, benzofuranyle, aminocarbonylphényle ou indolyle.

12. Composé selon la revendication 11 où A est -NH- et Ar est 2-benzofuranyle ou 2-indolyle.

13. Composé selon la revendication 1 où Ar est azaindolyle.

14. Composé selon la revendication 13 où Ar est 5- ou 6-azaindol-2-yle.

15. Composé selon la revendication 1 choisi dans le groupe consistant en :
4-{[6-(1H-indol-2-yl)pyrazin-2-yl]amino}-3-méthoxy-N-méthyl-N-(1-méthyl-pyrrolidin-3-yl)benzamide;
4-[5-amino-6-(1-benzofuran-2-yl)pyrazin-2-yl]-N-[(2S)-2,3-dihydroxypropyl]-benzamide;
5-{2-fluoro-4-[(4-méthylpipérazin-1-yl)carbonyl]phényl}-3-(1H-indol-2-yl)-pyrazin-2-amine;
N-[2-(diéthylamino)éthyl]-4-[5-(1H-indol-2-yl)pyridin-3-yl]benzamide;
3-(1-benzokran-2-yl)-5-{2-fluoro-4-[(4-méthylpipérazin-1-yl)carbonyl]phényl}-pyrazin-2-amine;
2-(5-{4-[(4-méthylpipérazin-1-yl)carbonyl]phényl}pyridin-3-yl)-1H-indole;
5-fluoro-2-{6-[4-(morpholin-4-ylcarbonyl)phényl]pyrazin-2-yl}-1H-indole;
2-{5-[4-(morpholin-4-ylcarbonyl)phényl]pyridin-3-yl}-1H-indole;
N-éthyl-N-(2-hydroxyéthyl)-4-[6-(1H-indol-2-yl)pyrazin-2-yl]benzamide;
4-{[6-(1H-indol-2-yl)pyrazin-2-yl]amino}-3-méthoxy-N-méthyl-N-(1-méthyl-pyrrolidin -3-yl)benzamide;
N-[2-(diéthylamino)éthyl]-4-{[6-(5-fluoro-1H-indol-2-yl)pyrazin-2-yl]amino}-3-méthoxybenzamide;
3-fluoro-4-[6-(6-fluoro-1H-indol-2-yl)pyrazin-2-yl]-N-méthyl-N-(1-méthyl-pyrrolidin-3-yl)benzamide ;
4-{[6-(5-fluoro-1H-indol-2-yl)pyrazin-2-yl]amino}-3-méthoxy-N-méthyl-N-(1-méthylpyrrolidin-3-yl)benzamide;
6-méthyl-2-{6-[4-(morpholin-4-ylcarbonyl)phényl]pyrazin-2-yl}-1H-indole;
2-{6-[4-(morpholin-4-ylcarbonyl)phényl]pyrazin-2-yl}-1H -indole;
4-[5-amino-6-(1-benzofuran-2-yl)pyrazin-2-yl]-N-(2,3-dihydroxypropyl)-benzamide ;
6-(5-fluoro-1H-indol-2-yl)-N-{2-méthoxy-4-[(4-méthylpipérazin-1-yl)carbonyl]-phényl}pyrazin-2-amine;
3-(1H-indol-2-yl)-5-[4-(morpholin-4-ylcarbonyl)phényl]pyrazin-2-amine;
N-[3-(diméthylamino)propyl]-4-{[6-(1H-indol-2-yl)pyrazin-2-yl]amino}-3-méthoxybenzamide;
2-(6-{2-fluoro-4-[(4-méthylpipérazin-1-yl)carbonyl]phényl}pyrazin-2-yl)-1Hindole;
3-[({4-[6-(1H-indol-2-yl)pyrazin-2-yl]benzoyl}amino)méthyl]pipéridine-1-carboxylate de tert-butyle ;
2-{6-[2-fluoro-4-(morpholin-4-ylcarbonyl)phényl]pyrazin-2-yl}-1H-indole;
3-{[6-(1-benzofuran-2-yl)pyrazin-2-yl]amino}-4-méthoxy-N-méthyl-N-(1-méthyl-pyrrolidin-3-yl)benzamide;
5-[2-fluoro-4-(morpholin-4-ylcarbonyl)phényl]-3-(1H-indol-2-yl)pyrazin-2-amine;
N-[2-(diéthylamino)éthyl]-4-{[6-(1H-indol-2-yl)pyrazin-2-yl]amino}-3-méthoxy-benzamide;
(1-{4-[6-(1H-indol-2-yl)pyrazin-2-yl]benzoyl}pyrrolidin-3-yl)carbamate de tert-butyle ;
6-(1H-indol-2-yl)-N-[2-méthoxy-4-(morpholin-4-ylcarbonyl)phényl]pyrazin-2-amine;
5-fluoro-2-(6-{4-[(4-méthylpipérazin-1-yl)carbonyl]phényl}pyrazin-2-yl)-1H-indole;
4-[5-amino-6-(1-benzofuran-2-yl)pyrazin-2-yl]-N-[1-(hydroxyméthyl)-2-méthyl-propyl]benzamide ;
6-(1H-indol-2-yl)-N-[2-méthoxy-4-(morpholin-4-ylcarbonyl)phényl]pyrazin-2-amine;
(1-{4-[5-amino-6-(1-benzofuran-2-yl)pyrazin-2-yl]benzoyl}pyrrolidin-3-yl)-carbamate de tert-butyle ;
3-(1-benzofuran-2-yl)-5-[4-(morpholin-4-ylcarbonyl)phényl]pyrazin-2-amine;
3-(4-{4-[6-(1H-indol-2-yl)pyrazin-2-yl]benzoyl}pipérazin-1-yl)-N,N-diméthyl-propan-1-amine;
4-{[6-(1-benzofuran-2-yl)pyrazin-2-yl]amino}-3-méthoxy-N-méthyl-N-(1-méthyl-pyrrolidin-3-yl)benzamide;
N-[(2S)-2,3-dihydroxypropyl]-4-[6-(1H-indol-2-yl)pyrazin-2-yl]benzamide;
N-[3-(diméthylamino)propyl]-4-[6-(1H-indol-2-yl)pyrazin-2-yl]-N-méthyl-benzamide;
N-(4-{[3-(diméthylamino)pyrrolidin-1-yl]carbonyl}-2-méthoxyphényl)-6-(1H-indol-2-yl)pyrazin-2-amine;
3-(1-benzofuran-2-yl)-5-{4-[(4-méthylpipérazin-1-yl)carbonyl]phényl}pyrazin-2-amine;
3-fluoro-5-[6-(6-fluoro-1H-indol-2-yl)pyrazin-2-yl]-N-méthyl-N-(1-méthyl-pyrrolidin-3-yl)benzamide ;
6-(1H-indol-2-yl)-N-{2-méthoxy-4-[(4-méthylpipérazin-1-yl)carbonyl]phényl}-pyrazin-2-amine ;
4-[5-amino-6-(1-benzofuran-2-yl)pyrazin-2-yl]-N-{3-[bis(2-hydroxyéthyl)amino]-propyl}benzamide ;
N-(2,3-dihydroxypropyl)-4-[6-(1H-indol-2-yl)pyrazin-2-yl]benzamide;
2-(4- 3-fluoro-5-[6-(6-fluoro-1H-indol-2-yl)pyrazin-2-yl]benzoyl}pipérazin-1-yl)-N,N-diméthyléthanamine;
4-[5-amino-6-(1-benzofuran-2-yl)pyrazin 2-yl]-N-éthyl-N-(2-hydroxyéthyl)-benzamide;
3-chloro-N-[2-(diéthylamino)éthyl]-4-{[6-(5-fluoro-1H-indol-2-yl)pyrazin-2-yl]amino}benzamide,
N-[2-chloro-4-(pipérazin-1-ylcarbonyl)phényl]-6-(5-fluoro-1H-indol-2yl)pyrazin-2-amine,
N-[2-chloro-5-méthoxy-4-(pipérazin-1-ylcarbonyl)phényl]-6-(5-fluoro-1H -indol-2-yl)pyrazin-2-amine,
4-{[6-(5-chloro-1H-indol-2-yl)pyrazin-2-yl]amino}-3-méthoxy-N-méthyl-N-(1-méthylpyrrolidin-3-yl)benzamide,
N-[2-chloro-4-({4-[3-(diméthylamino)propyl]pipérazin-1-yl}carbonyl)phényl]-6-(1H-indol-2-yl)pyrazin-2-amine,
N-[2-chloro-4-(pipérazin-1-ylcarbonyl)phényl]-6-(1H-indol-2-yl)pyrazin-2-amine,
N-[4-({4-[3-(diméthylamino)propyl]pipérazin-1-yl}carbonyl)-2-méthylphényl]-6-(5-fluoro-1H-indol-2-yl)pyrazin-2-amine,
N-[3-chloro-4-(pipérazin-1-ylcarbonyl)phényl]-6-(1H-indol-2-yl)pyrazin-2-amine,
4-{[6-(6-fluoro-1H-indol-2-yl)pyrazin-2-yl]amino}-3-méthoxy-N-méthyl-N-(1-méthylpyrrolidin-3-yl)-benzamide,
3-méthoxy-4-{[6-(5-méthoxy-1H-indol-2-yl)pyrazin-2-yl]amino}-N-méthyl-N-(1-méthylpyrrolidin-3-yl)benzamide,
4-{[6-(7-fluoro-1H-indol-2-yl)pyrazin-2-yl]amino}-3-méthoxy-N-méthyl-N-(1-méthylpyrrolidin-3-yl)benzamide,
4-{[6-(4-fluoro-1H-indol-2-yl)pyrazin-2-yl]amino}-3-méthoxy-N-méthyl-N-(1-méthylpyrrolidin-3-yl)benzamide,
2-[6-({4-[(4-méthylpipérazin-1-yl)carbonyl]phényl}amino)pyrazin-2-yl]-1H-indol-4-ol,
4-[[6-(5-fluoro-1H-indol-2-yl)pyrazin-2-yl](méthyl)amino]-N-méthyl-N-(1-méthyl-pyrrolidin-3-yl)benzamide,
3-méthoxy-4-{[6-(4-méthoxy-1H-indol-2-yl)pyrazin-2-yl]amino}-N-méthyl-N-(1-méthylpyrrolidin-3-yl)benzamide,
4-{[6-(6-chloro-1H-indol-2-yl)pyrazin-2-yl]amino}-3-méthoxy-N-méthyl-N-(1-méthylpyrrolidin-3-yl)benzamide,
4-{[6-(5-cyano-1H-indol-2-yl)pyrazin-2-yl]amino}-3-méthoxy-N-méthyl-N-(1-méthylpyrrolidin-3-yl)benzamide,
3-méthoxy-N-méthyl-4-{[6-(5-méthyl-1H -indol-2-yl)pyrazin-2-yl]amino}-N-(I-méthylpyrrolidin-3-yl)benzamide,
3-méthoxy-N-méthyl-4-{[6-(6-méthyl-1H-indol-2-yl)pyrazin-2-yl]amino}-N-(1-méthylpyrrolidin-3-yl)benzamide,
N-[2-(diéthylamino)éthyl]-2-fluoro-4-{[6-(1H-indol-2 yl)pyrazin -2-yl]amino}-benzamide,
N-(4-{[(3R)-3-(diméthylamino)pyrrolidin-1-yl]carbonyl}phényl)-6-(1H-indol-2-yl)pyrazin-2-amine,
N-(4-{[(3S)-3-(diméthylamino)pyrrolidin-1-yl]carbonyl}phényl)-6-(1H-indol-2-yl)pyrazin-2-amine,
N-[4-({4-[3-(diméthylamino)propyl]pipérazin-1-yl}carbonyl)-3-méthoxyphényl]-6-(1H-indol-2-yl)pyrazin-2-amine,
6-(6-méthoxy-1H-indol-2-yl)-N-[2- méthoxy-4-(pipérazin-1-ylcarbonyl)phényl]-pyrazin-2-amine,
6-(4-fluoro-1H-indol-2-yl)-N-[2-méthoxy-4-(pipérazin-1-ylcarbonyl)phényl]-pyrazin-2-amine,
N-(4-{[3-(diméthylamino)pyrrolidin-1-yl]carbonyl}-2-méthoxyphényl)-6-(1H-indol-2-yl)pyrazin-2-amine,
[1-(4-{[6-(5-fluoro-1H-indol-2-yl)pyrazin-2-yl]amino}-3-méthoxybenzoyl)-azétidin-3-yl]carbamate de tert-butyle,
N-[2-(diméthylamino)éthyl]-4-{[6-(1H-indol-2-yl)pyrazin-2-yl]amino}-3-méthoxy-N-méthylbenzamide,
N-azétidin-3-yl-4-{[6-(5-fluoro-1H-indol-2-yl)pyrazin-2-yl]amino}-3-méthoxy-benzamide,
N-{4-[(3-aminoazétidin-1-yl)carbonyl]-2-méthoxyphényl}-6-(5-fluoro-1H-indol-2-yl)pyrazin-2-amine,
3-[(4-{[6-(5-fluoro-1H-indol-2-yl)pyrazin -2-yl]amino}-3-méthoxybenzoyl)-amino]azétidine-1-carboxylate de tert-butyle,
N-(1,2-diéthylpyrazolidin-4-yl)-4-{[6-(1H-indol-2-yl)pyrazin-2-yl]amino}-3-méthoxybenzamide,
6-(5-fluoro-1H-indol-2-yl)-N-(2-méthoxy-4-{[(3S)-3-méthylpipérazin-1-yl]-carbonyl}phényl)pyrazin-2 -amine,
6-(5-fluoro-1H-indol-2-yl)-N-(2-méthoxy-4-{[(3R)-3-méthylpipérazin-1-yl]-carbonyl}phényl)pyrazin-2-amine,
N-(4-{[(3R,5S)-3,5-diméthylpipérazin-1-yl]carbonyl}-2-méthoxyphényl)-6-(1H-indol-2-yl)pyrazin-2-amine,
N-{4-[(4-éthylpipérazin-1-yl)carbonyl]-2-méthoxyphényl}-6-(1H-indol-2-yl)-pyrazin-2-amine,
N-(4-{[(3S)-3-(diméthylamino)pyrrolidin-1-yl]carbonyl}-2-méthoxyphényl)-6-(5-nitro-1H-indol-2-yl)pyrazin-2-amine,
6-(1H-indol-2-yl)-N-{4-[(4-isopropylpipérazin-1-yl)carbonyl]-2-méthoxyphényl}-pyrazin-2-amine,
N-(4-{[(3R)-3-(diméthylamino)pyrrolidin-1-yl]carbonyl}-2-méthoxyphényl)-6-(6-méthoxy-1H-indol-2-yl)pyrazin-2-amine,
6-(1H-indol-2-yl)-N-{2-méthoxy-4-[(4-méthyl-1,4-diazépan-1-yl)carbonyl]-phényl}pyrazin-2-amine,
N-[4-(2,7-diazaspiro[4.5]déc-2-ylcarbonyl)-2-méthoxyphényl]-6-(1H-indol-2-yl)pyrazin-2-amine,
6-(1H-indol-2-yl)-N-(2-méthoxy-4-{[(2S)-2-(pyrrolidin-1-ylméthyl)pyrrolidin-1-yl]carbonyl}phényl)pyrazin-2-amine,
N-(2-hydroxyéthyl)-4-{[6-(1H-indol-2-yl)pyrazin-2-yl]amino}-3-méthoxy-benzamide,
6-(1H-indol-2-yl)-N-(2-méthoxy-4-{[4-(2-méthoxyéthyl)pipérazin-1-yl]carbonyl}-phényl)pyrazin-2 -amine,
2-[4-(4-{[6-(1H-indol-2-yl)pyrazin-2-yl]amino}-3-méthoxybenzoyl)pipérazin-1-yl]éthanol,
4-{[6-(1H-indol-2-yl)pyrazin-2-yl]amino}-3-méthoxy-N-pipéridin-3-ylbenzamide,
N-(4-{[(3R)-3-aminopyrrolidin-1-yl]carbonyl}-2-méthoxyphényl)-6-(1H-indol-2-yl)pyrazin-2-amine,
acétate de 4-{[6-(1H-indol-2-yl)pyrazin-2-yl]amino}benzamide,
acétate de 6-(1H-indol-2-yl)-N-[4-(morpholin-4-ylcarbonyl)phényl]pyrazin-2-amine,
N-(4-{[(3S)-3-aminopyrrolidin-1-yl]carbonyl}-2-méthoxyphényl)-6-(1H-indol-2-yl)pyrazin-2-amine,
4-{[6-(1H-indol-2-yl)pyrazin-2-yl]amino}-3-méthoxy-N-[(3S)-pyrrolidin-3-yl]-benzamide,
4-{[6-(1H-indol-2-yl)pyrazin-2-yl]amino}-3-méthoxy-N-[(3R)-pyrrolidin-3-yl]-benzamide,
N-(4-{[(3R)-3-aminopyrrolidin-1-yl]carbonyl}-2-méthoxyphényl)-6-(5-fluoro-1H-indol-2-yl)pyrazin-2-amine,
2-{[6-(5-fluoro-1H-indol-2-yl)pyrazin-2-yl]amino}-5-(pipérazin-1-ylcarbonyl)-phénol,
2-[6-({4-[(4-méthylpipérazin-1-yl)carbonyl]phényl}amino)pyrazin-2-yl]-1H-indole-5-carboxamide,
N-{2-[2-(diméthylamino)éthoxy]-4-[(4-méthylpipérazin-1-yl)carbonyl]phényl}-6-(5-fluoro-1H-indol-2-yl)pyrazin-2-amine,
2-{2-{[6-(5-fluoro-1H-indol-2-yl)pyrazin-2-yl]amino}-5-[(4-méthylpipérazin-1-yl)carbonyl]phénoxy} -N,N-diméthylacétamide,
N-{2-(2-éthoxyéthoxy)-4-[(4-méthylpipérazin-1-yl)carbonyl]phényl}-6-(5-fluoro-1H-indol-2-yl)pyrazin-2-amine,
N-{4-[(4-méthylpipérazin-1-yl)carbonyl]phényl}-6-(1H-pyrrolo[2,3-c]pyridin-2-yl)pyrazin-2-amine,
N-(4-{[(3R)-3-(diméthylamino)pyrrolidin-1-yl]carbonyl}-2-méthoxyphényl)-6-(1H-pyrrolo [3,2-c]pyridin-2-yl)pyrazin-2-amine,
N-{5-[(4-éthylpipérazin-1-yl)carbonyl]pyridin-2-yl}-6-(1H-indol-2-yl)pyrazin-2-amine,
6-(1H-indol-2-yl)-N-{5-[(4-méthyl-1,4-diazépan-1-yl)carbonyl]pyridin-2yl}-pyrazin-2-amine,
6-(1H-indol-2-yl)-N-[5-(morpholin-4-ylcarbonyl)pyridin-2-yl]pyrazin-2-amine,
6-(5-fluoro-1H-indol-2-yl)-N-[6-(pipérazin-1-ylcarbonyl)pyridin-3-yl]pyrazin-2-amine,
N-{5-[(4-méthyl-1,4-diazépan-1-yl)carbonyl]pyridin-2-yl}-6-(1H-pyrrolo[3,2-c]-pyridin-2-yl)pyrazin-2-amine,
N-[5-(5-fluoro-1H-indol-2-yl)pyridin-3-yl]-5-(pipérazin-1-ylcarbonyl)pyridin-2-amine,
5-(1H-indol-2-yl)-N-{4-[(4-méthylpipérazin-1-yl)carbonyl]phényl}pyridin-3-amine,
5-(5-fluoro-1H-indol-2-yl)-N-[4-(pipérazin-1-ylcarbonyl)phényl]pyridin-3-amine,
5-(5-fluoro-1H-indol-2-yl)-N-{4-[(4-méthyl-1,4-diazépan-1-yl)carbonyl]phényl}-pyridin-3-amine,
5-(5-fluoro-1H-indol-2-yl)-N-[6-(pipérazin-1-ylcarbonyl)pyridin-3-yl]pyridin-3-amine et
4-[6-({4-[(4-méthylpipérazin-1-yl)carbonyl]phényl}amino)pyrazin-2-yl]-benzamide.

16. Composé selon l'une quelconque des revendications 1-15 destiné à être utilisé en thérapie.

17. Composé selon l'une quelconque des revendications 1-15 destiné à être utilisé dans le traitement ou la prophylaxie de l'obésité, des troubles de l'alimentation, de la cachexie, du diabète sucré, de l'hypertension, de la maladie coronarienne, de l'hypercholestérolémie, de la dyslipidémie, de l'hyperlipidémie, de l'hyperglycémie, de l'arthrose, des calculs biliaires, des apnées du sommeil, les troubles neurodégénératifs, du cancer, des affections inflammatoires et du diabète de type 2.

18. Utilisation d'un composé selon l'une quelconque des revendications 1-15 pour la fabrication d'un médicament destiné à être utilisé dans le traitement ou la prophylaxie d'un trouble lié à une activité indésirable des kinases MNK1 et/ou MNK2, comme l'obésité, les troubles de l'alimentation, la cachexie, le diabète sucré, l'hypertension, la maladie coronarienne, l'hypercholestérolémie, la dyslipidémie, l'hyperlipidémie, l'hyperglycémie, l'arthrose, les calculs biliaires, les apnées du sommeil, les troubles neurodégénératifs, le cancer, les affections inflammatoires et le diabète de type 2.

19. Formulation pharmaceutique comprenant comme ingrédient actif un composé selon l'une quelconque des revendications 1-15.

20. Formulation pharmaceutique selon la revendication 19 comprenant en outre un diluant ou vecteur pharmaceutiquement acceptable.
